# EUROPEAN PATENT SPECIFICATION

(11) **EP 1 000 144 B1**
(45) Date of publication and mention of the grant of the patent: **12.12.2007**
(21) Application number: 98925352.1
(22) Date of filing: 02.06.1998
(51) Int. Cl.: C12N 15/12, C07K 14/79, C07K 14/22, A61K 38/17, G01N 33/68, C12Q 1/68

(54) **LACTOFERRIN RECEPTOR GENE OF MORAXELLA**
LACTOFERRINREZEPTORGEN VON MORAXELLA
GENE DU RECEPTEUR DE LA LACTOFERRINE DE MORAXELLA

(30) Priority: 03.06.1997 US 867941; 08.05.1998 US 74658
(43) Date of publication of application: 17.05.2000
(60) Divisional application: 07021387.1
(73) Proprietor: Sanofi Pasteur Limited, Toronto, Ontario M2R 3T4 (CA)
(72) Inventor: LOOSMORE, Sheena, M., Aurora, Ontario L4G 4R4 (CA); DU, Run-Pan, Thornhill, Ontario L4J 7Y8 (CA); WANG, Quijun, Thornhill, Ontario L4J 7Y8 (CA); YANG, Yan-Ping, Toronto, Ontario M2N 6Y6 (CA); KLEIN, Michel, H., Toronto, Ontario M4N 1B9 (CA)
(74) Representative: Bizley, Richard Edward
(86) International application number: PCT/CA1998/000544
(87) International publication number: WO 1998/055606

(56) References cited:
- WO-A-90/12591
- CA-A- 2 162 193
- OGUNNARIWO J A AND SCHRYVERS A B: "Rapid identification of bacterial transferrin and lactoferrin receptor protein genes" JOURNAL OF BACTERIOLOGY, vol. 178, no. 24, December 1996, pages 7326-7328, XP002083300
- SCHRYVERS A B ET AL: "COMPARATIVE ANALYSIS OF THE TRANSFERRIN AND LACTOFERRIN BINDING PROTEINS IN THE FAMILY NEISSERIACEAE" CANADIAN JOURNAL OF MICROBIOLOGY, vol. 35, no. 5, May 1989, pages 409-415, XP002020995 cited in the application
- DU R-P ET AL: "Cloning and expression of the Moraxella catarrhalis lactoferrin receptor genes" INFECTION AND IMMUNITY, vol. 66, no. 8, August 1998, pages 3656-3665, XP002083301

### SEQUENCE LISTING

(1) GENERAL INFORMATION:
   (i) APPLICANT:
      (A) NAME: CONNAUGHT LABORATORIES LIMITED
      (B) STREET: 1755 Steeles Avenue West
      (C) CITY: North York
      (D) STATE: Ontario
      (E) COUNTRY: Canada
      (F) POSTAL CODE (ZIP): M2R 3T4

      (A) NAME: LOOSMORE, Sheena M.
      (B) STREET: 70 Crawford Rose Drive
      (C) CITY: Aurora
      (D) STATE: Ontario
      (E) COUNTRY: Canada
      (F) POSTAL CODE (ZIP): L4G 4R4

      (A) NAME: DU, Run-Pan
      (B) STREET: 299 Chelwood Drive
      (C) CITY: Thornhill
      (D) STATE: Ontario
      (E) COUNTRY: Canada
      (F) POSTAL CODE (ZIP): L4J 7Y8

      (A) NAME: WANG, Quijun
      (B) STREET: 299 Chelwood Drive
      (C) CITY: Thornhill
      (D) STATE: Ontario
      (E) COUNTRY: Canada
      (F) POSTAL CODE (ZIP): L4J 7Y8

      (A) NAME: YANG Yau-Ping
      (B) STREET: Apt. 709, 120 Torresdale Avenue
      (C) CITY: Willowdale
      (D) STATE: Ontario
      (E) COUNTRY: Canada
      (F) POSTAL CODE (ZIP): M2R 3N7

      (A) NAME: KLEIN, Michel H.
      (B) STREET: 16 Munro Boulevard
      (C) CITY: Willowdale
      (D) STATE: Ontario
      (E) COUNTRY: Canada
      (F) POSTAL CODE (ZIP): M2P 1B9
   (ii) TITLE OF INVENTION: LACTOFERRIN RECEPTOR GENES OF MORAXELLA
   (iii) NUMBER OF SEQUENCES: 77
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25 (EPO)
   (v) CURRENT APPLICATION DATA:
      APPLICATION NUMBER: PCT/CA98/00544
(2) INFORMATION FOR SEQ ID NO:1:
   (i) SEQUENCE CHARACTERISTICS:
      (A) LENGTH: 7650 base pairs
      (B) TYPE: nucleic acid
      (C) STRANDEDNESS: single
      (D) TOPOLOGY: linear
   (xi) SEQUENCE DESCRIPTION: SEQ ID NO:1:
   AAGCTTAGCA TGATGGCATC GGCTGATTGT CTTTTTGCCT TGTTGTGTGT TTGTGGGAGT 60
   TGATTGTACT TACCTTAGTG GTGGATGCTT GGGCTGATTT AATAAAGCGG TCTTCACAAC 120
   ACACCAAACG AGATATCACC ATGAGTACTG TCAAAACCCC CCACATTTTC TACCAAAAAC 180
   GCACCCTTAG CCTTGCCATC GCCAGTATTT TTGCTGCCTT GGTGATGACA GGCTGCCGCT 240
   CTGATGACAT CAGCGTCAAT GCACCCAATG TTACCCAACT GCCCCAAGGC ACGGTTTCAC 300
   CAATACCGAA CACAGGTCAT GACAACACCA ATAACACCAA CAATCAGGGC AACAACACGG 360
   ATAACAGCAC CAGCACAACT GACCCAAATG GCGATAACAA CCAACTGACA CAAGCACAAA 420
   AGACCGCCGC TGCCGCAGGG TTTTTTGTGA TGGGTAAAAT TCGTGATACC AGCCCAAAAA 480
   ATGACCCAGA TTATAGCAAT GATTTAGTAC AGCAGTGGCA AGGCAAATTA TATGTTGGTA 540
   TTGATGCCCA TCGCCCAGAT GGCATCGGCA CAGGTAAAAA CTTGCGTCAG CCCATCACCG 600
   CCAATGACAT CAAACCCTTG TATTTTAACA AATTCCCTGC ATTGTCTGAT TTGCATTTAG 660
   ACAGTGAACG CCACCGTTTT GACCCCAAAA AGCTAAACAC CATTAAAGTG TATGGTTATG 720
   GCAACTTAAC AACACCCTCT AAAAACAACA CTTACATCAA TCATCAGCAA GCTGATAATA 780
   AGAAAAATAA CAAGCCTGTT GACCCTTATG AAAATATCCG TTTTGGGTAT CTTGAACTAC 840
   AAGGAAGCAG TCTGACCCAA AAAAATGCCG ATACTCCAAA TGACAAAGAC CGCATTCCCA 900
   AACCCATGCC CATTTTGTTT TATCACGGAG AAAACGCCAG CAGCCAGCTG CCCAGTGCTG 960
   GTAAATTTAA CTACACAGGC AACTGGCTGT ACCTAAGTGA TGTCAAAAAA CGCCCTGCAC 1020
   TTTCAGCATC AGATGATCGA GTGGGGGTCT ATCTCAATGC CAGTGGCAAA TCCAATGAGG 1080
   GCGATGTCGT CAGTGCCGCC CACATTTATC TAAACGGCTT TCAATATAAG CACACGCCTG 1140
   CCACTTATCA GGTGGATTTT GACACAAACT CATTAACAGG CAAGCTGTCT TATTATGACA 1200
   ATCCCAACCA GCAAACTGCC CAAGGCAAAT ACATCAAAAG CCAATTTGAC ACTACCAAAA 1260
   AAGTCAATGA AACCGATGTG TATCAAATTG ATGCCAAAAT CAACGGCAAC CGCTTCGTCG 1320
   GTACGGCCAA ATCTTTGGTT AATGAGAACA CAGAAACCGC ACCTTTTATC AAAGAGCTGT 1380
   TCTCCAAAAA AGCCAATCCC AATAACCCAA ACCCTAATTC AGACACGCTA GAAGGCGGGT 1440
   TTTATGGTGA GTCGGGCGAT GAGCTGGCGG GTAAATTTTT ATCCAATGAC AACGCATCTT 1500
   ATGTGGTCTT TGGTGGTAAA CGAGACAAAA CAGACAAACC TGTCGCCACA AAAACGGTGT 1560
   ATTTTAGTGC AGGCTTTGAA AAACCTAGCA CCAGTTTTGT GGATAATGAA ACGATTGGCA 1620
   GAATTATTAA CAGCAAAAAG TTAAATGATG CGGTGAATGA GAAAATTGAT AATGGTGATA 1680
   TTCCTACCAG TGATGAACGC TATGATGAAT TTCCTTGGGG CGAAAAAAAA GCAGAATTCA 1740
   CCAAAAAAGT CAGCAGCAGC ACCCAAGCCG TGCCAGCTTA TTTTGGGCAA CATGATAAAT 1800
   TTTATTTTAA TGGCAACTAT TATGACCTAT CAGCCAGCAG TGTTGATAAA TTGGCCCCTG 1860
   CCGATGCTGT CAAAGCCAAC CAATCCATTA AAGAAAAATA CCCTAATGCC ACACTAAATA 1920
   AGGACAACCA AGTTACCGCC ATCGTGCTAC AAGAAGCCAA AGATAATAAG CCTTATACCG 1980
   CCATTCGTGC CAAAAGCTAT CAGCACATCA GTTTTGGCGA GACGCTGTAT AACGATGCCA 2040
   ACCAAACCCC AACACGCAGT TATTTTGTGC AAGGCGGTAG GGCAGATACC AGCACCACGC 2100
   TGCCCAAGGC AGGTAAATTC ACTTACAACG GTCTTTGGGC AGGCTATCTT ATCCAAAAAA 2160
   AGGACAAAGG TTATAGCAAT AATGAAGAAA CCATCAAGAA AAAAGGCCAT CAAGATTATC 2220
   TGTTAACCGA AGACTTCACC CCAGAAGATG ATGACGATGA TTTGACCGCA TCTGATGATT 2280
   CACAAGATGA TGATGCACAT GGCGATGATG ATTTGATTGC ATCTGATGAT TCACAAGATG 2340
   ATGACGCAGA TGGCGATGAC GATTCAGATG ATTTGGGTGA TGGTGCAGAT GACGCCGCCG 2400
   CAGGCAAAGT GTATCATGCA GGTAATATTC GCCCTGAATT TGAAAACAAA TACTTGCCCA 2460
   TTAATGAGCC TACTCATGAA AAAACCTTTG CCCTAGATGG TAAAAATAAA GCTAAGTTTG 2520
   ATGTGGATTT TGACACCAAC AGCCTAACTG GTAAATTAAA CGATGAGAGA GGTGATATCG 2580
   TCTTTGATAT CAAAAATGGC AAAATTGATG GCACAGGCTT TACCGCCAAA GCCGATGTGC 2640
   CAAACTATCG TGAAGAAGTG GGTAACAACC AAGGTGGCGG TTTCTTATAC AACATCAAAG 2700
   ATATTGATGT CAAGGGGCAA TTTTTTGGCA CAAATGGCGA AGAGTTGGCA GGGCAGTTAC 2760
   AGTACGACAA AGGCGATGGC ATCAATGACA CCGCCGAAAA AGCAGGGGCT GTCTTTGGGG 2820
   CTGTTAAAGA TAAATAAAGC CCCCTTCATC ATCGTTTAGT CGCTTGACCG ACAGTTGATG 2880
   ACGCCCTTGG CAATGTCTTA AAACAGCACT TTGAAACAGT GCCTTGGGCG AATTCTTGGA 2940
   TAAATGCACC AGATTTGCCT TGGGCTAATA TCTTGATAAA ACATCGCCAT AAAATAGAAA 3000
   ATAAAGTTTA GGATTTTTTT ATGTCAAAAT CTATCACAAA AACACAAACA CCATCAGTCC 3060
   ATACCATGAC CACGCACCGC TTAAACCTTG CCATCAAAGC GGCGTTATTT GGTGTGGCAG 3120
   TTTTACCCCT ATCCGTCTGG GCGCAAGAGA ACACTCAGAC AGATGCCAAC TCTGATGCCA 3180
   AAGACACAAA AACCCCTGTC GTCTATTTAG ATGCCATCAC GGTAACCGCC GCCCCATCTG 3240
   CCCCTGTTTC TCGGTTTGAC ACCGATGTAA CAGGGCTTGG CAAAACGGTC AAAACCGCTG 3300
   ACACGCTGGC AAAAGAACAA GTGCAGGGCA TTCGTGATTT GGTGCGTTAT GAAACTGGGG 3360
   TGAGTGTGGT TGAGCAGGGG CGTGGTGGCA GCAGCGGATT TGCCATTCAT GGCGTGGATA 3420
   AAAACCGAGT GGGCATTACC GTAGATGGCA TTGCCCAAAT TCAATCCTAC AAAGATGAAT 3480
   CCACCAAACG AGCTGGTGCA GGCTCTGGGG CGATGAATGA GATAGAGATT GAAAACATTG 3540
   CCGCCGTTGC CATCAATAAA GGTGGTAATG CCCTAGAAGC AGGCTCTGGT GCGTTGGGCG 3600
   GTTCGGTGGC GTTTCATACC AAAGATGTGA GCGATGTCTT AAAATCTGGT AAAAATCTTG 3660
   GCGCTCAAAG CAAAACCACT TATAACAGCA AAAATGACCA TTTTAGTCAG ACGCTGGCAG 3720
   CGGCAGGTAA AACCGAGCGT GTGGAAGCGA TGGTGCAATA TACCTACCGT AAAGGCAAAG 3780
   AAAACAAAGC ACACAGCGAC CTAAATGGCA TCAACCAAAG CCTATATCGC TTGGGTGCAT 3840
   GGCAACAAAA ATATGATTTA AGAAAGCCCA ATGAACTGTT TGCAGGCACA AGCTACATCA 3900
   CCGAAAGCTG TTTGGCAAGT GATGACCCAA AAAGCTGCGT ACAATACCCT TATGTCTACA 3960
   CCAAAGCCCG ACCAGATGGC ATCGGCAATC GCAATTTTTC TGAGTTAAGC GATGCTGAAA 4020
   AAGCACAATA TTTGGCATCC ACGCACCCCC ATGAGGTTGT CTCTGCCAAA GATTATACAG 4080
   GCATTTATCG GTTGTTACCT GACCCCATGG ACTATCGTTC AGACTCGTAT TTGGCACGCC 4140
   TTAACATCAA AATCACCCCA AATCTGGTCA GTAAACTGTT ATTAGAAGAC ACCAAGCAAA 4200
   CATACAACAT TCGTGATATG CGTCATTGTA GTTACCATGG GGCAAGATTG GGCAATGATG 4260
   GTAAGCCTGC CAATGGTGGC TCCATTGTTC TTTGCGATGA TTATCAAGAG TATCTAAACG 4320
   CCAATGACGC ATCACAAGCA TTATTTAGAC CAGGTGCTAA TGATGCCCCC ATTCCAAAAC 4380
   TGGCTTATGC CAGAAGCAGT GTGTTTAACC AAGAGCATGG CAAAACTCGC TATGGGTTAA 4440
   GTTTTGAGTT TAAGCCTGAC ACGCCATGGT TTAAGCAAGC AAAATTAAAC CTACACCAAC 4500
   AAAATATCCA AATCATTAAC CATGACATTA AAAAATCGTG CAGCCAATAT CCTAAGGTGG 4560
   ATTTAAATTG TGGCATCAGT GAAATTGGGC ATTATGAATA TCAAAATAAT TACCGTTATA 4620
   AAGAAGGGCG TGCCAGCTTG ACAGGCAAAC TTGATTTTAA TTTTGACCTG CTGGGTCAGC 4680
   ACGATTTGAC GGTGTTGGCT GGTGCAGATA AAGTTAAAAG CCAATTTCGT GCCAACAACC 4740
   CCAGACGCAC AATCATTGAC ACCACCCAAG GCGATGCCAT CATTGATGAA AGCACGCTGA 4800
   CAGCACAGGA GCAAGCCAAA TTTAAGCAAT CGGGGGCGGC ATGGATTGTC AAAAATCGCC 4860
   TTGGACGCTT AGAAGAAAAA GACGCCTGTG GCAATGCCAA TGAATGTGAA CGCGCCCCCA 4920
   TTCATGGCAG TAACCAATAT GTGGGCATTA ACAACCTTTA TACACCAAAT GATTATGTGG 4980
   ATTTAAGTTT TGGTGGACGC TTGGATAAAC AACGCATTCA CAGCACCGAT TCAAACATCA 5040
   TCAGCAAAAC TTACACCAAC AAAAGCTATA ATTTTGGAGC GGCGGTTCAT CTGACACCTG 5100
   ATTTTAGCCT GTTGTATAAA ACTGCCAAAG GCTTTCGTAC GCCAAGTTTT TATGAACTGT 5160
   ACAACTATAA CAGCACCGCC GCCCAGCATA AAAATGACCC TGATGTGTCT TTTCCCAAAC 5220
   GAGCGGTTGA TGTCAAACCT GAAACTTCCA ATACCAATGA ATACGGCTTT CGCTATCAGC 5280
   ACCCTTGGGG GGATGTTGAG ATGAGCATGT TCAAAAGCCG TTACAAGGAC ATGTTAGATA 5340
   AAGCCATACC GAACCTAACC AAAGCCCAAC AAGAGTATTG TAAGGCTCAT TTGGATTCCA 5400
   ATGAATGTGT TGGCAATCCG CCCACGCCCA AAACCAGTGA TGAGGTATTT GCCAACTTAT 5460
   ATAATGCCAC CATCAAAGGG GTGAGTGTCA AAGGCAAACT GGATTTGCAT GCCATGACAT 5520
   CAAAACTGCC AGATGGTCTT GAAATGACCT TGGGTTATGG TCATACCAAA TTGGGGAAAT 5580
   TTGATTACAT TGCACCCAAA GATGCCGATG GTTGGTATCA GGCTCGCCCT GCTTTTTGGG 5640
   ATGCCATCAC CCCAGCGCGC TATGTGGTCG GTCTAAACTA TGACCACCCC AGTCAAGTAT 5700
   GGGGCATTGG CACAACTTTA ACGCACAGCA AACAAAAAGA TGAAAATGAG CTAAGTGCCC 5760
   TTAGAATCCG AAATGGCAAA AGAGAAACAC AAACCTTAAC GCACACAATA CCCAAAGCCT 5820
   ATACCTTACT GGACATGACA GGCTATTATA GCCCAACTGA GAGCATCACC GCTCGTCTTG 5880
   GTATCAACAA TGTATTAAAC ACCCGCTACA CCACATGGGA AGCGGCACGC CAACTGCCCA 5940
   GCGAAGCTGC AAGCAGTACC CAATCAACCC GTTACATTGC ACCAGGTCGC AGTTACTTTG 6000
   CCAGTCTTGA AATGAAGTTT TAATATGACC TGTTTACCAA AGACCAACCC TGCTTTAAAA 6060
   GTCAAGCACA GATTTTTAAA GCAGGTGCTG TTATTGCTTT GTGTTGATAC ATTAACAGCA 6120
   CAGGCGTACG CCCACAGCCA TCATACGCCC ATTCATACAC CCACGCATGA GCTGCCATCT 6180
   GCTGATGCTT TATCAGATGA AGGCTTGGGT AAGGATTTGG GCAGTTTGGA CAGTTTGGAT 6240
   AGCCCAGATG GTTTGGGTGA TGGTTTAGGC GATGGTTTGG GTGATGGCTT AAAAAGTGAT 6300
   AAAGCCCCTT TACCCATCAA CGCCTTGACC GCCCATCAGA CCAATGAGAG CCAGCCTGCC 6360
   CCACCGAGCG TAGATGTCAA TTTTTTACTT GCCCAGCCAG AGGCATTTTA TCATGTCTTT 6420
   CATCAAGCGA TTGTGCAAGA TGATGTGGCA ACATTACGCT TGTTATTGCC ATTTTATGAC 6480
   CGCCTGCCTG ATGATTATCA AGATGATGTT TTGTTGTTAT TTGCCCAAAG TAAACTTGCC 6540
   CTAAGTGATG GCAATACCAA ATTGGCATTG AATCTGCTGA CCGATTTGAG TAACAAAGAG 6600
   CCAACACTTA CGGCGGTAAA ATTACAACTT GCTTCCTTGT TGCTGACCAA CAAGCACGAT 6660
   AAACACGCCC AAATGGTGCT AGATGAACTC AAAGATGATG CCCACTTTTT AAAATTAAGC 6720
   AAAAAAGAGC AAAGATGGGT GCTATCGCAA AGTCGCTATT TACATAAAAA ATATAAAATG 6780
   GGCTTGGATT TGGGCATCAA CTATCTGCAT TTGGATAATA TCAACGCCGC CTCCACCATC 6840
   ACCCAGCCCA ATATTAAAAA AGATGCCCCA AAACCTGCTC ATGGGCTTGC CTTATCGCTT 6900
   GGTGTGAATA AATACACGCC GCTTAGTCAT GGCATGAGTA TTTATACAGC CCTAGATGTT 6960
   GATGGTAAAT TTTATGATGA CAAAAGCCAC AATGAACTGG CGGTTTTTGC TCATGCTGGA 7020
   CTAAGAAAAG ATCACCAAAA AGGTTATGTT GATGTCGTAC CTTTTGTTGG GCGTATTTTT 7080
   GCCACCAATC AGCAGCATGG CAGATTATCC CCCAGAAAAG ACAGTCAGGG CGTGGCGTTT 7140
   GGCAGCCATC ATCGGATCAA TGATAAATGG CAAAATGCGT TTTTTGCACG CATGGAAAAA 7200
   GGCAATTATA CCGAGCGTTA TCAAGGTTAT GATGGCAAGC GTTATCATGT GAATGACACC 7260
   ATTTTGTTGC AAGATGGCCC AAATCGTCGT TACTCTTTGG GCGTGGGGTA TCAGCTTAGC 7320
   CATCTGCAAG ATGCAACAAA AAGCAGTCAT GCCACAAAGA TACATTTTGG GGTGTTGCAA 7380
   AGATTGCCAA ATGGTCTGAC CGTGCAAGGT AGAGTGAGTG CTGAGCGTGA GCGTTATCAT    7440
   GGTAAATTAT TGCGTCTGGT TAATCCTGAT GATGTGTATC GCACAGATAA AACCCTAACC    7500
   CTACAAACCT CCATTTGGCA CAAAGACATT CACTGGCTTG GATTAACGCC AAAGCTGACT    7560
   TATCGTTACA GTAAAAATAA CAGTAACTTA CCAGCACTTT ATAGCCATAA CAAACAAAAT    7620
   TTTTATTTGG AGCTTGGTCG GTCGTTTTAA                                     7650
(2) INFORMATION FOR SEQ ID NO:2:
   (i) SEQUENCE CHARACTERISTICS:
      (A) LENGTH: 2694 base pairs
      (B) TYPE: nucleic acid
      (C) STRANDEDNESS: single
      (D) TOPOLOGY: linear
   (xi) SEQUENCE DESCRIPTION: SEQ ID NO:2:
   ATGAGTACTG TCAAAACCCC CCACATTTTC TACCAAAAAC GCACCCTTAG CCTTGCCATC   60
   GCCAGTATTT TTGCTGCCTT GGTGATGACA GGCTGCCGCT CTGATGACAT CAGCGTCAAT  120
   GCACCCAATG TTACCCAACT GCCCCAAGGC ACGGTTTCAC CAATACCGAA CACAGGTCAT  180
   GACAACACCA ATAACACCAA CAATCAGGGC AACAACACGG ATAACAGCAC CAGCACAACT  240
   GACCCAAATG GCGATAACAA CCAACTGACA CAAGCACAAA AGACCGCCGC TGCCGCAGGG  300
   TTTTTTGTGA TGGGTAAAAT TCGTGATACC AGCCCAAAAA ATGACCCAGA TTATAGCAAT  360
   GATTTAGTAC AGCAGTGGCA AGGCAAATTA TATGTTGGTA TTGATGCCCA TCGCCCAGAT  420
   GGCATCGGCA CAGGTAAAAA CTTGCGTCAG CCCATCACCG CCAATGACAT CAAACCCTTG  480
   TATTTTAACA AATTCCCTGC ATTGTCTGAT TTGCATTTAG ACAGTGAACG CCACCGTTTT  540
   GACCCCAAAA AGCTAAACAC CATTAAAGTG TATGGTTATG GCAACTTAAC AACACCCTCT  600
   AAAAACAACA CTTACATCAA TCATCAGCAA GCTGATAATA AGAAAAATAA CAAGCCTGTT  660
   GACCCTTATG AAAATATCCG TTTTGGGTAT CTTGAACTAC AAGGAAGCAG TCTGACCCAA  720
   AAAAATGCCG ATACTCCAAA TGACAAAGAC CGCATTCCCA AACCCATGCC CATTTTGTTT  780
   TATCACGGAG AAAACGCCAG CAGCCAGCTG CCCAGTGCTG GTAAATTTAA CTACACAGGC  840
   AACTGGCTGT ACCTAAGTGA TGTCAAAAAA CGCCCTGCAC TTTCAGCATC AGATGATCGA  900
   GTGGGGGTCT ATCTCAATGC CAGTGGCAAA TCCAATGAGG GCGATGTCGT CAGTGCCGCC  960
   CACATTTATC TAAACGGCTT TCAATATAAG CACACGCCTG CCACTTATCA GGTGGATTTT 1020
   GACACAAACT CATTAACAGG CAAGCTGTCT TATTATGACA ATCCCAACCA GCAAACTGCC 1080
   CAAGGCAAAT ACATCAAAAG CCAATTTGAC ACTACCAAAA AAGTCAATGA AACCGATGTG 1140
   TATCAAATTG ATGCCAAAAT CAACGGCAAC CGCTTCGTCG GTACGGCCAA ATCTTTGGTT 1200
   AATGAGAACA CAGAAACCGC ACCTTTTATC AAAGAGCTGT TCTCCAAAAA AGCCAATCCC 1260
   AATAACCCAA ACCCTAATTC AGACACGCTA GAAGGCGGGT TTTATGGTGA GTCGGGCGAT 1320
   GAGCTGGCGG GTAAATTTTT ATCCAATGAC AACGCATCTT ATGTGGTCTT TGGTGGTAAA 1380
   CGAGACAAAA CAGACAAACC TGTCGCCACA AAAACGGTGT ATTTTAGTGC AGGCTTTGAA 1440
   AAACCTAGCA CCAGTTTTGT GGATAATGAA ACGATTGGCA GAATTATTAA CAGCAAAAAG 1500
   TTAAATGATG CGGTGAATGA GAAAATTGAT AATGGTGATA TTCCTACCAG TGATGAACGC 1560
   TATGATGAAT TTCCTTGGGG CGAAAAAAAA GCAGAATTCA CCAAAAAAGT CAGCAGCAGC 1620
   ACCCAAGCCG TGCCAGCTTA TTTTGGGCAA CATGATAAAT TTTATTTTAA TGGCAACTAT 1680
   TATGACCTAT CAGCCAGCAG TGTTGATAAA TTGGCCCCTG CCGATGCTGT CAAAGCCAAC 1740
   CAATCCATTA AAGAAAAATA CCCTAATGCC ACACTAAATA AGGACAACCA AGTTACCGCC 1800
   ATCGTGCTAC AAGAAGCCAA AGATAATAAG CCTTATACCG CCATTCGTGC CAAAAGCTAT 1860
   CAGCACATCA GTTTTGGCGA GACGCTGTAT AACGATGCCA ACCAAACCCC AACACGCAGT 1920
   TATTTTGTGC AAGGCGGTAG GGCAGATACC AGCACCACGC TGCCCAAGGC AGGTAAATTC 1980
   ACTTACAACG GTCTTTGGGC AGGCTATCTT ATCCAAAAAA AGGACAAAGG TTATAGCAAT 2040
   AATGAAGAAA CCATCAAGAA AAAAGGCCAT CAAGATTATC TGTTAACCGA AGACTTCACC 2100
   CCAGAAGATG ATGACGATGA TTTGACCGCA TCTGATGATT CACAAGATGA TGATGCACAT 2160
   GGCGATGATG ATTTGATTGC ATCTGATGAT TCACAAGATG ATGACGCAGA TGGCGATGAC 2220
   GATTCAGATG ATTTGGGTGA TGGTGCAGAT GACGCCGCCG CAGGCAAAGT GTATCATGCA 2280
   GGTAATATTC GCCCTGAATT TGAAAACAAA TACTTGCCCA TTAATGAGCC TACTCATGAA 2340
   AAAACCTTTG CCCTAGATGG TAAAAATAAA GCTAAGTTTG ATGTGGATTT TGACACCAAC 2400
   AGCCTAACTG GTAAATTAAA CGATGAGAGA GGTGATATCG TCTTTGATAT CAAAAATGGC 2460
   AAAATTGATG GCACAGGCTT TACCGCCAAA GCCGATGTGC CAAACTATCG TGAAGAAGTG 2520
   GGTAACAACC AAGGTGGCGG TTTCTTATAC AACATCAAAG ATATTGATGT CAAGGGGCAA 2580
   TTTTTTGGCA CAAATGGCGA AGAGTTGGCA GGGCAGTTAC AGTACGACAA AGGCGATGGC 2640
   ATCAATGACA CCGCCGAAAA AGCAGGGGCT GTCTTTGGGG CTGTTAAAGA TAAA 2694
(2) INFORMATION FOR SEQ ID NO:3:
   (i) SEQUENCE CHARACTERISTICS:
      (A) LENGTH: 3000 base pairs
      (B) TYPE: nucleic acid
      (C) STRANDEDNESS: single
      (D) TOPOLOGY: linear
   (xi) SEQUENCE DESCRIPTION: SEQ ID NO:3:
   ATGTCAAAAT CTATCACAAA AACACAAACA CCATCAGTCC ATACCATGAC CACGCACCGC 60
   TTAAACCTTG CCATCAAAGC GGCGTTATTT GGTGTGGCAG TTTTACCCCT ATCCGTCTGG 120
   GCGCAAGAGA ACACTCAGAC AGATGCCAAC TCTGATGCCA AAGACACAAA AACCCCTGTC 180
   GTCTATTTAG ATGCCATCAC GGTAACCGCC GCCCCATCTG CCCCTGTTTC TCGGTTTGAC 240
   ACCGATGTAA CAGGGCTTGG CAAAACGGTC AAAACCGCTG ACACGCTGGC AAAAGAACAA 300
   GTGCAGGGCA TTCGTGATTT GGTGCGTTAT GAAACTGGGG TGAGTGTGGT TGAGCAGGGG 360
   CGTGGTGGCA GCAGCGGATT TGCCATTCAT GGCGTGGATA AAAACCGAGT GGGCATTACC 420
   GTAGATGGCA TTGCCCAAAT TCAATCCTAC AAAGATGAAT CCACCAAACG AGCTGGTGCA 480
   GGCTCTGGGG CGATGAATGA GATAGAGATT GAAAACATTG CCGCCGTTGC CATCAATAAA 540
   GGTGGTAATG CCCTAGAAGC AGGCTCTGGT GCGTTGGGCG GTTCGGTGGC GTTTCATACC 600
   AAAGATGTGA GCGATGTCTT AAAATCTGGT AAAAATCTTG GCGCTCAAAG CAAAACCACT 660
   TATAACAGCA AAAATGACCA TTTTAGTCAG ACGCTGGCAG CGGCAGGTAA AACCGAGCGT 720
   GTGGAAGCGA TGGTGCAATA TACCTACCGT AAAGGCAAAG AAAACAAAGC ACACAGCGAC 780
   CTAAATGGCA TCAACCAAAG CCTATATCGC TTGGGTGCAT GGCAACAAAA ATATGATTTA 840
   AGAAAGCCCA ATGAACTGTT TGCAGGCACA AGCTACATCA CCGAAAGCTG TTTGGCAAGT 900
   GATGACCCAA AAAGCTGCGT ACAATACCCT TATGTCTACA CCAAAGCCCG ACCAGATGGC 960
   ATCGGCAATC GCAATTTTTC TGAGTTAAGC GATGCTGAAA AAGCACAATA TTTGGCATCC 1020
   ACGCACCCCC ATGAGGTTGT CTCTGCCAAA GATTATACAG GCATTTATCG GTTGTTACCT 1080
   GACCCCATGG ACTATCGTTC AGACTCGTAT TTGGCACGCC TTAACATCAA AATCACCCCA 1140
   AATCTGGTCA GTAAACTGTT ATTAGAAGAC ACCAAGCAAA CATACAACAT TCGTGATATG 1200
   CGTCATTGTA GTTACCATGG GGCAAGATTG GGCAATGATG GTAAGCCTGC CAATGGTGGC 1260
   TCCATTGTTC TTTGCGATGA TTATCAAGAG TATCTAAACG CCAATGACGC ATCACAAGCA 1320
   TTATTTAGAC CAGGTGCTAA TGATGCCCCC ATTCCAAAAC TGGCTTATGC CAGAAGCAGT 1380
   GTGTTTAACC AAGAGCATGG CAAAACTCGC TATGGGTTAA GTTTTGAGTT TAAGCCTGAC 1440
   ACGCCATGGT TTAAGCAAGC AAAATTAAAC CTACACCAAC AAAATATCCA AATCATTAAC 1500
   CATGACATTA AAAAATCGTG CAGCCAATAT CCTAAGGTGG ATTTAAATTG TGGCATCAGT 1560
   GAAATTGGGC ATTATGAATA TCAAAATAAT TACCGTTATA AAGAAGGGCG TGCCAGCTTG 1620
   ACAGGCAAAC TTGATTTTAA TTTTGACCTG CTGGGTCAGC ACGATTTGAC GGTGTTGGCT 1680
   GGTGCAGATA AAGTTAAAAG CCAATTTCGT GCCAACAACC CCAGACGCAC AATCATTGAC 1740
   ACCACCCAAG GCGATGCCAT CATTGATGAA AGCACGCTGA CAGCACAGGA GCAAGCCAAA 1800
   TTTAAGCAAT CGGGGGCGGC ATGGATTGTC AAAAATCGCC TTGGACGCTT AGAAGAAAAA 1860
   GACGCCTGTG GCAATGCCAA TGAATGTGAA CGCGCCCCCA TTCATGGCAG TAACCAATAT 1920
   GTGGGCATTA ACAACCTTTA TACACCAAAT GATTATGTGG ATTTAAGTTT TGGTGGACGC 1980
   TTGGATAAAC AACGCATTCA CAGCACCGAT TCAAACATCA TCAGCAAAAC TTACACCAAC 2040
   AAAAGCTATA ATTTTGGAGC GGCGGTTCAT CTGACACCTG ATTTTAGCCT GTTGTATAAA 2100
   ACTGCCAAAG GCTTTCGTAC GCCAAGTTTT TATGAACTGT ACAACTATAA CAGCACCGCC 2160
   GCCCAGCATA AAAATGACCC TGATGTGTCT TTTCCCAAAC GAGCGGTTGA TGTCAAACCT 2220
   GAAACTTCCA ATACCAATGA ATACGGCTTT CGCTATCAGC ACCCTTGGGG GGATGTTGAG 2280
   ATGAGCATGT TCAAAAGCCG TTACAAGGAC ATGTTAGATA AAGCCATACC GAACCTAACC 2340
   AAAGCCCAAC AAGAGTATTG TAAGGCTCAT TTGGATTCCA ATGAATGTGT TGGCAATCCG 2400
   CCCACGCCCA AAACCAGTGA TGAGGTATTT GCCAACTTAT ATAATGCCAC CATCAAAGGG 2460
   GTGAGTGTCA AAGGCAAACT GGATTTGCAT GCCATGACAT CAAAACTGCC AGATGGTCTT 2520
   GAAATGACCT TGGGTTATGG TCATACCAAA TTGGGGAAAT TTGATTACAT TGCACCCAAA 2580
   GATGCCGATG GTTGGTATCA GGCTCGCCCT GCTTTTTGGG ATGCCATCAC CCCAGCGCGC 2640
   TATGTGGTCG GTCTAAACTA TGACCACCCC AGTCAAGTAT GGGGCATTGG CACAACTTTA 2700
   ACGCACAGCA AACAAAAAGA TGAAAATGAG CTAAGTGCCC TTAGAATCCG AAATGGCAAA 2760
   AGAGAAACAC AAACCTTAAC GCACACAATA CCCAAAGCCT ATACCTTACT GGACATGACA 2820
   GGCTATTATA GCCCAACTGA GAGCATCACC GCTCGTCTTG GTATCAACAA TGTATTAAAC 2880
   ACCCGCTACA CCACATGGGA AGCGGCACGC CAACTGCCCA GCGAAGCTGC AAGCAGTACC 2940
   CAATCAACCC GTTACATTGC ACCAGGTCGC AGTTACTTTG CCAGTCTTGA AATGAAGTTT 3000
(2) INFORMATION FOR SEQ ID NO:4:
   (i) SEQUENCE CHARACTERISTICS:
      (A) LENGTH: 2955 base pairs
      (B) TYPE: nucleic acid
      (C) STRANDEDNESS: single
      (D) TOPOLOGY: linear
   (xi) SEQUENCE DESCRIPTION: SEQ ID NO:4:
   ATGACCACGC ACCGCTTAAA CCTTGCCATC AAAGCGGCGT TATTTGGTGT GGCAGTTTTA 60
   CCCCTATCCG TCTGGGCGCA AGAGAACACT CAGACAGATG CCAACTCTGA TGCCAAAGAC 120
   ACAAAAACCC CTGTCGTCTA TTTAGATGCC ATCACGGTAA CCGCCGCCCC ATCTGCCCCT 180
   GTTTCTCGGT TTGACACCGA TGTAACAGGG CTTGGCAAAA CGGTCAAAAC CGCTGACACG 240
   CTGGCAAAAG AACAAGTGCA GGGCATTCGT GATTTGGTGC GTTATGAAAC TGGGGTGAGT 300
   GTGGTTGAGC AGGGGCGTGG TGGCAGCAGC GGATTTGCCA TTCATGGCGT GGATAAAAAC 360
   CGAGTGGGCA TTACCGTAGA TGGCATTGCC CAAATTCAAT CCTACAAAGA TGAATCCACC 420
   AAACGAGCTG GTGCAGGCTC TGGGGCGATG AATGAGATAG AGATTGAAAA CATTGCCGCC 480
   GTTGCCATCA ATAAAGGTGG TAATGCCCTA GAAGCAGGCT CTGGTGCGTT GGGCGGTTCG 540
   GTGGCGTTTC ATACCAAAGA TGTGAGCGAT GTCTTAAAAT CTGGTAAAAA TCTTGGCGCT    600
   CAAAGCAAAA CCACTTATAA CAGCAAAAAT GACCATTTTA GTCAGACGCT GGCAGCGGCA    660
   GGTAAAACCG AGCGTGTGGA AGCGATGGTG CAATATACCT ACCGTAAAGG CAAAGAAAAC    720
   AAAGCACACA GCGACCTAAA TGGCATCAAC CAAAGCCTAT ATCGCTTGGG TGCATGGCAA    780
   CAAAAATATG ATTTAAGAAA GCCCAATGAA CTGTTTGCAG GCACAAGCTA CATCACCGAA    840
   AGCTGTTTGG CAAGTGATGA CCCAAAAAGC TGCGTACAAT ACCCTTATGT CTACACCAAA    900
   GCCCGACCAG ATGGCATCGG CAATCGCAAT TTTTCTGAGT TAAGCGATGC TGAAAAAGCA    960
   CAATATTTGG CATCCACGCA CCCCCATGAG GTTGTCTCTG CCAAAGATTA TACAGGCATT   1020
   TATCGGTTGT TACCTGACCC CATGGACTAT CGTTCAGACT CGTATTTGGC ACGCCTTAAC   1080
   ATCAAAATCA CCCCAAATCT GGTCAGTAAA CTGTTATTAG AAGACACCAA GCAAACATAC   1140
   AACATTCGTG ATATGCGTCA TTGTAGTTAC CATGGGGCAA GATTGGGCAA TGATGGTAAG   1200
   CCTGCCAATG GTGGCTCCAT TGTTCTTTGC GATGATTATC AAGAGTATCT AAACGCCAAT   1260
   GACGCATCAC AAGCATTATT TAGACCAGGT GCTAATGATG CCCCCATTCC AAAACTGGCT   1320
   TATGCCAGAA GCAGTGTGTT TAACCAAGAG CATGGCAAAA CTCGCTATGG GTTAAGTTTT   1380
   GAGTTTAAGC CTGACACGCC ATGGTTTAAG CAAGCAAAAT TAAACCTACA CCAACAAAAT   1440
   ATCCAAATCA TTAACCATGA CATTAAAAAA TCGTGCAGCC AATATCCTAA GGTGGATTTA   1500
   AATTGTGGCA TCAGTGAAAT TGGGCATTAT GAATATCAAA ATAATTACCG TTATAAAGAA   1560
   GGGCGTGCCA GCTTGACAGG CAAACTTGAT TTTAATTTTG ACCTGCTGGG TCAGCACGAT   1620
   TTGACGGTGT TGGCTGGTGC AGATAAAGTT AAAAGCCAAT TTCGTGCCAA CAACCCCAGA   1680
   CGCACAATCA TTGACACCAC CCAAGGCGAT GCCATCATTG ATGAAAGCAC GCTGACAGCA   1740
   CAGGAGCAAG CCAAATTTAA GCAATCGGGG GCGGCATGGA TTGTCAAAAA TCGCCTTGGA   1800
   CGCTTAGAAG AAAAAGACGC CTGTGGCAAT GCCAATGAAT GTGAACGCGC CCCCATTCAT   1860
   GGCAGTAACC AATATGTGGG CATTAACAAC CTTTATACAC CAAATGATTA TGTGGATTTA   1920
   AGTTTTGGTG GACGCTTGGA TAAACAACGC ATTCACAGCA CCGATTCAAA CATCATCAGC   1980
   AAAACTTACA CCAACAAAAG CTATAATTTT GGAGCGGCGG TTCATCTGAC ACCTGATTTT   2040
   AGCCTGTTGT ATAAAACTGC CAAAGGCTTT CGTACGCCAA GTTTTTATGA ACTGTACAAC   2100
   TATAACAGCA CCGCCGCCCA GCATAAAAAT GACCCTGATG TGTCTTTTCC CAAACGAGCG   2160
   GTTGATGTCA AACCTGAAAC TTCCAATACC AATGAATACG GCTTTCGCTA TCAGCACCCT   2220
   TGGGGGGATG TTGAGATGAG CATGTTCAAA AGCCGTTACA AGGACATGTT AGATAAAGCC   2280
   ATACCGAACC TAACCAAAGC CCAACAAGAG TATTGTAAGG CTCATTTGGA TTCCAATGAA   2340
   TGTGTTGGCA ATCCGCCCAC GCCCAAAACC AGTGATGAGG TATTTGCCAA CTTATATAAT   2400
   GCCACCATCA AAGGGGTGAG TGTCAAAGGC AAACTGGATT TGCATGCCAT GACATCAAAA   2460
   CTGCCAGATG GTCTTGAAAT GACCTTGGGT TATGGTCATA CCAAATTGGG GAAATTTGAT 2520
   TACATTGCAC CCAAAGATGC CGATGGTTGG TATCAGGCTC GCCCTGCTTT TTGGGATGCC 2580
   ATCACCCCAG CGCGCTATGT GGTCGGTCTA AACTATGACC ACCCCAGTCA AGTATGGGGC 2640
   ATTGGCACAA CTTTAACGCA CAGCAAACAA AAAGATGAAA ATGAGCTAAG TGCCCTTAGA 2700
   ATCCGAAATG GCAAAAGAGA AACACAAACC TTAACGCACA CAATACCCAA AGCCTATACC 2760
   TTACTGGACA TGACAGGCTA TTATAGCCCA ACTGAGAGCA TCACCGCTCG TCTTGGTATC 2820
   AACAATGTAT TAAACACCCG CTACACCACA TGGGAAGCGG CACGCCAACT GCCCAGCGAA 2880
   GCTGCAAGCA GTACCCAATC AACCCGTTAC ATTGCACCAG GTCGCAGTTA CTTTGCCAGT 2940
   CTTGAAATGA AGTTT 2955
(2) INFORMATION FOR SEQ ID NO:5:
   (i) SEQUENCE CHARACTERISTICS:
      (A) LENGTH: 1623 base pairs
      (B) TYPE: nucleic acid
      (C) STRANDEDNESS: single
      (D) TOPOLOGY: linear
   (xi) SEQUENCE DESCRIPTION: SEQ ID NO:5:
   ATGACCTGTT TACCAAAGAC CAACCCTGCT TTAAAAGTCA AGCACAGATT TTTAAAGCAG 60
   GTGCTGTTAT TGCTTTGTGT TGATACATTA ACAGCACAGG CGTACGCCCA CAGCCATCAT 120
   ACGCCCATTC ATACACCCAC GCATGAGCTG CCATCTGCTG ATGCTTTATC AGATGAAGGC 180
   TTGGGTAAGG ATTTGGGCAG TTTGGACAGT TTGGATAGCC CAGATGGTTT GGGTGATGGT 240
   TTAGGCGATG GTTTGGGTGA TGGCTTAAAA AGTGATAAAG CCCCTTTACC CATCAACGCC 300
   TTGACCGCCC ATCAGACCAA TGAGAGCCAG CCTGCCCCAC CGAGCGTAGA TGTCAATTTT 360
   TTACTTGCCC AGCCAGAGGC ATTTTATCAT GTCTTTCATC AAGCGATTGT GCAAGATGAT 420
   GTGGCAACAT TACGCTTGTT ATTGCCATTT TATGACCGCC TGCCTGATGA TTATCAAGAT 480
   GATGTTTTGT TGTTATTTGC CCAAAGTAAA CTTGCCCTAA GTGATGGCAA TACCAAATTG 540
   GCATTGAATC TGCTGACCGA TTTGAGTAAC AAAGAGCCAA CACTTACGGC GGTAAAATTA 600
   CAACTTGCTT CCTTGTTGCT GACCAACAAG CACGATAAAC ACGCCCAAAT GGTGCTAGAT 660
   GAACTCAAAG ATGATGCCCA CTTTTTAAAA TTAAGCAAAA AAGAGCAAAG ATGGGTGCTA 720
   TCGCAAAGTC GCTATTTACA TAAAAAATAT AAAATGGGCT TGGATTTGGG CATCAACTAT 780
   CTGCATTTGG ATAATATCAA CGCCGCCTCC ACCATCACCC AGCCCAATAT TAAAAAAGAT 840
   GCCCCAAAAC CTGCTCATGG GCTTGCCTTA TCGCTTGGTG TGAATAAATA CACGCCGCTT 900
   AGTCATGGCA TGAGTATTTA TACAGCCCTA GATGTTGATG GTAAATTTTA TGATGACAAA 960
   AGCCACAATG AACTGGCGGT TTTTGCTCAT GCTGGACTAA GAAAAGATCA CCAAAAAGGT     1020
   TATGTTGATG TCGTACCTTT TGTTGGGCGT ATTTTTGCCA CCAATCAGCA GCATGGCAGA     1080
   TTATCCCCCA GAAAAGACAG TCAGGGCGTG GCGTTTGGCA GCCATCATCG GATCAATGAT     1140
   AAATGGCAAA ATGCGTTTTT TGCACGCATG GAAAAAGGCA ATTATACCGA GCGTTATCAA     1200
   GGTTATGATG GCAAGCGTTA TCATGTGAAT GACACCATTT TGTTGCAAGA TGGCCCAAAT     1260
   CGTCGTTACT CTTTGGGCGT GGGGTATCAG CTTAGCCATC TGCAAGATGC AACAAAAAGC     1320
   AGTCATGCCA CAAAGATACA TTTTGGGGTG TTGCAAAGAT TGCCAAATGG TCTGACCGTG     1380
   CAAGGTAGAG TGAGTGCTGA GCGTGAGCGT TATCATGGTA AATTATTGCG TCTGGTTAAT     1440
   CCTGATGATG TGTATCGCAC AGATAAAACC CTAACCCTAC AAACCTCCAT TTGGCACAAA     1500
   GACATTCACT GGCTTGGATT AACGCCAAAG CTGACTTATC GTTACAGTAA AAATAACAGT     1560
   AACTTACCAG CACTTTATAG CCATAACAAA CAAAATTTTT ATTTGGAGCT TGGTCGGTCG     1620
   TTT                                                                  1623
(2) INFORMATION FOR SEQ ID NO:6:
   (i) SEQUENCE CHARACTERISTICS:
      (A) LENGTH: 7641 base pairs
      (B) TYPE: nucleic acid
      (C) STRANDEDNESS: single
      (D) TOPOLOGY: linear
   (xi) SEQUENCE DESCRIPTION: SEQ ID NO:6:
   AAGCTTAGCA TGATGGCATC GGCTGATTGT CTTTTTGCCT TGTTGTGTGT TTGTGGGAGT 60
   TGATTGTACT TACCTTAGTG GTGGATGCTT GGGCTGATTT AATTAAATTT AATCAAAGCG 120
   GTCTTCACAA CACACCAAAC GAGATATCAC CATGAGTACT GTCAAAACCC CCCATATTTT 180
   CTACCAAAAA CGCACCCTTA GCCTTGCCAT CGCCAGTATT TTTGCTGCCT TGGTGATGAC 240
   AGGCTGCCGC TCTGATGACA TCAGCGTCAA TGCACCCAAT GTTACCCAGC TGCCCCAAGG 300
   CACGGTTTCA CCAACGCCGA ACACAGGTCA TGACAACGCC AATAACACCA ACAATCAGGG 360
   CAACAACACG GATAACAGCA CCAGCACAAC TGACCCAAAT GGCGATAACA ACCAACTGAC 420
   ACAAGCGCAA AAAACTGCCG CCGCCGCAGG GTTTTTTGTG ATGGGTAAAA TTCGTGATAC 480
   CAGCGAAAAA AATGACCCAG ATTATAGTGA TGATTTAAAA CAGCAGTGGC TGGGCAAATT 540
   ATATGTTGGT ATTGATGCCC ATCGCCCAGA TGGCATCGGA AAAGGTAAAA ACTTGCGTCA 600
   GCCCATCACC GCCAATGACA TCAAACCCTT GTATTTTAAC AAATTCCCTG CATTGTCTGA 660
   TTTGCACTTA GACAGTGAAC GCCATCGTTT TGACCCCCAA AAGATAAACA CCATTAAAGT 720
   GTATGGTTAT GGTAACTTAA CAACACCATC CAACAACAAC ACTCACATCA ATCATCAGCA 780
   AGCTGATAAT AAGAAAAATA ACAAGCCTGT TGACCCTTAT GAAAATATCC GTTTTGGGTA 840
   TCTTGAACTA CAAGGAAGCA GCCTGACCCA AAAAAATGCC GATAATCAAA ATGAGCAAGA 900
   CCGCATTCCC AAACCCATGC CCATTTTGTT TTATCATGGA GAAAACGCCA GCAGCCAGCT 960
   GCCCAGCGCT GGTAAATTTA ACTACACAGG CAACTGGCTG TACCTAAGTG ATGTCAAAAA 1020
   ACGCCCTGCC CTTTCAGCAT CAGATGAGCG AGTGGGGGTC TATCTCAATG CCAGTGGCAA 1080
   AGCCAACGAG GGCGATGTCG TCAGTGCCGC CCACATTTAT CTAAACGGCT TTCAATATAA 1140
   GCACACGCCT GCCACTTATC AGGTGGATTT TGACACAAAC TCATTAACAG GCAAGCTGTC 1200
   CTATTATGAC AATCCCAATC AGCAAAATAA TAAAGGCGAA TATCTCAAAA GCCAATTTGA 1260
   CACTACCAAA AAAGTCAATG AAACCGATGT GTATCAAATT GATGCCAAAA TCAACGGTAA 1320
   CCGCTTTGTC GGTACGGCCA AATCTTTGGT TAATGAGAAA ACACAAACCG CACCTTTTAT 1380
   CAAAGAGCTG TTCTCCAAAA AAGCCAACCC CAATAACCCA AACCCTAATT CAGACACGCT 1440
   AGAAGGCGGA TTTTATGGTG AGTCGGGCGA TGAGCTGGCG GGTAAATTTT TATCCAATGA 1500
   CAACGCATCT TATGTGGTCT TTGGTGGCAA ACGAGACAAA ACGACTAAAC CTGTCGCCAC 1560
   AAAAACGGTG TATTTTAGTG CAGGCTTTGA AAAACCCAGC ACCAGTTTTG TGGATAATGA 1620
   AACGATTGGT GGAATTATTG ACCGTAAAGG GTTAAATAAT CACATTAATG AAGATGAAAT 1680
   TATTCCCAGT GATGATAGTT ATTATGGATA TACTTGGGGC AAGCCAGAGA AGCAGTTCAC 1740
   CAAAAAAGTC AGCAGCAGCA CCCAAGTCGT GCCAGCTTAT TTTGGGCAAC ATGATAAATT 1800
   TTATTTTAAT GGCAACTATT ATGACCTATC AGCCAGTCGT GTTGATAAAT TAGCCCCTGC 1860
   CGATGCTGTC AAAGCCAACC AATCCATTAA AGAAAAATAC CCTAATGCCA CACTAAATAA 1920
   GGACAACCAA GTTACCGCCA TCGTGCTACA AGAAGCCAAA GATAATAAGC CTTATACCGC 1980
   CATTCGTGCC AAAAGCTATC AGCACATCAG TTTTGGCGAG ACGCTGTATA ACGATGCCAA 2040
   CCAAACCCCA ACACGCAGTT ATTTTGTGCA AGGCGGTAGG GCAGATACCA GCACAACTTT 2100
   GCCCCAGGCA GGTAAATTCA CTTACAACGG TCTTTGGGCA GGCTACCTGA CCCAAAAAAA 2160
   GGACAAAGGT TATAGCGATA ATGCAGAAAC CATCAAGGAA AAAGGTCATC CAGGTTATCT 2220
   GTTAACCGAA AACTTCACCC CAGAAGATGA TGACGATGAT TTGACCGCAT CTGATGATTC 2280
   ACAAGATGAT AATACACATG GCGATGATGA TTTGATTGCA TCTGATGATT CACAAGATGA 2340
   TGACGCAGAT GGAGATGACG ATTCAGATGA TTTGGGTGAT GGTGCAGATG ATGACGCCGC 2400
   AGGCAAAGTG TATCATGCAG GTAATATTCG CCCTGAATTT GAAAACAAAT ACTTGCCCAT 2460
   TAATGAGCCT ACTCATGAAA AAACCTTTGC CCTAGATGGT AAAAATAAAG CTAAGTTTGA 2520
   AGTGGATTTT AACACCAACA GCCTAACTGG TAAATTAAAC GATGAGAGAG GTGATATCGT 2580
   CTTTGATATC AAAAATGGCA AAATTGATGG CACAGGATTT ACCGCCAAAG CCGATGTGCC 2640
   AAACTATCGT GAAGAAGTGG GTAACAACCA AGGTGGCGGT TTCTTATACA ACATCAAAGA 2700
   TATTGATGTT AAGGGGCAAT TTTTTGGCAC AAATGGCGAA GAGTTGGCAG GACAGTTACA 2760
   TCATGACAAA GGCGATGGCA TCAATGACAC CGCCGAAAAA GCAGGGGCTG TCTTTGGGGC 2820
   TGTTAAAGAT AAATAAAGCC CCCCTTCATC ATCGTTTAGT CGCTTGACCG ACAGTTGATG 2880
   ACGCCCTTGG CAATGTCTTA AAACAGCACT TTGAAACAGT GCCTTGGGCG AATTCTTGGA 2940
   TAAATGCACC AGATTTGCCT TGGGCTAATA TCTTGATAAA ACATCGCCAT AAAATAGAAA 3000
   ATAAAGTTTA GGATTTTTTT ATGTCAAAAT CTATCACAAA AACACAAACA CCATCAGTCC 3060
   ATACCATGAC CACGCACCGC TTAAACCTTG CCATCAAAGC GGCGTTATTT GGTGTGGCAG 3120
   TTTTACCCCT ATCCGTCTGG GCGCAAGAGA ACACTCAGAC AGATGCCAAC TCTGATGCCA 3180
   AAGACACAAA AACCCCTGTC GTCTATTTAG ATGCCATCAC GGTAACCGCC GCCCCATCTG 3240
   CCCCTGTTTC TCGGTTTGAC ACCGATGTAA CAGGGCTTGG CAAAACCGTC AAAACCGCTG 3300
   ACACGCTGGC AAAAGAACAA GTACAGGGCA TTCGTGATTT GGTGCGTTAT GAAACTGGGG 3360
   TGAGTGTGGT TGAGCAGGGG CGTGGTGGCA GCAGCGGATT TGCCATTCAT GGCGTGGATA 3420
   AAAACCGAGT GGGCATTACC GTAGATGGCA TTGCCCAAAT TCAATCCTAC AAAGACGAAT 3480
   CCACTAAGCG AGCTGGGGCA GGCTCTGGGG CGATGAACGA GATAGAGATT GAAAACATTG 3540
   CCGCCGTTGC CATCAATAAA GGCGGTAATG CCTTAGAAGC AGGCTCTGGT GCGTTGGGTG 3600
   GTTCGGTGGC GTTTCATACC AAAGATGTGA GCGATGTCTT AAAATCTGGT AACAATCTTG 3660
   GTGCTCAAAG CAAAACCACT TATAACAGCA AAAATGACCA TTTTAGTCAG ACGCTGGCAG 3720
   CGGCAGGTAA AACCGAGCGT GTGGAAGCGA TGGTGCAATA TACCTACCGT AAAGGCAAAG 3780
   AAAACAAAGC ACACAGCGAC CTAAATGGCA TCAACCAAAG CCTATATCGC TTGGGTGCAT 3840
   GGCAACAAAA ATATGATTTA AGAAAGCCTA ACGAACTGTT TGCAGGCACA AGCTATATCA 3900
   CCGAAAGCTG TTTGGCAAGT GATGACCCAA AAAGCTGCGT ACAATACCCT TATGTCTACA 3960
   CCAAAGCCCG ACCAGATGGT ATCGGCAATC GCAATTTTTC TGAGTTAAGC GATGCTGAAA 4020
   AAGCACAATA TTTGGCGTCC ACGCACCCCC ATGAGGTTGT CTCTGCCAAA GATTATACAG 4080
   GCACTTATCG GTTGTTACCT GACCCCATGG ACTATCGTTC AGACTCGTAT TTGGCACGCC 4140
   TTAACATCAA AATCACCCCA AATTTGGTCA GTAAACTGTT ATTAGAAGAC ACCAAGCAAA 4200
   CATACAACAT TCGTGATATG CGTCATTGTA GTTATCATGG GGCAAGATTG GGCAATGACG 4260
   GTAAGCCTGC CAATGGCGGC TCCATTGTCC TTTGCGATGA TTATCAAGAG TATCTAAATG 4320
   CCAATGACGC ATCACAAGCA TCATTTAGAC CAGGGGCTAA TGACGCCCCC ATTCCAAAAC 4380
   TGGCTTATGC CAGAAGCAGT GTGTTTAACC AAGAGCATGG CAAAACTCGC TATGGGTTAG 4440
   GTTTTGAGTT TAAGCCTGAC ACGCCATGGT TTAAACAAGC AAAATTAAAC CTACATCAAC 4500
   AAAATATCCA AATCATTAAC CATGACATTA AAAAATCGTG CAGCCAATAT CCCAAGGTGG 4560
   ATTTAAATTG TGGCATCAGT GAAATTGGGC ATTATGAATA TCAAAACAAT TACCGTTATA 4620
   AAGAAGGGCG TACCAGTTTG ACAGGCAAAC TTGATTTTAA TTTTGACCTG CTGGGCCAGC 4680
   ACGATTTGAC GGTGTTGGCT GGTGCAGATA AAGTTAAAAG CCAATTTCGT GCCAACAACC 4740
   CCAGACGCAC AATCATTGAC ACCACCCAAG GCGATGCCAT CATTGATGAA AGCACGCTGA 4800
   CAGCACAGGA GCAAGCCAAA TTTAAGCAAT CAGGGGCAGC ATGGATTGTC AAAAATCGCT 4860
   TAGGACGCTT AGAAGAAAAA GACGCCTGTG GCAATGCCAA TGAATGTGAA CGCGCGCCCA 4920
   TTCATGGCAG TAACCAATAT GTGGGCATTA ACAACCTTTA TACACCAAAT GATTATGTGG 4980
   ATTTAAGTTT TGGTGGACGC TTGGATAAAC AACGCATTCA CAGCACCGAT TCAAACATCA 5040
   TCAGCAAAAC TTACACCAAC AAAAGCTATA ATTTTGGAGC GGCGGTTCAT CTGACACCTG 5100
   ATTTTAGCCT GTTGTATAAA ACTGCCAAAG GCTTTCGTAC GCCAAGTTTT TATGAACTGT 5160
   ACAACTATAA CAGCACCGCC GCCCAGCATA AAAATGACCC TGATGTGTCT TTTCCCAAAC 5220
   GAGCGGTTGA TGTCAAACCT GAAACTTCCA ATACCAATGA ATACGGCTTT CGCTATCAGC 5280
   ACCCTTGGGG GGATATTGAG ATGAGCATGT TCAAAAGCCG TTACAAGGAC ATGTTAGATA 5340
   AAGCCATACC GAACCTAACC AAAGCCCAGC AAGAGTATTG TAAGGCTCAT TTGGATTCCA 5400
   ATGAATGTGT TGGTAATCCA CCCACGCCCA AAACCAGTGA TGAGGTATTT GCCAACTTAT 5460
   ATAATGCCAC CATCAAAGGG GTGAGTGTCA AAGGCAAACT GGATTTGCAT GCCATGACAT 5520
   CAAAACTGCC AGATGGTCTT GAAATGACCT TGGGTTATGG TCATACCAAA TTGGGGAAAT 5580
   TTGATTACAT TGCACCCAAA GATGCCGATG GTTGGTATCA GGCTCGCCCT GCTTTTTGGG 5640
   ATGCCATCAC CCCAGCGCGC TATGTGGTCG GTCTAAACTA TGACCACCCC AGTCAAGTAT 5700
   GGGGCATTGG CACAACTTTA ACGCACAGCA AACAAAAAGA TGAAAATGAG CTAAGTGCCC 5760
   TTAGAATCCG AAATGGCAAA AGAGAAATAC AAACCTTAAC GCACACAATA CCCAAAGCCT 5820
   ATACCTTACT GGACATGACA GGCTATTATA GCCCAACTGA GAGCATCACC GCTCGTCTTG 5880
   GTATCAACAA TGTATTAAAC ACCCGCTACA CCACATGGGA AGCGGCACGC CAACTGCCCA 5940
   GCGAAGCTGC AAGCAGTACC CAATCAACCC GTTACATTGC ACCAGGTCGC AGTTACTTTG 6000
   CCAGTCTTGA AATGAAGTTT TAATATGACC TGTTTACCAA AGACCAACCC TGCTTTAAAA 6060
   GTCAAGCACA GATTTTTAAA GCAGGTGCTG TTATTGCTTT GTGTTGATAC ATTAACAGCA 6120
   CAGGCGTACG CCCACAGCCA TCATACGCCC ATTCATACAC CCACGCATGA GCTGTCATCT 6180
   GCTGATGCTT TATCAGATGA AGGCTTGGGT AAGGATTTGG GCAGTTTGGA CAGCCCAGAT 6240
   GGTTTGGGTG ATGGTTTAGG CGATGGTTTG GGTGATGGCT TAAAAAGTGA TAAAACCCCT 6300
   TTACCCATCA ACGCCTTGAC CGTTAATCAG AGCAATGAGA GCCAGCCTGC CCCACCGAGC 6360
   GTAGATGTCA ATTTTTTACT TGCCCAGCCA GAGGCATTTT ATCATGTCTT TCATCAAGCG 6420
   ATTGTGCAAG ATGATGTGGC AACATTACGC TTGTTATTGC CATTTTATGA CCGCCTGCCT 6480
   GATGATTATC AAGATGATGT TTTGTTGTTA TTTGCCCAAA GTAAACTTGC CCTAAGTGAT 6540
   GGCAATACCA AATTGGCATT GAATCTGCTG ACCGATTTGA GTAACAAAGA GCCAACACTT 6600
   ACGGCGGTAA AATTACAACT TGCTTCCTTG TTGCTGACCA ACAAGCACGA TAAACACGCC   6660
   CAAATGGTGC TAGATGAACT CAAAGATGAT GCCCACTTTT TAAAATTAAG CAAAAAAGAG   6720
   CAAAGATGGG TGCTATCGCA AAGTCGCTAT TTACATAAAA AATATAAAAT GGGCTTGGAT   6780
   TTGGGCATCA ACTATCTGCA TTTGGATAAT ATCAACGCCG CCTCCACCAT CACCCAGCCC   6840
   AACATTAAAA AAGATGCCCC AAAACCTGCT CATGGGCTTG CCTTATCGCT TGGTGTGAAT   6900
   AAATACACGC CGCTTAGTCA TGGCATGAGT ATTTATACAG CCCTAGATGT TGATGGTAAA   6960
   TTTTATGATG ACAAAAGCCA CAATGAACTG GCGGTTTTTG CTCATGCTGG ACTAAGAAAA   7020
   GATCACCAAA AAGGTTATGT TGATGTCGTA CCTTTTGTTG GGCGTATTTT TGCCACCAAT   7080
   CAGCAGCATG GCAGATTATC CCCCAGAAAA GACAGTCAGG GCGTGGCGTT TGGCAGCCAT   7140
   CATCGGATCA ATGATAAATG GCAAAATGCG TTTTTTGCAC GCATGGAAAA AGGCAATTAT   7200
   ACCGAGCATT ATCAAGGTTA TGATGGCAAG CGTTATCATG TGAATGACAC CATTTTGTTG   7260
   CAAGATGGCC CAAATCGTCG TTACTCTTTG GGCGTGGGGT ATCAGCTTAG CCATCTGCAA   7320
   GATGCAACAA AAAGCAGTCA TGCCACAAAG ATACATTTTG GGGTGTTGCA AAGATTGCCA   7380
   AATGGTCTGA CCGTGCAAGG TAGAGTGAGT GCTGAGCGTG AGCGTTATCA TGGTAAATTA   7440
   TTGCGTCTGG TTAATCCTGA TGATGTGTAT CGCACAGATA AAACCCTAAC CCTACAAACC   7500
   TCCATTTGGC ACAAAGACAT TCACTGGCTT GGATTAACGC CAAAGCTGAC TTATCGTTAC   7560
   AGTAAAAATA ACAGTAACTT ACCAGCACTT TATAGCCATA ACAAACAAAA TTTTTATTTG   7620
   GAGCTTGGTC GGTCGTTTTA A                                             7641
(2) INFORMATION FOR SEQ ID NO:7:
   (i) SEQUENCE CHARACTERISTICS:
      (A) LENGTH: 2682 base pairs
      (B) TYPE: nucleic acid
      (C) STRANDEDNESS: single
      (D) TOPOLOGY: linear
   (xi) SEQUENCE DESCRIPTION: SEQ ID NO:7:
   ATGAGTACTG TCAAAACCCC CCATATTTTC TACCAAAAAC GCACCCTTAG CCTTGCCATC     60
   GCCAGTATTT TTGCTGCCTT GGTGATGACA GGCTGCCGCT CTGATGACAT CAGCGTCAAT    120
   GCACCCAATG TTACCCAGCT GCCCCAAGGC ACGGTTTCAC CAACGCCGAA CACAGGTCAT    180
   GACAACGCCA ATAACACCAA CAATCAGGGC AACAACACGG ATAACAGCAC CAGCACAACT    240
   GACCCAAATG GCGATAACAA CCAACTGACA CAAGCGCAAA AAACTGCCGC CGCCGCAGGG    300
   TTTTTTGTGA TGGGTAAAAT TCGTGATACC AGCGAAAAAA ATGACCCAGA TTATAGTGAT    360
   GATTTAAAAC AGCAGTGGCT GGGCAAATTA TATGTTGGTA TTGATGCCCA TCGCCCAGAT     420
   GGCATCGGAA AAGGTAAAAA CTTGCGTCAG CCCATCACCG CCAATGACAT CAAACCCTTG    480
   TATTTTAACA AATTCCCTGC ATTGTCTGAT TTGCACTTAG ACAGTGAACG CCATCGTTTT    540
   GACCCCCAAA AGATAAACAC CATTAAAGTG TATGGTTATG GTAACTTAAC AACACCATCC    600
   AACAACAACA CTCACATCAA TCATCAGCAA GCTGATAATA AGAAAAATAA CAAGCCTGTT    660
   GACCCTTATG AAAATATCCG TTTTGGGTAT CTTGAACTAC AAGGAAGCAG CCTGACCCAA    720
   AAAAATGCCG ATAATCAAAA TGAGCAAGAC CGCATTCCCA AACCCATGCC CATTTTGTTT    780
   TATCATGGAG AAAACGCCAG CAGCCAGCTG CCCAGCGCTG GTAAATTTAA CTACACAGGC    840
   AACTGGCTGT ACCTAAGTGA TGTCAAAAAA CGCCCTGCCC TTTCAGCATC AGATGAGCGA    900
   GTGGGGGTCT ATCTCAATGC CAGTGGCAAA GCCAACGAGG GCGATGTCGT CAGTGCCGCC    960
   CACATTTATC TAAACGGCTT TCAATATAAG CACACGCCTG CCACTTATCA GGTGGATTTT   1020
   GACACAAACT CATTAACAGG CAAGCTGTCC TATTATGACA ATCCCAATCA GCAAAATAAT   1080
   AAAGGCGAAT ATCTCAAAAG CCAATTTGAC ACTACCAAAA AAGTCAATGA AACCGATGTG   1140
   TATCAAATTG ATGCCAAAAT CAACGGTAAC CGCTTTGTCG GTACGGCCAA ATCTTTGGTT   1200
   AATGAGAAAA CACAAACCGC ACCTTTTATC AAAGAGCTGT TCTCCAAAAA AGCCAACCCC   1260
   AATAACCCAA ACCCTAATTC AGACACGCTA GAAGGCGGAT TTTATGGTGA GTCGGGCGAT   1320
   GAGCTGGCGG GTAAATTTTT ATCCAATGAC AACGCATCTT ATGTGGTCTT TGGTGGCAAA   1380
   CGAGACAAAA CGACTAAACC TGTCGCCACA AAAACGGTGT ATTTTAGTGC AGGCTTTGAA   1440
   AAACCCAGCA CCAGTTTTGT GGATAATGAA ACGATTGGTG GAATTATTGA CCGTAAAGGG   1500
   TTAAATAATC ACATTAATGA AGATGAAATT ATTCCCAGTG ATGATAGTTA TTATGGATAT   1560
   ACTTGGGGCA AGCCAGAGAA GCAGTTCACC AAAAAAGTCA GCAGCAGCAC CCAAGTCGTG   1620
   CCAGCTTATT TTGGGCAACA TGATAAATTT TATTTTAATG GCAACTATTA TGACCTATCA   1680
   GCCAGTCGTG TTGATAAATT AGCCCCTGCC GATGCTGTCA AAGCCAACCA ATCCATTAAA   1740
   GAAAAATACC CTAATGCCAC ACTAAATAAG GACAACCAAG TTACCGCCAT CGTGCTACAA   1800
   GAAGCCAAAG ATAATAAGCC TTATACCGCC ATTCGTGCCA AAGCTATCAA GCACATCAGT   1860
   TTTGGCGAGA CGCTGTATAA CGATGCCAAC CAAACCCCAA CACGCAGTTA TTTTGTGCAA   1920
   GGCGGTAGGG CAGATACCAG CACAACTTTG CCCCAGGCAG GTAAATTCAC TTACAACGGT   1980
   CTTTGGGCAG GCTACCTGAC CCAAAAAAAG GACAAAGGTT ATAGCGATAA TGCAGAAACC   2040
   ATCAAGGAAA AAGGTCATCC AGGTTATCTG TTAACCGAAA ACTTCACCCC AGAAGATGAT   2100
   GACGATGATT TGACCGCATC TGATGATTCA CAAGATGATA ATACACATGG CGATGATGAT   2160
   TTGATTGCAT CTGATGATTC ACAAGATGAT GACGCAGATG GAGATGACGA TTCAGATGAT   2220
   TTGGGTGATG GTGCAGATGA TGACGCCGCA GGCAAAGTGT ATCATGCAGG TAATATTCGC   2280
   CCTGAATTTG AAAACAAATA CTTGCCCATT AATGAGCCTA CTCATGAAAA AACCTTTGCC   2340
   CTAGATGGTA AAAATAAAGC TAAGTTTGAA GTGGATTTTA ACACCAACAG CCTAACTGGT    2400
   AAATTAAACG ATGAGAGAGG TGATATCGTC TTTGATATCA AAAATGGCAA AATTGATGGC    2460
   ACAGGATTTA CCGCCAAAGC CGATGTGCCA AACTATCGTG AAGAAGTGGG TAACAACCAA    2520
   GGTGGCGGTT TCTTATACAA CATCAAAGAT ATTGATGTTA AGGGGCAATT TTTTGGCACA    2580
   AATGGCGAAG AGTTGGCAGG ACAGTTACAT CATGACAAAG GCGATGGCAT CAATGACACC    2640
   GCCGAAAAAG CAGGGGCTGT CTTTGGGGCT GTTAAAGATA AA                       2682
(2) INFORMATION FOR SEQ ID NO:8:
   (i) SEQUENCE CHARACTERISTICS:
      (A) LENGTH: 3000 base pairs
      (B) TYPE: nucleic acid
      (C) STRANDEDNESS: single
      (D) TOPOLOGY: linear
   (xi) SEQUENCE DESCRIPTION: SEQ ID NO:8:
   ATGTCAAAAT CTATCACAAA AACACAAACA CCATCAGTCC ATACCATGAC CACGCACCGC     60
   TTAAACCTTG CCATCAAAGC GGCGTTATTT GGTGTGGCAG TTTTACCCCT ATCCGTCTGG    120
   GCGCAAGAGA ACACTCAGAC AGATGCCAAC TCTGATGCCA AAGACACAAA AACCCCTGTC    180
   GTCTATTTAG ATGCCATCAC GGTAACCGCC GCCCCATCTG CCCCTGTTTC TCGGTTTGAC    240
   ACCGATGTAA CAGGGCTTGG CAAAACCGTC AAAACCGCTG ACACGCTGGC AAAAGAACAA    300
   GTACAGGGCA TTCGTGATTT GGTGCGTTAT GAAACTGGGG TGAGTGTGGT TGAGCAGGGG    360
   CGTGGTGGCA GCAGCGGATT TGCCATTCAT GGCGTGGATA AAAACCGAGT GGGCATTACC    420
   GTAGATGGCA TTGCCCAAAT TCAATCCTAC AAAGACGAAT CCACTAAGCG AGCTGGGGCA    480
   GGCTCTGGGG CGATGAACGA GATAGAGATT GAAAACATTG CCGCCGTTGC CATCAATAAA    540
   GGCGGTAATG CCTTAGAAGC AGGCTCTGGT GCGTTGGGTG GTTCGGTGGC GTTTCATACC    600
   AAAGATGTGA GCGATGTCTT AAAATCTGGT AACAATCTTG GTGCTCAAAG CAAAACCACT    660
   TATAACAGCA AAAATGACCA TTTTAGTCAG ACGCTGGCAG CGGCAGGTAA AACCGAGCGT    720
   GTGGAAGCGA TGGTGCAATA TACCTACCGT AAAGGCAAAG AAAACAAAGC ACACAGCGAC    780
   CTAAATGGCA TCAACCAAAG CCTATATCGC TTGGGTGCAT GGCAACAAAA ATATGATTTA    840
   AGAAAGCCTA ACGAACTGTT TGCAGGCACA AGCTATATCA CCGAAAGCTG TTTGGCAAGT    900
   GATGACCCAA AAAGCTGCGT ACAATACCCT TATGTCTACA CCAAAGCCCG ACCAGATGGT    960
   ATCGGCAATC GCAATTTTTC TGAGTTAAGC GATGCTGAAA AAGCACAATA TTTGGCGTCC   1020
   ACGCACCCCC ATGAGGTTGT CTCTGCCAAA GATTATACAG GCACTTATCG GTTGTTACCT   1080
   GACCCCATGG ACTATCGTTC AGACTCGTAT TTGGCACGCC TTAACATCAA AATCACCCCA   1140
   AATTTGGTCA GTAAACTGTT ATTAGAAGAC ACCAAGCAAA CATACAACAT TCGTGATATG   1200
   CGTCATTGTA GTTATCATGG GGCAAGATTG GGCAATGACG GTAAGCCTGC CAATGGCGGC 1260
   TCCATTGTCC TTTGCGATGA TTATCAAGAG TATCTAAATG CCAATGACGC ATCACAAGCA 1320
   TCATTTAGAC CAGGGGCTAA TGACGCCCCC ATTCCAAAAC TGGCTTATGC CAGAAGCAGT 1380
   GTGTTTAACC AAGAGCATGG CAAAACTCGC TATGGGTTAG GTTTTGAGTT TAAGCCTGAC 1440
   ACGCCATGGT TTAAACAAGC AAAATTAAAC CTACATCAAC AAAATATCCA AATCATTAAC 1500
   CATGACATTA AAAAATCGTG CAGCCAATAT CCCAAGGTGG ATTTAAATTG TGGCATCAGT 1560
   GAAATTGGGC ATTATGAATA TCAAAACAAT TACCGTTATA AAGAAGGGCG TACCAGTTTG 1620
   ACAGGCAAAC TTGATTTTAA TTTTGACCTG CTGGGCCAGC ACGATTTGAC GGTGTTGGCT 1680
   GGTGCAGATA AAGTTAAAAG CCAATTTCGT GCCAACAACC CCAGACGCAC AATCATTGAC 1740
   ACCACCCAAG GCGATGCCAT CATTGATGAA AGCACGCTGA CAGCACAGGA GCAAGCCAAA 1800
   TTTAAGCAAT CAGGGGCAGC ATGGATTGTC AAAAATCGCT TAGGACGCTT AGAAGAAAAA 1860
   GACGCCTGTG GCAATGCCAA TGAATGTGAA CGCGCGCCCA TTCATGGCAG TAACCAATAT 1920
   GTGGGCATTA ACAACCTTTA TACACCAAAT GATTATGTGG ATTTAAGTTT TGGTGGACGC 1980
   TTGGATAAAC AACGCATTCA CAGCACCGAT TCAAACATCA TCAGCAAAAC TTACACCAAC 2040
   AAAAGCTATA ATTTTGGAGC GGCGGTTCAT CTGACACCTG ATTTTAGCCT GTTGTATAAA 2100
   ACTGCCAAAG GCTTTCGTAC GCCAAGTTTT TATGAACTGT ACAACTATAA CAGCACCGCC 2160
   GCCCAGCATA AAAATGACCC TGATGTGTCT TTTCCCAAAC GAGCGGTTGA TGTCAAACCT 2220
   GAAACTTCCA ATACCAATGA ATACGGCTTT CGCTATCAGC ACCCTTGGGG GGATATTGAG 2280
   ATGAGCATGT TCAAAAGCCG TTACAAGGAC ATGTTAGATA AAGCCATACC GAACCTAACC 2340
   AAAGCCCAGC AAGAGTATTG TAAGGCTCAT TTGGATTCCA ATGAATGTGT TGGTAATCCA 2400
   CCCACGCCCA AAACCAGTGA TGAGGTATTT GCCAACTTAT ATAATGCCAC CATCAAAGGG 2460
   GTGAGTGTCA AAGGCAAACT GGATTTGCAT GCCATGACAT CAAAACTGCC AGATGGTCTT 2520
   GAAATGACCT TGGGTTATGG TCATACCAAA TTGGGGAAAT TTGATTACAT TGCACCCAAA 2580
   GATGCCGATG GTTGGTATCA GGCTCGCCCT GCTTTTTGGG ATGCCATCAC CCCAGCGCGC 2640
   TATGTGGTCG GTCTAAACTA TGACCACCCC AGTCAAGTAT GGGGCATTGG CACAACTTTA 2700
   ACGCACAGCA AACAAAAAGA TGAAAATGAG CTAAGTGCCC TTAGAATCCG AAATGGCAAA 2760
   AGAGAAATAC AAACCTTAAC GCACACAATA CCCAAAGCCT ATACCTTACT GGACATGACA 2820
   GGCTATTATA GCCCAACTGA GAGCATCACC GCTCGTCTTG GTATCAACAA TGTATTAAAC 2880
   ACCCGCTACA CCACATGGGA AGCGGCACGC CAACTGCCCA GCGAAGCTGC AAGCAGTACC 2940
   CAATCAACCC GTTACATTGC ACCAGGTCGC AGTTACTTTG CCAGTCTTGA AATGAAGTTT 3000
(2) INFORMATION FOR SEQ ID NO:9:
   (i) SEQUENCE CHARACTERISTICS:
      (A) LENGTH: 2955 base pairs
      (B) TYPE: nucleic acid
      (C) STRANDEDNESS: single
      (D) TOPOLOGY: linear
   (xi) SEQUENCE DESCRIPTION: SEQ ID NO:9:
   ATGACCACGC ACCGCTTAAA CCTTGCCATC AAAGCGGCGT TATTTGGTGT GGCAGTTTTA     60
   CCCCTATCCG TCTGGGCGCA AGAGAACACT CAGACAGATG CCAACTCTGA TGCCAAAGAC    120
   ACAAAAACCC CTGTCGTCTA TTTAGATGCC ATCACGGTAA CCGCCGCCCC ATCTGCCCCT    180
   GTTTCTCGGT TTGACACCGA TGTAACAGGG CTTGGCAAAA CCGTCAAAAC CGCTGACACG    240
   CTGGCAAAAG AACAAGTACA GGGCATTCGT GATTTGGTGC GTTATGAAAC TGGGGTGAGT    300
   GTGGTTGAGC AGGGGCGTGG TGGCAGCAGC GGATTTGCCA TTCATGGCGT GGATAAAAAC    360
   CGAGTGGGCA TTACCGTAGA TGGCATTGCC CAAATTCAAT CCTACAAAGA CGAATCCACT    420
   AAGCGAGCTG GGGCAGGCTC TGGGGCGATG AACGAGATAG AGATTGAAAA CATTGCCGCC     480
   GTTGCCATCA ATAAAGGCGG TAATGCCTTA GAAGCAGGCT CTGGTGCGTT GGGTGGTTCG    540
   GTGGCGTTTC ATACCAAAGA TGTGAGCGAT GTCTTAAAAT CTGGTAACAA TCTTGGTGCT    600
   CAAAGCAAAA CCACTTATAA CAGCAAAAAT GACCATTTTA GTCAGACGCT GGCAGCGGCA    660
   GGTAAAACCG AGCGTGTGGA AGCGATGGTG CAATATACCT ACCGTAAAGG CAAAGAAAAC    720
   AAAGCACACA GCGACCTAAA TGGCATCAAC CAAAGCCTAT ATCGCTTGGG TGCATGGCAA    780
   CAAAAATATG ATTTAAGAAA GCCTAACGAA CTGTTTGCAG GCACAAGCTA TATCACCGAA    840
   AGCTGTTTGG CAAGTGATGA CCCAAAAAGC TGCGTACAAT ACCCTTATGT CTACACCAAA    900
   GCCCGACCAG ATGGTATCGG CAATCGCAAT TTTTCTGAGT TAAGCGATGC TGAAAAAGCA    960
   CAATATTTGG CGTCCACGCA CCCCCATGAG GTTGTCTCTG CCAAAGATTA TACAGGCACT   1020
   TATCGGTTGT TACCTGACCC CATGGACTAT CGTTCAGACT CGTATTTGGC ACGCCTTAAC   1080
   ATCAAAATCA CCCCAAATTT GGTCAGTAAA CTGTTATTAG AAGACACCAA GCAAACATAC   1140
   AACATTCGTG ATATGCGTCA TTGTAGTTAT CATGGGGCAA GATTGGGCAA TGACGGTAAG   1200
   CCTGCCAATG GCGGCTCCAT TGTCCTTTGC GATGATTATC AAGAGTATCT AAATGCCAAT   1260
   GACGCATCAC AAGCATCATT TAGACCAGGG GCTAATGACG CCCCCATTCC AAAACTGGCT   1320
   TATGCCAGAA GCAGTGTGTT TAACCAAGAG CATGGCAAAA CTCGCTATGG GTTAGGTTTT   1380
   GAGTTTAAGC CTGACACGCC ATGGTTTAAA CAAGCAAAAT TAAACCTACA TCAACAAAAT   1440
   ATCCAAATCA TTAACCATGA CATTAAAAAA TCGTGCAGCC AATATCCCAA GGTGGATTTA   1500
   AATTGTGGCA TCAGTGAAAT TGGGCATTAT GAATATCAAA ACAATTACCG TTATAAAGAA   1560
   GGGCGTACCA GTTTGACAGG CAAACTTGAT TTTAATTTTG ACCTGCTGGG CCAGCACGAT 1620
   TTGACGGTGT TGGCTGGTGC AGATAAAGTT AAAAGCCAAT TTCGTGCCAA CAACCCCAGA 1680
   CGCACAATCA TTGACACCAC CCAAGGCGAT GCCATCATTG ATGAAAGCAC GCTGACAGCA 1740
   CAGGAGCAAG CCAAATTTAA GCAATCAGGG GCAGCATGGA TTGTCAAAAA TCGCTTAGGA 1800
   CGCTTAGAAG AAAAAGACGC CTGTGGCAAT GCCAATGAAT GTGAACGCGC GCCCATTCAT 1860
   GGCAGTAACC AATATGTGGG CATTAACAAC CTTTATACAC CAAATGATTA TGTGGATTTA 1920
   AGTTTTGGTG GACGCTTGGA TAAACAACGC ATTCACAGCA CCGATTCAAA CATCATCAGC 1980
   AAAACTTACA CCAACAAAAG CTATAATTTT GGAGCGGCGG TTCATCTGAC ACCTGATTTT 2040
   AGCCTGTTGT ATAAAACTGC CAAAGGCTTT CGTACGCCAA GTTTTTATGA ACTGTACAAC 2100
   TATAACAGCA CCGCCGCCCA GCATAAAAAT GACCCTGATG TGTCTTTTCC CAAACGAGCG 2160
   GTTGATGTCA AACCTGAAAC TTCCAATACC AATGAATACG GCTTTCGCTA TCAGCACCCT 2220
   TGGGGGGATA TTGAGATGAG CATGTTCAAA AGCCGTTACA AGGACATGTT AGATAAAGCC 2280
   ATACCGAACC TAACCAAAGC CCAGCAAGAG TATTGTAAGG CTCATTTGGA TTCCAATGAA 2340
   TGTGTTGGTA ATCCACCCAC GCCCAAAACC AGTGATGAGG TATTTGCCAA CTTATATAAT 2400
   GCCACCATCA AAGGGGTGAG TGTCAAAGGC AAACTGGATT TGCATGCCAT GACATCAAAA 2460
   CTGCCAGATG GTCTTGAAAT GACCTTGGGT TATGGTCATA CCAAATTGGG GAAATTTGAT 2520
   TACATTGCAC CCAAAGATGC CGATGGTTGG TATCAGGCTC GCCCTGCTTT TTGGGATGCC 2580
   ATCACCCCAG CGCGCTATGT GGTCGGTCTA AACTATGACC ACCCCAGTCA AGTATGGGGC 2640
   ATTGGCACAA CTTTAACGCA CAGCAAACAA AAAGATGAAA ATGAGCTAAG TGCCCTTAGA 2700
   ATCCGAAATG GCAAAAGAGA AATACAAACC TTAACGCACA CAATACCCAA AGCCTATACC 2760
   TTACTGGACA TGACAGGCTA TTATAGCCCA ACTGAGAGCA TCACCGCTCG TCTTGGTATC 2820
   AACAATGTAT TAAACACCCG CTACACCACA TGGGAAGCGG CACGCCAACT GCCCAGCGAA 2880
   GCTGCAAGCA GTACCCAATC AACCCGTTAC ATTGCACCAG GTCGCAGTTA CTTTGCCAGT 2940
   CTTGAAATGA AGTTT 2955
(2) INFORMATION FOR SEQ ID NO:10:
   (i) SEQUENCE CHARACTERISTICS:
      (A) LENGTH: 1614 base pairs
      (B) TYPE: nucleic acid
      (C) STRANDEDNESS: single
      (D) TOPOLOGY: linear
   (xi) SEQUENCE DESCRIPTION: SEQ ID NO:10:
   ATGACCTGTT TACCAAAGAC CAACCCTGCT TTAAAAGTCA AGCACAGATT TTTAAAGCAG     60
   GTGCTGTTAT TGCTTTGTGT TGATACATTA ACAGCACAGG CGTACGCCCA CAGCCATCAT    120
   ACGCCCATTC ATACACCCAC GCATGAGCTG TCATCTGCTG ATGCTTTATC AGATGAAGGC    180
   TTGGGTAAGG ATTTGGGCAG TTTGGACAGC CCAGATGGTT TGGGTGATGG TTTAGGCGAT    240
   GGTTTGGGTG ATGGCTTAAA AAGTGATAAA ACCCCTTTAC CCATCAACGC CTTGACCGTT    300
   AATCAGAGCA ATGAGAGCCA GCCTGCCCCA CCGAGCGTAG ATGTCAATTT TTTACTTGCC    360
   CAGCCAGAGG CATTTTATCA TGTCTTTCAT CAAGCGATTG TGCAAGATGA TGTGGCAACA    420
   TTACGCTTGT TATTGCCATT TTATGACCGC CTGCCTGATG ATTATCAAGA TGATGTTTTG    480
   TTGTTATTTG CCCAAAGTAA ACTTGCCCTA AGTGATGGCA ATACCAAATT GGCATTGAAT    540
   CTGCTGACCG ATTTGAGTAA CAAAGAGCCA ACACTTACGG CGGTAAAATT ACAACTTGCT    600
   TCCTTGTTGC TGACCAACAA GCACGATAAA CACGCCCAAA TGGTGCTAGA TGAACTCAAA    660
   GATGATGCCC ACTTTTTAAA ATTAAGCAAA AAAGAGCAAA GATGGGTGCT ATCGCAAAGT    720
   CGCTATTTAC ATAAAAAATA TAAAATGGGC TTGGATTTGG GCATCAACTA TCTGCATTTG    780
   GATAATATCA ACGCCGCCTC CACCATCACC CAGCCCAACA TTAAAAAAGA TGCCCCAAAA    840
   CCTGCTCATG GGCTTGCCTT ATCGCTTGGT GTGAATAAAT ACACGCCGCT TAGTCATGGC    900
   ATGAGTATTT ATACAGCCCT AGATGTTGAT GGTAAATTTT ATGATGACAA AAGCCACAAT    960
   GAACTGGCGG TTTTTGCTCA TGCTGGACTA AGAAAAGATC ACCAAAAAGG TTATGTTGAT   1020
   GTCGTACCTT TTGTTGGGCG TATTTTTGCC ACCAATCAGC AGCATGGCAG ATTATCCCCC   1080
   AGAAAAGACA GTCAGGGCGT GGCGTTTGGC AGCCATCATC GGATCAATGA TAAATGGCAA   1140
   AATGCGTTTT TTGCACGCAT GGAAAAAGGC AATTATACCG AGCATTATCA AGGTTATGAT   1200
   GGCAAGCGTT ATCATGTGAA TGACACCATT TTGTTGCAAG ATGGCCCAAA TCGTCGTTAC   1260
   TCTTTGGGCG TGGGGTATCA GCTTAGCCAT CTGCAAGATG CAACAAAAAG CAGTCATGCC   1320
   ACAAAGATAC ATTTTGGGGT GTTGCAAAGA TTGCCAAATG GTCTGACCGT GCAAGGTAGA   1380
   GTGAGTGCTG AGCGTGAGCG TTATCATGGT AAATTATTGC GTCTGGTTAA TCCTGATGAT   1440
   GTGTATCGCA CAGATAAAAC CCTAACCCTA CAAACCTCCA TTTGGCACAA AGACATTCAC   1500
   TGGCTTGGAT TAACGCCAAA GCTGACTTAT CGTTACAGTA AAAATAACAG TAACTTACCA   1560
   GCACTTTATA GCCATAACAA ACAAAATTTT TATTTGGAGC TTGGTCGGTC GTTT         1614
(2) INFORMATION FOR SEQ ID NO:11:
   (i) SEQUENCE CHARACTERISTICS:
      (A) LENGTH: 898 amino acids
      (B) TYPE: amino acid
      (C) STRANDEDNESS: single
      (D) TOPOLOGY: linear
   (xi) SEQUENCE DESCRIPTION: SEQ ID NO:11:
   Met Ser Thr Val Lys Thr Pro His Ile Phe Tyr Gln Lys Arg Thr Leu
   1               5                   10                  15
   Ser Leu Ala Ile Ala Ser Ile Phe Ala Ala Leu Val Met Thr Gly Cys
               20                  25                  30
   Arg Ser Asp Asp Ile Ser Val Asn Ala Pro Asn Val Thr Gln Leu Pro
           35                  40                  45
   Gln Gly Thr Val Ser Pro Ile Pro Asn Thr Gly His Asp Asn Thr Asn
       50                  55                  60
   Asn Thr Asn Asn Gln Gly Asn Asn Thr Asp Asn Ser Thr Ser Thr Thr
   65                  70                  75                  80
   Asp Pro Asn Gly Asp Asn Asn Gln Leu Thr Gln Ala Gln Lys Thr Ala
                   85                  90                  95
   Ala Ala Ala Gly Phe Phe Val Met Gly Lys Ile Arg Asp Thr Ser Pro
               100                 105                 110
   Lys Asn Asp Pro Asp Tyr Ser Asn Asp Leu Val Gln Gln Trp Gln Gly
           115                 120                 125
   Lys Leu Tyr Val Gly Ile Asp Ala His Arg Pro Asp Gly Ile Gly Thr
       130                 135                 140
   Gly Lys Asn Leu Arg Gln Pro Ile Thr Ala Asn Asp Ile Lys Pro Leu
   145                 150                 155                 160
   Tyr Phe Asn Lys Phe Pro Ala Leu Ser Asp Leu His Leu Asp Ser Glu
                   165                 170                 175
   Arg His Arg Phe Asp Pro Lys Lys Leu Asn Thr Ile Lys Val Tyr Gly
               180                 185                 190
   Tyr Gly Asn Leu Thr Thr Pro Ser Lys Asn Asn Thr Tyr Ile Asn His
           195                 200                 205
   Gln Gln Ala Asp Asn Lys Lys Asn Asn Lys Pro Val Asp Pro Tyr Glu
       210                 215                 220
   Asn Ile Arg Phe Gly Tyr Leu Glu Leu Gln Gly Ser Ser Leu Thr Gln
   225                 230                 235                 240
   Lys Asn Ala Asp Thr Pro Asn Asp Lys Asp Arg Ile Pro Lys Pro Met
                   245                 250                 255
   Pro Ile Leu Phe Tyr His Gly Glu Asn Ala Ser Ser Gln Leu Pro Ser
               260                 265                 270
   Ala Gly Lys Phe Asn Tyr Thr Gly Asn Trp Leu Tyr Leu Ser Asp Val
           275                 280                 285
   Lys Lys Arg Pro Ala Leu Ser Ala Ser Asp Asp Arg Val Gly Val Tyr
       290                 295                 300
   Leu Asn Ala Ser Gly Lys Ser Asn Glu Gly Asp Val Val Ser Ala Ala
   305                 310                 315                 320
   His Ile Tyr Leu Asn Gly Phe Gln Tyr Lys His Thr Pro Ala Thr Tyr
                   325                 330                 335
   Gln Val Asp Phe Asp Thr Asn Ser Leu Thr Gly Lys Leu Ser Tyr Tyr
               340                 345                 350
   Asp Asn Pro Asn Gln Gln Thr Ala Gln Gly Lys Tyr Ile Lys Ser Gln
           355                 360                 365
   Phe Asp Thr Thr Lys Lys Val Asn Glu Thr Asp Val Tyr Gln Ile Asp
       370                 375                 380
   Ala Lys Ile Asn Gly Asn Arg Phe Val Gly Thr Ala Lys Ser Leu Val
   385                 390                 395                 400
   Asn Glu Asn Thr Glu Thr Ala Pro Phe Ile Lys Glu Leu Phe Ser Lys
                   405                 410                 415
   Lys Ala Asn Pro Asn Asn Pro Asn Pro Asn Ser Asp Thr Leu Glu Gly
               420                 425                 430
   Gly Phe Tyr Gly Glu Ser Gly Asp Glu Leu Ala Gly Lys Phe Leu Ser
           435                 440                 445
   Asn Asp Asn Ala Ser Tyr Val Val Phe Gly Gly Lys Arg Asp Lys Thr
       450                 455                 460
   Asp Lys Pro Val Ala Thr Lys Thr Val Tyr Phe Ser Ala Gly Phe Glu
   465                 470                 475                 480
   Lys Pro Ser Thr Ser Phe Val Asp Asn Glu Thr Ile Gly Arg Ile Ile
                   485                 490                 495
   Asn Ser Lys Lys Leu Asn Asp Ala Val Asn Glu Lys Ile Asp Asn Gly
               500                 505                 510
   Asp Ile Pro Thr Ser Asp Glu Arg Tyr Asp Glu Phe Pro Trp Gly Glu
           515                 520                 525
   Lys Lys Ala Glu Phe Thr Lys Lys Val Ser Ser Ser Thr Gln Ala Val
       530                 535                 540
   Pro Ala Tyr Phe Gly Gln His Asp Lys Phe Tyr Phe Asn Gly Asn Tyr
   545                 550                 555                 560
   Tyr Asp Leu Ser Ala Ser Ser Val Asp Lys Leu Ala Pro Ala Asp Ala
                   565                 570                 575
   Val Lys Ala Asn Gln Ser Ile Lys Glu Lys Tyr Pro Asn Ala Thr Leu
               580                 585                 590
   Asn Lys Asp Asn Gln Val Thr Ala Ile Val Leu Gln Glu Ala Lys Asp
           595                 600                 605
   Asn Lys Pro Tyr Thr Ala Ile Arg Ala Lys Ser Tyr Gln His Ile Ser
       610                 615                 620
   Phe Gly Glu Thr Leu Tyr Asn Asp Ala Asn Gln Thr Pro Thr Arg Ser
   625                 630                 635                 640
   Tyr Phe Val Gln Gly Gly Arg Ala Asp Thr Ser Thr Thr Leu Pro Lys
                   645                 650                 655
   Ala Gly Lys Phe Thr Tyr Asn Gly Leu Trp Ala Gly Tyr Leu Ile Gln
               660                 665                 670
   Lys Lys Asp Lys Gly Tyr Ser Asn Asn Glu Glu Thr Ile Lys Lys Lys
           675                 680                 685
   Gly His Gln Asp Tyr Leu Leu Thr Glu Asp Phe Thr Pro Glu Asp Asp
       690                 695                 700
   Asp Asp Asp Leu Thr Ala Ser Asp Asp Ser Gln Asp Asp Asp Ala His
   705                 710                 715                 720
   Gly Asp Asp Asp Leu Ile Ala Ser Asp Asp Ser Gln Asp Asp Asp Ala
                   725                 730                 735
   Asp Gly Asp Asp Asp Ser Asp Asp Leu Gly Asp Gly Ala Asp Asp Ala
               740                 745                 750
   Ala Ala Gly Lys Val Tyr His Ala Gly Asn Ile Arg Pro Glu Phe Glu
           755                 760                 765
   Asn Lys Tyr Leu Pro Ile Asn Glu Pro Thr His Glu Lys Thr Phe Ala
       770                 775                 780
   Leu Asp Gly Lys Asn Lys Ala Lys Phe Asp Val Asp Phe Asp Thr Asn
   785                 790                 795                 800
   Ser Leu Thr Gly Lys Leu Asn Asp Glu Arg Gly Asp Ile Val Phe Asp
                   805                 810                 815
   Ile Lys Asn Gly Lys Ile Asp Gly Thr Gly Phe Thr Ala Lys Ala Asp
               820                 825                 830
   Val Pro Asn Tyr Arg Glu Glu Val Gly Asn Asn Gln Gly Gly Gly Phe
           835                 840                 845
   Leu Tyr Asn Ile Lys Asp Ile Asp Val Lys Gly Gln Phe Phe Gly Thr
       850                 855                 860
   Asn Gly Glu Glu Leu Ala Gly Gln Leu Gln Tyr Asp Lys Gly Asp Gly
   865                 870                 875                 880
   Ile Asn Asp Thr Ala Glu Lys Ala Gly Ala Val Phe Gly Ala Val Lys
                   885                 890                 895
   Asp Lys
(2) INFORMATION FOR SEQ ID NO:12:
   (i) SEQUENCE CHARACTERISTICS:
      (A) LENGTH: 1000 amino acids
      (B) TYPE: amino acid
      (C) STRANDEDNESS: single
      (D) TOPOLOGY: linear
   (xi) SEQUENCE DESCRIPTION: SEQ ID NO:12:
   Met Ser Lys Ser Ile Thr Lys Thr Gln Thr Pro Ser Val His Thr Met
   1               5                   10                  15
   Thr Thr His Arg Leu Asn Leu Ala Ile Lys Ala Ala Leu Phe Gly Val
               20                  25                  30
   Ala Val Leu Pro Leu Ser Val Trp Ala Gln Glu Asn Thr Gln Thr Asp
           35                  40                  45
   Ala Asn Ser Asp Ala Lys Asp Thr Lys Thr Pro Val Val Tyr Leu Asp
       50                  55                  60
   Ala Ile Thr Val Thr Ala Ala Pro Ser Ala Pro Val Ser Arg Phe Asp
   65                  70                  75                  80
   Thr Asp Val Thr Gly Leu Gly Lys Thr Val Lys Thr Ala Asp Thr Leu
                   85                  90                  95
   Ala Lys Glu Gln Val Gln Gly Ile Arg Asp Leu Val Arg Tyr Glu Thr
               100                 105                 110
   Gly Val Ser Val Val Glu Gln Gly Arg Gly Gly Ser Ser Gly Phe Ala
           115                 120                 125
   Ile His Gly Val Asp Lys Asn Arg Val Gly Ile Thr Val Asp Gly Ile
       130                 135                 140
   Ala Gln Ile Gln Ser Tyr Lys Asp Glu Ser Thr Lys Arg Ala Gly Ala
   145                 150                 155                 160
   Gly Ser Gly Ala Met Asn Glu Ile Glu Ile Glu Asn Ile Ala Ala Val
                   165                 170                 175
   Ala Ile Asn Lys Gly Gly Asn Ala Leu Glu Ala Gly Ser Gly Ala Leu
               180                 185                 190
   Gly Gly Ser Val Ala Phe His Thr Lys Asp Val Ser Asp Val Leu Lys
           195                 200                 205
   Ser Gly Lys Asn Leu Gly Ala Gln Ser Lys Thr Thr Tyr Asn Ser Lys
       210                 215                 220
   Asn Asp His Phe Ser Gln Thr Leu Ala Ala Ala Gly Lys Thr Glu Arg
   225                 230                 235                 240
   Val Glu Ala Met Val Gln Tyr Thr Tyr Arg Lys Gly Lys Glu Asn Lys
                   245                 250                 255
   Ala His Ser Asp Leu Asn Gly Ile Asn Gln Ser Leu Tyr Arg Leu Gly
               260                 265                 270
   Ala Trp Gln Gln Lys Tyr Asp Leu Arg Lys Pro Asn Glu Leu Phe Ala
           275                 280                 285
   Gly Thr Ser Tyr Ile Thr Glu Ser Cys Leu Ala Ser Asp Asp Pro Lys
       290                 295                 300
   Ser Cys Val Gln Tyr Pro Tyr Val Tyr Thr Lys Ala Arg Pro Asp Gly
   305                 310                 315                 320
   Ile Gly Asn Arg Asn Phe Ser Glu Leu Ser Asp Ala Glu Lys Ala Gln
                   325                 330                 335
   Tyr Leu Ala Ser Thr His Pro His Glu Val Val Ser Ala Lys Asp Tyr
               340                 345                 350
   Thr Gly Ile Tyr Arg Leu Leu Pro Asp Pro Met Asp Tyr Arg Ser Asp
           355                 360                 365
   Ser Tyr Leu Ala Arg Leu Asn Ile Lys Ile Thr Pro Asn Leu Val Ser
       370                 375                 380
   Lys Leu Leu Leu Glu Asp Thr Lys Gln Thr Tyr Asn Ile Arg Asp Met
   385                 390                 395                 400
   Arg His Cys Ser Tyr His Gly Ala Arg Leu Gly Asn Asp Gly Lys Pro
                   405                 410                 415
   Ala Asn Gly Gly Ser Ile Val Leu Cys Asp Asp Tyr Gln Glu Tyr Leu
               420                 425                 430
   Asn Ala Asn Asp Ala Ser Gln Ala Leu Phe Arg Pro Gly Ala Asn Asp
           435                 440                 445
   Ala Pro Ile Pro Lys Leu Ala Tyr Ala Arg Ser Ser Val Phe Asn Gln
       450                 455                 460
   Glu His Gly Lys Thr Arg Tyr Gly Leu Ser Phe Glu Phe Lys Pro Asp
   465                 470                 475                 480
   Thr Pro Trp Phe Lys Gln Ala Lys Leu Asn Leu His Gln Gln Asn Ile
                   485                 490                 495
   Gln Ile Ile Asn His Asp Ile Lys Lys Ser Cys Ser Gln Tyr Pro Lys
               500                 505                 510
   Val Asp Leu Asn Cys Gly Ile Ser Glu Ile Gly His Tyr Glu Tyr Gln
           515                 520                 525
   Asn Asn Tyr Arg Tyr Lys Glu Gly Arg Ala Ser Leu Thr Gly Lys Leu
       530                 535                 540
   Asp Phe Asn Phe Asp Leu Leu Gly Gln His Asp Leu Thr Val Leu Ala
   545                 550                 555                 560
   Gly Ala Asp Lys Val Lys Ser Gln Phe Arg Ala Asn Asn Pro Arg Arg
                   565                 570                 575
   Thr Ile Ile Asp Thr Thr Gln Gly Asp Ala Ile Ile Asp Glu Ser Thr
               580                 585                 590
   Leu Thr Ala Gln Glu Gln Ala Lys Phe Lys Gln Ser Gly Ala Ala Trp
           595                 600                 605
   Ile Val Lys Asn Arg Leu Gly Arg Leu Glu Glu Lys Asp Ala Cys Gly
       610                 615                 620
   Asn Ala Asn Glu Cys Glu Arg Ala Pro Ile His Gly Ser Asn Gln Tyr
   625                 630                 635                 640
   Val Gly Ile Asn Asn Leu Tyr Thr Pro Asn Asp Tyr Val Asp Leu Ser
                   645                 650                 655
   Phe Gly Gly Arg Leu Asp Lys Gln Arg Ile His Ser Thr Asp Ser Asn
               660                 665                 670
   Ile Ile Ser Lys Thr Tyr Thr Asn Lys Ser Tyr Asn Phe Gly Ala Ala
           675                 680                 685
   Val His Leu Thr Pro Asp Phe Ser Leu Leu Tyr Lys Thr Ala Lys Gly
       690                 695                 700
   Phe Arg Thr Pro Ser Phe Tyr Glu Leu Tyr Asn Tyr Asn Ser Thr Ala
   705                 710                 715                 720
   Ala Gln His Lys Asn Asp Pro Asp Val Ser Phe Pro Lys Arg Ala Val
                   725                 730                 735
   Asp Val Lys Pro Glu Thr Ser Asn Thr Asn Glu Tyr Gly Phe Arg Tyr
               740                 745                 750
   Gln His Pro Trp Gly Asp Val Glu Met Ser Met Phe Lys Ser Arg Tyr
           755                 760                 765
   Lys Asp Met Leu Asp Lys Ala Ile Pro Asn Leu Thr Lys Ala Gln Gln
       770                 775                 780
   Glu Tyr Cys Lys Ala His Leu Asp Ser Asn Glu Cys Val Gly Asn Pro
   785                 790                 795                 800
   Pro Thr Pro Lys Thr Ser Asp Glu Val Phe Ala Asn Leu Tyr Asn Ala
                   805                 810                 815
   Thr Ile Lys Gly Val Ser Val Lys Gly Lys Leu Asp Leu His Ala Met
               820                 825                 830
   Thr Ser Lys Leu Pro Asp Gly Leu Glu Met Thr Leu Gly Tyr Gly His
           835                 840                 845
   Thr Lys Leu Gly Lys Phe Asp Tyr Ile Ala Pro Lys Asp Ala Asp Gly
       850                 855                 860
   Trp Tyr Gln Ala Arg Pro Ala Phe Trp Asp Ala Ile Thr Pro Ala Arg
   865                 870                 875                 880
   Tyr Val Val Gly Leu Asn Tyr Asp His Pro Ser Gln Val Trp Gly Ile
                   885                 890                 895
   Gly Thr Thr Leu Thr His Ser Lys Gln Lys Asp Glu Asn Glu Leu Ser
               900                 905                 910
   Ala Leu Arg Ile Arg Asn Gly Lys Arg Glu Thr Gln Thr Leu Thr His
           915                 920                 925
   Thr Ile Pro Lys Ala Tyr Thr Leu Leu Asp Met Thr Gly Tyr Tyr Ser
       930                 935                 940
   Pro Thr Glu Ser Ile Thr Ala Arg Leu Gly Ile Asn Asn Val Leu Asn
   945                 950                 955                 960
   Thr Arg Tyr Thr Thr Trp Glu Ala Ala Arg Gln Leu Pro Ser Glu Ala
                   965                 970                 975
   Ala Ser Ser Thr Gln Ser Thr Arg Tyr Ile Ala Pro Gly Arg Ser Tyr
               980                 985                 990
   Phe Ala Ser Leu Glu Met Lys Phe
           995                 1000
(2) INFORMATION FOR SEQ ID NO:13:
   (i) SEQUENCE CHARACTERISTICS:
      (A) LENGTH: 985 amino acids
      (B) TYPE: amino acid
      (C) STRANDEDNESS: single
      (D) TOPOLOGY: linear
   (xi) SEQUENCE DESCRIPTION: SEQ ID NO:13:
   Met Thr Thr His Arg Leu Asn Leu Ala Ile Lys Ala Ala Leu Phe Gly
   1               5                   10                  15
   Val Ala Val Leu Pro Leu Ser Val Trp Ala Gln Glu Asn Thr Gln Thr
               20                  25                  30
   Asp Ala Asn Ser Asp Ala Lys Asp Thr Lys Thr Pro Val Val Tyr Leu
           35                  40                  45
   Asp Ala Ile Thr Val Thr Ala Ala Pro Ser Ala Pro Val Ser Arg Phe
       50                  55                  60
   Asp Thr Asp Val Thr Gly Leu Gly Lys Thr Val Lys Thr Ala Asp Thr
   65                  70                  75                  80
   Leu Ala Lys Glu Gln Val Gln Gly Ile Arg Asp Leu Val Arg Tyr Glu
                   85                  90                  95
   Thr Gly Val Ser Val Val Glu Gln Gly Arg Gly Gly Ser Ser Gly Phe
               100                 105                 110
   Ala Ile His Gly Val Asp Lys Asn Arg Val Gly Ile Thr Val Asp Gly
           115                 120                 125
   Ile Ala Gln Ile Gln Ser Tyr Lys Asp Glu Ser Thr Lys Arg Ala Gly
       130                 135                 140
   Ala Gly Ser Gly Ala Met Asn Glu Ile Glu Ile Glu Asn Ile Ala Ala
   145                 150                 155                 160
   Val Ala Ile Asn Lys Gly Gly Asn Ala Leu Glu Ala Gly Ser Gly Ala
                   165                 170                 175
   Leu Gly Gly Ser Val Ala Phe His Thr Lys Asp Val Ser Asp Val Leu
               180                 185                 190
   Lys Ser Gly Lys Asn Leu Gly Ala Gln Ser Lys Thr Thr Tyr Asn Ser
           195                 200                 205
   Lys Asn Asp His Phe Ser Gln Thr Leu Ala Ala Ala Gly Lys Thr Glu
       210                 215                 220
   Arg Val Glu Ala Met Val Gln Tyr Thr Tyr Arg Lys Gly Lys Glu Asn
   225                 230                 235                 240
   Lys Ala His Ser Asp Leu Asn Gly Ile Asn Gln Ser Leu Tyr Arg Leu
                   245                 250                 255
   Gly Ala Trp Gln Gln Lys Tyr Asp Leu Arg Lys Pro Asn Glu Leu Phe
               260                 265                 270
   Ala Gly Thr Ser Tyr Ile Thr Glu Ser Cys Leu Ala Ser Asp Asp Pro
           275                 280                 285
   Lys Ser Cys Val Gln Tyr Pro Tyr Val Tyr Thr Lys Ala Arg Pro Asp
       290                 295                 300
   Gly Ile Gly Asn Arg Asn Phe Ser Glu Leu Ser Asp Ala Glu Lys Ala
   305 310 315 320
   Gln Tyr Leu Ala Ser Thr His Pro His Glu Val Val Ser Ala Lys Asp
   325 330 335
   Tyr Thr Gly Ile Tyr Arg Leu Leu Pro Asp Pro Met Asp Tyr Arg Ser
   340 345 350
   Asp Ser Tyr Leu Ala Arg Leu Asn Ile Lys Ile Thr Pro Asn Leu Val
   355 360 365
   Ser Lys Leu Leu Leu Glu Asp Thr Lys Gln Thr Tyr Asn Ile Arg Asp
   370 375 380
   Met Arg His Cys Ser Tyr His Gly Ala Arg Leu Gly Asn Asp Gly Lys
   385 390 395 400
   Pro Ala Asn Gly Gly Ser Ile Val Leu Cys Asp Asp Tyr Gln Glu Tyr
   405 410 415
   Leu Asn Ala Asn Asp Ala Ser Gln Ala Leu Phe Arg Pro Gly Ala Asn
   420 425 430
   Asp Ala Pro Ile Pro Lys Leu Ala Tyr Ala Arg Ser Ser Val Phe Asn
   435 440 445
   Gln Glu His Gly Lys Thr Arg Tyr Gly Leu Ser Phe Glu Phe Lys Pro
   450 455 460
   Asp Thr Pro Trp Phe Lys Gln Ala Lys Leu Asn Leu His Gln Gln Asn
   465 470 475 480
   Ile Gln Ile Ile Asn His Asp Ile Lys Lys Ser Cys Ser Gln Tyr Pro
   485 490 495
   Lys Val Asp Leu Asn Cys Gly Ile Ser Glu Ile Gly His Tyr Glu Tyr
   500 505 510
   Gln Asn Asn Tyr Arg Tyr Lys Glu Gly Arg Ala Ser Leu Thr Gly Lys
   515 520 525
   Leu Asp Phe Asn Phe Asp Leu Leu Gly Gln His Asp Leu Thr Val Leu
   530 535 540
   Ala Gly Ala Asp Lys Val Lys Ser Gln Phe Arg Ala Asn Asn Pro Arg
   545 550 555 560
   Arg Thr Ile Ile Asp Thr Thr Gln Gly Asp Ala Ile Ile Asp Glu Ser
   565 570 575
   Thr Leu Thr Ala Gln Glu Gln Ala Lys Phe Lys Gln Ser Gly Ala Ala
   580 585 590
   Trp Ile Val Lys Asn Arg Leu Gly Arg Leu Glu Glu Lys Asp Ala Cys
   595 600 605
   Gly Asn Ala Asn Glu Cys Glu Arg Ala Pro Ile His Gly Ser Asn Gln
   610 615 620
   Tyr Val Gly Ile Asn Asn Leu Tyr Thr Pro Asn Asp Tyr Val Asp Leu
   625 630 635 640
   Ser Phe Gly Gly Arg Leu Asp Lys Gln Arg Ile His Ser Thr Asp Ser
   645 650 655
   Asn Ile Ile Ser Lys Thr Tyr Thr Asn Lys Ser Tyr Asn Phe Gly Ala
   660 665 670
   Ala Val His Leu Thr Pro Asp Phe Ser Leu Leu Tyr Lys Thr Ala Lys
   675 680 685
   Gly Phe Arg Thr Pro Ser Phe Tyr Glu Leu Tyr Asn Tyr Asn Ser Thr
   690 695 700
   Ala Ala Gln His Lys Asn Asp Pro Asp Val Ser Phe Pro Lys Arg Ala
   705 710 715 720
   Val Asp Val Lys Pro Glu Thr Ser Asn Thr Asn Glu Tyr Gly Phe Arg
   725 730 735
   Tyr Gln His Pro Trp Gly Asp Val Glu Met Ser Met Phe Lys Ser Arg
   740 745 750
   Tyr Lys Asp Met Leu Asp Lys Ala Ile Pro Asn Leu Thr Lys Ala Gln
   755 760 765
   Gln Glu Tyr Cys Lys Ala His Leu Asp Ser Asn Glu Cys Val Gly Asn
   770 775 780
   Pro Pro Thr Pro Lys Thr Ser Asp Glu Val Phe Ala Asn Leu Tyr Asn
   785 790 795 800
   Ala Thr Ile Lys Gly Val Ser Val Lys Gly Lys Leu Asp Leu His Ala
   805 810 815
   Met Thr Ser Lys Leu Pro Asp Gly Leu Glu Met Thr Leu Gly Tyr Gly
   820 825 830
   His Thr Lys Leu Gly Lys Phe Asp Tyr Ile Ala Pro Lys Asp Ala Asp
   835 840 845
   Gly Trp Tyr Gln Ala Arg Pro Ala Phe Trp Asp Ala Ile Thr Pro Ala
   850 855 860
   Arg Tyr Val Val Gly Leu Asn Tyr Asp His Pro Ser Gln Val Trp Gly
   865 870 875 880
   Ile Gly Thr Thr Leu Thr His Ser Lys Gln Lys Asp Glu Asn Glu Leu
   885 890 895
   Ser Ala Leu Arg Ile Arg Asn Gly Lys Arg Glu Thr Gln Thr Leu Thr
   900 905 910
   His Thr Ile Pro Lys Ala Tyr Thr Leu Leu Asp Met Thr Gly Tyr Tyr
   915 920 925
   Ser Pro Thr Glu Ser Ile Thr Ala Arg Leu Gly Ile Asn Asn Val Leu
   930 935 940
   Asn Thr Arg Tyr Thr Thr Trp Glu Ala Ala Arg Gln Leu Pro Ser Glu
   945 950 955 960
   Ala Ala Ser Ser Thr Gln Ser Thr Arg Tyr Ile Ala Pro Gly Arg Ser
   965 970 975
   Tyr Phe Ala Ser Leu Glu Met Lys Phe
   980 985
(2) INFORMATION FOR SEQ ID NO:14:
   (i) SEQUENCE CHARACTERISTICS:
      (A) LENGTH: 541 amino acids
      (B) TYPE: amino acid
      (C) STRANDEDNESS: single
      (D) TOPOLOGY: linear
   (xi) SEQUENCE DESCRIPTION: SEQ ID NO:14:
   Met Thr Cys Leu Pro Lys Thr Asn Pro Ala Leu Lys Val Lys His Arg
   1               5                   10                  15
   Phe Leu Lys Gln Val Leu Leu Leu Leu Cys Val Asp Thr Leu Thr Ala
               20                  25                  30
   Gln Ala Tyr Ala His Ser His His Thr Pro Ile His Thr Pro Thr His
           35                  40                  45
   Glu Leu Pro Ser Ala Asp Ala Leu Ser Asp Glu Gly Leu Gly Lys Asp
       50                  55                  60
   Leu Gly Ser Leu Asp Ser Leu Asp Ser Pro Asp Gly Leu Gly Asp Gly
   65                  70                  75                  80
   Leu Gly Asp Gly Leu Gly Asp Gly Leu Lys Ser Asp Lys Ala Pro Leu
                   85                  90                  95
   Pro Ile Asn Ala Leu Thr Ala His Gln Thr Asn Glu Ser Gln Pro Ala
               100                 105                 110
   Pro Pro Ser Val Asp Val Asn Phe Leu Leu Ala Gln Pro Glu Ala Phe
           115                 120                 125
   Tyr His Val Phe His Gln Ala Ile Val Gln Asp Asp Val Ala Thr Leu
       130                 135                 140
   Arg Leu Leu Leu Pro Phe Tyr Asp Arg Leu Pro Asp Asp Tyr Gln Asp
   145                 150                 155                 160
   Asp Val Leu Leu Leu Phe Ala Gln Ser Lys Leu Ala Leu Ser Asp Gly
                   165                 170                 175
   Asn Thr Lys Leu Ala Leu Asn Leu Leu Thr Asp Leu Ser Asn Lys Glu
               180                 185                 190
   Pro Thr Leu Thr Ala Val Lys Leu Gln Leu Ala Ser Leu Leu Leu Thr
           195                 200                 205
   Asn Lys His Asp Lys His Ala Gln Met Val Leu Asp Glu Leu Lys Asp
       210                 215                 220
   Asp Ala His Phe Leu Lys Leu Ser Lys Lys Glu Gln Arg Trp Val Leu
   225                 230                 235                 240
   Ser Gln Ser Arg Tyr Leu His Lys Lys Tyr Lys Met Gly Leu Asp Leu
                   245                 250                 255
   Gly Ile Asn Tyr Leu His Leu Asp Asn Ile Asn Ala Ala Ser Thr Ile
               260                 265                 270
   Thr Gln Pro Asn Ile Lys Lys Asp Ala Pro Lys Pro Ala His Gly Leu
           275                 280                 285
   Ala Leu Ser Leu Gly Val Asn Lys Tyr Thr Pro Leu Ser His Gly Met
       290                 295                 300
   Ser Ile Tyr Thr Ala Leu Asp Val Asp Gly Lys Phe Tyr Asp Asp Lys
   305                 310                 315                 320
   Ser His Asn Glu Leu Ala Val Phe Ala His Ala Gly Leu Arg Lys Asp
                   325                 330                 335
   His Gln Lys Gly Tyr Val Asp Val Val Pro Phe Val Gly Arg Ile Phe
               340                 345                 350
   Ala Thr Asn Gln Gln His Gly Arg Leu Ser Pro Arg Lys Asp Ser Gln
           355                 360                 365
   Gly Val Ala Phe Gly Ser His His Arg Ile Asn Asp Lys Trp Gln Asn
       370                 375                 380
   Ala Phe Phe Ala Arg Met Glu Lys Gly Asn Tyr Thr Glu Arg Tyr Gln
   385                 390                 395                 400
   Gly Tyr Asp Gly Lys Arg Tyr His Val Asn Asp Thr Ile Leu Leu Gln
                   405                 410                 415
   Asp Gly Pro Asn Arg Arg Tyr Ser Leu Gly Val Gly Tyr Gln Leu Ser
               420                 425                 430
   His Leu Gln Asp Ala Thr Lys Ser Ser His Ala Thr Lys Ile His Phe
           435                 440                 445
   Gly Val Leu Gln Arg Leu Pro Asn Gly Leu Thr Val Gln Gly Arg Val
       450                 455                 460
   Ser Ala Glu Arg Glu Arg Tyr His Gly Lys Leu Leu Arg Leu Val Asn
   465                 470                 475                 480
   Pro Asp Asp Val Tyr Arg Thr Asp Lys Thr Leu Thr Leu Gln Thr Ser
                   485                 490                 495
   Ile Trp His Lys Asp Ile His Trp Leu Gly Leu Thr Pro Lys Leu Thr
               500                 505                 510
   Tyr Arg Tyr Ser Lys Asn Asn Ser Asn Leu Pro Ala Leu Tyr Ser His
           515                 520                 525
   Asn Lys Gln Asn Phe Tyr Leu Glu Leu Gly Arg Ser Phe
       530                 535                 540
(2) INFORMATION FOR SEQ ID NO:15:
   (i) SEQUENCE CHARACTERISTICS:
      (A) LENGTH: 894 amino acids
      (B) TYPE: amino acid
      (C) STRANDEDNESS: single
      (D) TOPOLOGY: linear
   (xi) SEQUENCE DESCRIPTION: SEQ ID NO:15:
   Met Ser Thr Val Lys Thr Pro His Ile Phe Tyr Gln Lys Arg Thr Leu
   1               5                   10                  15
   Ser Leu Ala Ile Ala Ser Ile Phe Ala Ala Leu Val Met Thr Gly Cys
               20                  25                  30
   Arg Ser Asp Asp Ile Ser Val Asn Ala Pro Asn Val Thr Gln Leu Pro
           35                  40                  45
   Gln Gly Thr Val Ser Pro Thr Pro Asn Thr Gly His Asp Asn Ala Asn
       50                  55                  60
   Asn Thr Asn Asn Gln Gly Asn Asn Thr Asp Asn Ser Thr Ser Thr Thr
   65                  70                  75                  80
   Asp Pro Asn Gly Asp Asn Asn Gln Leu Thr Gln Ala Gln Lys Thr Ala
                   85                  90                  95
   Ala Ala Ala Gly Phe Phe Val Met Gly Lys Ile Arg Asp Thr Ser Glu
               100                 105                 110
   Lys Asn Asp Pro Asp Tyr Ser Asp Asp Leu Lys Gln Gln Trp Leu Gly
           115                 120                 125
   Lys Leu Tyr Val Gly Ile Asp Ala His Arg Pro Asp Gly Ile Gly Lys
       130                 135                 140
   Gly Lys Asn Leu Arg Gln Pro Ile Thr Ala Asn Asp Ile Lys Pro Leu
   145                 150                 155                 160
   Tyr Phe Asn Lys Phe Pro Ala Leu Ser Asp Leu His Leu Asp Ser Glu
                   165                 170                 175
   Arg His Arg Phe Asp Pro Gln Lys Ile Asn Thr Ile Lys Val Tyr Gly
               180                 185                 190
   Tyr Gly Asn Leu Thr Thr Pro Ser Asn Asn Asn Thr His Ile Asn His
           195                 200                 205
   Gln Gln Ala Asp Asn Lys Lys Asn Asn Lys Pro Val Asp Pro Tyr Glu
       210                 215                 220
   Asn Ile Arg Phe Gly Tyr Leu Glu Leu Gln Gly Ser Ser Leu Thr Gln
   225                 230                 235                 240
   Lys Asn Ala Asp Asn Gln Asn Glu Gln Asp Arg Ile Pro Lys Pro Met
                   245                 250                 255
   Pro Ile Leu Phe Tyr His Gly Glu Asn Ala Ser Ser Gln Leu Pro Ser
               260                 265                 270
   Ala Gly Lys Phe Asn Tyr Thr Gly Asn Trp Leu Tyr Leu Ser Asp Val
           275                 280                 285
   Lys Lys Arg Pro Ala Leu Ser Ala Ser Asp Glu Arg Val Gly Val Tyr
       290                 295                 300
   Leu Asn Ala Ser Gly Lys Ala Asn Glu Gly Asp Val Val Ser Ala Ala
   305                 310                 315                 320
   His Ile Tyr Leu Asn Gly Phe Gln Tyr Lys His Thr Pro Ala Thr Tyr
                   325                 330                 335
   Gln Val Asp Phe Asp Thr Asn Ser Leu Thr Gly Lys Leu Ser Tyr Tyr
               340                 345                 350
   Asp Asn Pro Asn Gln Gln Asn Asn Lys Gly Glu Tyr Leu Lys Ser Gln
           355                 360                 365
   Phe Asp Thr Thr Lys Lys Val Asn Glu Thr Asp Val Tyr Gln Ile Asp
       370                 375                 380
   Ala Lys Ile Asn Gly Asn Arg Phe Val Gly Thr Ala Lys Ser Leu Val
   385                 390                 395                 400
   Asn Glu Lys Thr Gln Thr Ala Pro Phe Ile Lys Glu Leu Phe Ser Lys
                   405                 410                 415
   Lys Ala Asn Pro Asn Asn Pro Asn Pro Asn Ser Asp Thr Leu Glu Gly
               420                 425                 430
   Gly Phe Tyr Gly Glu Ser Gly Asp Glu Leu Ala Gly Lys Phe Leu Ser
           435                 440                 445
   Asn Asp Asn Ala Ser Tyr Val Val Phe Gly Gly Lys Arg Asp Lys Thr
       450                 455                 460
   Thr Lys Pro Val Ala Thr Lys Thr Val Tyr Phe Ser Ala Gly Phe Glu
   465                 470                 475                 480
   Lys Pro Ser Thr Ser Phe Val Asp Asn Glu Thr Ile Gly Gly Ile Ile
                   485                 490                 495
   Asp Arg Lys Gly Leu Asn Asn His Ile Asn Glu Asp Glu Ile Ile Pro
               500                 505                 510
   Ser Asp Asp Ser Tyr Tyr Gly Tyr Thr Trp Gly Lys Pro Glu Lys Gln
           515                 520                 525
   Phe Thr Lys Lys Val Ser Ser Ser Thr Gln Val Val Pro Ala Tyr Phe
       530                 535                 540
   Gly Gln His Asp Lys Phe Tyr Phe Asn Gly Asn Tyr Tyr Asp Leu Ser
   545                 550                 555                 560
   Ala Ser Arg Val Asp Lys Leu Ala Pro Ala Asp Ala Val Lys Ala Asn
                   565                 570                 575
   Gln Ser Ile Lys Glu Lys Tyr Pro Asn Ala Thr Leu Asn Lys Asp Asn
               580                 585                 590
   Gln Val Thr Ala Ile Val Leu Gln Glu Ala Lys Asp Asn Lys Pro Tyr
           595                 600                 605
   Thr Ala Ile Arg Ala Lys Ser Tyr Gln His Ile Ser Phe Gly Glu Thr
       610                 615                 620
   Leu Tyr Asn Asp Ala Asn Gln Thr Pro Thr Arg Ser Tyr Phe Val Gln
   625                 630                 635                 640
   Gly Gly Arg Ala Asp Thr Ser Thr Thr Leu Pro Gln Ala Gly Lys Phe
                   645                 650                 655
   Thr Tyr Asn Gly Leu Trp Ala Gly Tyr Leu Thr Gln Lys Lys Asp Lys
               660                 665                 670
   Gly Tyr Ser Asp Asn Ala Glu Thr Ile Lys Glu Lys Gly His Pro Gly
           675                 680                 685
   Tyr Leu Leu Thr Glu Asn Phe Thr Pro Glu Asp Asp Asp Asp Asp Leu
       690                 695                 700
   Thr Ala Ser Asp Asp Ser Gln Asp Asp Asn Thr His Gly Asp Asp Asp
   705                 710                 715                 720
   Leu Ile Ala Ser Asp Asp Ser Gln Asp Asp Asp Ala Asp Gly Asp Asp
                   725                 730                 735
   Asp Ser Asp Asp Leu Gly Asp Gly Ala Asp Asp Asp Ala Ala Gly Lys
               740                 745                 750
   Val Tyr His Ala Gly Asn Ile Arg Pro Glu Phe Glu Asn Lys Tyr Leu
           755                 760                 765
   Pro Ile Asn Glu Pro Thr His Glu Lys Thr Phe Ala Leu Asp Gly Lys
       770                 775                 780
   Asn Lys Ala Lys Phe Glu Val Asp Phe Asn Thr Asn Ser Leu Thr Gly
   785                 790                 795                 800
   Lys Leu Asn Asp Glu Arg Gly Asp Ile Val Phe Asp Ile Lys Asn Gly
                   805                 810                 815
   Lys Ile Asp Gly Thr Gly Phe Thr Ala Lys Ala Asp Val Pro Asn Tyr
               820                 825                 830
   Arg Glu Glu Val Gly Asn Asn Gln Gly Gly Gly Phe Leu Tyr Asn Ile
           835                 840                 845
   Lys Asp Ile Asp Val Lys Gly Gln Phe Phe Gly Thr Asn Gly Glu Glu
       850                 855                 860
   Leu Ala Gly Gln Leu His His Asp Lys Gly Asp Gly Ile Asn Asp Thr
   865                 870                 875                 880
   Ala Glu Lys Ala Gly Ala Val Phe Gly Ala Val Lys Asp Lys
                   885                 890
(2) INFORMATION FOR SEQ ID NO:16:
   (i) SEQUENCE CHARACTERISTICS:
      (A) LENGTH: 1000 amino acids
      (B) TYPE: amino acid
      (C) STRANDEDNESS: single
      (D) TOPOLOGY: linear
   (xi) SEQUENCE DESCRIPTION: SEQ ID NO:16:
   Met Ser Lys Ser Ile Thr Lys Thr Gln Thr Pro Ser Val His Thr Met
   1               5                   10                  15
   Thr Thr His Arg Leu Asn Leu Ala Ile Lys Ala Ala Leu Phe Gly Val
               20                  25                  30
   Ala Val Leu Pro Leu Ser Val Trp Ala Gln Glu Asn Thr Gln Thr Asp
   35 40 45
   Ala Asn Ser Asp Ala Lys Asp Thr Lys Thr Pro Val Val Tyr Leu Asp
   50 55 60
   Ala Ile Thr Val Thr Ala Ala Pro Ser Ala Pro Val Ser Arg Phe Asp
   65 70 75 80
   Thr Asp Val Thr Gly Leu Gly Lys Thr Val Lys Thr Ala Asp Thr Leu
   85 90 95
   Ala Lys Glu Gln Val Gln Gly Ile Arg Asp Leu Val Arg Tyr Glu Thr
   100 105 110
   Gly Val Ser Val Val Glu Gln Gly Arg Gly Gly Ser Ser Gly Phe Ala
   115 120 125
   Ile His Gly Val Asp Lys Asn Arg Val Gly Ile Thr Val Asp Gly Ile
   130 135 140
   Ala Gln Ile Gln Ser Tyr Lys Asp Glu Ser Thr Lys Arg Ala Gly Ala
   145 150 155 160
   Gly Ser Gly Ala Met Asn Glu Ile Glu Ile Glu Asn Ile Ala Ala Val
   165 170 175
   Ala Ile Asn Lys Gly Gly Asn Ala Leu Glu Ala Gly Ser Gly Ala Leu
   180 185 190
   Gly Gly Ser Val Ala Phe His Thr Lys Asp Val Ser Asp Val Leu Lys
   195 200 205
   Ser Gly Asn Asn Leu Gly Ala Gln Ser Lys Thr Thr Tyr Asn Ser Lys
   210 215 220
   Asn Asp His Phe Ser Gln Thr Leu Ala Ala Ala Gly Lys Thr Glu Arg
   225 230 235 240
   Val Glu Ala Met Val Gln Tyr Thr Tyr Arg Lys Gly Lys Glu Asn Lys
   245 250 255
   Ala His Ser Asp Leu Asn Gly Ile Asn Gln Ser Leu Tyr Arg Leu Gly
   260 265 270
   Ala Trp Gln Gln Lys Tyr Asp Leu Arg Lys Pro Asn Glu Leu Phe Ala
   275 280 285
   Gly Thr Ser Tyr Ile Thr Glu Ser Cys Leu Ala Ser Asp Asp Pro Lys
   290 295 300
   Ser Cys Val Gln Tyr Pro Tyr Val Tyr Thr Lys Ala Arg Pro Asp Gly
   305 310 315 320
   Ile Gly Asn Arg Asn Phe Ser Glu Leu Ser Asp Ala Glu Lys Ala Gln
   325 330 335
   Tyr Leu Ala Ser Thr His Pro His Glu Val Val Ser Ala Lys Asp Tyr
   340 345 350
   Thr Gly Thr Tyr Arg Leu Leu Pro Asp Pro Met Asp Tyr Arg Ser Asp
   355 360 365
   Ser Tyr Leu Ala Arg Leu Asn Ile Lys Ile Thr Pro Asn Leu Val Ser
       370                 375                 380
   Lys Leu Leu Leu Glu Asp Thr Lys Gln Thr Tyr Asn Ile Arg Asp Met
   385                 390                 395                 400
   Arg His Cys Ser Tyr His Gly Ala Arg Leu Gly Asn Asp Gly Lys Pro
                   405                 410                 415
   Ala Asn Gly Gly Ser Ile Val Leu Cys Asp Asp Tyr Gln Glu Tyr Leu
               420                 425                 430
   Asn Ala Asn Asp Ala Ser Gln Ala Ser Phe Arg Pro Gly Ala Asn Asp
           435                 440                 445
   Ala Pro Ile Pro Lys Leu Ala Tyr Ala Arg Ser Ser Val Phe Asn Gln
       450                 455                 460
   Glu His Gly Lys Thr Arg Tyr Gly Leu Gly Phe Glu Phe Lys Pro Asp
   465                 470                 475                 480
   Thr Pro Trp Phe Lys Gln Ala Lys Leu Asn Leu His Gln Gln Asn Ile
                   485                 490                 495
   Gln Ile Ile Asn His Asp Ile Lys Lys Ser Cys Ser Gln Tyr Pro Lys
               500                 505                 510
   Val Asp Leu Asn Cys Gly Ile Ser Glu Ile Gly His Tyr Glu Tyr Gln
           515                 520                 525
   Asn Asn Tyr Arg Tyr Lys Glu Gly Arg Thr Ser Leu Thr Gly Lys Leu
       530                 535                 540
   Asp Phe Asn Phe Asp Leu Leu Gly Gln His Asp Leu Thr Val Leu Ala
   545                 550                 555                 560
   Gly Ala Asp Lys Val Lys Ser Gln Phe Arg Ala Asn Asn Pro Arg Arg
                   565                 570                 575
   Thr Ile Ile Asp Thr Thr Gln Gly Asp Ala Ile Ile Asp Glu Ser Thr
               580                 585                 590
   Leu Thr Ala Gln Glu Gln Ala Lys Phe Lys Gln Ser Gly Ala Ala Trp
           595                 600                 605
   Ile Val Lys Asn Arg Leu Gly Arg Leu Glu Glu Lys Asp Ala Cys Gly
       610                 615                 620
   Asn Ala Asn Glu Cys Glu Arg Ala Pro Ile His Gly Ser Asn Gln Tyr
   625                 630                 635                 640
   Val Gly Ile Asn Asn Leu Tyr Thr Pro Asn Asp Tyr Val Asp Leu Ser
                   645                 650                 655
   Phe Gly Gly Arg Leu Asp Lys Gln Arg Ile His Ser Thr Asp Ser Asn
               660                 665                 670
   Ile Ile Ser Lys Thr Tyr Thr Asn Lys Ser Tyr Asn Phe Gly Ala Ala
           675                 680                 685
   Val His Leu Thr Pro Asp Phe Ser Leu Leu Tyr Lys Thr Ala Lys Gly
       690                 695                 700
   Phe Arg Thr Pro Ser Phe Tyr Glu Leu Tyr Asn Tyr Asn Ser Thr Ala
   705                 710                 715                 720
   Ala Gln His Lys Asn Asp Pro Asp Val Ser Phe Pro Lys Arg Ala Val
                   725                 730                 735
   Asp Val Lys Pro Glu Thr Ser Asn Thr Asn Glu Tyr Gly Phe Arg Tyr
               740                 745                 750
   Gln His Pro Trp Gly Asp Ile Glu Met Ser Met Phe Lys Ser Arg Tyr
           755                 760                 765
   Lys Asp Met Leu Asp Lys Ala Ile Pro Asn Leu Thr Lys Ala Gln Gln
       770                 775                 780
   Glu Tyr Cys Lys Ala His Leu Asp Ser Asn Glu Cys Val Gly Asn Pro
   785                 790                 795                 800
   Pro Thr Pro Lys Thr Ser Asp Glu Val Phe Ala Asn Leu Tyr Asn Ala
                   805                 810                 815
   Thr Ile Lys Gly Val Ser Val Lys Gly Lys Leu Asp Leu His Ala Met
               820                 825                 830
   Thr Ser Lys Leu Pro Asp Gly Leu Glu Met Thr Leu Gly Tyr Gly His
           835                 840                 845
   Thr Lys Leu Gly Lys Phe Asp Tyr Ile Ala Pro Lys Asp Ala Asp Gly
       850                 855                 860
   Trp Tyr Gln Ala Arg Pro Ala Phe Trp Asp Ala Ile Thr Pro Ala Arg
   865                 870                 875                 880
   Tyr Val Val Gly Leu Asn Tyr Asp His Pro Ser Gln Val Trp Gly Ile
                   885                 890                 895
   Gly Thr Thr Leu Thr His Ser Lys Gln Lys Asp Glu Asn Glu Leu Ser
               900                 905                 910
   Ala Leu Arg Ile Arg Asn Gly Lys Arg Glu Ile Gln Thr Leu Thr His
           915                 920                 925
   Thr Ile Pro Lys Ala Tyr Thr Leu Leu Asp Met Thr Gly Tyr Tyr Ser
       930                 935                 940
   Pro Thr Glu Ser Ile Thr Ala Arg Leu Gly Ile Asn Asn Val Leu Asn
   945                 950                 955                 960
   Thr Arg Tyr Thr Thr Trp Glu Ala Ala Arg Gln Leu Pro Ser Glu Ala
                   965                 970                 975
   Ala Ser Ser Thr Gln Ser Thr Arg Tyr Ile Ala Pro Gly Arg Ser Tyr
               980                 985                 990
   Phe Ala Ser Leu Glu Met Lys Phe
           995                 1000
(2) INFORMATION FOR SEQ ID NO:17:
   (i) SEQUENCE CHARACTERISTICS:
      (A) LENGTH: 985 amino acids
      (B) TYPE: amino acid
      (C) STRANDEDNESS: single
      (D) TOPOLOGY: linear
   (xi) SEQUENCE DESCRIPTION: SEQ ID NO:17:
   Met Thr Thr His Arg Leu Asn Leu Ala Ile Lys Ala Ala Leu Phe Gly
   1               5                   10                  15
   Val Ala Val Leu Pro Leu Ser Val Trp Ala Gln Glu Asn Thr Gln Thr
               20                  25                  30
   Asp Ala Asn Ser Asp Ala Lys Asp Thr Lys Thr Pro Val Val Tyr Leu
           35                  40                  45
   Asp Ala Ile Thr Val Thr Ala Ala Pro Ser Ala Pro Val Ser Arg Phe
       50                  55                  60
   Asp Thr Asp Val Thr Gly Leu Gly Lys Thr Val Lys Thr Ala Asp Thr
   65                  70                  75                  80
   Leu Ala Lys Glu Gln Val Gln Gly Ile Arg Asp Leu Val Arg Tyr Glu
                   85                  90                  95
   Thr Gly Val Ser Val Val Glu Gln Gly Arg Gly Gly Ser Ser Gly Phe
               100                 105                 110
   Ala Ile His Gly Val Asp Lys Asn Arg Val Gly Ile Thr Val Asp Gly
           115                 120                 125
   Ile Ala Gln Ile Gln Ser Tyr Lys Asp Glu Ser Thr Lys Arg Ala Gly
       130                 135                 140
   Ala Gly Ser Gly Ala Met Asn Glu Ile Glu Ile Glu Asn Ile Ala Ala
   145                 150                 155                 160
   Val Ala Ile Asn Lys Gly Gly Asn Ala Leu Glu Ala Gly Ser Gly Ala
                   165                 170                 175
   Leu Gly Gly Ser Val Ala Phe His Thr Lys Asp Val Ser Asp Val Leu
               180                 185                 190
   Lys Ser Gly Asn Asn Leu Gly Ala Gln Ser Lys Thr Thr Tyr Asn Ser
           195                 200                 205
   Lys Asn Asp His Phe Ser Gln Thr Leu Ala Ala Ala Gly Lys Thr Glu
       210                 215                 220
   Arg Val Glu Ala Met Val Gln Tyr Thr Tyr Arg Lys Gly Lys Glu Asn
   225                 230                 235                 240
   Lys Ala His Ser Asp Leu Asn Gly Ile Asn Gln Ser Leu Tyr Arg Leu
                   245                 250                 255
   Gly Ala Trp Gln Gln Lys Tyr Asp Leu Arg Lys Pro Asn Glu Leu Phe
               260                 265                 270
   Ala Gly Thr Ser Tyr Ile Thr Glu Ser Cys Leu Ala Ser Asp Asp Pro
           275                 280                 285
   Lys Ser Cys Val Gln Tyr Pro Tyr Val Tyr Thr Lys Ala Arg Pro Asp
       290                 295                 300
   Gly Ile Gly Asn Arg Asn Phe Ser Glu Leu Ser Asp Ala Glu Lys Ala
   305 310 315 320
   Gln Tyr Leu Ala Ser Thr His Pro His Glu Val Val Ser Ala Lys Asp
   325 330 335
   Tyr Thr Gly Thr Tyr Arg Leu Leu Pro Asp Pro Met Asp Tyr Arg Ser
   340 345 350
   Asp Ser Tyr Leu Ala Arg Leu Asn Ile Lys Ile Thr Pro Asn Leu Val
   355 360 365
   Ser Lys Leu Leu Leu Glu Asp Thr Lys Gln Thr Tyr Asn Ile Arg Asp
   370 375 380
   Met Arg His Cys Ser Tyr His Gly Ala Arg Leu Gly Asn Asp Gly Lys
   385 390 395 400
   Pro Ala Asn Gly Gly Ser Ile Val Leu Cys Asp Asp Tyr Gln Glu Tyr
   405 410 415
   Leu Asn Ala Asn Asp Ala Ser Gln Ala Ser Phe Arg Pro Gly Ala Asn
   420 425 430
   Asp Ala Pro Ile Pro Lys Leu Ala Tyr Ala Arg Ser Ser Val Phe Asn
   435 440 445
   Gln Glu His Gly Lys Thr Arg Tyr Gly Leu Gly Phe Glu Phe Lys Pro
   450 455 460
   Asp Thr Pro Trp Phe Lys Gln Ala Lys Leu Asn Leu His Gln Gln Asn
   465 470 475 480
   Ile Gln Ile Ile Asn His Asp Ile Lys Lys Ser Cys Ser Gln Tyr Pro
   485 490 495
   Lys Val Asp Leu Asn Cys Gly Ile Ser Glu Ile Gly His Tyr Glu Tyr
   500 505 510
   Gln Asn Asn Tyr Arg Tyr Lys Glu Gly Arg Thr Ser Leu Thr Gly Lys
   515 520 525
   Leu Asp Phe Asn Phe Asp Leu Leu Gly Gln His Asp Leu Thr Val Leu
   530 535 540
   Ala Gly Ala Asp Lys Val Lys Ser Gln Phe Arg Ala Asn Asn Pro Arg
   545 550 555 560
   Arg Thr Ile Ile Asp Thr Thr Gln Gly Asp Ala Ile Ile Asp Glu Ser
   565 570 575
   Thr Leu Thr Ala Gln Glu Gln Ala Lys Phe Lys Gln Ser Gly Ala Ala
   580 585 590
   Trp Ile Val Lys Asn Arg Leu Gly Arg Leu Glu Glu Lys Asp Ala Cys
   595 600 605
   Gly Asn Ala Asn Glu Cys Glu Arg Ala Pro Ile His Gly Ser Asn Gln
   610 615 620
   Tyr Val Gly Ile Asn Asn Leu Tyr Thr Pro Asn Asp Tyr Val Asp Leu
   625 630 635 640
   Ser Phe Gly Gly Arg Leu Asp Lys Gln Arg Ile His Ser Thr Asp Ser
   645 650 655
   Asn Ile Ile Ser Lys Thr Tyr Thr Asn Lys Ser Tyr Asn Phe Gly Ala
   660 665 670
   Ala Val His Leu Thr Pro Asp Phe Ser Leu Leu Tyr Lys Thr Ala Lys
   675 680 685
   Gly Phe Arg Thr Pro Ser Phe Tyr Glu Leu Tyr Asn Tyr Asn Ser Thr
   690 695 700
   Ala Ala Gln His Lys Asn Asp Pro Asp Val Ser Phe Pro Lys Arg Ala
   705 710 715 720
   Val Asp Val Lys Pro Glu Thr Ser Asn Thr Asn Glu Tyr Gly Phe Arg
   725 730 735
   Tyr Gln His Pro Trp Gly Asp Ile Glu Met Ser Met Phe Lys Ser Arg
   740 745 750
   Tyr Lys Asp Met Leu Asp Lys Ala Ile Pro Asn Leu Thr Lys Ala Gln
   755 760 765
   Gln Glu Tyr Cys Lys Ala His Leu Asp Ser Asn Glu Cys Val Gly Asn
   770 775 780
   Pro Pro Thr Pro Lys Thr Ser Asp Glu Val Phe Ala Asn Leu Tyr Asn
   785 790 795 800
   Ala Thr Ile Lys Gly Val Ser Val Lys Gly Lys Leu Asp Leu His Ala
   805 810 815
   Met Thr Ser Lys Leu Pro Asp Gly Leu Glu Met Thr Leu Gly Tyr Gly
   820 825 830
   His Thr Lys Leu Gly Lys Phe Asp Tyr Ile Ala Pro Lys Asp Ala Asp
   835 840 845
   Gly Trp Tyr Gln Ala Arg Pro Ala Phe Trp Asp Ala Ile Thr Pro Ala
   850 855 860
   Arg Tyr Val Val Gly Leu Asn Tyr Asp His Pro Ser Gln Val Trp Gly
   865 870 875 880
   Ile Gly Thr Thr Leu Thr His Ser Lys Gln Lys Asp Glu Asn Glu Leu
   885 890 895
   Ser Ala Leu Arg Ile Arg Asn Gly Lys Arg Glu Ile Gln Thr Leu Thr
   900 905 910
   His Thr Ile Pro Lys Ala Tyr Thr Leu Leu Asp Met Thr Gly Tyr Tyr
   915 920 925
   Ser Pro Thr Glu Ser Ile Thr Ala Arg Leu Gly Ile Asn Asn Val Leu
   930 935 940
   Asn Thr Arg Tyr Thr Thr Trp Glu Ala Ala Arg Gln Leu Pro Ser Glu
   945 950 955 960
   Ala Ala Ser Ser Thr Gln Ser Thr Arg Tyr Ile Ala Pro Gly Arg Ser
   965 970 975
   Tyr Phe Ala Ser Leu Glu Met Lys Phe
               980                 985
(2) INFORMATION FOR SEQ ID NO:18:
   (i) SEQUENCE CHARACTERISTICS:
      (A) LENGTH: 538 amino acids
      (B) TYPE: amino acid
      (C) STRANDEDNESS: single
      (D) TOPOLOGY: linear
   (xi) SEQUENCE DESCRIPTION: SEQ ID NO:18:
   Met Thr Cys Leu Pro Lys Thr Asn Pro Ala Leu Lys Val Lys His Arg
   1               5                   10                  15
   Phe Leu Lys Gln Val Leu Leu Leu Leu Cys Val Asp Thr Leu Thr Ala
               20                  25                  30
   Gln Ala Tyr Ala His Ser His His Thr Pro Ile His Thr Pro Thr His
           35                  40                  45
   Glu Leu Ser Ser Ala Asp Ala Leu Ser Asp Glu Gly Leu Gly Lys Asp
       50                  55                  60
   Leu Gly Ser Leu Asp Ser Pro Asp Gly Leu Gly Asp Gly Leu Gly Asp
   65                  70                  75                  80
   Gly Leu Gly Asp Gly Leu Lys Ser Asp Lys Thr Pro Leu Pro Ile Asn
                   85                  90                  95
   Ala Leu Thr Val Asn Gln Ser Asn Glu Ser Gln Pro Ala Pro Pro Ser
               100                 105                 110
   Val Asp Val Asn Phe Leu Leu Ala Gln Pro Glu Ala Phe Tyr His Val
           115                 120                 125
   Phe His Gln Ala Ile Val Gln Asp Asp Val Ala Thr Leu Arg Leu Leu
       130                 135                 140
   Leu Pro Phe Tyr Asp Arg Leu Pro Asp Asp Tyr Gln Asp Asp Val Leu
   145                 150                 155                 160
   Leu Leu Phe Ala Gln Ser Lys Leu Ala Leu Ser Asp Gly Asn Thr Lys
                   165                 170                 175
   Leu Ala Leu Asn Leu Leu Thr Asp Leu Ser Asn Lys Glu Pro Thr Leu
               180                 185                 190
   Thr Ala Val Lys Leu Gln Leu Ala Ser Leu Leu Leu Thr Asn Lys His
           195                 200                 205
   Asp Lys His Ala Gln Met Val Leu Asp Glu Leu Lys Asp Asp Ala His
       210                 215                 220
   Phe Leu Lys Leu Ser Lys Lys Glu Gln Arg Trp Val Leu Ser Gln Ser
   225                 230                 235                 240
   Arg Tyr Leu His Lys Lys Tyr Lys Met Gly Leu Asp Leu Gly Ile Asn
                   245                 250                 255
   Tyr Leu His Leu Asp Asn Ile Asn Ala Ala Ser Thr Ile Thr Gln Pro
   260 265 270
   Asn Ile Lys Lys Asp Ala Pro Lys Pro Ala His Gly Leu Ala Leu Ser
   275 280 285
   Leu Gly Val Asn Lys Tyr Thr Pro Leu Ser His Gly Met Ser Ile Tyr
   290 295 300
   Thr Ala Leu Asp Val Asp Gly Lys Phe Tyr Asp Asp Lys Ser His Asn
   305 310 315 320
   Glu Leu Ala Val Phe Ala His Ala Gly Leu Arg Lys Asp His Gln Lys
   325 330 335
   Gly Tyr Val Asp Val Val Pro Phe Val Gly Arg Ile Phe Ala Thr Asn
   340 345 350
   Gln Gln His Gly Arg Leu Ser Pro Arg Lys Asp Ser Gln Gly Val Ala
   355 360 365
   Phe Gly Ser His His Arg Ile Asn Asp Lys Trp Gln Asn Ala Phe Phe
   370 375 380
   Ala Arg Met Glu Lys Gly Asn Tyr Thr Glu His Tyr Gln Gly Tyr Asp
   385 390 395 400
   Gly Lys Arg Tyr His Val Asn Asp Thr Ile Leu Leu Gln Asp Gly Pro
   405 410 415
   Asn Arg Arg Tyr Ser Leu Gly Val Gly Tyr Gln Leu Ser His Leu Gln
   420 425 430
   Asp Ala Thr Lys Ser Ser His Ala Thr Lys Ile His Phe Gly Val Leu
   435 440 445
   Gln Arg Leu Pro Asn Gly Leu Thr Val Gln Gly Arg Val Ser Ala Glu
   450 455 460
   Arg Glu Arg Tyr His Gly Lys Leu Leu Arg Leu Val Asn Pro Asp Asp
   465 470 475 480
   Val Tyr Arg Thr Asp Lys Thr Leu Thr Leu Gln Thr Ser Ile Trp His
   485 490 495
   Lys Asp Ile His Trp Leu Gly Leu Thr Pro Lys Leu Thr Tyr Arg Tyr
   500 505 510
   Ser Lys Asn Asn Ser Asn Leu Pro Ala Leu Tyr Ser His Asn Lys Gln
   515 520 525
   Asn Phe Tyr Leu Glu Leu Gly Arg Ser Phe
   530 535
(2) INFORMATION FOR SEQ ID NO:19:
   (i) SEQUENCE CHARACTERISTICS:
      (A) LENGTH: 1076 amino acids
      (B) TYPE: amino acid
      (C) STRANDEDNESS: single
      (D) TOPOLOGY: linear
   (xi) SEQUENCE DESCRIPTION: SEQ ID NO:19:
   Met Asn Gln Ser Lys Gln Asn Asn Lys Ser Lys Lys Ser Lys Gln Val
   1               5                   10                  15
   Leu Lys Leu Ser Ala Leu Ser Leu Gly Leu Leu Asn Ile Thr Gln Val
               20                  25                  30
   Ala Leu Ala Asn Thr Thr Ala Asp Lys Ala Glu Ala Thr Asp Lys Thr
           35                  40                  45
   Asn Leu Val Val Val Leu Asp Glu Thr Val Val Thr Ala Lys Lys Asn
       50                  55                  60
   Ala Pro Val Ser Arg Lys Ala Asn Glu Val Thr Gly Leu Gly Lys Val
   65                  70                  75                  80
   Val Lys Thr Ala Glu Thr Ile Asn Lys Glu Gln Val Leu Asn Ile Arg
                   85                  90                  95
   Asp Leu Thr Arg Tyr Asp Pro Gly Ile Ala Val Val Glu Gln Gly Arg
               100                 105                 110
   Gly Ala Ser Ser Gly Tyr Ser Ile Arg Gly Met Asp Lys Asn Arg Val
           115                 120                 125
   Ala Val Leu Val Asp Gly Ile Asn Gln Ala Gln His Tyr Gln Gly Pro
       130                 135                 140
   Val Ala Gly Lys Asn Tyr Ala Ala Gly Gly Ala Ile Asn Glu Ile Glu
   145                 150                 155                 160
   Tyr Glu Asn Val Arg Ser Val Glu Ile Ser Lys Gly Ala Asn Ser Ser
                   165                 170                 175
   Glu Tyr Gly Ser Gly Ala Leu Ser Gly Ser Val Ala Phe Val Thr Lys
               180                 185                 190
   Thr Ala Asp Asp Ile Ile Lys Asp Gly Lys Asp Trp Gly Val Gln Thr
           195                 200                 205
   Lys Thr Ala Tyr Ala Ser Lys Asn Asn Ala Trp Val Asn Ser Val Ala
       210                 215                 220
   Ala Ala Gly Lys Ala Gly Ser Phe Ser Gly Leu Ile Ile Tyr Thr Asp
   225                 230                 235                 240
   Arg Arg Gly Gln Glu Tyr Lys Ala His Asp Asp Ala Tyr Gln Gly Ser
                   245                 250                 255
   Gln Ser Phe Asp Arg Ala Val Ala Thr Thr Asp Pro Asn Asn Arg Thr
               260                 265                 270
   Phe Leu Ile Ala Asn Glu Cys Ala Asn Gly Asn Tyr Glu Ala Cys Ala
           275                 280                 285
   Ala Gly Gly Gln Thr Lys Leu Gln Ala Lys Pro Thr Asn Val Arg Asp
       290                 295                 300
   Lys Val Asn Val Lys Asp Tyr Thr Gly Pro Asn Arg Leu Ile Pro Asn
   305                 310                 315                 320
   Pro Leu Thr Gln Asp Ser Lys Ser Leu Leu Leu Arg Pro Gly Tyr Gln
                   325                 330                 335
   Leu Asn Asp Lys His Tyr Val Gly Gly Val Tyr Glu Ile Thr Lys Gln
               340                 345                 350
   Asn Tyr Ala Met Gln Asp Lys Thr Val Pro Ala Tyr Leu Ala Val His
           355                 360                 365
   Asp Ile Glu Lys Ser Arg Leu Ser Asn His Ala Gln Ala Asn Gly Tyr
       370                 375                 380
   Tyr Gln Gly Asn Asn Leu Gly Glu Arg Ile Arg Asp Thr Ile Gly Pro
   385                 390                 395                 400
   Asp Ser Gly Tyr Gly Ile Asn Tyr Ala His Gly Val Phe Tyr Asp Glu
                   405                 410                 415
   Lys His Gln Lys Asp Arg Leu Gly Leu Glu Tyr Val Tyr Asp Ser Lys
               420                 425                 430
   Gly Glu Asn Lys Trp Phe Asp Asp Val Arg Val Ser Tyr Asp Lys Gln
           435                 440                 445
   Asp Ile Thr Leu Arg Ser Gln Leu Thr Asn Thr His Cys Ser Thr Tyr
       450                 455                 460
   Pro His Ile Asp Lys Asn Cys Thr Pro Asp Val Asn Lys Pro Phe Ser
   465                 470                 475                 480
   Val Lys Glu Val Asp Asn Asn Ala Tyr Lys Glu Gln His Asn Leu Ile
                   485                 490                 495
   Lys Ala Val Phe Asn Lys Lys Met Ala Leu Gly Ser Thr His His His
               500                 505                 510
   Ile Asn Leu Gln Val Gly Tyr Asp Lys Phe Asn Ser Ser Leu Ser Arg
           515                 520                 525
   Val Glu Tyr Arg Leu Ala Thr His Gln Ser Tyr Gln Lys Leu Asp Tyr
       530                 535                 540
   Thr Pro Pro Ser Asn Pro Leu Pro Asp Lys Phe Lys Pro Ile Leu Gly
   545                 550                 555                 560
   Ser Asn Asn Lys Pro Ile Cys Leu Asp Ala Tyr Gly Tyr Gly His Asp
                   565                 570                 575
   His Pro Gln Ala Cys Asn Ala Lys Asn Ser Thr Tyr Gln Asn Phe Ala
               580                 585                 590
   Ile Lys Lys Gly Ile Glu Gln Tyr Asn Gln Lys Thr Asn Thr Asp Lys
           595                 600                 605
   Ile Asp Tyr Gln Ala Ile Ile Asp Gln Tyr Asp Lys Gln Asn Pro Asn
       610                 615                 620
   Ser Thr Leu Lys Pro Phe Glu Lys Ile Lys Gln Ser Leu Gly Gln Glu
   625                 630                 635                 640
   Lys Tyr Asn Lys Ile Asp Glu Leu Gly Phe Lys Ala Tyr Lys Asp Leu
                   645                 650                 655
   Arg Asn Glu Trp Ala Gly Trp Thr Asn Asp Asn Ser Gln Gln Asn Ala
               660                 665                 670
   Asn Lys Gly Thr Asp Asn Ile Tyr Gln Pro Asn Gln Ala Thr Val Val
           675                 680                 685
   Lys Asp Asp Lys Cys Lys Tyr Ser Glu Thr Asn Ser Tyr Ala Asp Cys
       690                 695                 700
   Ser Thr Thr Pro Arg His Ile Ser Gly Asp Asn Tyr Phe Ile Ala Leu
   705                 710                 715                 720
   Lys Asp Asn Met Thr Ile Asn Lys Tyr Val Asp Leu Gly Leu Gly Ala
                   725                 730                 735
   Arg Tyr Asp Arg Ile Lys His Lys Ser Asp Val Pro Leu Val Asp Asn
               740                 745                 750
   Ser Ala Ser Asn Gln Leu Ser Trp Asn Phe Gly Val Val Val Lys Pro
           755                 760                 765
   Thr Asn Trp Leu Asp Ile Ala Tyr Arg Ser Ser Gln Gly Phe Arg Met
       770                 775                 780
   Pro Ser Phe Ser Glu Met Tyr Gly Glu Arg Phe Gly Val Thr Ile Gly
   785                 790                 795                 800
   Lys Gly Thr Gln His Gly Cys Lys Gly Leu Tyr Tyr Ile Cys Gln Gln
                   805                 810                 815
   Thr Val His Gln Thr Lys Leu Lys Pro Glu Lys Ser Phe Asn Gln Glu
               820                 825                 830
   Ile Gly Ala Thr Leu His Asn His Leu Gly Ser Leu Glu Val Ser Tyr
           835                 840                 845
   Phe Lys Asn Arg Tyr Thr Asp Leu Ile Val Gly Lys Ser Glu Glu Ile
       850                 855                 860
   Arg Thr Leu Thr Gln Gly Asp Asn Ala Gly Lys Gln Arg Gly Lys Gly
   865                 870                 875                 880
   Asp Leu Gly Phe His Asn Gly Gln Asp Ala Asp Leu Thr Gly Ile Asn
                   885                 890                 895
   Ile Leu Gly Arg Leu Asp Leu Asn Ala Ala Asn Ser Arg Leu Pro Tyr
               900                 905                 910
   Gly Leu Tyr Ser Thr Leu Ala Tyr Asn Lys Val Asp Val Lys Gly Lys
           915                 920                 925
   Thr Leu Asn Pro Thr Leu Ala Gly Thr Asn Ile Leu Phe Asp Ala Ile
       930                 935                 940
   Gln Pro Ser Arg Tyr Val Val Gly Leu Gly Tyr Asp Ala Pro Ser Gln
   945                 950                 955                 960
   Lys Trp Gly Ala Asn Ala Ile Phe Thr His Ser Asp Ala Lys Asn Pro
                   965                 970                 975
   Ser Glu Leu Leu Ala Asp Lys Asn Leu Gly Asn Gly Asn Ile Gln Thr
               980                 985                 990
   Lys Gln Ala Thr Lys Ala Lys Ser Thr Pro Trp Gln Thr Leu Asp Leu
           995                 1000                1005
   Ser Gly Tyr Val Asn Ile Lys Asp Asn Phe Thr Leu Arg Ala Gly Val
       1010                1015                1020
   Tyr Asn Val Phe Asn Thr Tyr Tyr Thr Thr Trp Glu Ala Leu Arg Gln
   1025                1030                1035                1040
   Thr Ala Lys Gly Ala Val Asn Gln His Thr Gly Leu Ser Gln Asp Lys
                   1045                1050                1055
   His Tyr Gly Arg Tyr Ala Ala Pro Gly Arg Asn Tyr Gln Leu Ala Leu
               1060                1065                1070
   Glu Met Lys Phe
           1075
(2) INFORMATION FOR SEQ ID NO:20:
   (i) SEQUENCE CHARACTERISTICS:
      (A) LENGTH: 753 amino acids
      (B) TYPE: amino acid
      (C) STRANDEDNESS: single
      (D) TOPOLOGY: linear
   (xi) SEQUENCE DESCRIPTION: SEQ ID NO:20:
   Gln Tyr Thr Arg Lys Gly Glu Asn Lys Ala His Ser Asp Leu Asn Gly
   1               5                   10                  15
   Ile Asn Gln Ser Leu Tyr Arg Leu Gly Ala Trp Gln Gln Lys Tyr Asp
               20                  25                  30
   Leu Arg Lys Pro Asn Glu Leu Phe Ala Gly Thr Ser Tyr Ile Thr Glu
           35                  40                  45
   Ser Cys Leu Ala Ser Asp Asp Pro Lys Ser Cys Val Gln Tyr Pro Tyr
       50                  55                  60
   Val Tyr Thr Lys Ala Arg Pro Asp Gly Ile Gly Asn Arg Asn Phe Ser
   65                  70                  75                  80
   Glu Leu Ser Asp Ala Glu Lys Ala Gln Tyr Leu Ala Ser Thr His Pro
                   85                  90                  95
   His Glu Val Val Ser Ala Lys Asp Tyr Thr Gly Ile Tyr Arg Leu Leu
               100                 105                 110
   Pro Asp Pro Met Asp Tyr Arg Ser Asp Ser Tyr Leu Ala Arg Leu Asn
           115                 120                 125
   Ile Lys Ile Thr Pro Asn Leu Val Xaa Lys Leu Leu Leu Glu Asp Thr
       130                 135                 140
   Lys Gln Thr Tyr Asn Ile Arg Asp Met Arg His Cys Ser Tyr His Gly
   145                 150                 155                 160
   Ala Arg Leu Gly Asn Asp Gly Lys Pro Ala Asn Gly Gly Ser Ile Val
                   165                 170                 175
   Leu Cys Asp Asp Tyr Gln Glu Tyr Leu Asn Ala Asn Asp Ala Ser Gln
               180                 185                 190
   Ala Leu Phe Arg Pro Gly Ala Asn Asp Ala Pro Ile Pro Lys Leu Ala
           195                 200                 205
   Tyr Ala Arg Ser Ser Val Phe Asn Gln Glu His Gly Lys Thr Arg Tyr
       210                 215                 220
   Gly Leu Ser Phe Glu Phe Lys Pro Asp Thr Pro Trp Phe Lys Gln Ala
   225                 230                 235                 240
   Lys Leu Asn Leu His Gln Gln Asn Ile Gln Ile Ile Asn His Asp Ile
                   245                 250                 255
   Lys Lys Ser Cys Ser Gln Tyr Pro Lys Val Asp Ser Asn Cys Gly Ile
               260                 265                 270
   Ser Glu Ile Gly His Tyr Glu Tyr Gln Xaa Asn Tyr Arg Tyr Lys Glu
           275                 280                 285
   Gly Arg Ala Ser Leu Thr Gly Lys Leu Asp Phe Asn Phe Asp Leu Leu
       290                 295                 300
   Gly Gln His Asp Leu Thr Val Leu Ala Gly Thr Asp Lys Val Lys Ser
   305                 310                 315                 320
   Gln Phe Arg Ala Asn Asn Pro Arg Arg Thr Ile Ile Asp Thr Thr Gln
                   325                 330                 335
   Gly Asp Ala Ile Ile Asp Glu Ser Thr Leu Thr Ala Gln Glu Gln Ala
               340                 345                 350
   Lys Phe Lys Gln Ser Gly Ala Ala Trp Ile Val Lys Asn Arg Leu Gly
           355                 360                 365
   Arg Leu Glu Glu Lys Asp Ala Cys Gly Asn Ala Asn Glu Cys Glu Arg
       370                 375                 380
   Ala Pro Ile His Gly Ser Asn Gln Tyr Val Gly Ile Asn Asn Leu Tyr
   385                 390                 395                 400
   Thr Pro Asn Asp Tyr Val Asp Xaa Ser Phe Gly Gly Arg Leu Asp Lys
                   405                 410                 415
   Gln Arg Ile His Ser Thr Asp Ser Asn Ile Ile Ser Lys Thr Tyr Thr
               420                 425                 430
   Asn Lys Ser Tyr Asn Phe Gly Ala Ala Val His Leu Thr Pro Asp Phe
           435                 440                 445
   Ser Leu Leu Tyr Lys Thr Ala Lys Gly Phe Arg Thr Pro Ser Phe Tyr
       450                 455                 460
   Glu Leu Tyr Asn Tyr Asn Ser Thr Ala Ala Gln His Lys Asn Asp Pro
   465                 470                 475                 480
   Asp Val Ser Phe Pro Lys Arg Ala Val Asp Val Lys Pro Glu Thr Ser
                   485                 490                 495
   Asn Thr Asn Glu Tyr Gly Phe Arg Tyr Gln His Pro Trp Gly Asp Val
               500                 505                 510
   Glu Met Ser Met Phe Lys Ser Arg Tyr Lys Asp Met Leu Asp Lys Ala
           515                 520                 525
   Ile Pro Asn Leu Thr Lys Ala Gln Gln Glu Tyr Cys Arg Ala His Leu
       530                 535                 540
   Asp Ser Asn Glu Cys Val Gly Asn Pro Pro Thr Pro Lys Thr Ser Asp
   545                 550                 555                 560
   Glu Val Phe Ala Asn Leu Tyr Asn Ala Thr Ile Lys Gly Val Ser Val
                   565                 570                 575
   Lys Gly Lys Leu Asp Leu His Ala Met Thr Ser Lys Leu Pro Asp Gly
               580                 585                 590
   Leu Glu Met Thr Leu Gly Tyr Gly His Thr Lys Leu Gly Lys Phe Xaa
           595                 600                 605
   Tyr Ile Ala Pro Lys Asp Ala Asp Gly Trp Tyr Gln Ala Arg Pro Ala
       610                 615                 620
   Phe Trp Asp Ala Ile Thr Pro Ala Arg Tyr Val Val Gly Leu Asn Tyr
   625                 630                 635                 640
   Asp His Pro Ser Gln Val Trp Gly Ile Gly Ala Thr Leu Thr His Ser
                   645                 650                 655
   Lys Gln Lys Asp Glu Asn Glu Leu Ser Ala Leu Arg Ile Arg Asn Gly
               660                 665                 670
   Lys Arg Glu Thr Gln Thr Leu Thr His Thr Ile Pro Lys Ala Tyr Thr
           675                 680                 685
   Leu Leu Asp Met Thr Gly Tyr Tyr Ser Pro Thr Glu Ser Ile Thr Ala
       690                 695                 700
   Arg Leu Gly Ile Asn Asn Val Leu Asn Thr Arg Tyr Thr Thr Trp Glu
   705                 710                 715                 720
   Ala Ala Arg Gln Leu Pro Ser Glu Ala Ala Ser Ser Thr Gln Ser Thr
                   725                 730                 735
   Arg Tyr Ile Ala Pro Gly Arg Ser Tyr Phe Ala Ser Leu Glu Met Lys
               740                 745                 750
   Phe
(2) INFORMATION FOR SEQ ID NO:21:
   (i) SEQUENCE CHARACTERISTICS:
      (A) LENGTH: 585 amino acids
      (B) TYPE: amino acid
      (C) STRANDEDNESS: single
      (D) TOPOLOGY: linear
   (xi) SEQUENCE DESCRIPTION: SEQ ID NO:21:
   Gln Tyr Thr Arg Lys Gly Glu Asn Lys Ala His Ser Asp Leu Asn Gly
   1               5                   10                  15
   Ile Asn Gln Ser Leu Tyr Arg Leu Gly Ala Trp Gln Gln Lys Tyr Asp
               20                  25                  30
   Leu Arg Lys Pro Asn Glu Leu Phe Ala Gly Thr Ser Tyr Ile Thr Glu
           35                  40                  45
   Ser Cys Leu Ala Ser Asp Asp Pro Lys Ser Cys Val Gln Tyr Pro Tyr
       50                  55                  60
   Val Tyr Thr Lys Ala Arg Pro Asp Gly Ile Gly Asn Arg Asn Phe Ser
   65                  70                  75                  80
   Glu Leu Ser Asp Ala Glu Lys Ala Gln Tyr Leu Ala Ser Thr His Pro
                   85                  90                  95
   His Glu Val Val Ser Ala Lys Asp Tyr Thr Gly Thr Tyr Arg Leu Leu
               100                 105                 110
   Pro Asp Pro Met Asp Tyr Arg Ser Asp Ser Tyr Leu Ala Arg Leu Asn
           115                 120                 125
   Ile Lys Ile Thr Pro Asn Leu Val Ser Lys Leu Leu Leu Glu Asp Thr
       130                 135                 140
   Lys Gln Thr Tyr Asn Ile Arg Asp Met Arg His Cys Ser Tyr His Gly
   145                 150                 155                 160
   Ala Arg Leu Gly Asn Asp Gly Lys Pro Ala Asn Gly Gly Ser Ile Val
                   165                 170                 175
   Leu Cys Asp Asp Tyr Gln Glu Tyr Leu Asn Ala Asn Asp Ala Ser Gln
               180                 185                 190
   Ala Ser Phe Arg Pro Gly Ala Asn Asp Ala Pro Ile Pro Lys Leu Ala
           195                 200                 205
   Tyr Ala Arg Ser Ser Val Phe Asn Gln Glu His Gly Lys Thr Arg Tyr
       210                 215                 220
   Gly Leu Gly Phe Glu Phe Lys Pro Asp Thr Pro Trp Phe Lys Gln Ala
   225                 230                 235                 240
   Lys Leu Asn Leu His Gln Gln Asn Ile Gln Ile Ile Asn Thr Asp Ser
                   245                 250                 255
   Asn Ile Ile Ser Lys Thr Tyr Thr Asn Lys Ser Tyr Asn Phe Gly Ala
               260                 265                 270
   Ala Val His Xaa Thr Pro Asp Phe Ser Leu Leu Tyr Lys Thr Ala Lys
           275                 280                 285
   Gly Phe Arg Thr Pro Ser Phe Tyr Glu Leu Tyr Asn Tyr Asn Ser Thr
       290                 295                 300
   Ala Ala Gln His Lys Asn Asp Pro Asp Val Ser Phe Pro Lys Arg Ala
   305                 310                 315                 320
   Val Asp Val Lys Pro Glu Thr Ser Asn Thr Asn Glu Tyr Gly Phe Arg
                   325                 330                 335
   Tyr Gln His Pro Trp Gly Asp Ile Glu Met Ser Met Phe Lys Ser Arg
               340                 345                 350
   Tyr Lys Asp Met Leu Asp Lys Ala Ile Pro Asn Leu Thr Lys Ala Gln
           355                 360                 365
   Gln Glu Tyr Cys Lys Ala His Leu Asp Ser Asn Glu Cys Val Gly Asn
       370                 375                 380
   Pro Pro Thr Pro Lys Thr Ser Asp Glu Val Phe Ala Asn Leu Tyr Asn
   385                 390                 395                 400
   Ala Thr Ile Lys Gly Val Ser Val Lys Gly Lys Leu Asp Leu His Ala
                   405                 410                 415
   Met Thr Ser Lys Leu Pro Asp Gly Leu Glu Met Thr Leu Gly Tyr Gly
               420                 425                 430
   His Thr Lys Leu Gly Lys Phe Xaa Tyr Ile Ala Pro Lys Asp Ala Asp
           435                 440                 445
   Gly Trp Tyr Gln Ala Arg Pro Ala Phe Trp Asp Ala Ile Thr Pro Ala
       450                 455                 460
   Arg Tyr Val Val Gly Leu Asn Tyr Asp His Pro Ser Gln Val Trp Gly
   465                 470                 475                 480
   Ile Gly Thr Thr Leu Thr His Ser Lys Gln Lys Asp Glu Asn Glu Leu
                   485                 490                 495
   Ser Ala Leu Arg Ile Arg Asn Gly Lys Arg Glu Ile Gln Thr Leu Thr
               500                 505                 510
   His Thr Ile Pro Lys Ala Tyr Thr Leu Leu Asp Met Thr Gly Tyr Tyr
           515                 520                 525
   Ser Pro Thr Glu Ser Ile Thr Ala Arg Leu Gly Ile Asn Asn Val Leu
       530                 535                 540
   Asn Thr Arg Tyr Thr Thr Trp Glu Ala Ala Arg Gln Leu Pro Ser Glu
   545                 550                 555                 560
   Ala Ala Ser Ser Thr Gln Ser Thr Arg Tyr Ile Ala Pro Gly Arg Ser
                   565                 570                 575
   Tyr Phe Ala Ser Leu Glu Met Lys Phe
               580                 585
(2) INFORMATION FOR SEQ ID NO:22:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (xi) SEQUENCE DESCRIPTION: SEQ ID NO:22:
   Met Val Gln Tyr Thr Tyr Arg Lys Gly Lys Glu Asn Lys Ala His
   1               5                   10                  15
(2) INFORMATION FOR SEQ ID NO:23:
   (i) SEQUENCE CHARACTERISTICS:
      (A) LENGTH: 944 amino acids
      (B) TYPE: amino acid
      (C) STRANDEDNESS: single
      (D) TOPOLOGY: linear
   (xi) SEQUENCE DESCRIPTION: SEQ ID NO:23:
   Met Asn Lys Lys His Gly Phe Gln Leu Thr Leu Thr Ala Leu Ala Val
   1               5                   10                  15
   Ala Ala Ala Phe Pro Ser Tyr Ala Ala Asn Pro Glu Thr Ala Ala Pro
               20                  25                  30
   Asp Ala Ala Gln Thr Gln Ser Leu Lys Glu Val Thr Val Arg Ala Ala
           35                  40                  45
   Lys Val Gly Arg Arg Ser Lys Glu Ala Val Thr Gly Leu Gly Lys Ile
       50                  55                  60
   Ala Lys Thr Ser Glu Thr Leu Asn Lys Glu Gln Val Leu Gly Ile Arg
   65                  70                  75                  80
   Asp Leu Thr Arg Tyr Asp Pro Gly Val Ala Val Val Glu Gln Gly Asn
                   85                  90                  95
   Gly Ala Ser Gly Gly Tyr Ser Ile Arg Gly Val Asp Lys Asn Arg Val
               100                 105                 110
   Ala Val Ser Val Asp Gly Val Ala Gln Ile Gln Ala Phe Thr Val Gln
           115                 120                 125
   Gly Ser Leu Ser Gly Tyr Gly Gly Arg Gly Gly Ser Gly Ala Ile Asn
       130                 135                 140
   Glu Ile Glu Tyr Glu Asn Ile Ser Thr Val Glu Ile Asp Lys Gly Ala
   145                 150                 155                 160
   Gly Ser Ser Asp His Gly Ser Gly Ala Leu Gly Gly Ala Val Ala Phe
                   165                 170                 175
   Arg Thr Lys Glu Ala Ala Asp Leu Ile Ser Asp Gly Lys Ser Trp Gly
               180                 185                 190
   Ile Gln Ala Lys Thr Ala Tyr Gly Ser Lys Asn Arg Gln Phe Met Lys
           195                 200                 205
   Ser Leu Gly Ala Gly Phe Ser Lys Asp Gly Trp Glu Gly Leu Leu Ile
       210                 215                 220
   Arg Thr Glu Arg Gln Gly Arg Glu Thr His Pro His Gly Asp Ile Ala
   225                 230                 235                 240
   Asp Gly Val Ala Tyr Gly Ile Asn Arg Leu Asp Ala Phe Arg Gln Thr
                   245                 250                 255
   Tyr Gly Ile Lys Lys Pro Ser Glu Gly Gly Glu Tyr Phe Leu Ala Glu
               260                 265                 270
   Gly Glu Ser Glu Leu Lys Pro Val Ala Lys Val Ala Gly Asn Gly Asn
   275 280 285
   Tyr Leu Asn Asn Gln Leu Asn Arg Trp Val Lys Glu Arg Ile Glu Gln
   290 295 300
   Asn Gln Pro Leu Ser Ala Glu Glu Glu Ala Met Val Arg Glu Ala Gln
   305 310 315 320
   Ala Arg His Glu Asn Leu Ser Ala Gln Ala Tyr Thr Gly Gly Gly Arg
   325 330 335
   Ile Leu Pro Asp Pro Met Asp Tyr Arg Ser Gly Ser Trp Leu Ala Lys
   340 345 350
   Leu Gly Tyr Arg Phe Gly Gly Arg His Tyr Val Gly Gly Val Phe Glu
   355 360 365
   Asp Thr Lys Gln Arg Tyr Asp Ile Arg Asp Met Thr Glu Lys Gln Tyr
   370 375 380
   Tyr Gly Thr Asp Glu Ala Lys Lys Phe Arg Asp Lys Ser Gly Val Tyr
   385 390 395 400
   Asp Gly Asp Asp Phe Arg Asp Gly Leu Tyr Phe Val Pro Asn Ile Glu
   405 410 415
   Glu Trp Lys Gly Asp Gln Lys Leu Ile Arg Gly Ile Gly Leu Lys Tyr
   420 425 430
   Ser Arg Thr Lys Phe Ile Asp Glu His His Arg Arg Arg Arg Met Gly
   435 440 445
   Leu Leu Tyr Arg Tyr Glu Asn Glu Lys Tyr Ser Asp Asn Trp Ala Asp
   450 455 460
   Lys Ala Val Leu Ser Phe Asp Lys Gln Gly Val Ala Thr Asp Asn Asn
   465 470 475 480
   Thr Leu Lys Leu Asn Cys Ala Val Tyr Pro Ala Val Asp Lys Ser Cys
   485 490 495
   Arg Ala Ser Ala Asp Lys Pro Tyr Ser Tyr Asp Ser Ser Asp Arg Phe
   500 505 510
   His Tyr Arg Glu Gln His Asn Val Leu Asn Ala Ser Phe Glu Lys Ser
   515 520 525
   Leu Lys Asn Lys Trp Thr Lys His His Leu Thr Leu Gly Phe Gly Tyr
   530 535 540
   Asp Ala Ser Asn Ala Ile Ser Arg Pro Glu Gln Leu Ser His Asn Ala
   545 550 555 560
   Ala Arg Ile Ser Glu Tyr Ser Asp Tyr Thr Asp Lys Gly Asp Lys Tyr
   565 570 575
   Leu Leu Gly Lys Pro Glu Val Val Glu Gly Ser Val Cys Gly Tyr Ile
   580 585 590
   Glu Thr Leu Arg Ser Arg Lys Cys Val Pro Arg Lys Ile Asn Gly Ser
   595 600 605
   Asn Ile His Ile Ser Leu Asn Asp Arg Phe Ser Ile Gly Lys Tyr Phe
       610                 615                 620
   Asp Phe Ser Leu Gly Gly Arg Tyr Asp Arg Lys Asn Phe Thr Thr Ser
   625                 630                 635                 640
   Glu Glu Leu Val Arg Ser Gly Arg Tyr Val Asp Arg Ser Trp Asn Ser
                   645                 650                 655
   Gly Ile Val Phe Lys Pro Asn Arg His Phe Ser Leu Ser Tyr Arg Ala
               660                 665                 670
   Ser Ser Gly Phe Arg Thr Pro Ser Phe Gln Glu Leu Phe Gly Ile Asp
           675                 680                 685
   Ile Tyr His Asp Tyr Pro Lys Gly Trp Gln Arg Pro Ala Leu Lys Ser
       690                 695                 700
   Glu Lys Ala Ala Asn Arg Glu Ile Gly Leu Gln Trp Lys Gly Asp Phe
   705                 710                 715                 720
   Gly Phe Leu Glu Ile Ser Ser Phe Arg Asn Arg Tyr Thr Asp Met Ile
                   725                 730                 735
   Ala Val Ala Asp His Lys Thr Lys Leu Pro Asn Gln Ala Gly Gln Leu
               740                 745                 750
   Thr Glu Ile Asp Ile Arg Asp Tyr Tyr Asn Ala Gln Asn Met Ser Leu
           755                 760                 765
   Gln Gly Val Asn Ile Leu Gly Lys Ile Asp Trp Asn Gly Val Tyr Gly
       770                 775                 780
   Lys Leu Pro Glu Gly Leu Tyr Thr Thr Leu Ala Tyr Asn Arg Ile Lys
   785                 790                 795                 800
   Pro Lys Ser Val Ser Asn Arg Pro Gly Leu Ser Leu Arg Ser Tyr Ala
                   805                 810                 815
   Leu Asp Ala Val Gln Pro Ser Arg Tyr Val Leu Gly Phe Gly Tyr Asp
               820                 825                 830
   Gln Pro Glu Gly Lys Trp Gly Ala Asn Ile Met Leu Thr Tyr Ser Lys
           835                 840                 845
   Gly Lys Asn Pro Asp Glu Leu Ala Tyr Leu Ala Gly Asp Gln Lys Arg
       850                 855                 860
   Tyr Ser Thr Lys Arg Ala Ser Ser Ser Trp Ser Thr Ala Asp Val Ser
   865                 870                 875                 880
   Ala Tyr Leu Asn Leu Lys Lys Arg Leu Thr Leu Arg Ala Ala Ile Tyr
                   885                 890                 895
   Asn Ile Gly Asn Tyr Arg Tyr Val Thr Trp Glu Ser Leu Arg Gln Thr
               900                 905                 910
   Ala Glu Ser Thr Ala Asn Arg His Gly Gly Asp Ser Asn Tyr Gly Arg
           915                 920                 925
   Tyr Ala Ala Pro Gly Arg Asn Phe Ser Leu Ala Leu Glu Met Lys Phe
       930                 935                 940
(2) INFORMATION FOR SEQ ID NO:24:
   (i) SEQUENCE CHARACTERISTICS:
      (A) LENGTH: 944 amino acids
      (B) TYPE: amino acid
      (C) STRANDEDNESS: single
      (D) TOPOLOGY: linear
   (xi) SEQUENCE DESCRIPTION: SEQ ID NO:24:
   Met Asn Lys Lys His Gly Phe Pro Leu Thr Leu Thr Ala Leu Ala Ile
   1               5                   10                  15
   Ala Thr Ala Phe Pro Ala Tyr Ala Ala Gln Ala Gly Ala Ala Ala Leu
               20                  25                  30
   Asp Ala Ala Gln Ser Gln Ser Leu Lys Glu Val Thr Val Arg Ala Ala
           35                  40                  45
   Lys Val Gly Arg Arg Ser Lys Pro Glu Ala Thr Gly Leu Gly Lys Ile
       50                  55                  60
   Ala Lys Thr Ser Glu Thr Leu Asn Lys Glu Gln Val Leu Gly Ile Arg
   65                  70                  75                  80
   Asp Leu Thr Arg Tyr Asp Pro Gly Val Ala Val Val Glu Gln Gly Asn
                   85                  90                  95
   Gly Ala Ser Gly Gly Tyr Ser Ile Arg Gly Val Asp Lys Asn Arg Val
               100                 105                 110
   Ala Val Ser Val Asp Gly Val Ala Gln Ile Gln Ala Phe Thr Val Gln
           115                 120                 125
   Gly Ser Leu Ser Gly Tyr Gly Gly Arg Gly Gly Ser Gly Ala Ile Asn
       130                 135                 140
   Glu Ile Glu Tyr Glu Asn Ile Ser Thr Val Glu Ile Asp Lys Gly Ala
   145                 150                 155                 160
   Gly Ser Ser Asp His Gly Ser Gly Ala Leu Gly Gly Ala Val Ala Phe
                   165                 170                 175
   Arg Thr Lys Glu Ala Ala Asp Leu Ile Ser Asp Gly Lys Ser Trp Gly
               180                 185                 190
   Ile Gln Ala Lys Thr Ala Tyr Gly Ser Lys Asn Arg Gln Phe Met Lys
           195                 200                 205
   Ser Leu Gly Ala Gly Phe Ser Lys Asp Gly Trp Glu Gly Leu Leu Ile
       210                 215                 220
   Arg Thr Glu Arg Gln Gly Arg Glu Thr Arg Pro His Gly Asp Ile Ala
   225                 230                 235                 240
   Asp Gly Val Glu Tyr Gly Ile Asp Arg Leu Asp Ala Phe Arg Gln Thr
                   245                 250                 255
   Tyr Asp Ile Lys Arg Lys Thr Thr Glu Pro Phe Phe Leu Val Glu Gly
               260                 265                 270
   Glu Asn Thr Leu Lys Pro Val Ala Lys Leu Ala Gly Tyr Gly Ile Tyr
           275                 280                 285
   Leu Asn Arg Gln Leu Asn Arg Trp Val Lys Glu Arg Ile Glu Gln Asn
       290                 295                 300
   Gln Pro Leu Ser Ala Glu Glu Glu Ala Gln Val Arg Glu Ala Gln Ala
   305                 310                 315                 320
   Arg His Glu Asn Leu Ser Ala Gln Ala Tyr Thr Gly Gly Gly Arg Ile
                   325                 330                 335
   Leu Pro Asp Pro Met Asp Tyr Arg Ser Gly Ser Trp Leu Ala Lys Leu
               340                 345                 350
   Gly Tyr Arg Phe Gly Gly Arg His Tyr Val Gly Gly Val Phe Glu Asp
           355                 360                 365
   Thr Lys Gln Arg Tyr Asp Ile Arg Asp Met Thr Glu Lys Gln Tyr Tyr
       370                 375                 380
   Gly Thr Asp Glu Ala Glu Lys Phe Arg Asp Lys Ser Gly Val Tyr Asp
   385                 390                 395                 400
   Gly Asp Asp Phe Arg Asp Gly Leu Tyr Phe Val Pro Asn Ile Glu Glu
                   405                 410                 415
   Trp Lys Gly Asp Lys Asn Leu Val Lys Gly Ile Gly Leu Lys Tyr Ser
               420                 425                 430
   Arg Thr Lys Phe Ile Asp Glu His His Arg Arg Arg Arg Met Gly Leu
           435                 440                 445
   Leu Tyr Arg Tyr Glu Asn Glu Lys Tyr Ser Asp Asn Trp Ala Asp Lys
       450                 455                 460
   Ala Val Leu Ser Phe Asp Lys Gln Gly Val Ala Thr Asp Asn Asn Thr
   465                 470                 475                 480
   Leu Lys Leu Asn Cys Ala Val Tyr Pro Ala Val Asp Lys Ser Cys Arg
                   485                 490                 495
   Ala Ser Ala Asp Lys Pro Tyr Ser Tyr Asp Ser Ser Asp Arg Phe His
               500                 505                 510
   Tyr Arg Glu Gln His Asn Val Leu Asn Ala Ser Phe Glu Lys Ser Leu
           515                 520                 525
   Lys Asn Lys Trp Thr Lys His His Leu Thr Leu Gly Phe Gly Tyr Asp
       530                 535                 540
   Ala Ser Lys Ala Val Ser Arg Pro Glu Gln Leu Ser His Asn Ala Ala
   545                 550                 555                 560
   Arg Ile Ser Glu Ser Thr Gly Phe Asp Glu Lys Asn Gln Asp Lys Tyr
                   565                 570                 575
   Arg Leu Gly Lys Pro Glu Val Val Glu Gly Ser Val Cys Gly Tyr Ile
               580                 585                 590
   Glu Thr Leu Arg Ser Arg Lys Cys Val Pro Arg Lys Ile Asn Gly Ser
           595                 600                 605
   Asn Ile His Ile Ser Leu Asn Asp Arg Phe Ser Ile Gly Lys Tyr Phe
       610                 615                 620
   Asp Phe Ser Leu Gly Gly Arg Tyr Asp Arg Lys Asn Phe Thr Thr Ser
   625                 630                 635                 640
   Glu Glu Leu Val Arg Ser Gly Arg Tyr Ala Asp Arg Ser Trp Asn Ser
                   645                 650                 655
   Gly Ile Val Phe Lys Pro Asn Arg His Phe Ser Val Ser Tyr Arg Ala
               660                 665                 670
   Ser Ser Gly Phe Arg Thr Pro Ser Phe Gln Glu Leu Phe Gly Ile Asp
           675                 680                 685
   Ile Tyr His Asp Tyr Pro Lys Gly Trp Gln Arg Pro Ala Leu Lys Ser
       690                 695                 700
   Glu Lys Ala Ala Asn Arg Glu Ile Gly Leu Gln Trp Lys Gly Asp Phe
   705                 710                 715                 720
   Gly Phe Leu Glu Ile Ser Ser Phe Arg Asn Arg Tyr Thr Asp Met Ile
                   725                 730                 735
   Ala Val Ala Asp Gln Lys Thr Lys Leu Pro Asp Ser Ala Gly Arg Leu
               740                 745                 750
   Thr Glu Ile Asp Ile Arg Asp Tyr Tyr Asn Ala Gln Asn Met Ser Leu
           755                 760                 765
   Gln Gly Ile Asn Ile Leu Gly Lys Ile Asp Trp Asn Gly Val Tyr Gly
       770                 775                 780
   Lys Leu Pro Glu Gly Leu Tyr Thr Thr Leu Ala Tyr Asn Arg Ile Lys
   785                 790                 795                 800
   Pro Lys Ser Val Ser Asn Arg Pro Asp Leu Ser Leu Arg Ser Tyr Ala
                   805                 810                 815
   Leu Asp Ala Val Gln Pro Ser Arg Tyr Val Leu Gly Phe Gly Tyr Asp
               820                 825                 830
   Gln Pro Glu Gly Lys Trp Gly Ala Asn Ile Met Leu Thr Tyr Ser Lys
           835                 840                 845
   Gly Lys Asn Pro Asp Glu Leu Ala Tyr Leu Ala Gly Asp Gln Lys Arg
       850                 855                 860
   Tyr Ser Ala Gly Arg Val Thr Ser Ser Trp Lys Thr Ala Asp Val Ser
   865                 870                 875                 880
   Ala Tyr Leu Asn Leu Lys Lys Arg Leu Thr Leu Arg Ala Ala Ile Tyr
                   885                 890                 895
   Asn Ile Gly Asn Tyr Arg Tyr Val Thr Trp Glu Ser Leu Arg Gln Thr
               900                 905                 910
   Ala Glu Ser Thr Ala Asn Arg His Gly Gly Asp Ser Asn Tyr Gly Arg
           915                 920                 925
   Tyr Ala Ala Pro Gly Arg Asn Phe Ser Leu Ala Leu Glu Met Lys Phe
       930                 935                 940
(2) INFORMATION FOR SEQ ID NO:25:
   (i) SEQUENCE CHARACTERISTICS:
      (A) LENGTH: 702 amino acids
      (B) TYPE: amino acid
      (C) STRANDEDNESS: single
      (D) TOPOLOGY: linear
   (xi) SEQUENCE DESCRIPTION: SEQ ID NO:25:
   Met Lys His Ile Pro Leu Thr Thr Leu Cys Val Ala Ile Ser Ala Val
   1               5                   10                  15
   Leu Leu Thr Ala Cys Gly Gly Ser Gly Gly Ser Asn Pro Pro Ala Pro
               20                  25                  30
   Thr Pro Ile Pro Asn Ala Ser Gly Ser Gly Asn Thr Gly Asn Thr Gly
           35                  40                  45
   Asn Ala Gly Gly Thr Asp Asn Thr Ala Asn Ala Gly Asn Thr Gly Gly
       50                  55                  60
   Thr Asn Ser Gly Thr Gly Ser Ala Asn Thr Pro Glu Pro Lys Tyr Gln
   65                  70                  75                  80
   Asp Val Pro Thr Glu Lys Asn Glu Lys Asp Lys Val Ser Ser Ile Gln
                   85                  90                  95
   Glu Pro Ala Met Gly Tyr Gly Met Ala Leu Ser Lys Ile Asn Leu His
               100                 105                 110
   Asn Arg Gln Asp Thr Pro Leu Asp Glu Lys Asn Ile Ile Thr Leu Asp
           115                 120                 125
   Gly Lys Lys Gln Val Ala Glu Gly Lys Lys Ser Pro Leu Pro Phe Ser
       130                 135                 140
   Leu Asp Val Glu Asn Lys Leu Leu Asp Gly Tyr Ile Ala Lys Met Asn
   145                 150                 155                 160
   Val Ala Asp Lys Asn Ala Ile Gly Asp Arg Ile Lys Lys Gly Asn Lys
                   165                 170                 175
   Glu Ile Ser Asp Glu Glu Leu Ala Lys Gln Ile Lys Glu Ala Val Arg
               180                 185                 190
   Lys Ser His Glu Phe Gln Gln Val Leu Ser Ser Leu Glu Asn Lys Ile
           195                 200                 205
   Phe His Ser Asn Asp Gly Thr Thr Lys Ala Thr Thr Arg Asp Leu Lys
       210                 215                 220
   Tyr Val Asp Tyr Gly Tyr Tyr Leu Ala Asn Asp Gly Asn Tyr Leu Thr
   225                 230                 235                 240
   Val Lys Thr Asp Lys Leu Trp Asn Leu Gly Pro Val Gly Gly Val Phe
                   245                 250                 255
   Tyr Asn Gly Thr Thr Thr Ala Lys Glu Leu Pro Thr Gln Asp Ala Val
               260                 265                 270
   Lys Tyr Lys Gly His Trp Asp Phe Met Thr Asp Val Ala Asn Arg Arg
   275 280 285
   Asn Arg Phe Ser Glu Val Lys Glu Asn Ser Gln Ala Gly Trp Tyr Tyr
   290 295 300
   Gly Ala Ser Ser Lys Asp Glu Tyr Asn Arg Leu Leu Thr Lys Glu Asp
   305 310 315 320
   Ser Ala Pro Asp Gly His Ser Gly Glu Tyr Gly His Ser Ser Glu Phe
   325 330 335
   Thr Val Asn Phe Lys Glu Lys Lys Leu Thr Gly Lys Leu Phe Ser Asn
   340 345 350
   Leu Gln Asp Arg His Lys Gly Asn Val Thr Lys Thr Glu Arg Tyr Asp
   355 360 365
   Ile Asp Ala Asn Ile His Gly Asn Arg Phe Arg Gly Ser Ala Thr Ala
   370 375 380
   Ser Asn Lys Asn Asp Thr Ser Lys His Pro Phe Thr Ser Asp Ala Asn
   385 390 395 400
   Asn Arg Leu Glu Gly Gly Phe Tyr Gly Pro Lys Gly Glu Glu Leu Ala
   405 410 415
   Gly Lys Phe Leu Thr Asn Asp Asn Lys Leu Phe Gly Val Phe Gly Ala
   420 425 430
   Lys Arg Glu Ser Lys Ala Glu Glu Lys Thr Glu Ala Ile Leu Asp Ala
   435 440 445
   Tyr Ala Leu Gly Thr Phe Asn Thr Ser Asn Ala Thr Thr Phe Thr Pro
   450 455 460
   Phe Thr Glu Lys Gln Leu Asp Asn Phe Gly Asn Ala Lys Lys Leu Val
   465 470 475 480
   Leu Gly Ser Thr Val Ile Asp Leu Val Pro Thr Asp Ala Thr Lys Asn
   485 490 495
   Glu Phe Thr Lys Asp Lys Pro Glu Ser Ala Thr Asn Glu Ala Gly Glu
   500 505 510
   Thr Leu Met Val Asn Asp Glu Val Ser Val Lys Thr Tyr Gly Lys Asn
   515 520 525
   Phe Glu Tyr Leu Lys Phe Gly Glu Leu Ser Ile Gly Gly Ser His Ser
   530 535 540
   Val Phe Leu Gln Gly Glu Arg Thr Ala Thr Thr Gly Glu Lys Ala Val
   545 550 555 560
   Pro Thr Thr Gly Thr Ala Lys Tyr Leu Gly Asn Trp Val Gly Tyr Ile
   565 570 575
   Thr Gly Lys Asp Thr Gly Thr Gly Thr Gly Lys Ser Phe Thr Asp Ala
   580 585 590
   Gln Asp Val Ala Asp Phe Asp Ile Asp Phe Gly Asn Lys Ser Val Ser
   595 600 605
   Gly Lys Leu Ile Thr Lys Gly Arg Gln Asp Pro Val Phe Ser Ile Thr
       610                 615                 620
   Gly Gln Ile Ala Gly Asn Gly Trp Thr Gly Thr Ala Ser Thr Thr Lys
   625                 630                 635                 640
   Ala Asp Ala Gly Gly Tyr Lys Ile Asp Ser Ser Ser Thr Gly Lys Ser
                   645                 650                 655
   Ile Ala Ile Lys Asp Ala Asn Val Thr Gly Gly Phe Tyr Gly Pro Asn
               660                 665                 670
   Ala Asn Glu Met Gly Gly Ser Phe Thr His Asn Ala Asp Asp Ser Lys
           675                 680                 685
   Ala Ser Val Val Phe Gly Thr Lys Arg Gln Gln Glu Val Lys
       690                 695                 700
(2) INFORMATION FOR SEQ ID NO:26:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (xi) SEQUENCE DESCRIPTION: SEQ ID NO:26:
   Leu Glu Met Lys Phe
   1               5
(2) INFORMATION FOR SEQ ID NO:27:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (xi) SEQUENCE DESCRIPTION: SEQ ID NO:27:
   Leu Glu Gly Gly Phe Tyr
   1               5
(2) INFORMATION FOR SEQ ID NO:28:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (xi) SEQUENCE DESCRIPTION: SEQ ID NO:28:
   Gln Tyr Thr Arg Lys Gly Glu Asn Lys Ala
   1               5                   10
(2) INFORMATION FOR SEQ ID NO:29:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:29:
      CAATATACCG TAAAGGTGAA AATAAAGC 28
(2) INFORMATION FOR SEQ ID NO:30:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:30:
      CAATATACCG TAAAGGTGAA AATAAAGC 28
(2) INFORMATION FOR SEQ ID NO:31:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:31:
      CAATATACCG TAAAGGTGAA AACAAAGC 28
(2) INFORMATION FOR SEQ ID NO:32:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:32:
      CAATATACCG TAAAGGCGAA AATAAAGC 28
(2) INFORMATION FOR SEQ ID NO:33:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:33:
      CAATATACCG CAAAGGCGAA AACAAAGC 28
(2) INFORMATION FOR SEQ ID NO:34:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:34:
      CAATATACCG CAAAGGCGAA AATAAAGC 28
(2) INFORMATION FOR SEQ ID NO:35:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:35:
      CAATATACCG CAAAGGTGAA AATAAAGC 28
(2) INFORMATION FOR SEQ ID NO:36:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:36:
      CAATATACCG CAAAGGTGAA AACAAAGC 28
(2) INFORMATION FOR SEQ ID NO:37:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:37:
      CTTGAAATGA AGTTTTAA 18
(2) INFORMATION FOR SEQ ID NO:38:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:38:
      GAACTTTACT TCAAAATT 18
(2) INFORMATION FOR SEQ ID NO:39:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (xi) SEQUENCE DESCRIPTION: SEQ ID NO:39:
   Asp Gly Leu Gly
   1
(2) INFORMATION FOR SEQ ID NO:40:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (xi) SEQUENCE DESCRIPTION: SEQ ID NO:40:
   Met Ser Lys Ser Ile Thr
   1               5
(2) INFORMATION FOR SEQ ID NO:41:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:41:
      GGAATTCCAT ATGTCAAAAT CTATCACAAA 30
(2) INFORMATION FOR SEQ ID NO:42:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (xi) SEQUENCE DESCRIPTION: SEQ ID NO:42:
   Leu Asp Ala Ile Thr Val Thr Ala Ala
   1               5
(2) INFORMATION FOR SEQ ID NO:43:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:43:
      TTTAGATGCC ATCACGGTAA CCGCCGCCCC 30
(2) INFORMATION FOR SEQ ID NO:44:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:44:
      AAATCTACGG TAGTGCCATT GGCGGCGGGG 30
(2) INFORMATION FOR SEQ ID NO:45:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (xi) SEQUENCE DESCRIPTION: SEQ ID NO:45:
   Gly Lys Leu Asp Leu His Ala Met Thr Ser
   1               5                   10
(2) INFORMATION FOR SEQ ID NO:46:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:46:
      GGCAAACTGG ATTTGCATGC CATGACATCA 30
(2) INFORMATION FOR SEQ ID NO:47:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (xi) SEQUENCE DESCRIPTION: SEQ ID NO:47:
   Ser Leu Glu Met Lys Phe
   1               5
(2) INFORMATION FOR SEQ ID NO:48:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:48:
      AGTCTTGAAA TGAAGTTTTA A 21
(2) INFORMATION FOR SEQ ID NO:49:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO:49:
      TCAGAACTTT ACTTCAAAAT TGCCCTAGGG C 31
(2) INFORMATION FOR SEQ ID NO:50:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (xi) SEQUENCE DESCRIPTION: SEQ ID NO:50:
   Met Thr Thr His Arg Leu
   1               5
(2) INFORMATION FOR SEQ ID NO:51:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:51:
      GGAATTCCAT ATGACCACGC ACCGCTTAAA 30
(2) INFORMATION FOR SEQ ID NO:52:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (xi) SEQUENCE DESCRIPTION: SEQ ID NO:52:
   Met Ser Thr Val Lys Thr Pro His Ile
   1               5
(2) INFORMATION FOR SEQ ID NO:53:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:53:
      GGAATTCCAT ATGAGTACTG TCAAAACCCC CCACA 35
(2) INFORMATION FOR SEQ ID NO:54:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (xi) SEQUENCE DESCRIPTION: SEQ ID NO:54:
   Ile Pro Asn Thr Gly His Asp Asn Thr Asn
   1               5                   10
(2) INFORMATION FOR SEQ ID NO:55:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO:55:
      AATACCGAAC ACAGGTCATG ACAACACCAA T 31
(2) INFORMATION FOR SEQ ID NO:56:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO:56:
      TTATGGCTTG TGTCCAGTAC TGTTGTGGTT A 31
(2) INFORMATION FOR SEQ ID NO:57:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (xi) SEQUENCE DESCRIPTION: SEQ ID NO:57:
   Asn Glu Pro Thr His Glu Lys Thr Phe Ala
   1               5                   10
(2) INFORMATION FOR SEQ ID NO:58
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:58
      AATGAGCCTA CTCATGAAAA AACCTTTGCC 30
(2) INFORMATION FOR SEQ ID NO:59
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (xi) SEQUENCE DESCRIPTION: SEQ ID NO:59:
   Gly Ala Val Phe Gly Ala Val Lys Asp Lys
   1               5                   10
(2) INFORMATION FOR SEQ ID NO:60:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:60:
      GGGCTGTCTT TGGGGCTGTT AAAGATAAAT AA 32
(2) INFORMATION FOR SEQ ID NO:61:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO:61:
      CCCGACAGAA ACCCCGACAA TTTCTATTTA TTCCTAGGGC 40
(2) INFORMATION FOR SEQ ID NO:62:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (xi) SEQUENCE DESCRIPTION: SEQ ID NO:62:
   Met Cys Arg Ser Asp Asp Ile Ser Val Asn
   1               5                   10
(2) INFORMATION FOR SEQ ID NO:63:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO:63:
      GGAATTCCAT ATGTGCCGCT CTGATGACAT CAGCGTCAAT 40
(2) INFORMATION FOR SEQ ID NO:64:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (xi) SEQUENCE DESCRIPTION: SEQ ID NO:64:
   Phe Leu Lys Gln Val
   1               5
(2) INFORMATION FOR SEQ ID NO:65:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (xi) SEQUENCE DESCRIPTION: SEQ ID NO:65:
      TTTTTAAAGC AGGTG 15
(2) INFORMATION FOR SEQ ID NO:66:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (xi) SEQUENCE DESCRIPTION: SEQ ID NO:66:
      AAAAATTTCG TC 12
(2) INFORMATION FOR SEQ ID NO:67:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (xi) SEQUENCE DESCRIPTION: SEQ ID NO:67:
   Met Val Gln Tyr Thr Arg Lys Gly Glu Asn Lys Ala His
   1               5                   10
(2) INFORMATION FOR SEQ ID NO: 68:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO: 68:
      TTAGCCCAAG GCAAATCTGG TGCA 24
(2) INFORMATION FOR SEQ ID NO: 69:
   (i) SEQUENCE CHARACTERISTICS:
      (A) LENGTH: 2718 base pairs
      (B) TYPE: nucleic acid
      (C) STRANDEDNESS: single
      (D) TOPOLOGY: linear
   (xi) SEQUENCE DESCRIPTION: SEQ ID NO: 69:
   ATGAGTACTG TCAAAGTCCC CCACATTTTC TACCAAAAAC GCACCCTTAG CCTTGCCATC 60
   GCCAGTATTT TTGCTGCCGT GGTGATGACA GGCTGCCGCT CTGATGACAT CAGCGTCAAT 120
   GCACCCAATG TTACCCAACT GCCCCAAGGC ACGGTTTCAC CAATACCGAA CACAGGTCAT 180
   GACAACACCA ATAACACCAA CAATCAGGGC AACAACACGG ATAACAGCAC CAGCACAACT 240
   GACCCAAATG GCGATAACAA CCAACTGACA CAAGCACAAA AAACTGCCGC CGCCGCAGGG 300
   TTTTTTGTGA TGGGTAAAAT TCGTGATACC AGCGAAAAAA ATGACCCAGA TTATACCAAA 360
   GATTTACAAG GCAGCGTACA TACAGCAGGG CAAGGCTTAC AGTACTTAGG CACCAAAGAG 420
   CCTCGGCCAG ATGGCACAGG TACAGGTAAA AACTTACGCC AGCCCATCAC AGCTGATGAC 480
   ATTACACCAC TTTATTTTGA TAAATTCCCC AAAATATCCG ATCTGCACCT AGAAAACAGC 540
   GAGCATGTGT TTGATGCTAA AAAAGCAAAT AACATCAAAA TATATGGTTA TGGTGCATTG 600
   TCATCACCTG CCAAAAACCC AACCTACATG AATTATCAAC AAGAACAAAA CATCAAAAAC 660
   AAAAAACCAG GCGATGATTA TCAAAACATT CGTTTTGGCT ATATGGAGCT AAGAGAGCTG 720
   GACCTAAATA AAAAAGGTGC AGACACCCAG AGCGACAAGA ACCGTGCCAT CATTTTCACC 780
   ACACCTACTT TATTTTATCA TGGTGAGAAT GCCAGCACCC ATCTGCCAAA GGCGGGTAAA 840
   TTTGACTATG AGGGCAATTG GTTGTATCTG ACCGATGTCA AAAAACGCCC ATTTTTAGAT 900
   AAAACAGACG ATAAAGTAGG CACTTATTTT AACTCAACCA GAAAATCAAA TGAAGGCGAT 960
   TTGGTGAGTG CAGCACACAT TTATCTAAAC AGCTTTAAAT ATAAACACAC CCCGGCCACT 1020
   TATAGCGTGG ACTTTGATCA AAATACCCTA AAAGGCAAAT TGTCTTATTA TGACAACCCA 1080
   AACAAGCAAA CAGCCGATGG GCGTTATATC AGAAGTCAGT TTGATACCGA CAAAAAGGTC 1140
   AATGAAGCCG ATGTCTATGA GATTGACGCC AAGATTAATG GCAACCGCTT TACTGGCACA 1200
   GCCAAATCTT TGATTGATGA TAACACCAAT ACCGCACCTT TTGTTAAAGA GCTGTTCTCC 1260
   AAAAAAGCCA ATCCCAACAA CCCAGACCCC AACTCAGATA CGCTAGAAGG CGGGTTTTAT 1320
   GGTGAGTCGG GCGATGAGCT GGCGGGTAAA TTTTTATCCA ATGACAACGC AACTTTTGTG 1380
   GTCTTTGGTG GCAAACGAGA CAAAACGACC GAACCTGTCG CCACAAAAAC GGTGTATTTT 1440
   AGTACAGGAT TTGAAAAACC CAGCACCAGC TTTGTTGGCA ATGAAGAGAT TGGTAGCATT 1500
   ATTGACGGTA AAAAGTTAAA TGATGAAGTC AATAATCAAA TTGAAGATGA AACTGTCCCT 1560
   GTCAGTAATA AAGAATATTA TGAATATAAT TATGGACGAC CCAACAAACA ATTCACCAAA    1620
   AAAATAAACG CCAGCGTCCA AAAAAACCCT GCTTATTTTG GTCAGCATGA TAAGTTTTAT    1680
   TTTAATGGTA ACTATTATGA CTTATCAGCC AAAGAAGCAA ACAAGCTTGG TGTCTCCCAA    1740
   GATACCAGCA CCAATAAGAG TATTTTGGCT AAATACCCAG ATGCCAAAGT AAGCACAGAC    1800
   AATAAAGTTA CCAAAATCGT TCTACAACAA GCCAAAGATA AGCCGTATAC CGCCATTCAT    1860
   GCCAAAAGCT ATGACCACAT CAGTTTTGGT GAAGTATTGT ATAATGATAA CAAAGGCAAC    1920
   CCAACACGCA GTTATTTTGT GCAAGGCGGT CAAGCGGATG TCAGTACTCA GCTGCCCAGT    1980
   GCAGGTAAAT TCACCTATAA TGGTCTTTGG GCAGGCTACC TGACCCAGAA AAAAGACAAA    2040
   GGTTATAGCA AAGATGAGGA TACCATCAAG CAAAAAGGTC TTAAAGATTA TATATTGACC    2100
   AAAGACTTTA TCCCACAAGA TGACGATGAC GATGACGATA GTTTGACCGC ATCTGATGAT    2160
   TCACAAGATG ATAATACACA TGGCGATGAT GATTTGATTG CATCTGATGA TTCACAAGAT    2220
   GATGACACAG ATGGCGATGA CGATTCAGAT GATTTGGGTG ATGGTGCAGA TGATGACGCC    2280
   GCAGGCAAAG TGTATCATGC AGGTAATATT CGCCCTGAAT TTGAAAACAA ATACTTGCCC    2340
   ATTAATGAGC CTACTCATGA AAAAACCTTT GCCCTAGATG GTAAAAATAA GGCTAAGTTT    2400
   GATGTAAACT TTGACACCAA CAGCCTAACT GGTAAATTAA ACGATGAGAG AGGTGATATC    2460
   GTCTTTGATA TCAAAAATGG CAAAATTGAT GGCACAGGAT TTACCGCCAA AGCCGATGTG    2520
   CCAAACTATC GTGAAGAAGT GGGTAACAAC CAAGGTGGCG GTTTCTTATA CAACATCAAA    2580
   GATATTGATG TTAAGGGGCA ATTTTTTGGC ACAAATGGCG AAGAGTTGGC AGGACGGTTA    2640
   CATCATGACA AAGGCGATGG CATCACTGAC ACCGCCGAAA AAGCAGGGGC TGTCTTTGGG    2700
   GCTGTTAAAG ATAAATAA                                                  2718
(2) INFORMATION FOR SEQ ID NO: 70:
   (i) SEQUENCE CHARACTERISTICS:
      (A) LENGTH: 905 amino acids
      (B) TYPE: amino acid
      (C) STRANDEDNESS: single
      (D) TOPOLOGY: linear
   (xi) SEQUENCE DESCRIPTION: SEQ ID NO: 70:
   Met Ser Thr Val Lys Val Pro His Ile Phe Tyr Gln Lys Arg Thr Leu
   1               5                   10                  15
   Ser Leu Ala Ile Ala Ser Ile Phe Ala Ala Val Val Met Thr Gly Cys
               20                  25                  30
   Arg Ser Asp Asp Ile Ser Val Asn Ala Pro Asn Val Thr Gln Leu Pro
           35                  40                  45
   Gln Gly Thr Val Ser Pro Ile Pro Asn Thr Gly His Asp Asn Thr Asn
       50                  55                  60
   Asn Thr Asn Asn Gln Gly Asn Asn Thr Asp Asn Ser Thr Ser Thr Thr
   65 70 75 80
   Asp Pro Asn Gly Asp Asn Asn Gln Leu Thr Gln Ala Gln Lys Thr Ala
   85 90 95
   Ala Ala Ala Gly Phe Phe Val Met Gly Lys Ile Arg Asp Thr Ser Glu
   100 105 110
   Lys Asn Asp Pro Asp Tyr Thr Lys Asp Leu Gln Gly Ser Val His Thr
   115 120 125
   Ala Gly Gln Gly Leu Gln Tyr Leu Gly Thr Lys Glu Pro Arg Pro Asp
   130 135 140
   Gly Thr Gly Thr Gly Lys Asn Leu Arg Gln Pro Ile Thr Ala Asp Asp
   145 150 155 160
   Ile Thr Pro Leu Tyr Phe Asp Lys Phe Pro Lys Ile Ser Asp Leu His
   165 170 175
   Leu Glu Asn Ser Glu His Val Phe Asp Ala Lys Lys Ala Asn Asn Ile
   180 185 190
   Lys Ile Tyr Gly Tyr Gly Ala Leu Ser Ser Pro Ala Lys Asn Pro Thr
   195 200 205
   Tyr Met Asn Tyr Gln Gln Glu Gln Asn Ile Lys Asn Lys Lys Pro Gly
   210 215 220
   Asp Asp Tyr Gln Asn Ile Arg Phe Gly Tyr Met Glu Leu Arg Glu Leu
   225 230 235 240
   Asp Leu Asn Lys Lys Gly Ala Asp Asn Gln Ser Asp Lys Asn Arg Ala
   245 250 255
   Ile Ile Phe Thr Thr Pro Thr Leu Phe Tyr His Gly Glu Asn Ala Ser
   260 265 270
   Thr His Leu Pro Lys Ala Gly Lys Phe Asp Tyr Glu Gly Asn Trp Leu
   275 280 285
   Tyr Leu Thr Asp Val Lys Lys Arg Pro Phe Leu Asp Lys Thr Asp Asp
   290 295 300
   Lys Val Gly Thr Tyr Phe Asn Ser Thr Arg Lys Ser Asn Glu Gly Asp
   305 310 315 320
   Leu Val Ser Ala Ala His Ile Tyr Leu Asn Ser Phe Lys Tyr Lys His
   325 330 335
   Thr Pro Ala Thr Tyr Ser Val Asp Phe Asp Gln Asn Thr Leu Lys Gly
   340 345 350
   Lys Leu Ser Tyr Tyr Asp Asn Pro Asn Lys Gln Thr Ala Asp Gly Arg
   355 360 365
   Tyr Ile Arg Ser Gln Phe Asp Thr Asp Lys Lys Val Asn Glu Ala Asp
   370 375 380
   Val Tyr Glu Ile Asp Ala Lys Ile Asn Gly Asn Arg Phe Thr Gly Thr
   385 390 395 400
   Ala Lys Ser Leu Ile Asp Asp Asn Thr Asn Thr Ala Pro Phe Val Lys
                   405                 410                 415
   Glu Leu Phe Ser Lys Lys Ala Asn Pro Asn Asn Pro Asp Pro Asn Ser
               420                 425                 430
   Asp Thr Leu Glu Gly Gly Phe Tyr Gly Glu Ser Gly Asp Glu Leu Ala
           435                 440                 445
   Gly Lys Phe Leu Ser Asn Asp Asn Ala Thr Phe Val Val Phe Gly Gly
       450                 455                 460
   Lys Arg Asp Lys Thr Thr Glu Pro Val Ala Thr Lys Thr Val Tyr Phe
   465                 470                 475                 480
   Ser Thr Gly Phe Glu Lys Pro Ser Thr Ser Phe Val Gly Asn Glu Glu
                   485                 490                 495
   Ile Gly Ser Ile Ile Asp Gly Lys Gly Leu Asn Asp Glu Val Asn Asn
               500                 505                 510
   Gln Ile Glu Asp Glu Thr Val Pro Val Ser Asn Lys Glu Tyr Tyr Glu
           515                 520                 525
   Tyr Asn Tyr Gly Arg Pro Asn Lys Gln Phe Thr Lys Lys Ile Asn Ala
       530                 535                 540
   Ser Val Gln Lys Asn Pro Ala Tyr Phe Gly Gln His Asp Lys Phe Tyr
   545                 550                 555                 560
   Phe Asn Gly Asn Tyr Tyr Asp Leu Ser Ala Lys Glu Ala Asn Lys Leu
                   565                 570                 575
   Gly Val Ser Gln Asp Thr Ser Thr Asn Lys Ser Ile Leu Ala Lys Tyr
               580                 585                 590
   Pro Asp Ala Lys Val Ser Thr Asp Asn Lys Val Thr Lys Ile Val Leu
           595                 600                 605
   Gln Gln Ala Lys Asp Lys Pro Tyr Thr Ala Ile His Ala Lys Ser Tyr
       610                 615                 620
   Asp His Ile Ser Phe Gly Glu Val Leu Tyr Asn Asp Asn Lys Gly Asn
   625                 630                 635                 640
   Pro Thr Arg Ser Tyr Phe Val Gln Gly Gly Gln Ala Asp Val Ser Thr
                   645                 650                 655
   Gln Leu Pro Ser Ala Gly Lys Phe Thr Tyr Asn Gly Leu Trp Ala Gly
               660                 665                 670
   Tyr Leu Thr Gln Lys Lys Asp Lys Gly Tyr Ser Lys Asp Glu Asp Thr
           675                 680                 685
   Ile Lys Gln Lys Gly Leu Lys Asp Tyr Ile Leu Thr Lys Asp Phe Ile
       690                 695                 700
   Pro Gln Asp Asp Asp Asp Asp Asp Asp Ser Leu Thr Ala Ser Asp Asp
   705                 710                 715                 720
   Ser Gln Asp Asp Asn Thr His Gly Asp Asp Asp Leu Ile Ala Ser Asp
                   725                 730                 735
   Asp Ser Gln Asp Asp Asp Thr Asp Gly Asp Asp Asp Ser Asp Asp Leu
   740 745 750
   Gly Asp Gly Ala Asp Asp Asp Ala Ala Gly Lys Val Tyr His Ala Gly
   755 760 765
   Asn Ile Arg Pro Glu Phe Glu Asn Lys Tyr Leu Pro Ile Asn Glu Pro
   770 775 780
   Thr His Glu Lys Thr Phe Ala Leu Asp Gly Lys Asn Lys Ala Lys Phe
   785 790 795 800
   Asp Val Asn Phe Asp Thr Asn Ser Leu Thr Gly Lys Leu Asn Asp Glu
   805 810 815
   Arg Gly Asp Ile Val Phe Asp Ile Lys Asn Gly Lys Ile Asp Gly Thr
   820 825 830
   Gly Phe Thr Ala Lys Ala Asp Val Pro Asn Tyr Arg Glu Glu Val Gly
   835 840 845
   Asn Asn Gln Gly Gly Gly Phe Leu Tyr Asn Ile Lys Asp Ile Asp Val
   850 855 860
   Lys Gly Arg Phe Phe Gly Thr Asn Gly Glu Glu Leu Ala Gly Gln Leu
   865 870 875 880
   His His Asp Lys Gly Asp Gly Ile Thr Asp Thr Ala Glu Lys Ala Gly
   885 890 895
   Ala Val Phe Gly Ala Val Lys Asp Lys
   900 905
(2) INFORMATION FOR SEQ ID NO: 71:
   (i) SEQUENCE CHARACTERISTICS:
      (A) LENGTH: 7 amino acids
      (B) TYPE: amino acid
      (C) STRANDEDNESS: single
      (D) TOPOLOGY: linear
   (xi) SEQUENCE DESCRIPTION: SEQ ID NO: 71:
   Leu Glu Gly Gly Phe Tyr Gly
   1 5
(2) INFORMATION FOR SEQ ID NO: 72:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (xi) SEQUENCE DESCRIPTION: SEQ ID NO: 72:
   Gly Lys Asn Leu Arg Gly Pro Ile
   1               5
(2) INFORMATION FOR SEQ ID NO: 73:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO: 73:
      GGTAAAAACT TGCGTCAGCC CATC 24
(2) INFORMATION FOR SEQ ID NO: 74:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO: 74:
      CCATTTTTGA ACGCAGTCGG GTAG 24
(2) INFORMATION FOR SEQ ID NO: 75:
   (i) SEQUENCE CHARACTERISTICS:
      (A) LENGTH: 946 amino acids
      (B) TYPE: amino acid
      (C) STRANDEDNESS: single
      (D) TOPOLOGY: linear
   (xi) SEQUENCE DESCRIPTION: SEQ ID NO: 75:
   Met Asn Lys Lys His Gly Phe Pro Leu Thr Leu Thr Ala Leu Ala Ile
   1               5                   10                  15
   Ala Thr Ala Phe Pro Ala Tyr Ala Ala Gln Ala Gly Ala Ala Ala Leu
               20                  25                  30
   Asp Ala Ala Gln Ser Gln Ser Leu Lys Glu Val Thr Val Arg Ala Ala
           35                  40                  45
   Lys Val Gly Arg Arg Ser Lys Glu Ala Thr Gly Leu Gly Lys Ile Val
       50                  55                  60
   Lys Thr Ser Glu Thr Leu Asn Lys Glu Gln Val Leu Gly Ile Arg Asp
   65                  70                  75                  80
   Leu Thr Arg Tyr Asp Pro Gly Val Ala Val Val Glu Gln Gly Asn Gly
                   85                  90                  95
   Ala Ser Gly Gly Tyr Ser Ile Arg Gly Val Asp Lys Asn Arg Val Ala
               100                 105                 110
   Val Ser Val Asp Gly Val Ala Gln Ile Gln Ala Phe Thr Val Gln Gly
   115 120 125
   Ser Leu Ser Gly Tyr Gly Gly Arg Gly Gly Ser Gly Ala Ile Asn Glu
   130 135 140
   Ile Glu Tyr Glu Asn Ile Ser Thr Val Glu Ile Asp Lys Gly Ala Gly
   145 150 155 160
   Ser Ser Asp His Gly Ser Gly Ala Leu Gly Gly Ala Val Ala Phe Arg
   165 170 175
   Thr Lys Glu Ala Ala Asp Leu Ile Ser Asp Gly Lys Ser Trp Gly Ile
   180 185 190
   Gln Ala Lys Thr Ala Tyr Gly Ser Lys Asn Arg Gln Phe Met Lys Ser
   195 200 205
   Leu Gly Ala Gly Phe Ser Lys Asp Gly Trp Glu Gly Leu Leu Ile Arg
   210 215 220
   Thr Glu Arg Gln Gly Arg Glu Thr Arg Pro His Gly Asp Ile Ala Asp
   225 230 235 240
   Gly Val Ala Tyr Gly Ile Asp Arg Leu Asp Ala Phe Arg Gln Thr Tyr
   245 250 255
   Asp Ile Lys Arg Lys Thr Thr Lys Pro Ala Phe Leu Ala Glu Gly Glu
   260 265 270
   Asn Thr Leu Lys Pro Val Ala Lys Leu Ala Gly Tyr Gly Ile Tyr Leu
   275 280 285
   Asn Arg Gln Leu Asn Arg Trp Val Lys Glu Arg Ile Glu Gln Asn Gln
   290 295 300
   Pro Leu Ser Ala Glu Glu Glu Ala Gln Val Arg Glu Ala Gln Ala Arg
   305 310 315 320
   His Glu Asn Leu Ser Ala Gln Ala Tyr Thr Gly Gly Gly Arg Ile Leu
   325 330 335
   Pro Asp Pro Met Asp Tyr Arg Ser Gly Ser Trp Leu Ala Lys Leu Gly
   340 345 350
   Tyr Arg Phe Gly Gly Arg His Tyr Val Gly Gly Val Phe Glu Asp Thr
   355 360 365
   Lys Gln Arg Tyr Asp Ile Arg Asp Met Thr Glu Lys Gln Tyr Tyr Gly
   370 375 380
   Thr Asp Glu Ala Glu Lys Phe Arg Asp Lys Ser Gly Val Tyr Asp Gly
   385 390 395 400
   Asp Asp Phe Arg Asp Gly Leu Tyr Phe Val Pro Asn Ile Glu Glu Trp
   405 410 415
   Lys Gly Asp Lys Asn Leu Val Lys Gly Ile Gly Leu Lys Tyr Ser Arg
   420 425 430
   Thr Lys Phe Ile Asp Glu His His Arg Arg Arg Arg Met Gly Leu Leu
   435 440 445
   Tyr Arg Tyr Glu Asn Glu Lys Tyr Ser Asp Asn Trp Ala Asp Lys Ala
       450                 455                 460
   Val Leu Ser Phe Asp Lys Gln Gly Val Ala Thr Asp Asn Asn Thr Leu
   465                 470                 475                 480
   Lys Leu Asn Cys Ala Val Tyr Pro Ala Val Asp Lys Ala Cys Arg Ala
                   485                 490                 495
   Ser Ala Asp Lys Pro Tyr Ser Tyr Asp Ser Ser Asp Arg Phe His Tyr
               500                 505                 510
   Arg Glu Gln His Asn Val Leu Asn Ala Ser Phe Glu Lys Ser Leu Lys
           515                 520                 525
   Asn Lys Trp Thr Lys His His Leu Thr Leu Gly Phe Gly Tyr Asp Ala
       530                 535                 540
   Ser Asn Ala Ile Ser Arg Pro Glu Gln Leu Ser His Asn Ala Ala Arg
   545                 550                 555                 560
   Ile Ser Glu Ser Thr Gly Phe Asp Glu Lys Asn Gln Asp Lys Tyr Arg
                   565                 570                 575
   Leu Gly Lys Pro Glu Val Val Glu Gly Ser Val Cys Gly Tyr Ile Glu
               580                 585                 590
   Thr Leu Arg Ser Arg Lys Cys Val Pro Arg Lys Ile Asn Gly Ser Asn
           595                 600                 605
   Ile His Ile Ser Leu Asn Asp Arg Phe Ser Ile Gly Lys Tyr Phe Asp
       610                 615                 620
   Phe Ser Leu Gly Gly Arg Tyr Asp Arg Lys Asn Phe Thr Thr Ser Glu
   625                 630                 635                 640
   Glu Leu Val Arg Ser Gly Arg Tyr Ala Asp Arg Ser Trp Asn Ser Gly
                   645                 650                 655
   Ile Val Phe Lys Pro Asn Arg His Phe Ser Val Ser Tyr Arg Ala Ser
               660                 665                 670
   Ser Gly Phe Arg Thr Pro Ser Phe Gln Glu Leu Phe Gly Ile Asp Ile
           675                 680                 685
   Tyr His Asp Tyr Pro Lys Gly Trp Gln Arg Pro Ala Leu Lys Ser Glu
       690                 695                 700
   Lys Ala Ala Asn Arg Glu Ile Gly Leu Gln Trp Lys Gly Asp Phe Gly
   705                 710                 715                 720
   Phe Leu Glu Ile Ser Ser Phe Arg Asn Arg Tyr Thr Asp Met Ile Ala
                   725                 730                 735
   Val Ala Asp Gln Lys Thr Lys Leu Pro Asp Ser Ala Gly Arg Leu Thr
               740                 745                 750
   Glu Ile Asp Ile Arg Asp Tyr Tyr Gly Asn Pro Asn Ala Gln Asn Met
           755                 760                 765
   Ser Leu Gln Gly Ile Asn Ile Leu Gly Lys Ile Asp Trp Asn Gly Val
       770                 775                 780
   Tyr Gly Lys Leu Pro Glu Gly Leu Tyr Thr Thr Leu Ala Tyr Asn Arg
   785                 790                 795                 800
   Ile Lys Pro Lys Ser Val Ser Asn Arg Pro Asp Leu Ser Leu Arg Ser
                   805                 810                 815
   Tyr Ala Leu Asp Ala Val Gln Pro Ser Arg Tyr Val Leu Gly Phe Gly
               820                 825                 830
   Tyr Asp Gln Pro Glu Gly Lys Trp Gly Ala Asn Ile Met Leu Thr Tyr
           835                 840                 845
   Ser Lys Gly Lys Asn Pro Asp Glu Leu Ala Tyr Leu Ala Gly Asp Gln
       850                 855                 860
   Lys Arg Tyr Ser Ala Gly Arg Val Thr Ser Ser Trp Lys Thr Ala Asp
   865                 870                 875                 880
   Val Ser Ala Tyr Leu Asn Leu Lys Lys Arg Leu Thr Leu Arg Ala Ala
                   885                 890                 895
   Ile Tyr Asn Ile Gly Asn Tyr Arg Tyr Val Thr Trp Glu Ser Leu Arg
               900                 905                 910
   Gln Thr Ala Glu Ser Thr Ala Asn Arg His Gly Gly Asp Ser Asn Tyr
           915                 920                 925
   Gly Arg Tyr Ala Ala Pro Gly Arg Asn Phe Ser Leu Ala Leu Glu Met
       930                 935                 940
   Lys Phe
   945
(2) INFORMATION FOR SEQ ID NO: 76:
   (i) SEQUENCE CHARACTERISTICS:
      (A) LENGTH: 946 amino acids
      (B) TYPE: amino acid
      (C) STRANDEDNESS: single
      (D) TOPOLOGY: linear
   (xi) SEQUENCE DESCRIPTION: SEQ ID NO: 76:
   Met Asn Lys Lys His Gly Phe Gln Leu Thr Leu Thr Ala Leu Ala Val
   1               5                   10                  15
   Ala Ala Ala Phe Pro Ser Tyr Ala Ala Asn Pro Glu Thr Ala Ala Pro
               20                  25                  30
   Asp Ala Ala Gln Thr Gln Ser Leu Lys Glu Val Thr Val Arg Ala Ala
           35                  40                  45
   Lys Val Gly Arg Arg Ser Lys Glu Ala Thr Gly Leu Gly Lys Ile Ala
       50                  55                  60
   Lys Thr Ser Glu Thr Leu Asn Lys Glu Gln Val Leu Gly Ile Arg Asp
   65                  70                  75                  80
   Leu Thr Arg Tyr Asp Pro Gly Val Ala Val Val Glu Gln Gly Asn Gly
                   85                  90                  95
   Ala Ser Gly Gly Tyr Ser Ile Arg Gly Val Asp Lys Asn Arg Val Ala
   100 105 110
   Val Ser Val Asp Gly Val Ala Gln Ile Gln Ala Phe Thr Val Gln Gly
   115 120 125
   Ser Leu Ser Gly Tyr Gly Gly Arg Gly Gly Ser Gly Ala Ile Asn Glu
   130 135 140
   Ile Glu Tyr Glu Asn Ile Ser Thr Val Glu Ile Asp Lys Gly Ala Gly
   145 150 155 160
   Ser Ser Asp His Gly Ser Gly Ala Leu Gly Gly Ala Val Ala Phe Arg
   165 170 175
   Thr Lys Glu Ala Ala Asp Leu Ile Ser Asp Gly Lys Ser Trp Gly Ile
   180 185 190
   Gln Ala Lys Thr Ala Tyr Gly Ser Lys Asn Arg Gln Phe Met Lys Ser
   195 200 205
   Leu Gly Ala Gly Phe Ser Lys Asp Gly Trp Glu Gly Leu Leu Ile Arg
   210 215 220
   Thr Glu Arg Gln Gly Arg Glu Thr His Pro His Gly Asp Ile Ala Asp
   225 230 235 240
   Gly Val Ala Tyr Gly Ile Asn Arg Leu Asp Ala Phe Arg Gln Thr Tyr
   245 250 255
   Gly Ile Lys Lys Pro Ser Glu Gly Gly Glu Tyr Phe Leu Ala Glu Gly
   260 265 270
   Glu Ser Glu Leu Lys Pro Val Ala Lys Val Ala Gly Asn Gly Asn Tyr
   275 280 285
   Leu Asn Asn Gln Leu Asn Arg Trp Val Lys Glu Arg Ile Glu Gln Asn
   290 295 300
   Gln Pro Leu Ser Ala Glu Glu Glu Ala Met Val Arg Glu Ala Gln Ala
   305 310 315 320
   Arg His Glu Asn Leu Ser Ala Gln Ala Tyr Thr Gly Gly Gly Arg Ile
   325 330 335
   Leu Pro Asp Pro Met Asp Tyr Arg Ser Gly Ser Trp Leu Ala Lys Leu
   340 345 350
   Gly Tyr Arg Phe Gly Gly Arg His Tyr Val Gly Gly Val Phe Glu Asp
   355 360 365
   Thr Lys Gln Arg Tyr Asp Ile Arg Asp Met Thr Glu Lys Gln Tyr Tyr
   370 375 380
   Gly Thr Asp Glu Ala Lys Lys Phe Arg Asp Lys Ser Gly Val Tyr Asp
   385 390 395 400
   Gly Asp Asp Phe Arg Asp Gly Leu Tyr Phe Val Pro Asn Ile Glu Glu
   405 410 415
   Trp Lys Gly Asp Gln Lys Leu Ile Arg Gly Ile Gly Leu Lys Tyr Ser
   420 425 430
   Arg Thr Lys Phe Ile Asp Glu His His Arg Arg Arg Arg Met Gly Leu
   435 440 445
   Leu Tyr Arg Tyr Glu Asn Glu Lys Tyr Ser Asp Asn Trp Ala Asp Lys
   450 455 460
   Ala Val Leu Ser Phe Asp Lys Gln Gly Val Ala Thr Asp Asn Asn Thr
   465 470 475 480
   Leu Lys Leu Asn Cys Ala Val Tyr Pro Ala Val Asp Lys Ser Cys Arg
   485 490 495
   Ala Ser Ala Asp Lys Pro Tyr Ser Tyr Asp Ser Ser Asp Arg Phe His
   500 505 510
   Tyr Arg Glu Gln His Asn Val Leu Asn Ala Ser Phe Glu Lys Ser Leu
   515 520 525
   Lys Asn Lys Trp Thr Lys His His Leu Thr Leu Gly Phe Gly Tyr Asp
   530 535 540
   Ala Ser Asn Ala Ile Ser Arg Pro Glu Gln Leu Ser His Asn Ala Ala
   545 550 555 560
   Arg Ile Ser Glu Tyr Ser Asp Tyr Thr Asp Lys Gly Asp Lys Tyr Leu
   565 570 575
   Leu Gly Lys Pro Glu Val Val Glu Gly Ser Val Cys Gly Tyr Ile Glu
   580 585 590
   Thr Leu Arg Ser Arg Lys Cys Val Pro Arg Lys Ile Asn Gly Ser Asn
   595 600 605
   Ile His Ile Ser Leu Asn Asp Arg Phe Ser Ile Gly Lys Tyr Phe Asp
   610 615 620
   Phe Ser Leu Gly Gly Arg Tyr Asp Arg Lys Asn Phe Thr Thr Ser Glu
   625 630 635 640
   Glu Leu Val Arg Ser Gly Arg Tyr Val Asp Arg Ser Trp Asn Ser Gly
   645 650 655
   Ile Val Phe Lys Pro Asn Arg His Phe Ser Leu Ser Tyr Arg Ala Ser
   660 665 670
   Ser Gly Phe Arg Thr Pro Ser Phe Gln Glu Leu Phe Gly Ile Asp Ile
   675 680 685
   Tyr His Asp Tyr Pro Lys Gly Trp Gln Arg Pro Ala Leu Lys Ser Glu
   690 695 700
   Lys Ala Ala Asn Arg Glu Ile Gly Leu Gln Trp Lys Gly Asp Phe Gly
   705 710 715 720
   Phe Leu Glu Ile Ser Ser Phe Arg Asn Arg Tyr Thr Asp Met Ile Ala
   725 730 735
   Val Ala Asp His Lys Thr Lys Leu Pro Asn Gln Ala Gly Gln Leu Thr
   740 745 750
   Glu Ile Asp Ile Arg Asp Tyr Tyr Gly Asn Pro Asn Ala Gln Asn Met
   755 760 765
   Ser Leu Gln Gly Val Asn Ile Leu Gly Lys Ile Asp Trp Asn Gly Val
       770                 775                 780
   Tyr Gly Lys Leu Pro Glu Gly Leu Tyr Thr Thr Leu Ala Tyr Asn Arg
   785                 790                 795                 800
   Ile Lys Pro Lys Ser Val Ser Asn Arg Pro Gly Leu Ser Leu Arg Ser
                   805                 810                 815
   Tyr Ala Leu Asp Ala Val Gln Pro Ser Arg Tyr Val Leu Gly Phe Gly
               820                 825                 830
   Tyr Asp Gln Pro Glu Gly Lys Trp Gly Ala Asn Ile Met Leu Thr Tyr
           835                 840                 845
   Ser Lys Gly Lys Asn Pro Asp Glu Leu Ala Tyr Leu Ala Gly Asp Gln
       850                 855                 860
   Lys Arg Tyr Ser Thr Lys Arg Ala Ser Ser Ser Trp Ser Thr Ala Asp
   865                 870                 875                 880
   Val Ser Ala Tyr Leu Asn Leu Lys Lys Arg Leu Thr Leu Arg Ala Ala
                   885                 890                 895
   Ile Tyr Asn Ile Gly Asn Tyr Arg Tyr Val Thr Trp Glu Ser Leu Arg
               900                 905                 910
   Gln Thr Ala Glu Ser Thr Ala Asn Arg His Gly Gly Asp Ser Asn Tyr
           915                 920                 925
   Gly Arg Tyr Ala Ala Pro Gly Arg Asn Phe Ser Leu Ala Leu Glu Met
       930                 935                 940
   Lys Phe
   945
(2) INFORMATION FOR SEQ ID NO: 77:
   (i) SEQUENCE CHARACTERISTICS:
      (A) LENGTH: 943 amino acids
      (B) TYPE: amino acid
      (C) STRANDEDNESS: single
      (D) TOPOLOGY: linear
   (xi) SEQUENCE DESCRIPTION: SEQ ID NO: 77:
   Met Asn Lys Lys His Ser Phe Pro Leu Thr Leu Thr Ala Leu Ala Ile
   1               5                   10                  15
   Ala Thr Ala Phe Pro Ser Tyr Ala Ala Asn Ser Glu Thr Ala Ala Gln
               20                  25                  30
   Thr Gln Ser Leu Lys Glu Val Thr Val Arg Ala Ala Lys Val Gly Arg
           35                  40                  45
   Arg Ser Lys Glu Val Thr Gly Leu Gly Lys Ile Val Lys Thr Ser Glu
       50                  55                  60
   Thr Leu Asn Lys Glu Gln Val Leu Gly Ile Arg Asp Leu Thr Arg Tyr
   65                  70                  75                  80
   Asp Pro Gly Val Ala Val Val Glu Gln Gly Asn Gly Ala Ser Gly Gly
   85 90 95
   Tyr Ser Ile Arg Gly Val Asp Lys Asn Arg Val Ala Val Ser Val Asp
   100 105 110
   Gly Val Ala Gln Ile Gln Ala Phe Thr Val Gln Gly Ser Leu Ser Gly
   115 120 125
   Tyr Gly Gly Arg Gly Gly Ser Gly Ala Ile Asn Glu Ile Glu Tyr Glu
   130 135 140
   Asn Ile Ser Thr Val Glu Ile Asp Lys Gly Ala Gly Ser Ser Asp His
   145 150 155 160
   Gly Ser Gly Ala Leu Gly Gly Ala Val Ala Phe Arg Thr Lys Glu Ala
   165 170 175
   Ala Asp Leu Ile Ser Asp Gly Lys Ser Trp Gly Ile Gln Ala Lys Thr
   180 185 190
   Ala Tyr Gly Ser Lys Asn Arg Gln Phe Met Lys Ser Leu Gly Ala Gly
   195 200 205
   Phe Ser Lys Asp Gly Trp Glu Gly Leu Leu Ile Arg Thr Glu Arg Gln
   210 215 220
   Gly Arg Glu Thr Arg Pro His Gly Asp Ile Ala Asp Gly Val Ala Tyr
   225 230 235 240
   Gly Ile Asp Arg Leu Asp Ala Phe Arg Gln Thr Tyr Asp Ile Gln Lys
   245 250 255
   Gln Asn Lys Lys Ala Glu Tyr Phe Leu Ala Glu Gly Glu Ser Glu Leu
   260 265 270
   Lys Pro Ala Ala Lys Leu Ala Gly Asn Gly Asn Tyr Leu Asn Asn Gln
   275 280 285
   Leu Asn Arg Trp Val Glu Glu Arg Lys Lys Asn Asn Gln Ser Leu Ser
   290 295 300
   Ala Glu Glu Glu Ala Met Val Arg Glu Ala Gln Ala Arg His Glu Asn
   305 310 315 320
   Leu Ser Ala Gln Ala Tyr Thr Gly Gly Gly Arg Ile Leu Pro Asp Pro
   325 330 335
   Met Asp Tyr Arg Ser Gly Ser Trp Leu Ala Lys Leu Gly Tyr Arg Phe
   340 345 350
   Gly Gly Arg His Tyr Val Gly Gly Val Phe Glu Asp Thr Lys Gln Arg
   355 360 365
   Tyr Asp Ile Arg Asp Met Thr Glu Lys Gln Tyr Tyr Gly Thr Asp Glu
   370 375 380
   Ala Thr Lys Phe Ser Asp Lys Ser Gly Val Tyr Asp Gly Asp Asp Phe
   385 390 395 400
   Arg Asp Gly Leu Tyr Phe Val Pro Asn Ile Glu Glu Trp Lys Gly Asp
   405 410 415
   Lys Asn Leu Val Lys Gly Ile Gly Leu Lys Tyr Ser Arg Thr Lys Phe
               420                 425                 430
   Ile Asp Glu His His Arg Arg Arg Arg Met Gly Leu Leu Tyr Arg Tyr
           435                 440                 445
   Glu Asn Glu Ala Tyr Ser Asp Asn Trp Ala Asp Lys Ala Val Leu Ser
       450                 455                 460
   Phe Asp Lys Gln Gly Val Ala Thr Asp Asn Asn Thr Leu Lys Leu Asn
   465                 470                 475                 480
   Cys Ala Val Tyr Pro Ala Val Asp Lys Ala Cys Arg Ala Ser Ala Asp
                   485                 490                 495
   Lys Pro Tyr Ser Tyr Asp Ser Ser Asp Arg Phe His Tyr Arg Glu Gln
               500                 505                 510
   His Asn Val Leu Asn Ala Leu Phe Glu Lys Ser Leu Lys Asn Lys Trp
           515                 520                 525
   Thr Lys His His Leu Thr Leu Gly Phe Gly Tyr Asp Ala Ser Asn Ala
       530                 535                 540
   Ile Ser Arg Pro Glu Gln Leu Ser His Asn Ala Ala Arg Ile Ser Glu
   545                 550                 555                 560
   Phe Ser Asp Tyr Ala Asp Asp Gly Lys Tyr Lys Tyr Leu Arg Leu Lys
                   565                 570                 575
   Pro Glu Val Val Glu Gly Ser Val Cys Gly Tyr Ile Glu Thr Leu Arg
               580                 585                 590
   Ser Arg Lys Cys Val Pro Arg Lys Ile Asn Gly Ser Asn Ile His Ile
           595                 600                 605
   Ser Leu Asn Asp Arg Phe Ser Ile Gly Lys Tyr Phe Asp Phe Ser Leu
       610                 615                 620
   Gly Gly Arg Tyr Asp Arg Gln Asn Phe Thr Thr Ser Glu Glu Leu Val
   625                 630                 635                 640
   Arg Ser Gly Arg Tyr Thr Asp Arg Ser Trp Asn Ser Gly Ile Val Phe
                   645                 650                 655
   Lys Pro Ser Arg His Leu Ser Leu Ser Tyr Arg Ala Ser Ser Gly Phe
               660                 665                 670
   Arg Thr Pro Ser Phe Gln Glu Leu Phe Gly Ile Asp Ile Tyr His Asp
           675                 680                 685
   Tyr Pro Lys Gly Trp Gln Arg Pro Ala Leu Lys Ser Glu Lys Ala Ala
       690                 695                 700
   Asn Arg Glu Ile Gly Leu Gln Trp Lys Gly Asp Phe Gly Phe Leu Glu
   705                 710                 715                 720
   Ile Ser Ser Phe Arg Asn Arg Tyr Thr Asp Met Ile Ala Val Ala Asp
                   725                 730                 735
   His Lys Thr Lys Leu Pro Asn Gln Ala Gly Arg Leu Thr Glu Ile Asp
               740                 745                 750
   Ile Arg Asp Tyr Tyr Gly Asn Pro Asn Ala Gln Asn Met Ser Leu Gln
           755                 760                 765
   Gly Val Asn Ile Leu Gly Lys Ile Asp Trp Asn Gly Val Tyr Gly Lys
       770                 775                 780
   Leu Pro Glu Gly Leu Tyr Thr Thr Leu Ala Tyr Asn Arg Ile Lys Pro
   785                 790                 795                 800
   Lys Ser Val Ser Asn Arg Pro Asp Leu Ser Leu Arg Ser Tyr Ala Leu
                   805                 810                 815
   Asp Ala Gly Gln Pro Ser Arg Tyr Val Leu Gly Phe Gly Tyr Asp Gln
               820                 825                 830
   Pro Glu Gly Lys Trp Gly Ala Asn Ile Met Leu Thr Tyr Ser Lys Gly
           835                 840                 845
   Lys Asn Pro Asp Glu Leu Ala Tyr Leu Ala Gly Asp Gln Lys Arg Tyr
       850                 855                 860
   Ser Thr Lys Arg Ala Ser Ser Ser Trp Ser Thr Ala Asp Val Ser Ala
   865                 870                 875                 880
   Tyr Leu Asn Leu Lys Lys Arg Leu Thr Leu Arg Ala Ala Ile Tyr Asn
                   885                 890                 895
   Ile Gly Asn Tyr Arg Tyr Val Thr Trp Glu Ser Leu Arg Gln Thr Ala
               900                 905                 910
   Glu Ser Thr Ala Asn Arg His Gly Gly Asp Ser Asn Tyr Gly Arg Tyr
           915                 920                 925
   Ala Ala Pro Gly Arg Asn Phe Ser Leu Ala Leu Glu Met Lys Phe
       930                 935                 940

## Description

The present invention relates to (i) purified and isolated nucleic acid molecules encoding lactoferrin receptor protein 2 (Lbp2) of *Moraxella catarrhalis* Q8, (ii) immunogenic compositions comprising such nucleic acid, (iii) expression vectors comprising such a nucleic acid molecule for transformation of a host cell transformed in culture, (iv) methods for preparing such receptor protein as a substantially pure recombinant lactoferrin receptor protein, (v) methods of determining the presence, in a sample, of nucleic acid encoding the aforesaid Lbp2 and (vi) diagnostic kits for determining the presence, in a sample, of nucleic acid encoding the aforesaid Lbp2.

*Moraxella (Branhamella) catarrhalis* bacteria are Gram-negative diplococcal pathogens which are carried asymptomatically in the healthy human respiratory tract. However, in recent years, *M. catarrhalis* has been recognized as an important causative agent of otitis media. In addition, *M. catarrhalis* has been associated with sinusitis, conjunctivitis, and urogenital infections, as well as with a number of inflammatory diseases of the lower respiratory tract in children and adults, including pneumonia, chronic bronchitis, tracheitis, and emphysema (refs. 1 to 8). (Throughout this application, various references are cited in parentheses to describe more fully the state of the art to which this invention pertains. Full bibliographic information for each citation is found at the end of the specification, immediately preceding the claims). Occasionally, *M. catarrhalis* invades to cause septicaemia, arthritis, endocarditis, and meningitis (refs. 9 to 13).

*M. catarrhalis* colonizes the human upper respiratory tract and is an important cause of otitis media in infants and children as well as lower respiratory tract infections in adults with chronic obstructive pulmonary disease.

Otitis media is one of the most common illnesses of early childhood and approximately 80% of all children suffer at least one middle ear infection before the age of three (ref. 14). Chronic otitis media has been associated with auditory and speech impairment in children, and in some cases, has been associated with learning disabilities. Conventional treatments for otitis media include antibiotic administration and surgical procedures, including tonsillectomies, adenoidectomies, and tympanocentesis. In the United States, treatment costs for otitis media are estimated to be between one and two billion dollars per year.

In otitis media cases, *M. catarrhalis* is commonly co-isolated from middle ear fluid along with *Streptococcus pneumoniae* and non-typable *Haemophilus influenzae,* which are believed to be responsible for 50% and 30% of otitis media infections, respectively. *M. catarrhalis* is believed to be responsible for approximately 20% of otitis media infections (ref. 15). Epidemiological reports indicate that the number of cases of otitis media attributable to *M. catarrhalis* is increasing, along with the number of antibioticresistant isolates of *M. catarrhalis.* Thus, prior to 1970, no β-lactamase-producing *M. catarrhalis* isolates had been reported, but since the mid-seventies, an increasing number of β-lactamase-expressing isolates have been detected. Recent surveys suggest that up to 80 to 85% of clinical isolates produce β-lactamase (ref. 16, 22, 23).

Iron-restriction is a general host defence mechanism against microbial pathogens. A number of bacterial species including *Neisseria meningitidis* (ref. 17, 24), *N. gonorrhoeae* (ref. 25) and *M. catarrhalis* (ref. 17), express outer membrane proteins which specifically bind human lactoferrin.

*M. catarrhalis* infection may lead to serious disease. It would be advantageous to provide a recombinant source of lactoferrin binding proteins as antigens in immunogenic preparations including vaccines, carriers for other antigens and immunogens and the generation of diagnostic reagents. The genes encoding lactoferrin binding proteins and fragments thereof are particularly desirable and useful in the specific identification and diagnosis of *Moraxella* and for immunization against disease caused by *M. catarrhalis* and for the generation of diagnostic reagents.

The present invention is directed towards the provision of purified and isolated nucleic acid molecules encoding a lactoferrin receptor protein 2 (Lbp2) of *Moraxella catarrhalis Q8*. The nucleic acid molecules and isolated and purified lactoferrin binding proteins provided herein are useful for the specific detection of strains of *Moraxella* and for diagnosis of infection by *Moraxella.* The purified and isolated nucleic acid molecules provided herein, such as DNA. are also useful for expressing the *lbp* gene encoding Lbp2 of *Moraxella catarrhalis* Q8 by recombinant DNA means for providing, in an economical manner, purified and isolated lactoferrin receptor protein 2 free of other *Moraxella* proteins.

The lactoferrin receptor, subunits or fragments thereof, as well as nucleic acid molecules encoding the same and vectors containing such nucleic acid molecules, are useful in immunogenic compositions for vaccinating against diseases caused by *Moraxella,* the diagnosis of infection by *Moraxella,* and as tools for the generation of immunological reagents.

Monoclonal antibodies or mono-specific antisera (antibodies) raised against the lactoferrin receptor protein produced in accordance with aspects of the present invention are useful for the diagnosis of infection by *Moraxella,* the specific detection of *Moraxella* (in, for example, *in vitro* and *in vivo* assays) and for the treatment of diseases caused by *Moraxella.*

In accordance with one aspect of the present invention, there is provided a purified and isolated nucleic acid molecule encoding a lactoferrin receptor protein 2 (Lbp2) of *Moraxella catarrhalis Q8.* A fragment of the lactoferrin receptor protein is a portion of the protein which retains the immunological properties of the protein.

A nucleic acid molecule according to the invention encodes the Lbp2 protein of *Moraxella catarrhalis Q8.*

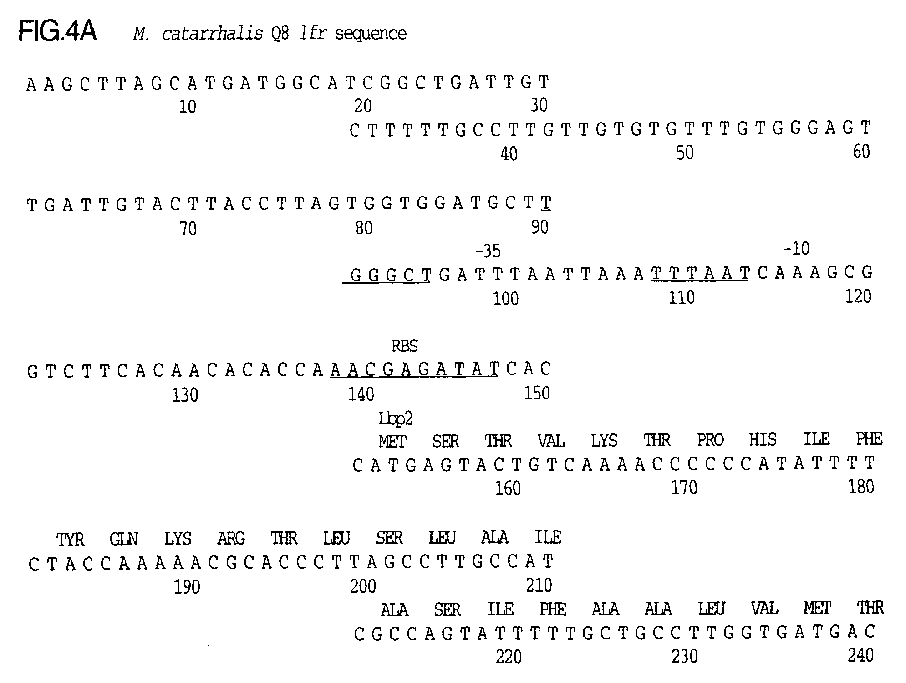

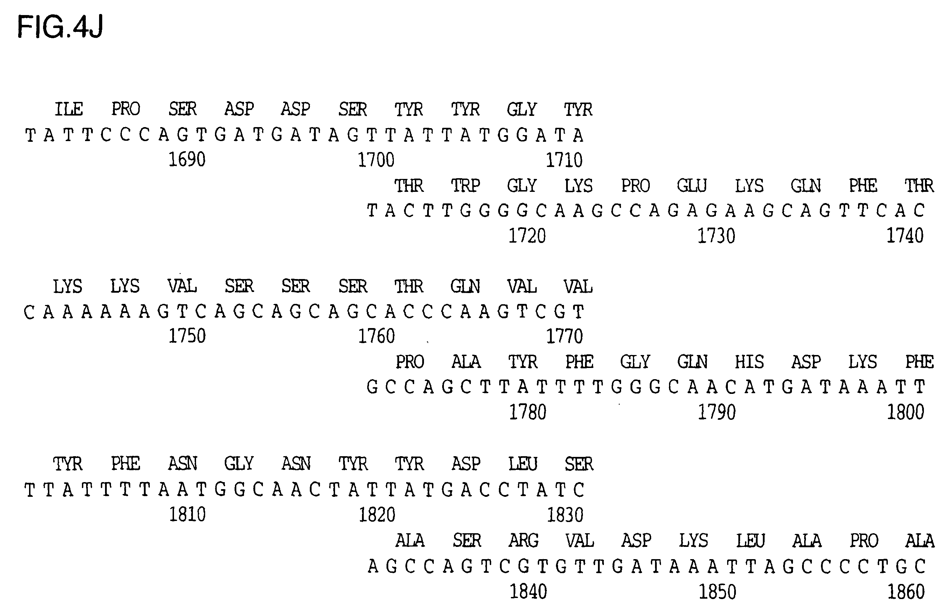

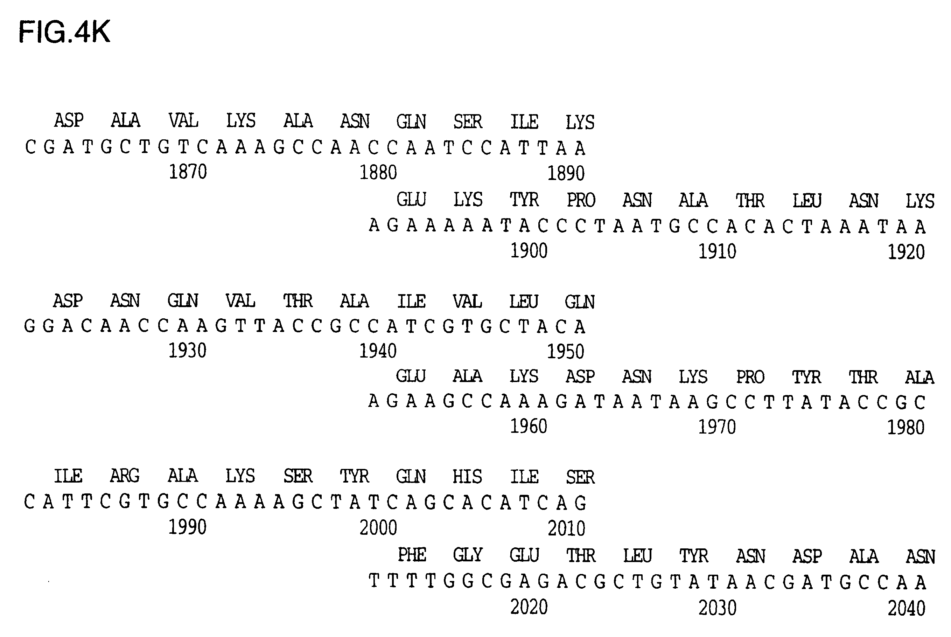

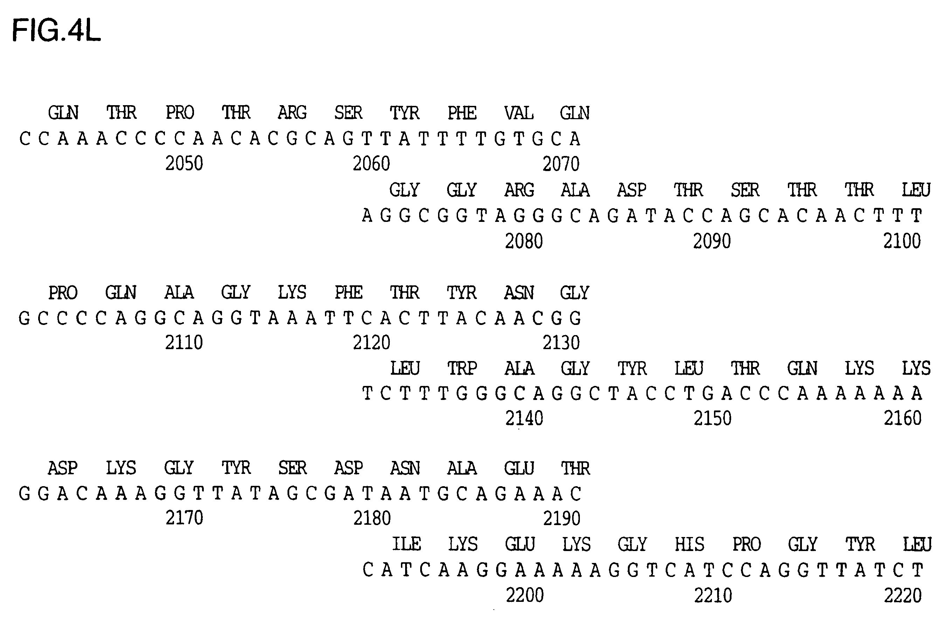

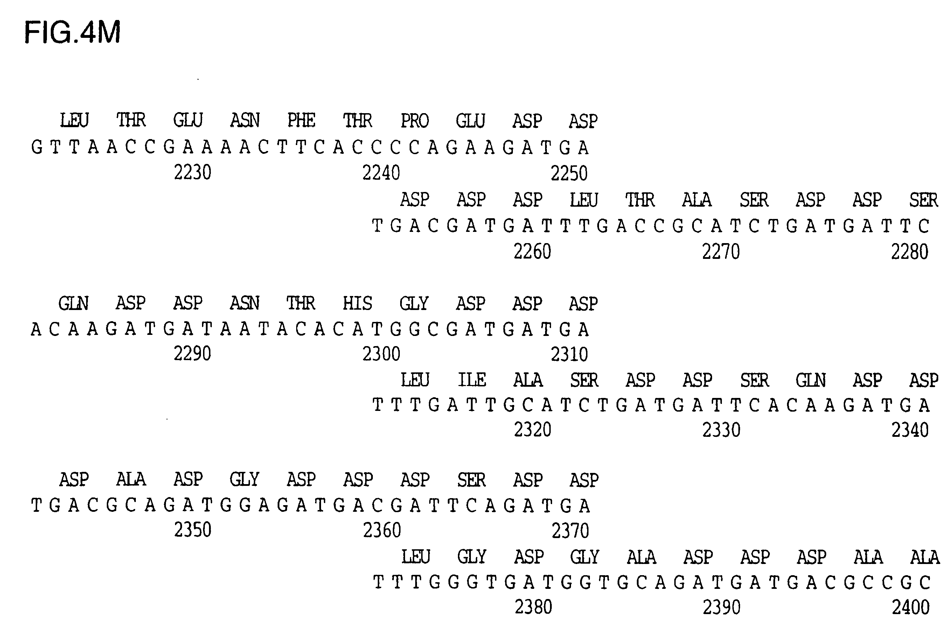

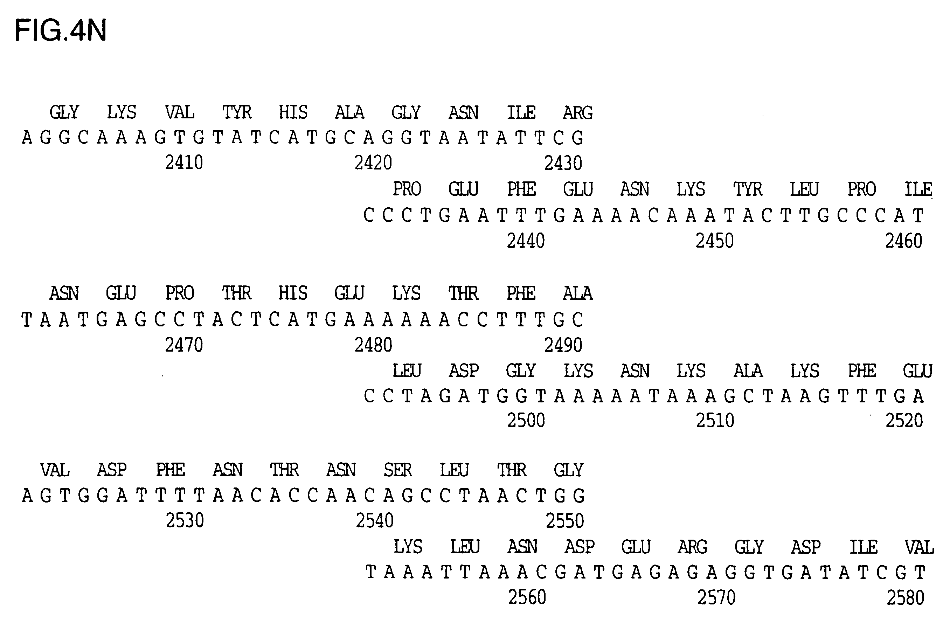

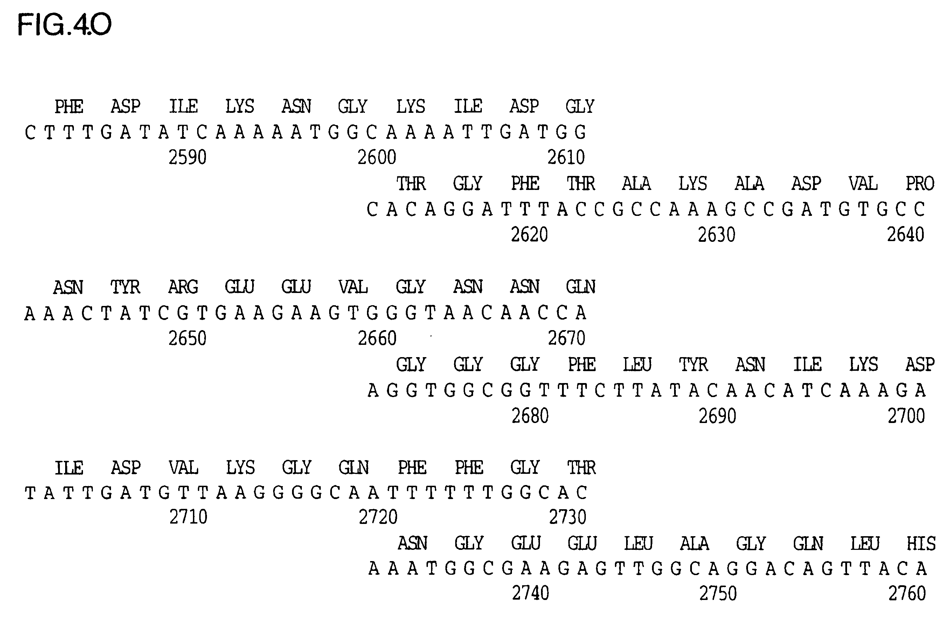

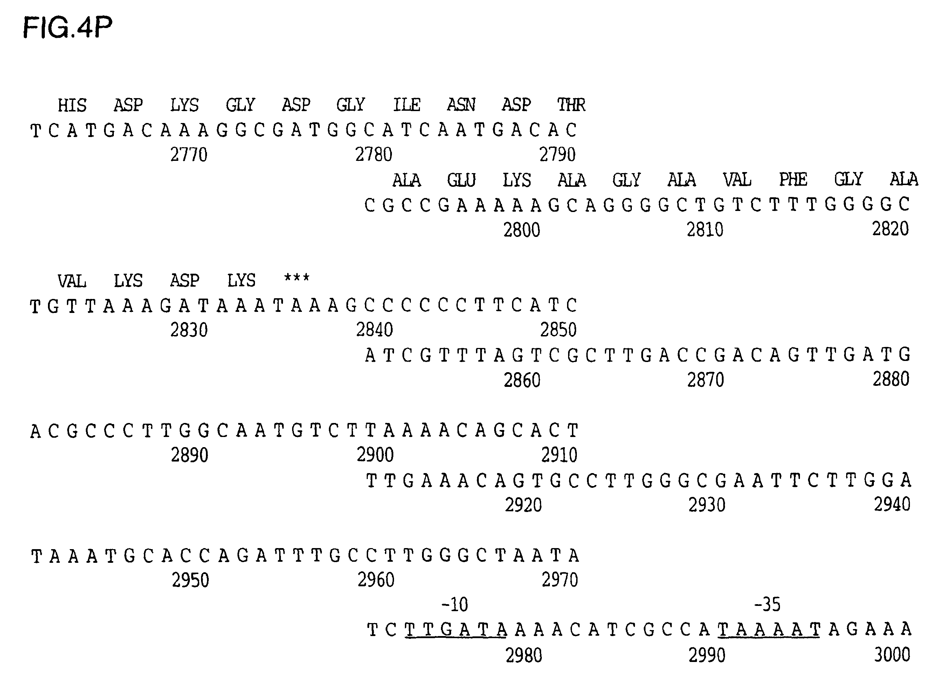

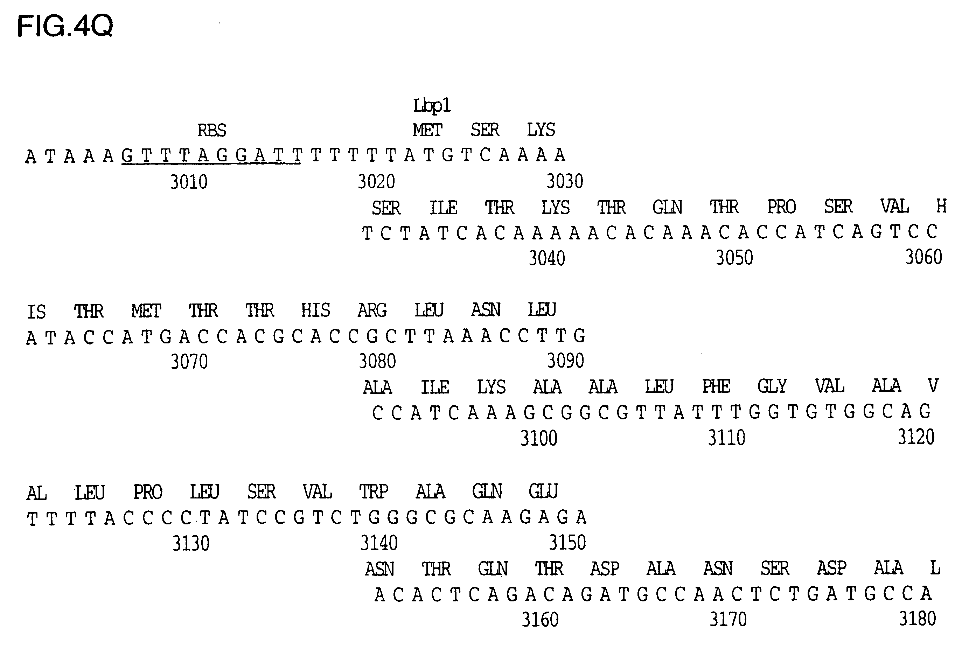

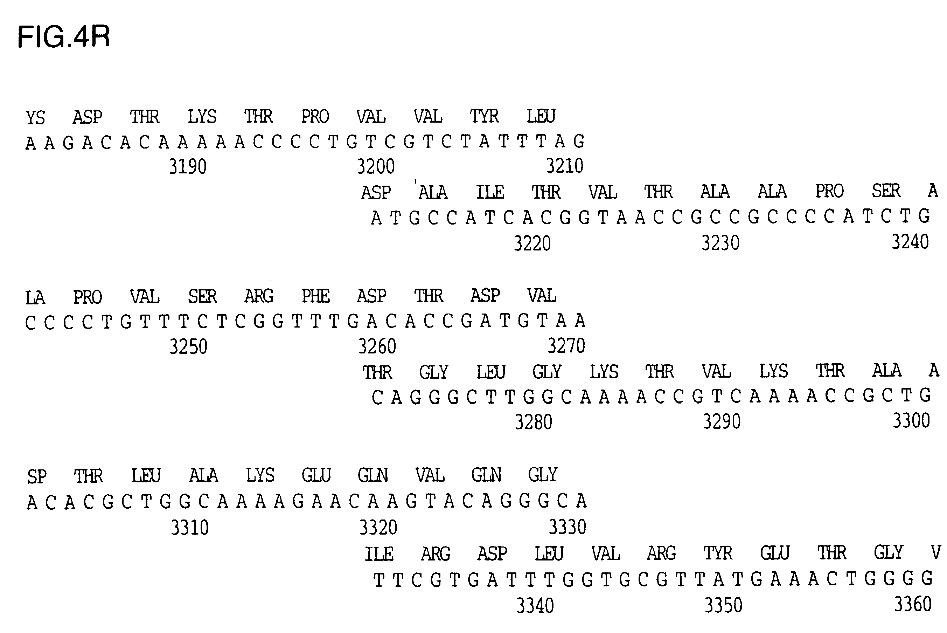

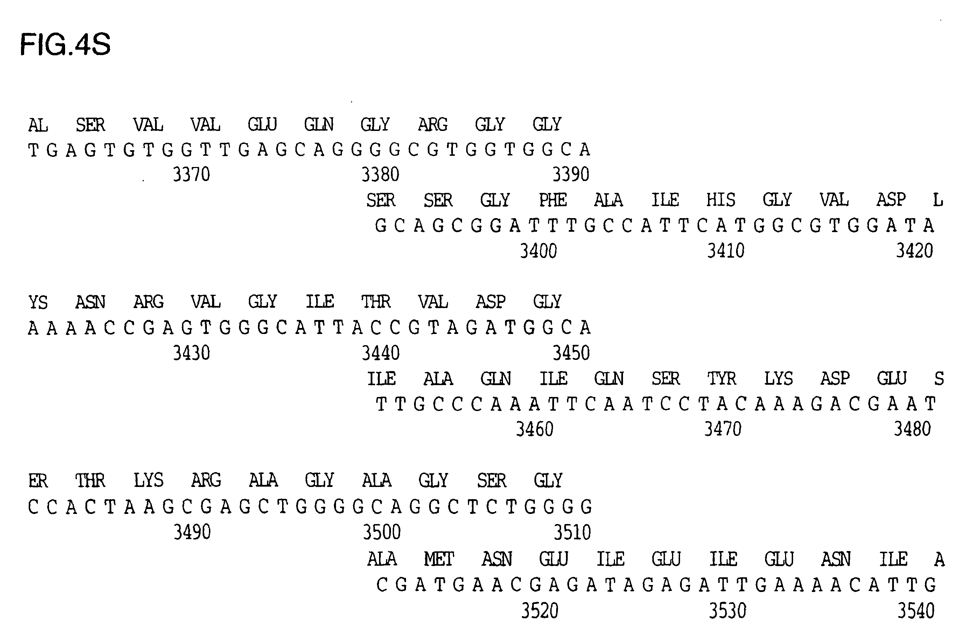

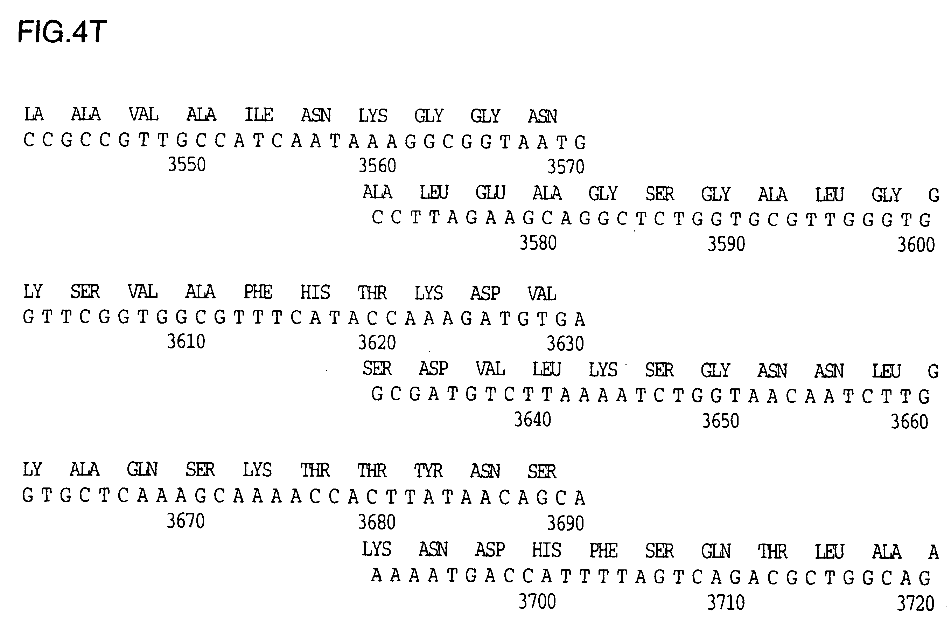

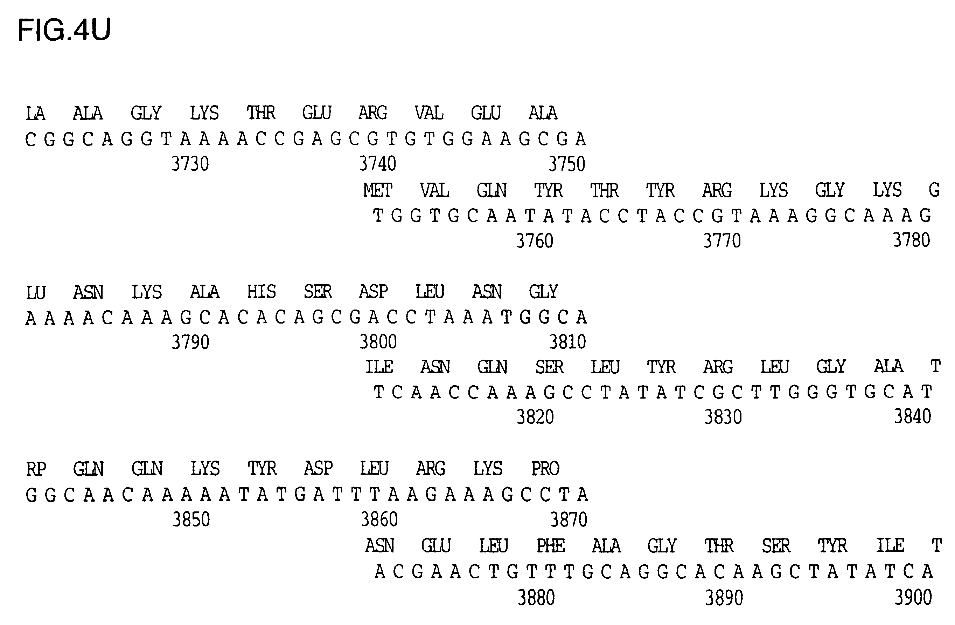

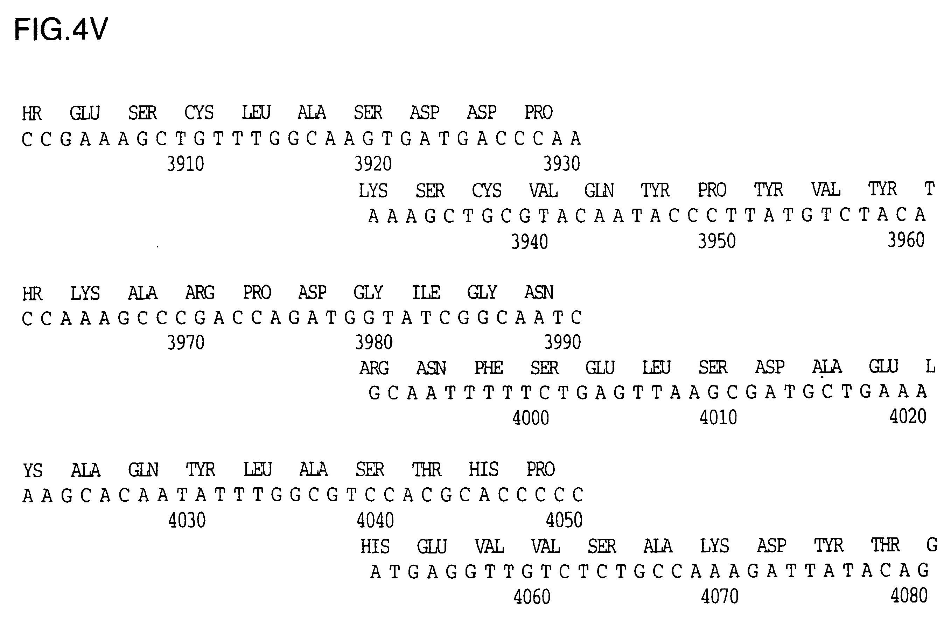

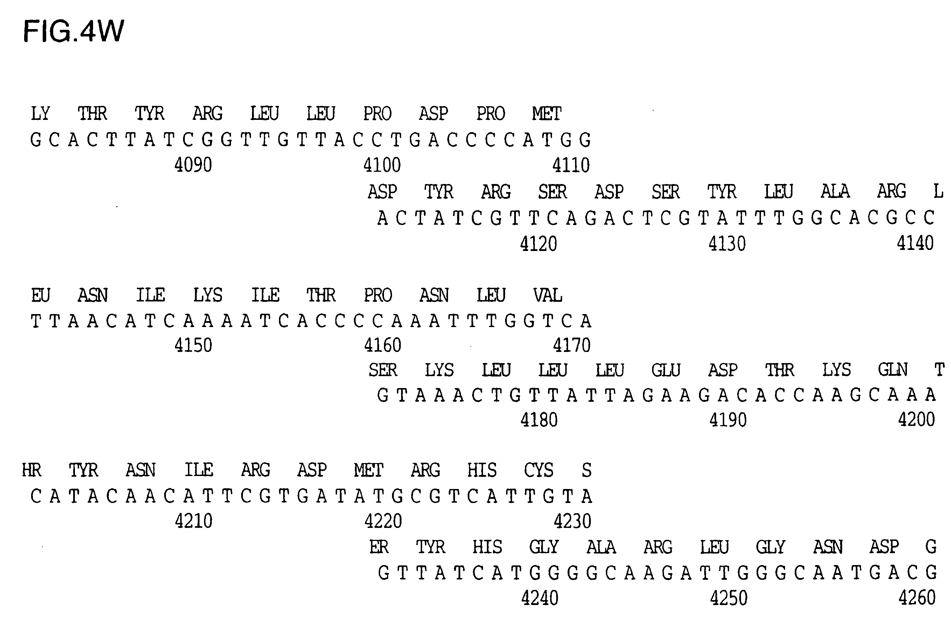

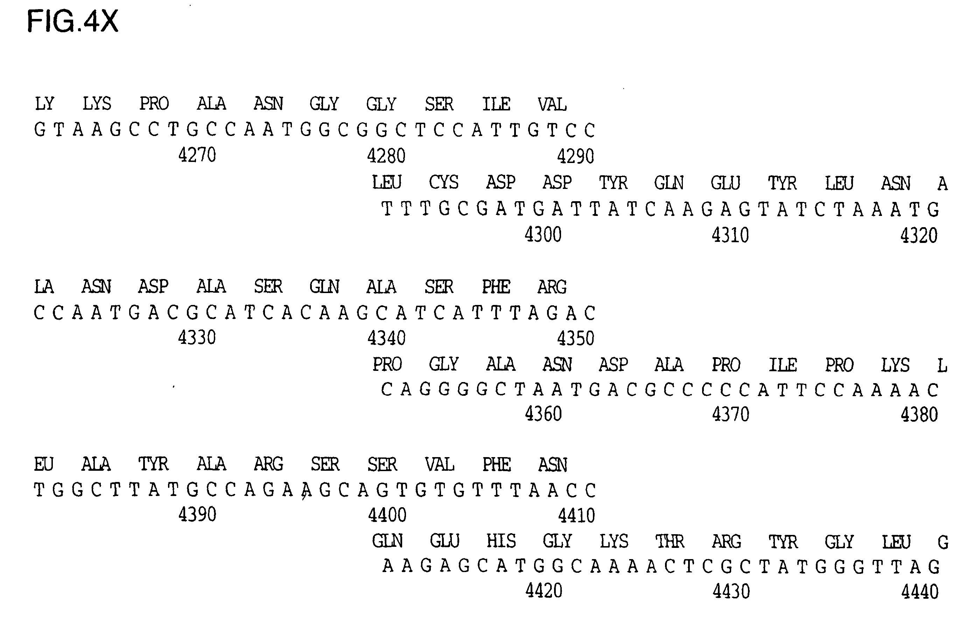

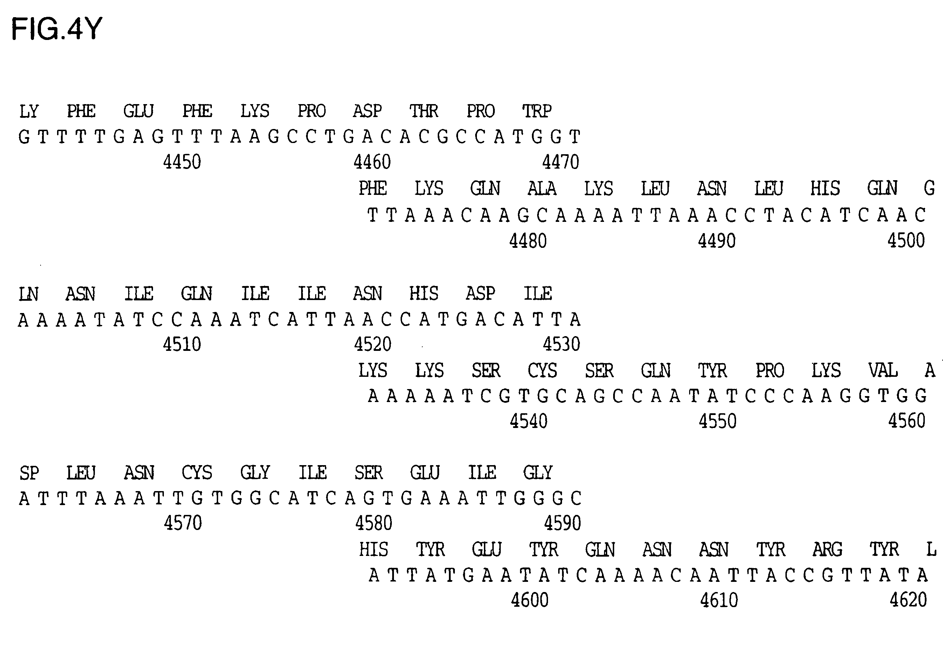

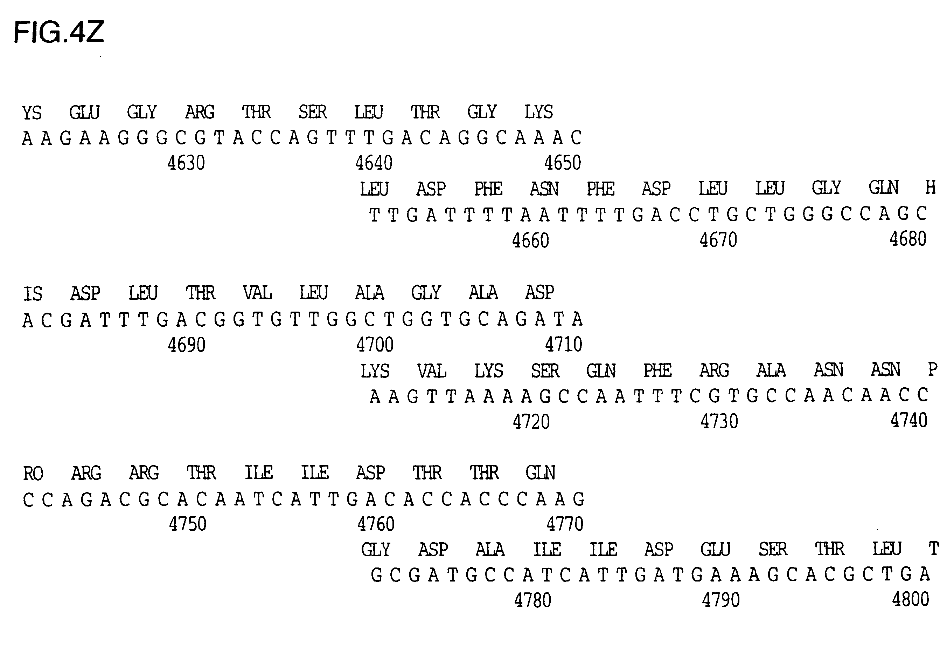

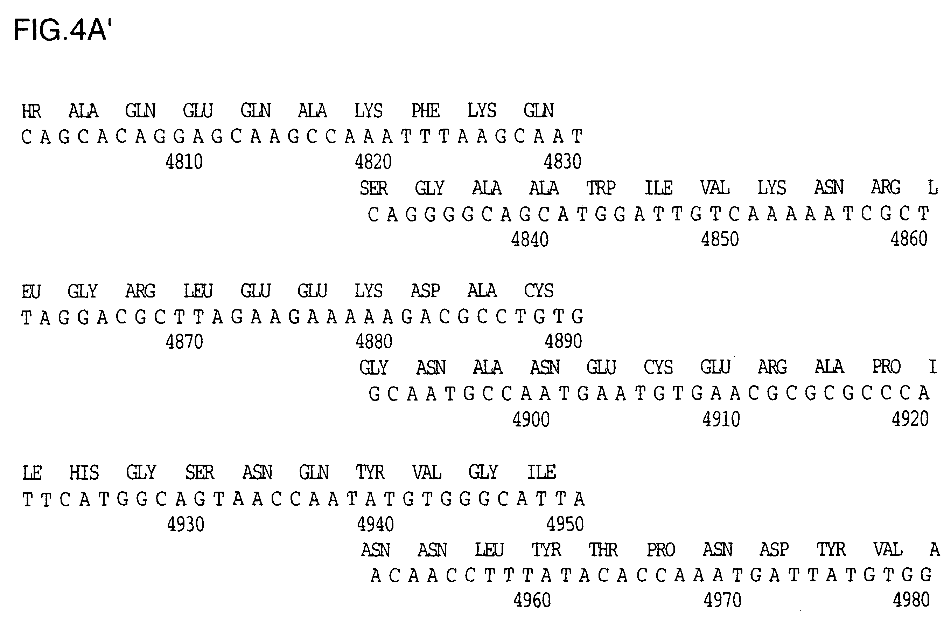

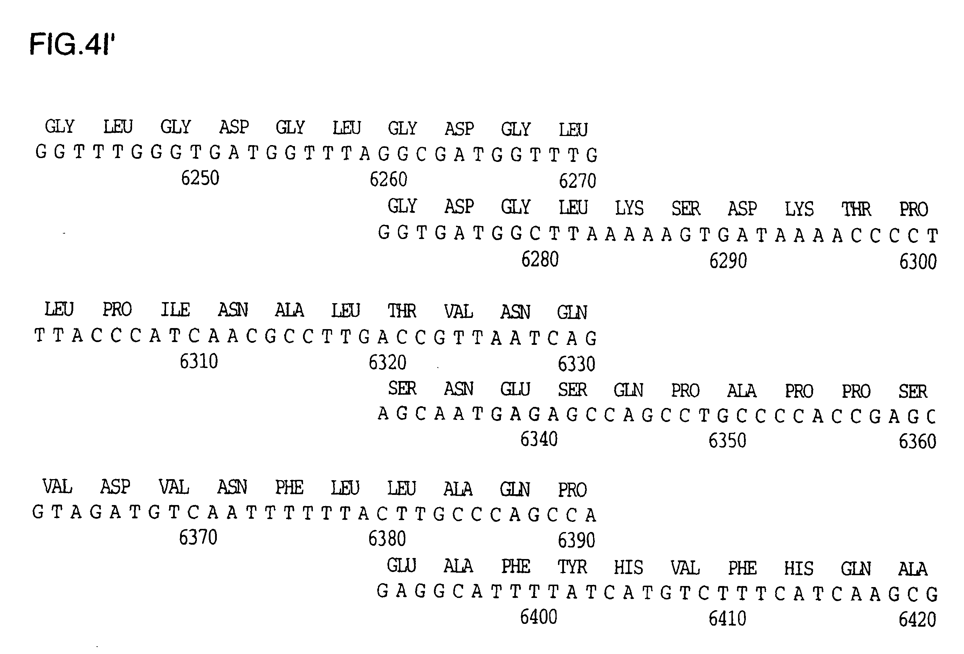

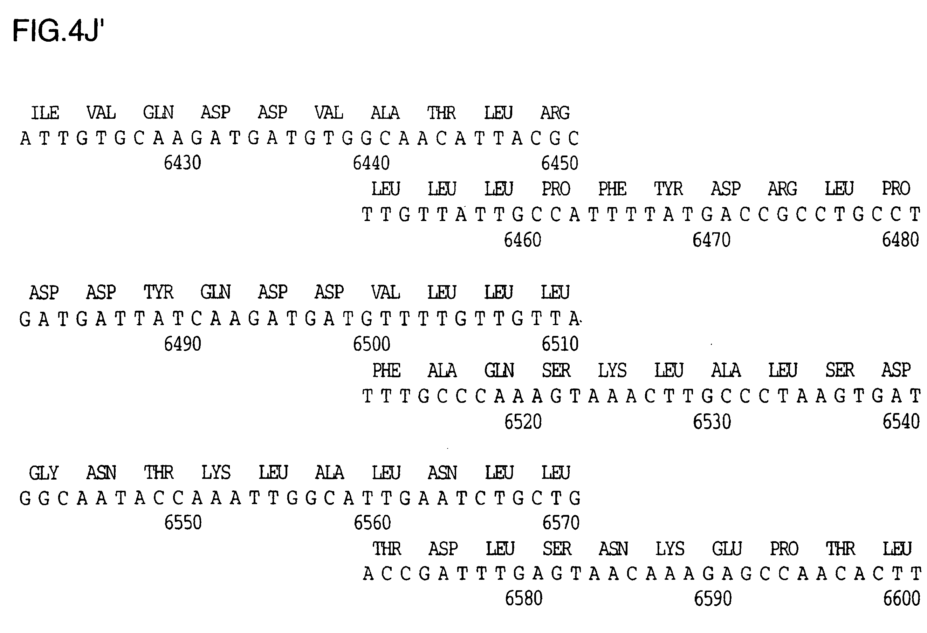

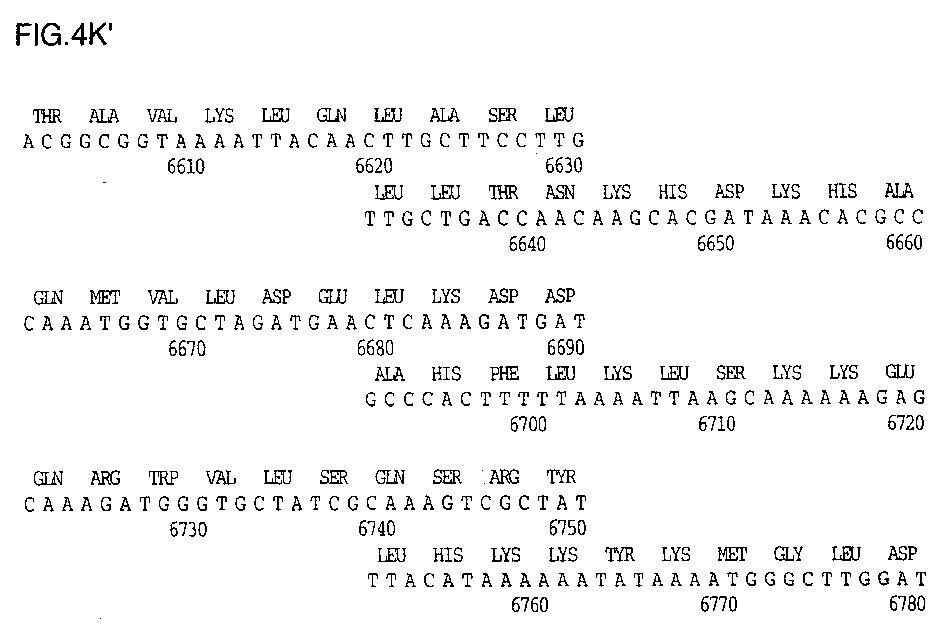

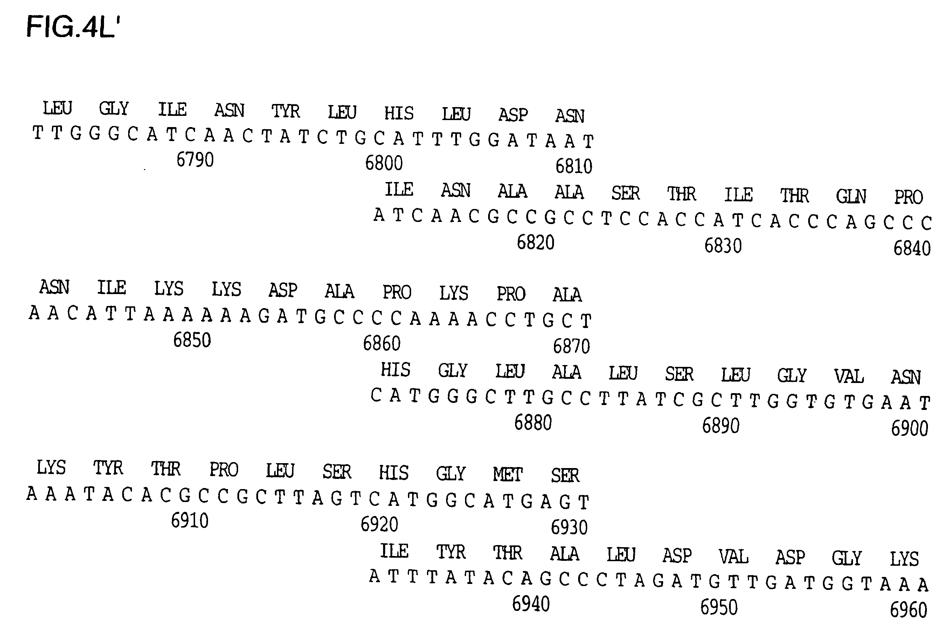

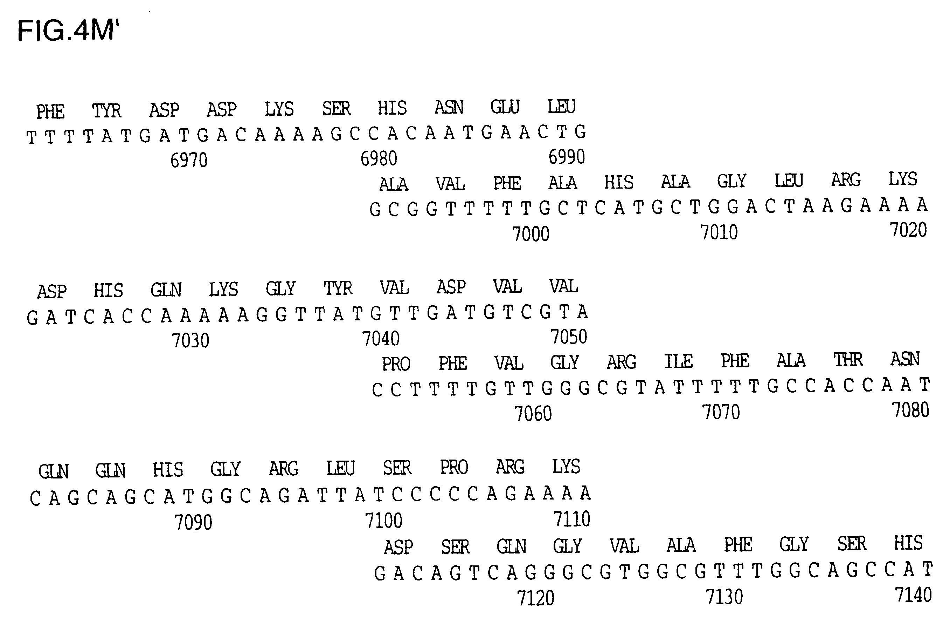

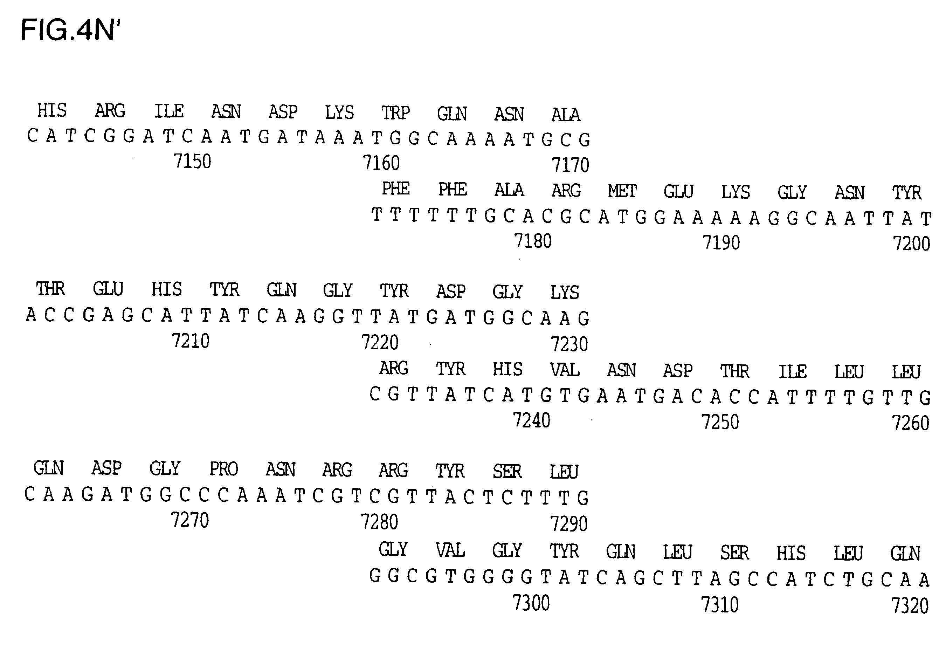

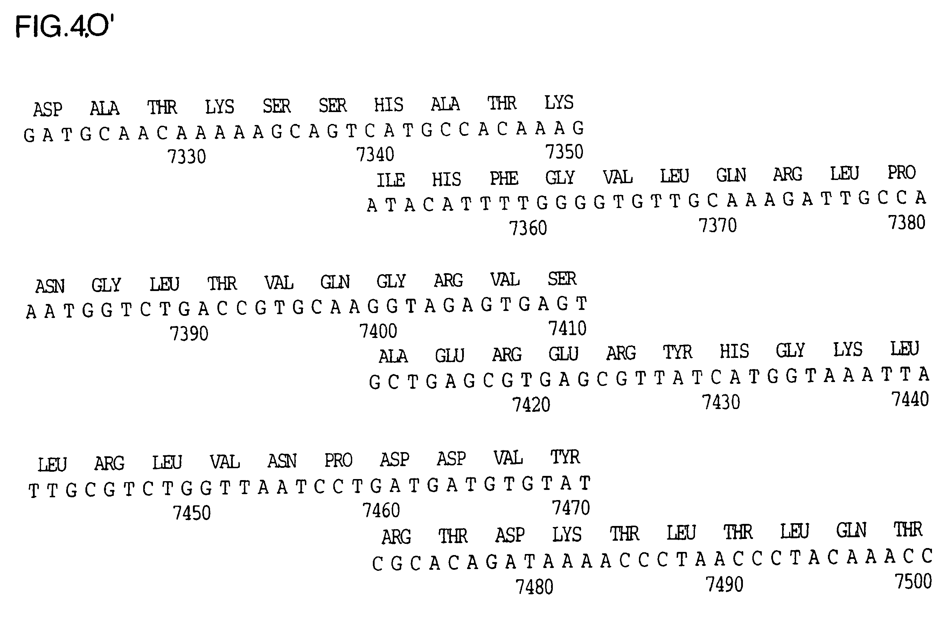

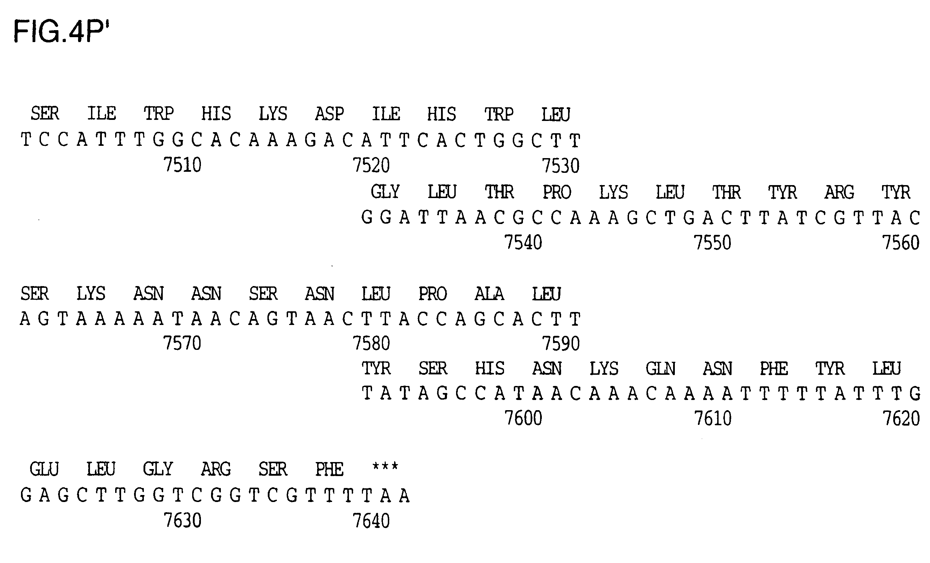

An isolated and purified nucleic acid molecule of the invention has (a) a DNA sequence which encodes an amino acid sequence consisting of amino acids 1 to 894 of the amino acid sequence as set out in Figures 4A to 4P; or (b) a DNA sequence complementary to the DNA sequence of (a).

In another aspect of the present invention, there is provided a purified and isolated nucleic acid molecule having a DNA sequence consisting of a DNA sequence consisting of nucleotides 152 to 2833 of the DNA sequence as set out in Figures 4A to 4P, or the complementary DNA sequence thereto.

In an additional aspect, the present invention includes a vector adapted for transformation of a host, comprising a nucleic acid molecule as provided herein.

The vector may be adapted for expression of the encoded lactoferrin receptor protein in a heterologous or homologous host, in either a lipidated or non-lipidated form. Furthermore, expression vectors falling within the scope of the present invention may be adapted for transformation of a host and comprise a nucleic acid molecule as provided herein and expression means operatively coupled to the nucleic acid molecule for expression by the host of the lactoferrin receptor protein.

In specific embodiments of this aspect of the invention, the nucleic acid molecule may encode substantially the Lbp2 protein of the *Moraxella* strain.

The expression means may include a nucleic acid portion encoding a leader sequence for secretion from the host of the lactoferrin receptor protein. The expression means also may include a nucleic acid portion encoding a lipidation signal for expression from the host of a lipidated form of the lactoferrin receptor protein. The host may be selected from, for example, *Escherichia coli, Bacillus, Bordetella, Haemophilus, Moraxella,* fungi, yeast or baculovirus and Semliki Forest virus expression system may be used.

In an additional aspect of the invention, there is provided a host cell transformed in culture, containing an expression vector as provided herein. The invention further includes a recombinant lactoferrin receptor protein 2 (Lbp2) of *Moraxella catarrhalis Q8,* producible by the transformed host.

Such recombinant lactoferrin receptor protein may be provided in substantially pure form according to a further aspect of the invention, which provides a method of forming a substantially pure recombinant lactoferrin receptor protein, which comprises growing the transformed host provided herein and isolating and purifying the lactoferrin receptor protein. The lactoferrin receptor protein may be expressed in inclusion bodies, which may be purified free from cellular material and soluble proteins and lactoferrin receptor protein solubilized from the purified inclusion bodies, and the lactoferrin receptor protein purified free from other solubilized materials. The recombinant protein is generally at least about 70% pure, preferably at least about 90% pure.

The recombinantly-produced Lbp2 protein of the invention, as prepared according to the above method is devoid of the Lbp1 and ORF3 proteins of the *Moraxella* strain and any other protein of the *Moraxella* strain.

In accordance with another aspect of the invention, an immunogenic composition is provided which comprised at least one active component selected from at least one nucleic acid molecule as provided herein or at least one recombinant protein as provided herein and a pharmaceutically acceptable carrier therefor. The at least one active component produces an immune response when administered to a host.

The immunogenic compositions provided herein may be formulated as a vaccine for *in vivo* administration to a host to provide protection against disease caused by *M. catarrhalis.* For such purpose, the compositions may be formulated as a microparticle, capsule, ISCOM or liposome preparation. The immunogenic composition may be provided in combination with a targeting molecule for delivery to specific cells of the immune system or to mucosal surfaces. The immunogenic compositions of the invention (including vaccines) may further comprise at least one other immunogenic or immunostimulating material and the immunostimulating material may be at least one adjuvant or at least one cytokine.

Suitable adjuvants for use in the present invention include (but are not limited to) aluminum phosphate, aluminum hydroxide, QS21, Quil A, derivatives and components thereof, ISCOM matrix, calcium phosphate, calcium hydroxide, zinc hydroxide, a glycolipid analog, an octadecyl ester of an amino acid, a muramyl dipeptide, polyphosphazene, ISCOPREP, DC-chol, DDBA and a lipoprotein and other adjuvants to induce a TH1 response. Advantageous combination of adjuvants are described in copending United States Patent Applications No. 08/261,194 filed June 16, 1994 and 08/483,856 filed June 7, 1995, assigned to the assignee hereof and WO 95/34308, published November 21,1995.

Methods for generating an immune response in a host can be employed using the compositions of the present invention. Such methods comprise the step of administering to a susceptible host, such as a human, an effective amount of the immunogenic composition as recited above. The immune response may be humoral or a cell-mediated immune response and may provide protection against disease caused by *Moraxella.* Hosts in which protection against disease may be conferred include primates, including humans.

Also, the nucleic acid molecules of the invention can be used in a live vector for delivery of lactoferrin receptor protein 2 to a host. Such vectors comprise a vector containing the nucleic acid molecule as described above. The vector may be selected from *Salmonella, Mycobacterium bovis,* BCG, adenovirus, poxvirus, vaccinia and poliovirus.

The nucleic acid molecules provided herein are also useful in diagnostic applications. Accordingly, in a further aspect of the invention, there is provided a method of determining the presence, in a sample, of nucleic acid encoding a lactoferrin receptor protein 2 of *Moraxella catarrhalis Q8,* comprising the steps of:
a) contacting the sample with a nucleic acid molecule as provided herein to produce duplexes comprising the nucleic acid molecule and any nucleic acid molecule encoding the lactoferrin receptor protein present in the sample and specifically hybridizable therewith; and
b) determining the production of the duplexes.

In addition, the present invention provides a diagnostic kit for determining the presence, in a sample, of nucleic acid encoding a lactoferrin receptor protein 2 of *Moraxella catarrhalis Q8,* comprising:
a) a nucleic acid molecule as provided herein;
b) means for contacting the nucleic acid molecule with the sample to produce duplexes comprising the nucleic acid molecule and any such nucleic acid present in the sample and hybridizable with the nucleic acid present in the sample and hybridizable with the nucleic acid molecule; and
c) means for determining production of the duplexes.

The invention further includes the use of the nucleic acid molecules and proteins provided herein as medicines. The invention additionally encompasses the use of the nucleic acid molecules and proteins provided herein in the manufacture of medicaments for protection against disease caused by strains of *Moraxella.*

Advantages of the present invention include:
- an isolated and purified nucleic acid molecule encoding a lactoferrin receptor protein 2 of *Moraxella Q8*;
- recombinantly-produced lactoferrin receptor protein (Lbp2) encoded therein, free from other *Moraxella* proteins;
- diagnostic kits and immunological reagents for specific identification of *Moraxella.*

### BRIEF DESCRIPTION OF DRAWINGS

The present invention will be further understood from the following description with reference to the drawings, in which:

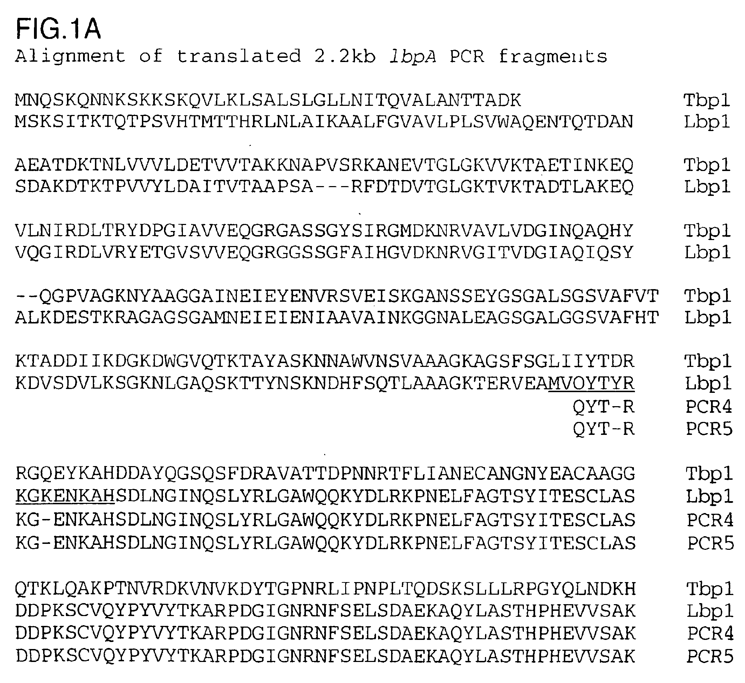

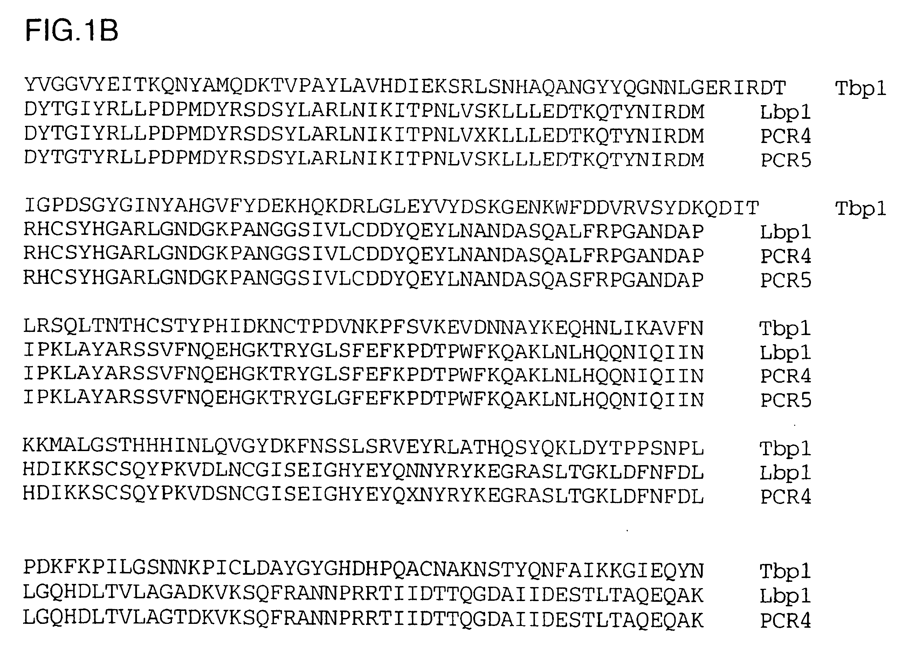

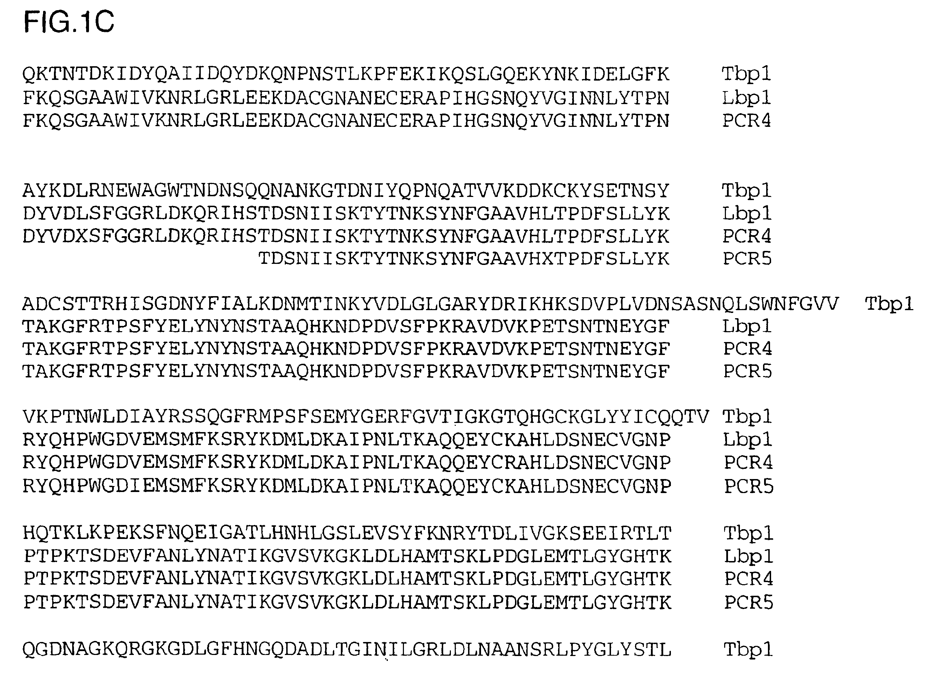

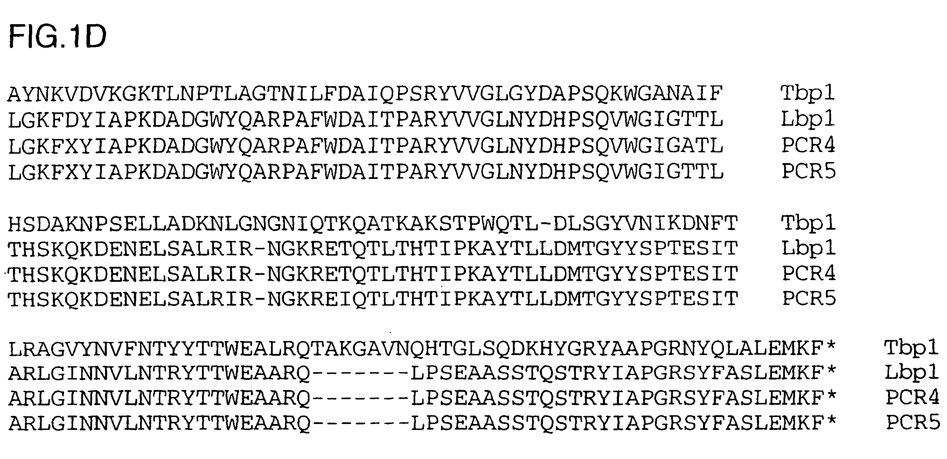

Figure 1 shows partial sequence of the 2.2 kb PCR amplified fragments of the *lbpA* genes from *M. catarrhalis* 4223 or Q8, which were used to probe the phage libraries. In the figure, Tbp1 is the deduced 4223 Tbp1 sequence (as described in United. States Patent Application No. 08/613,009 filed March 8, 1996, assigned to the assignee hereof (SEQ ID No: 19), Lbp1 is the deduced full-length 4223 Lbp1 sequence (SEQ ID No: 3) used here solely for aligning the PCR fragments, PCR4 is the 4223 PCR fragment (SEQ ID No: 20), and PCR5 is a partial sequence of the Q8 PCR fragment (SEQ ID No: 21). Only single strand sequence was obtained for the PCR fragments and "X" has been inserted where there was a doubtful sequence. Dashes have been used for maximum alignment. The underlined sequence in Lbp1 (MVQYTYRKGKENKAH - SEQ ID No: 22) represents the position of a CNBr peptide used to generate the 5'-PCR primer.

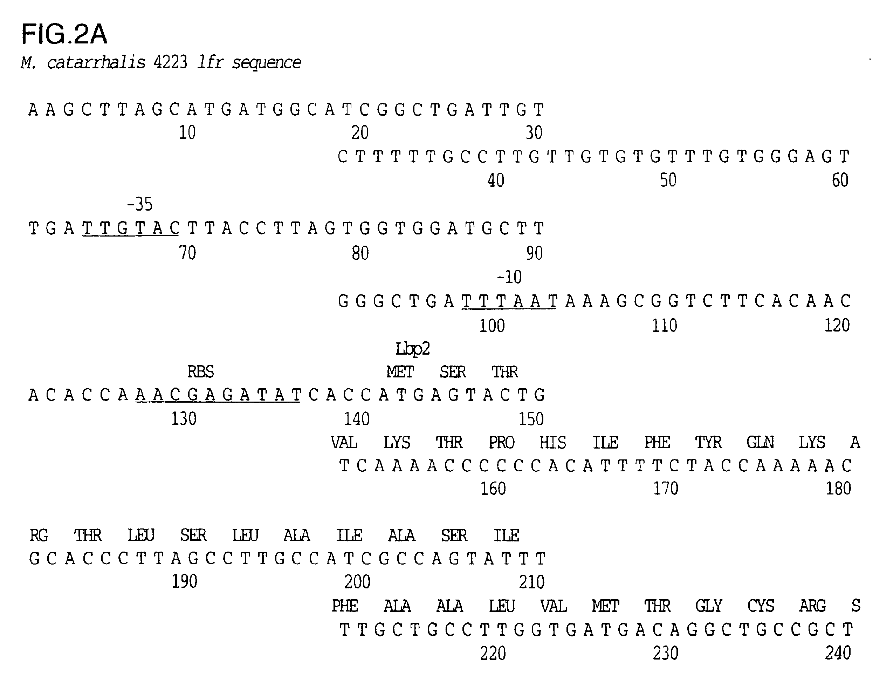

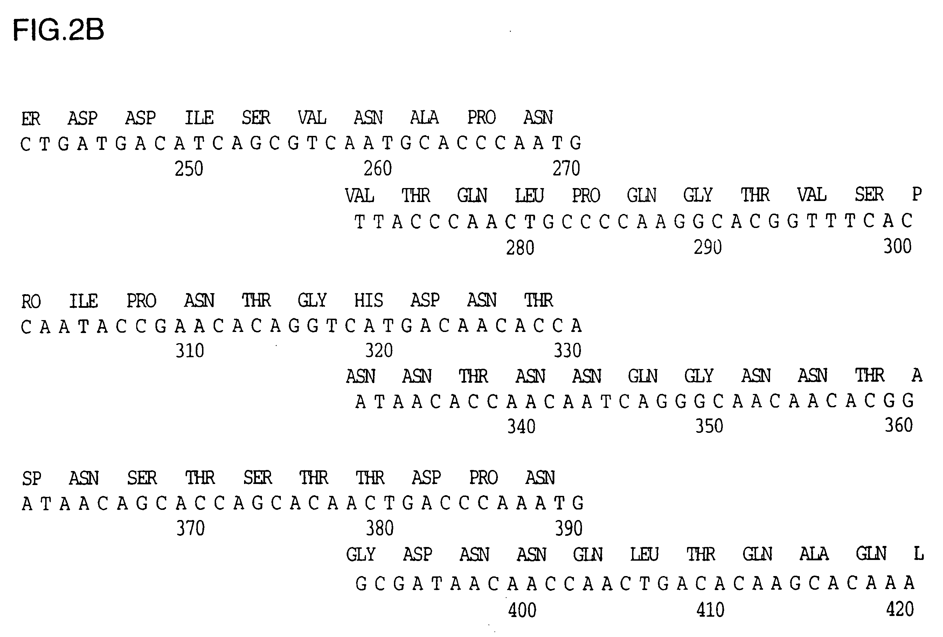

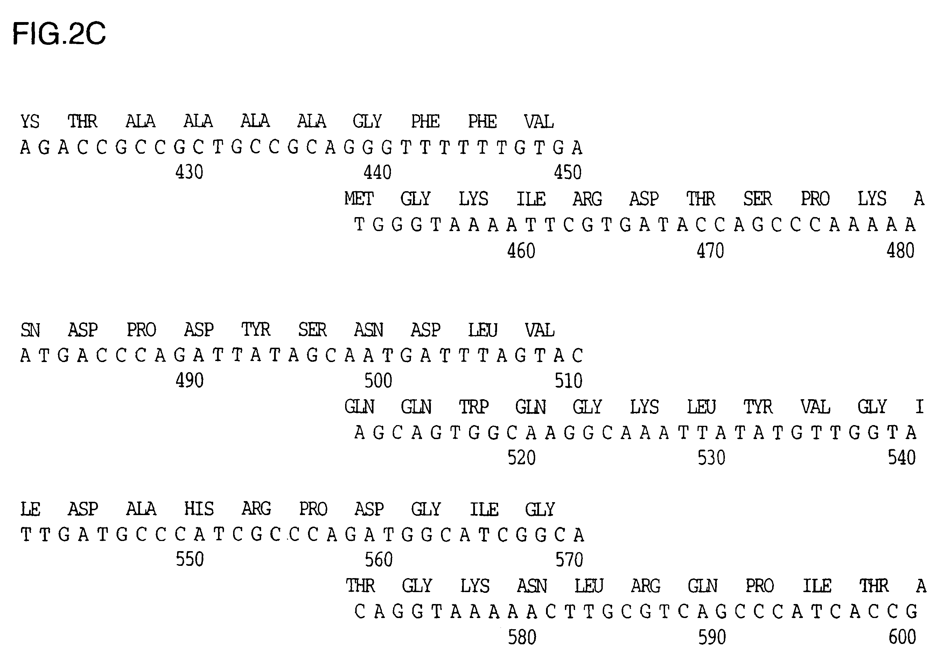

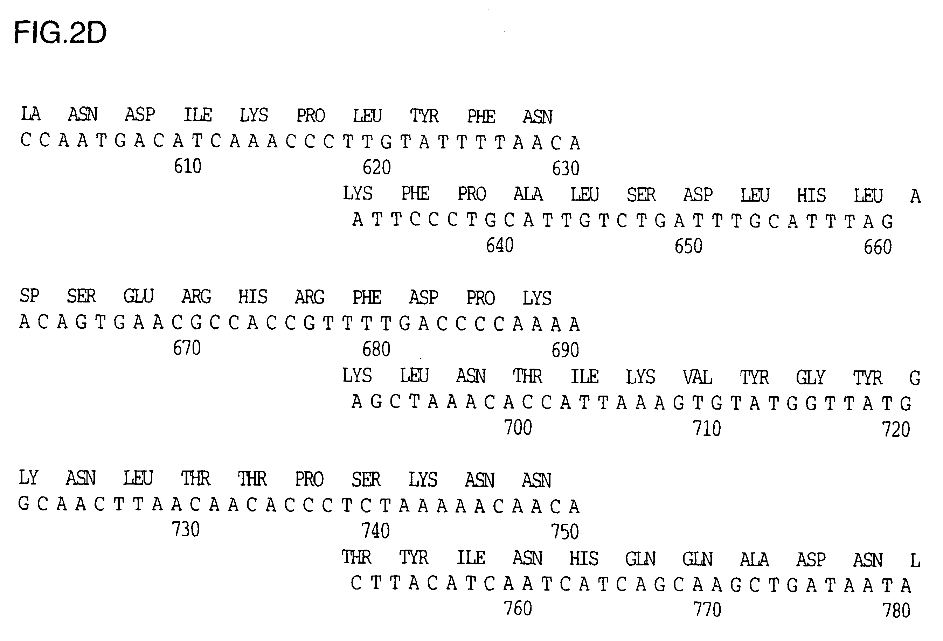

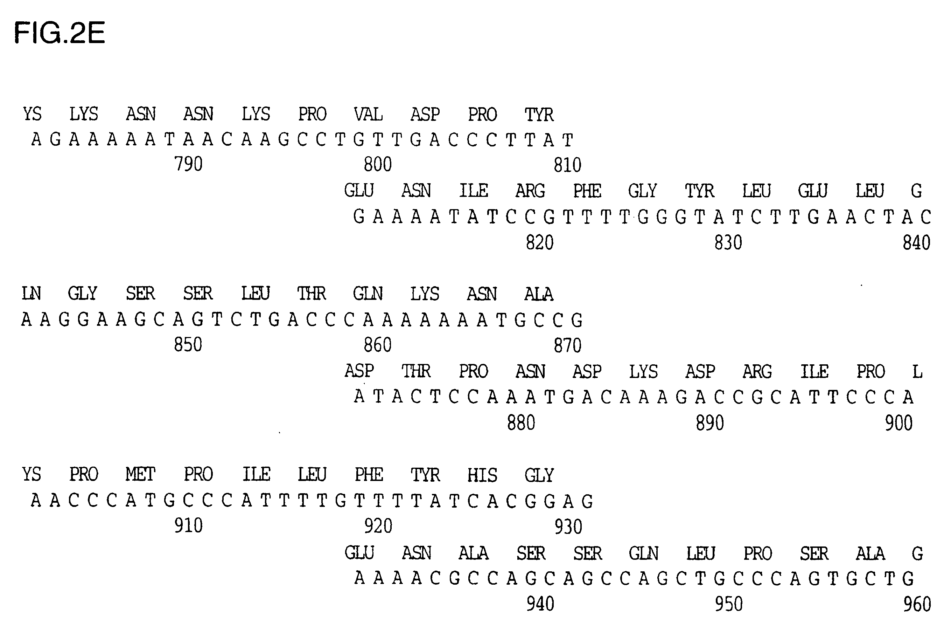

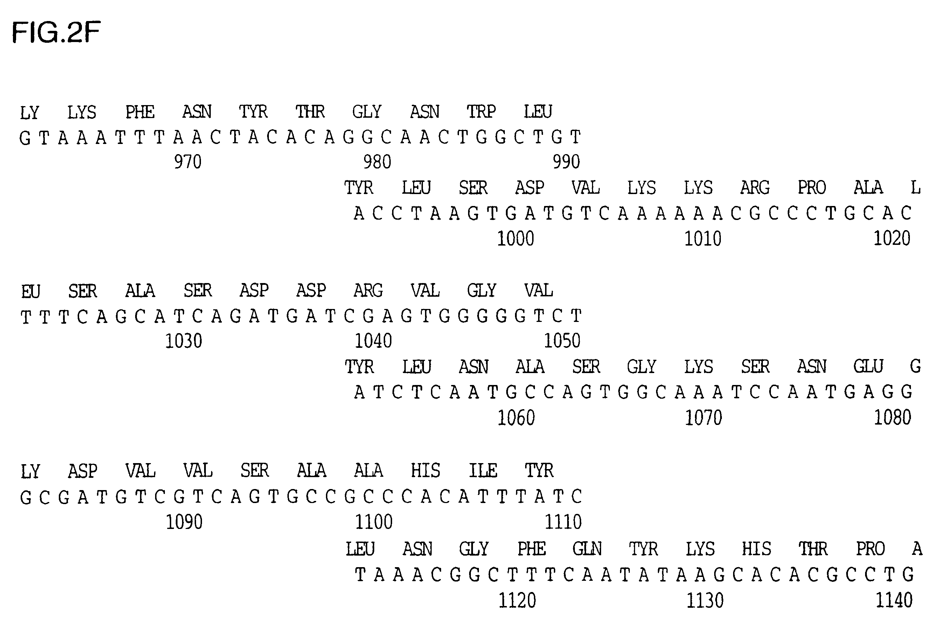

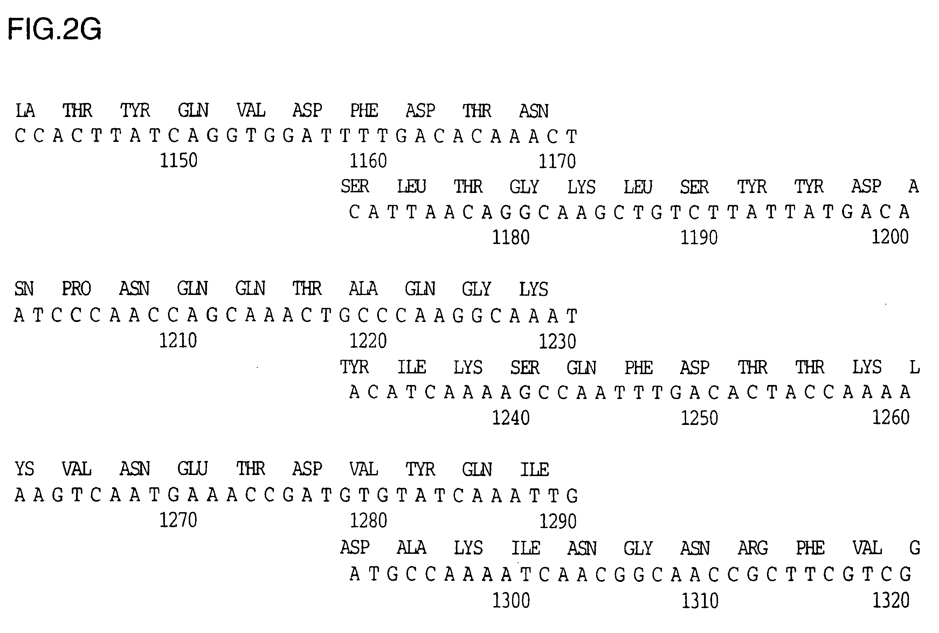

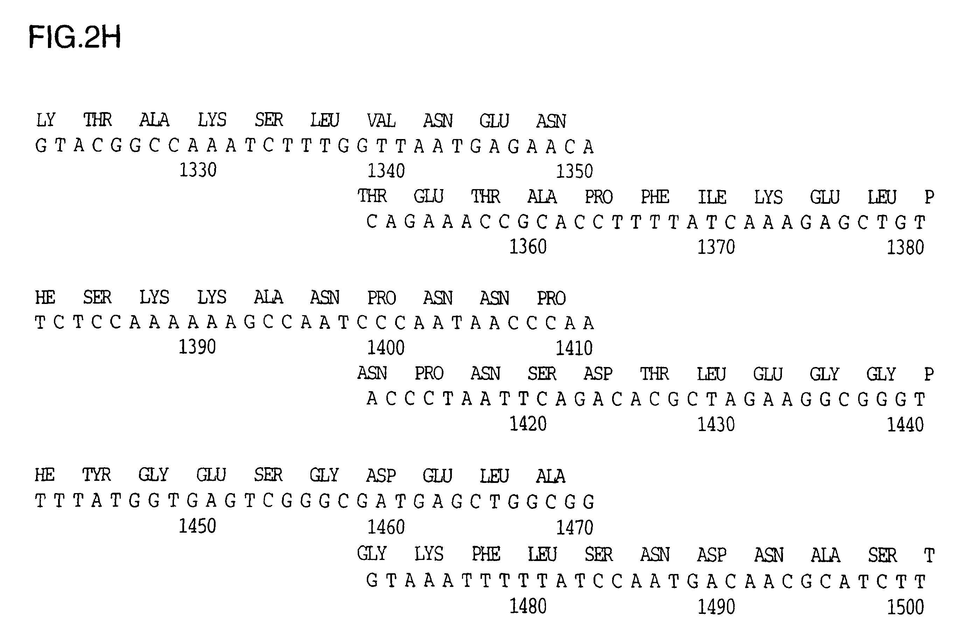

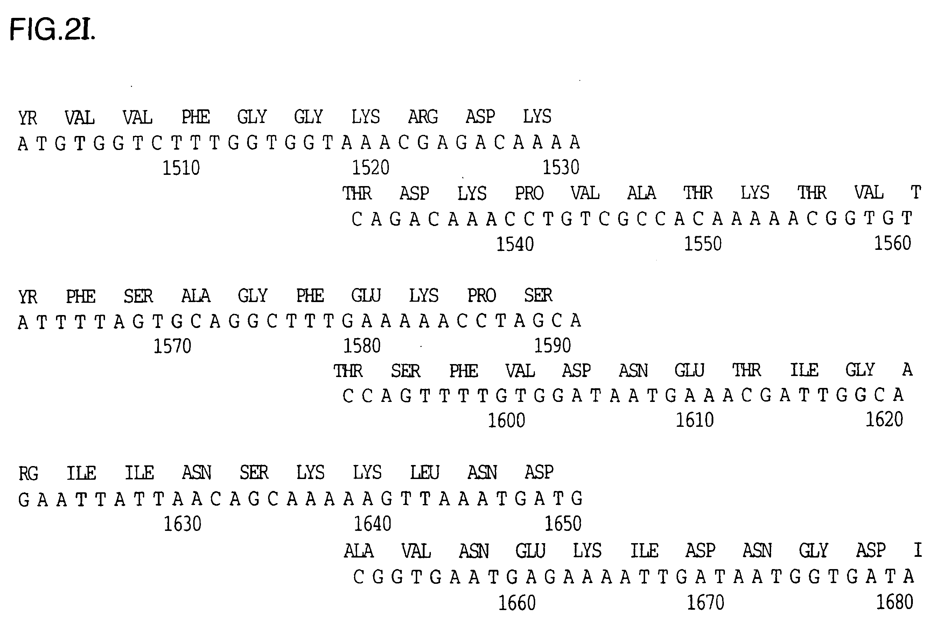

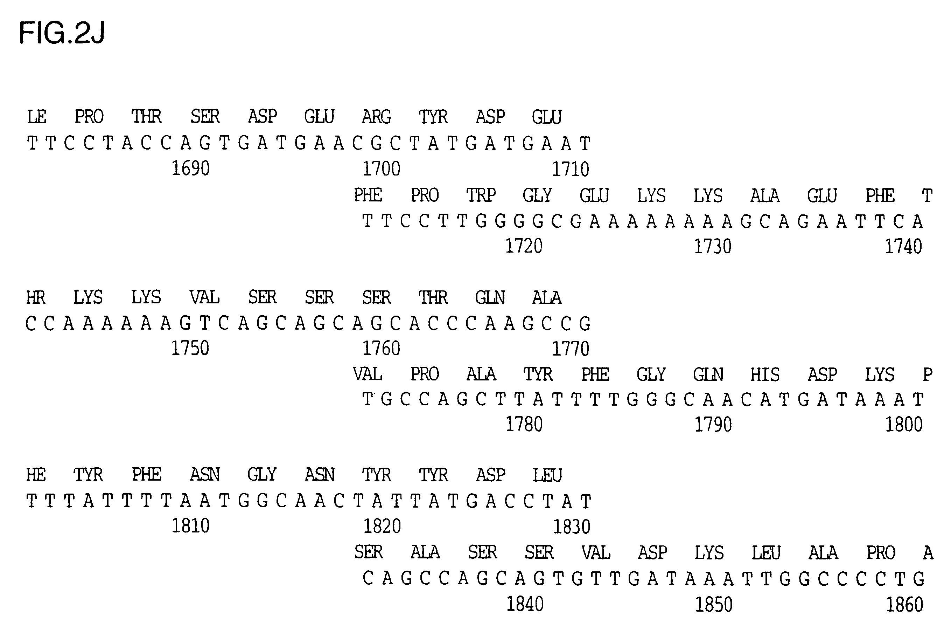

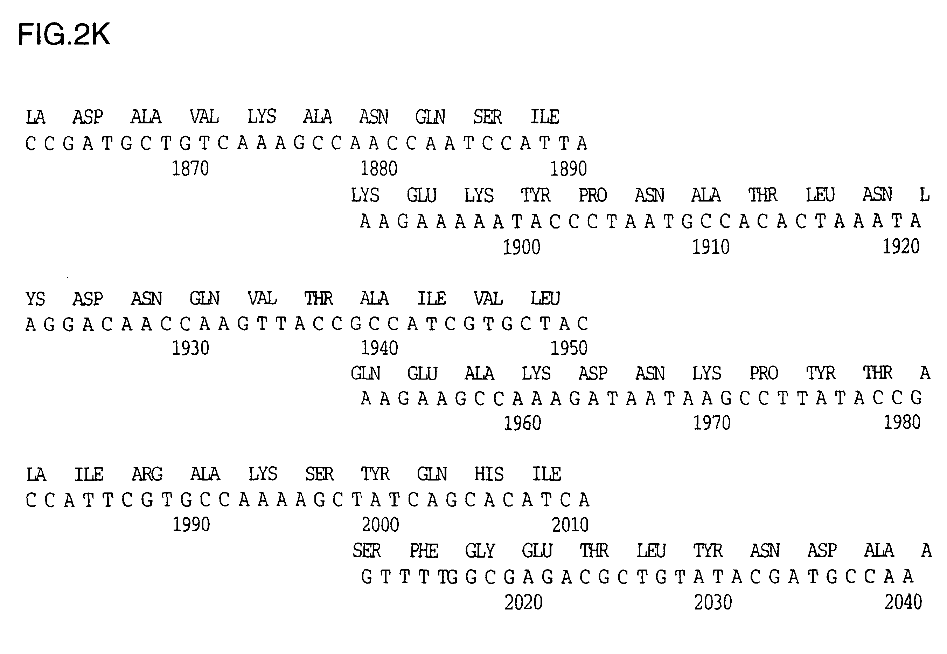

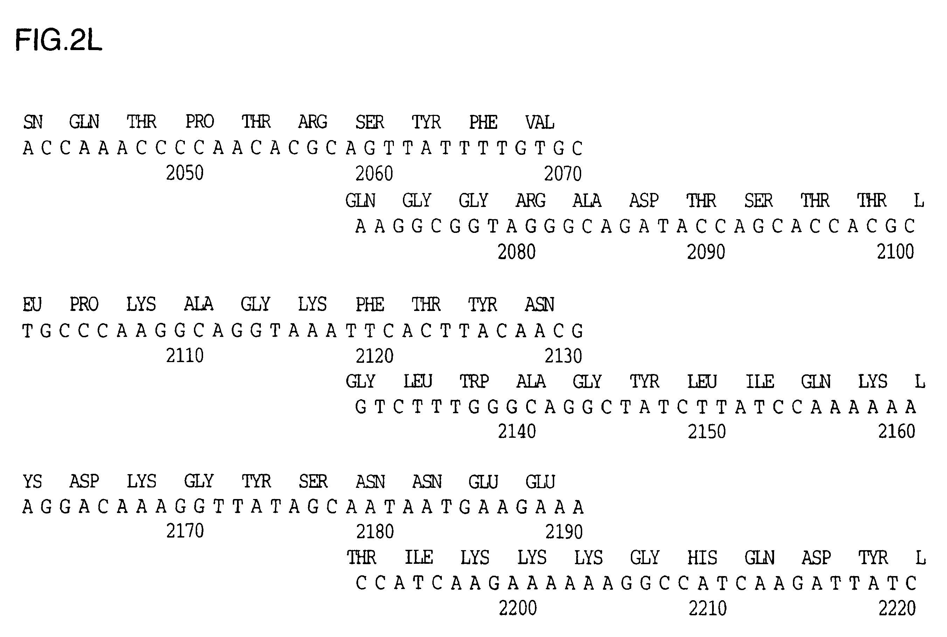

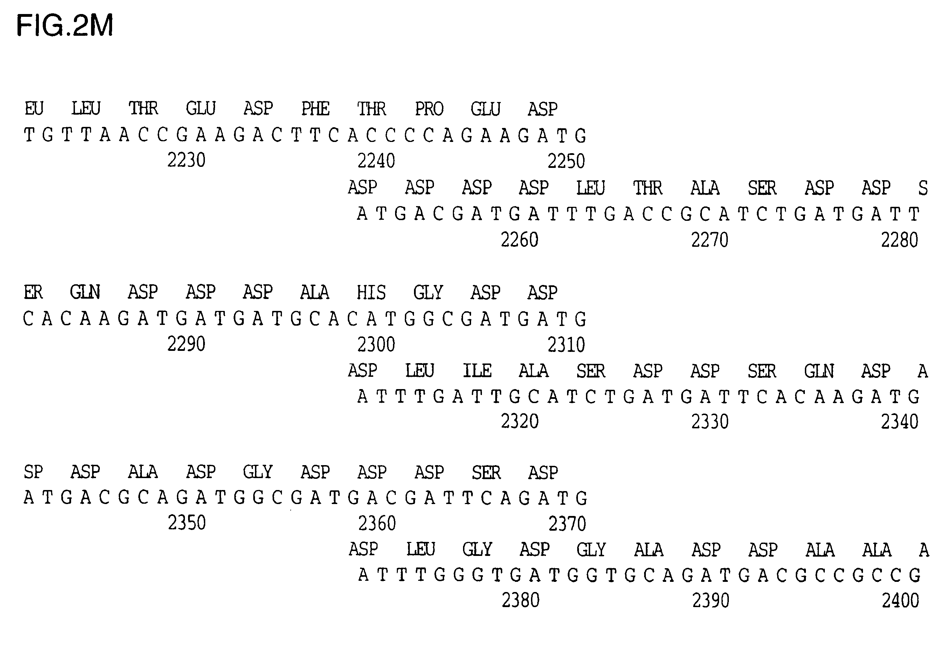

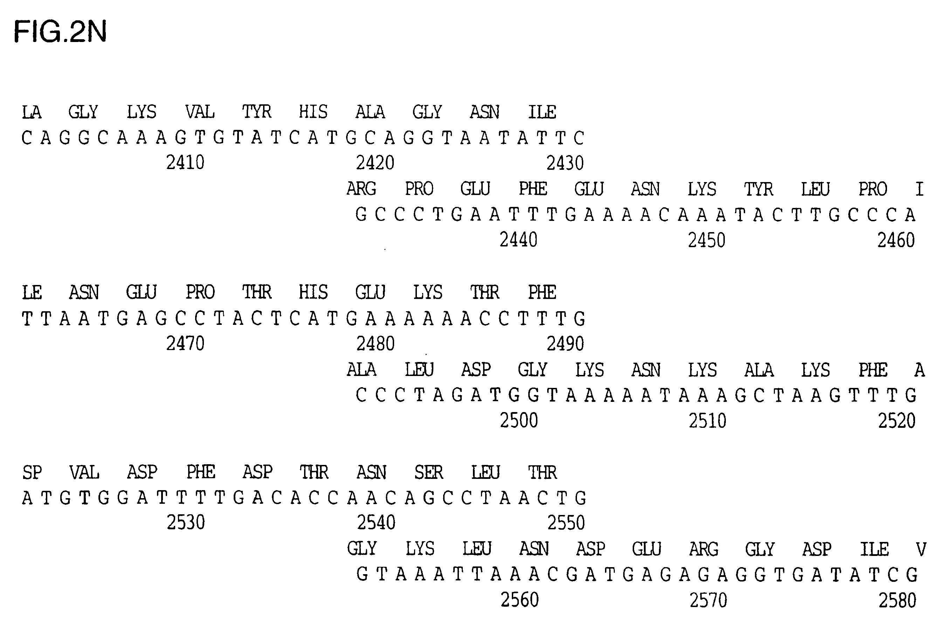

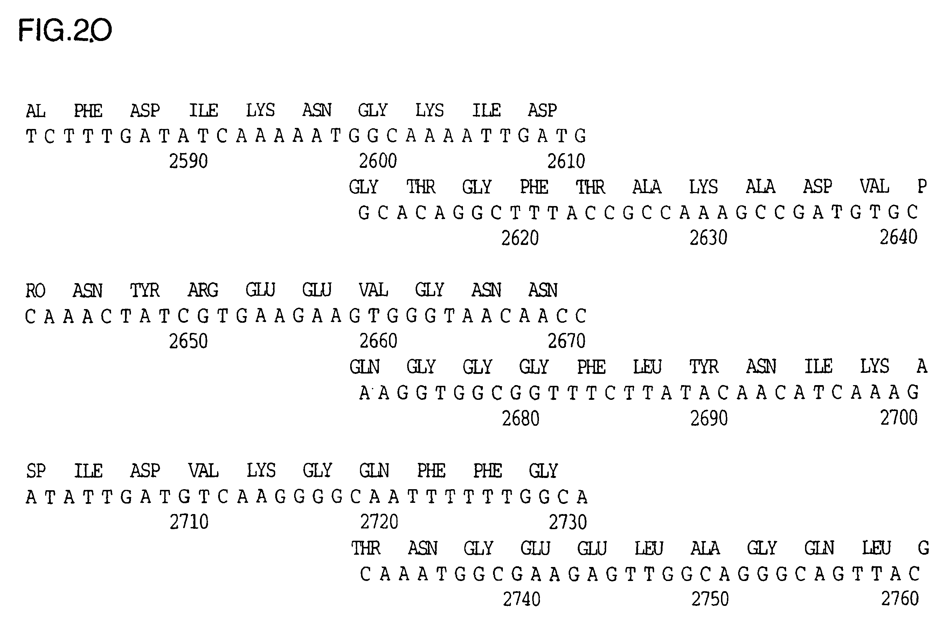

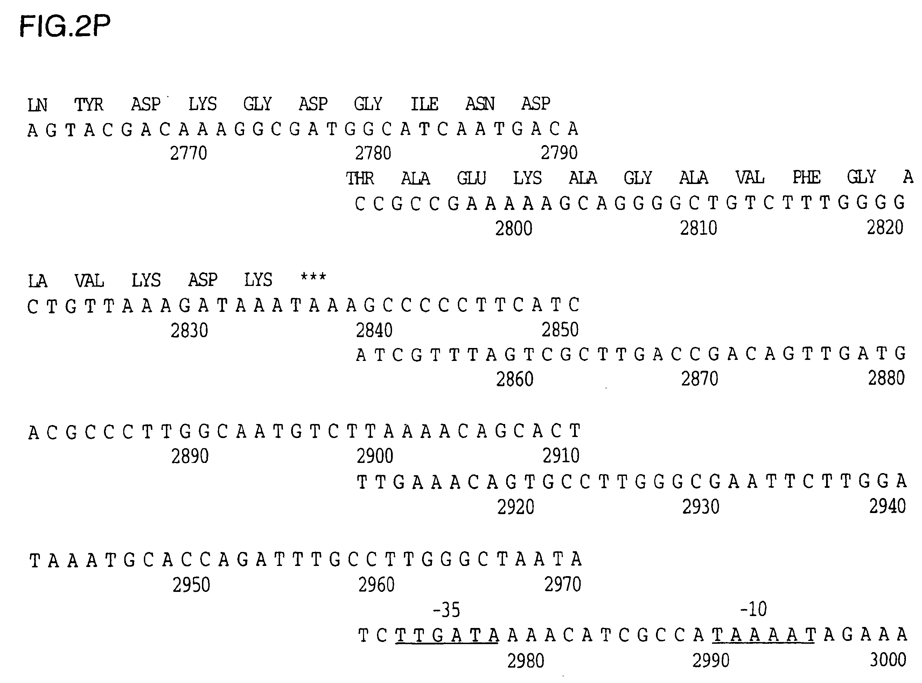

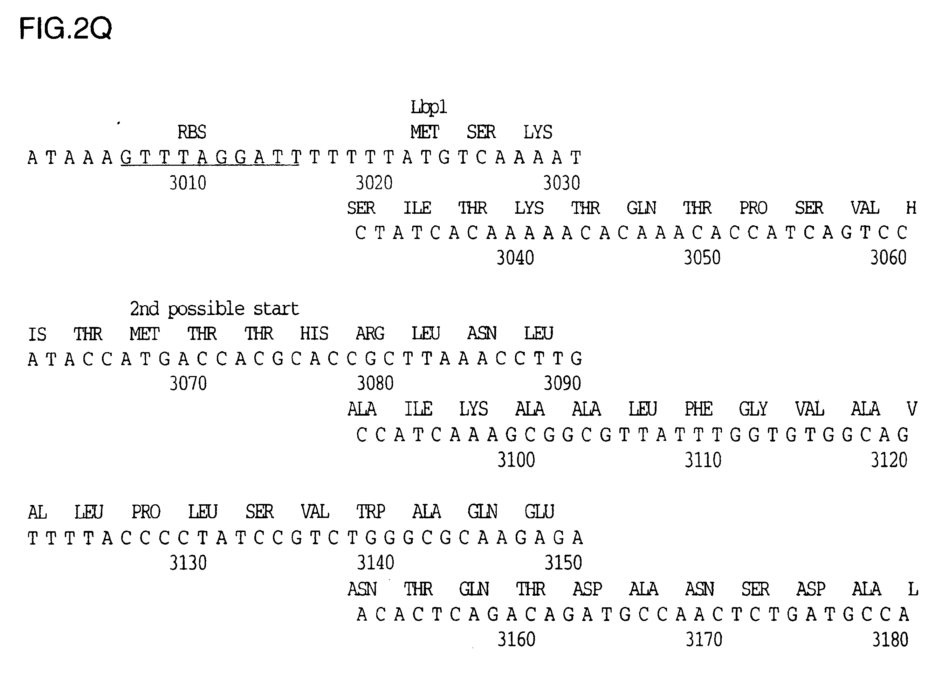

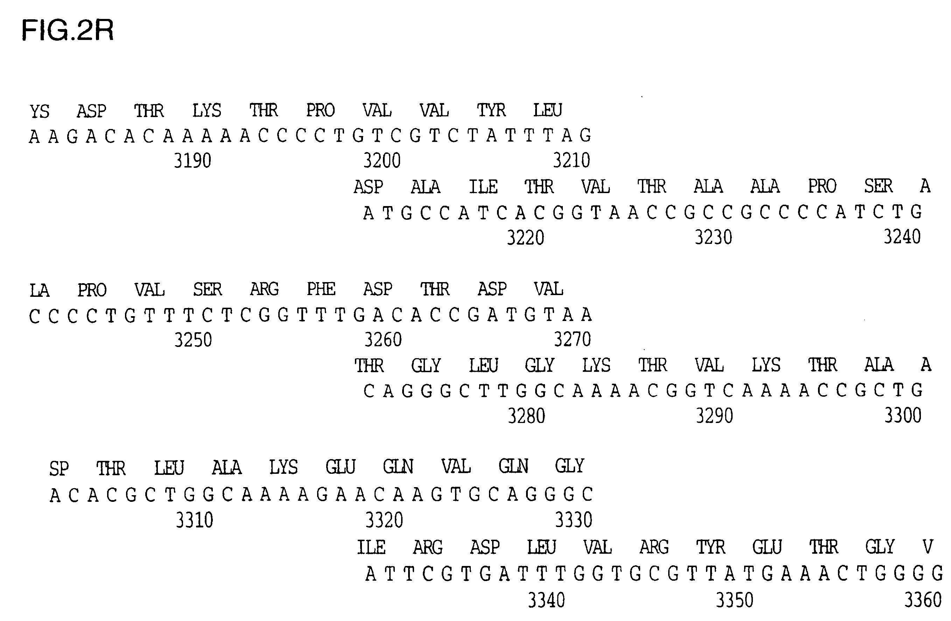

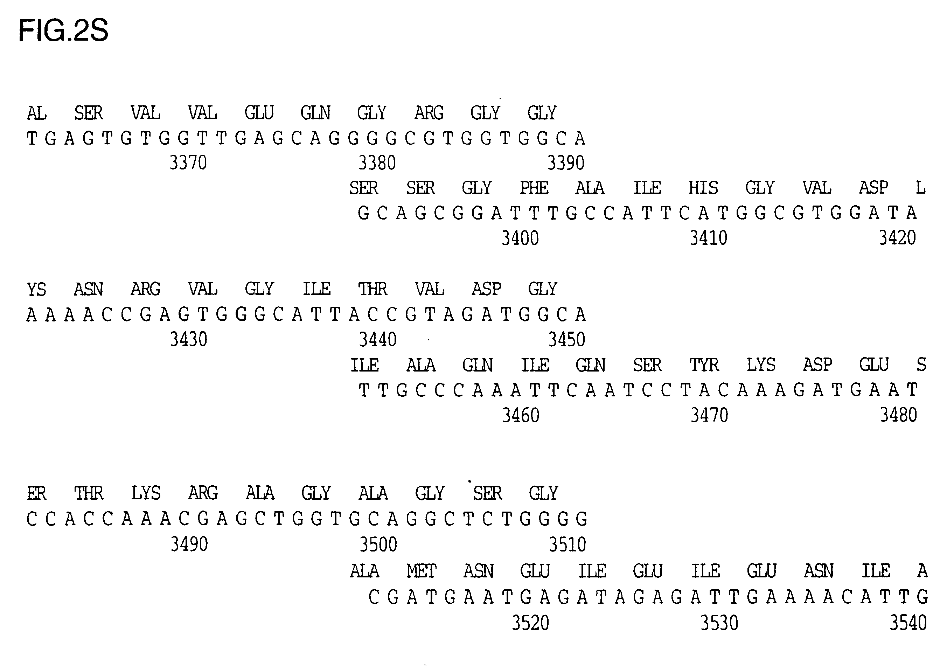

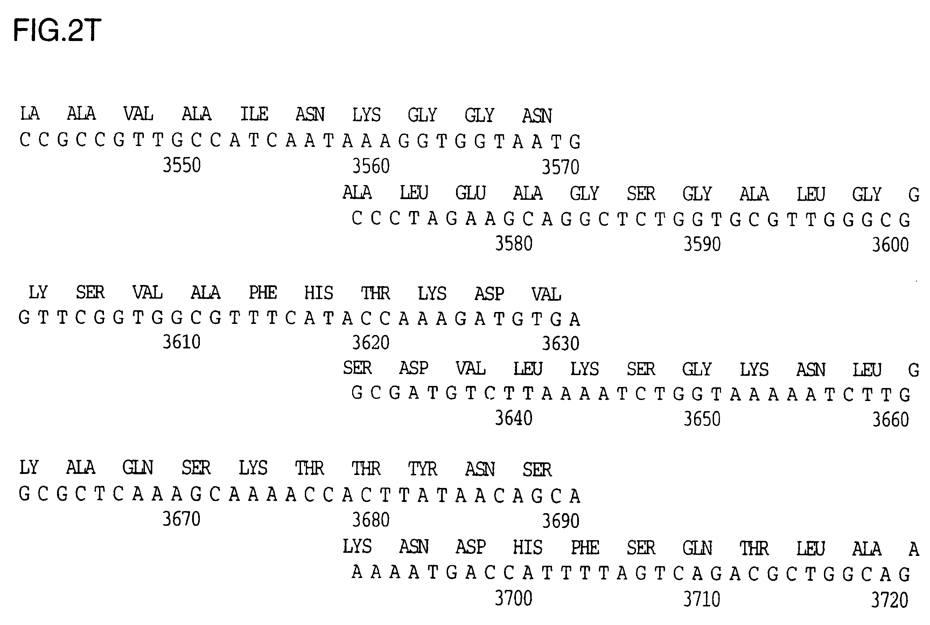

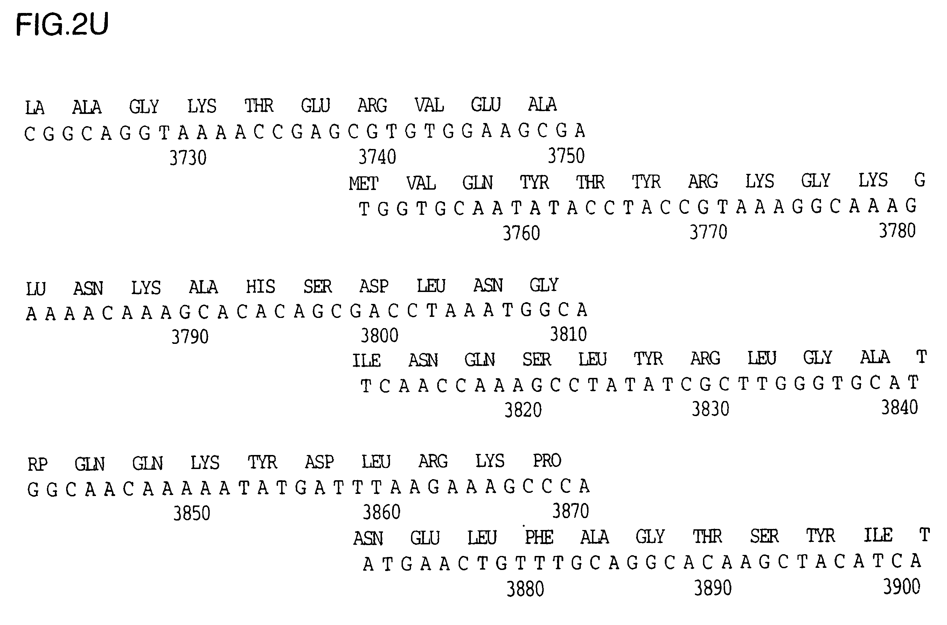

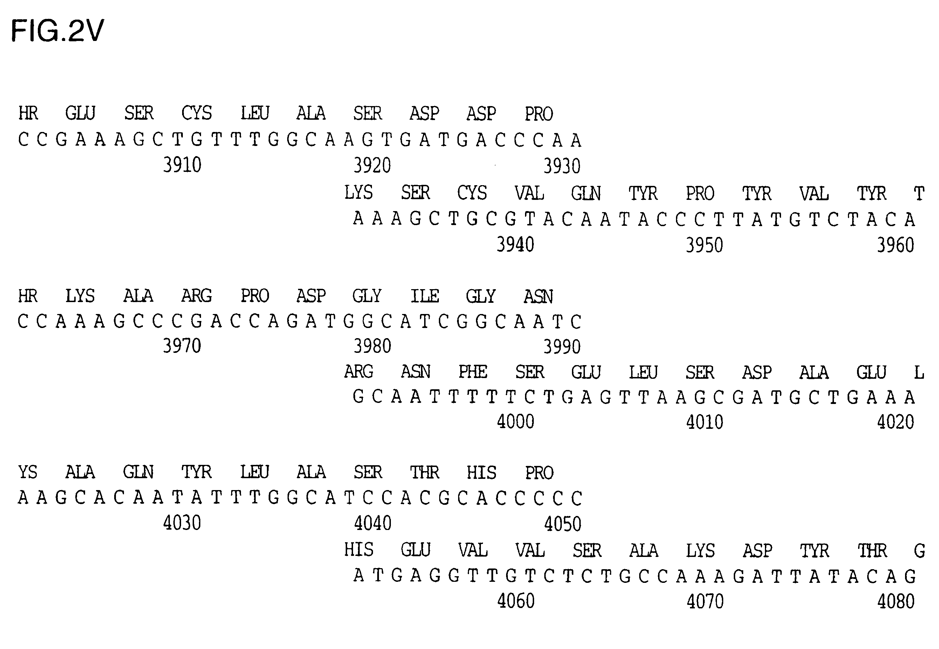

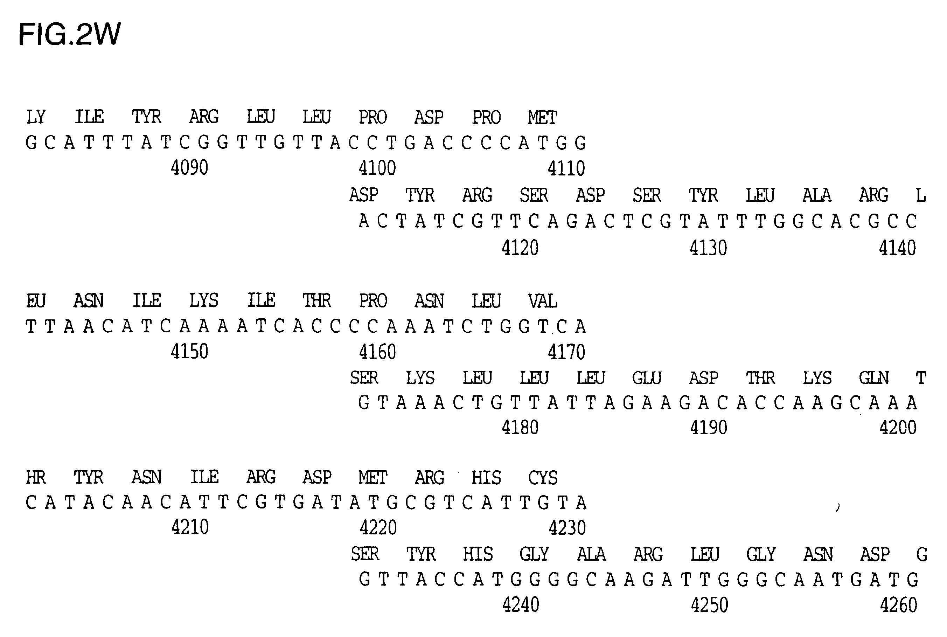

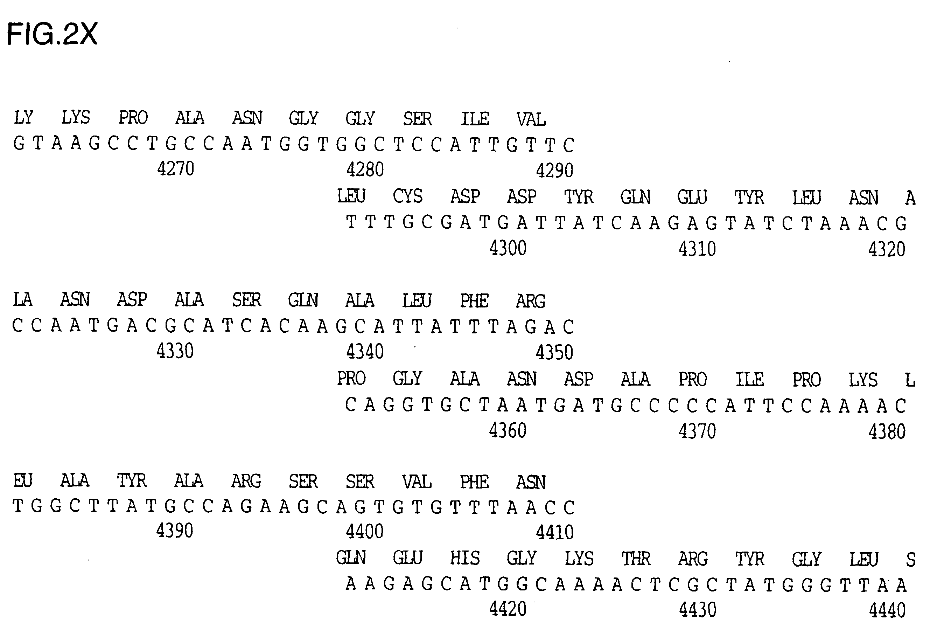

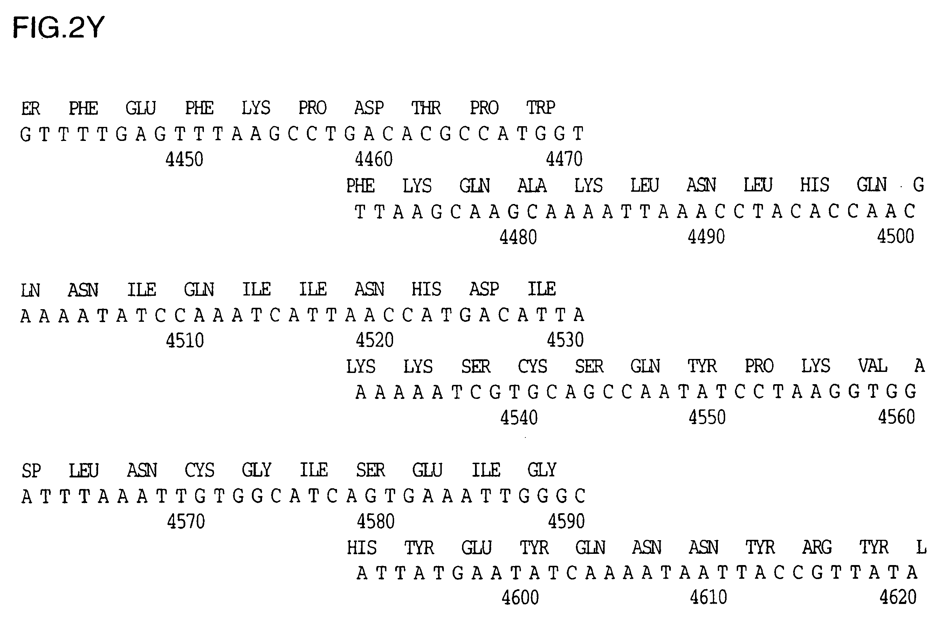

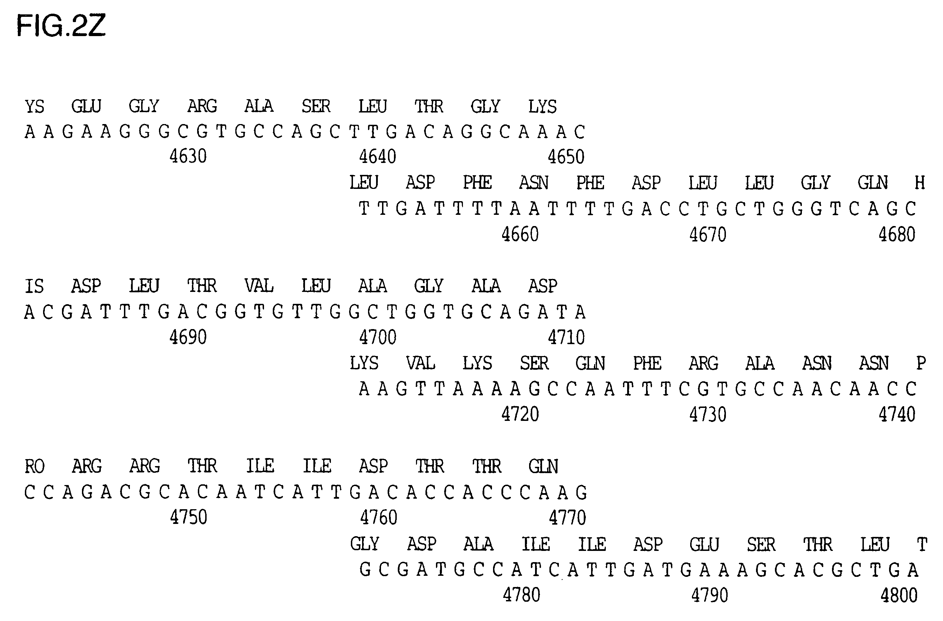

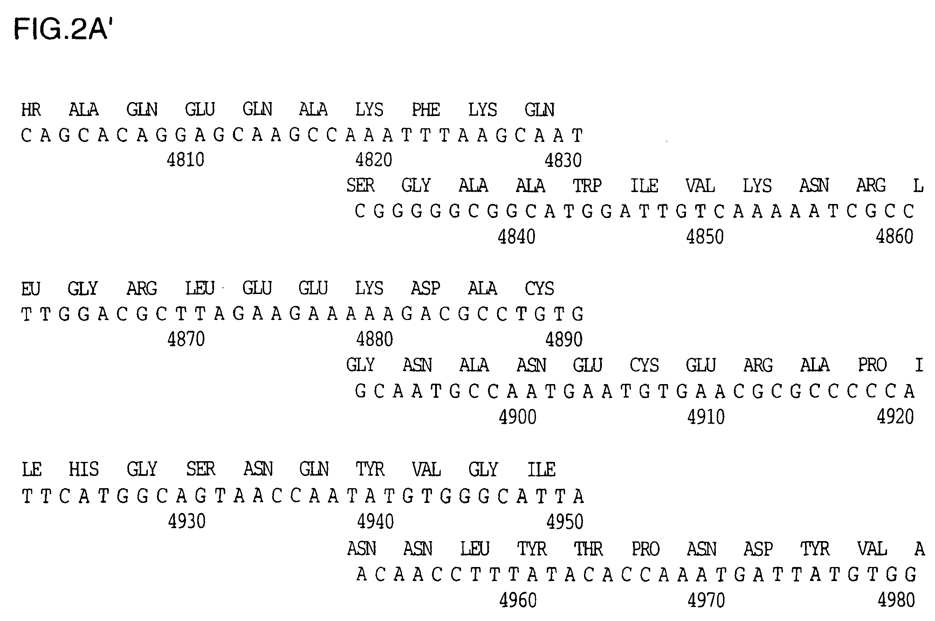

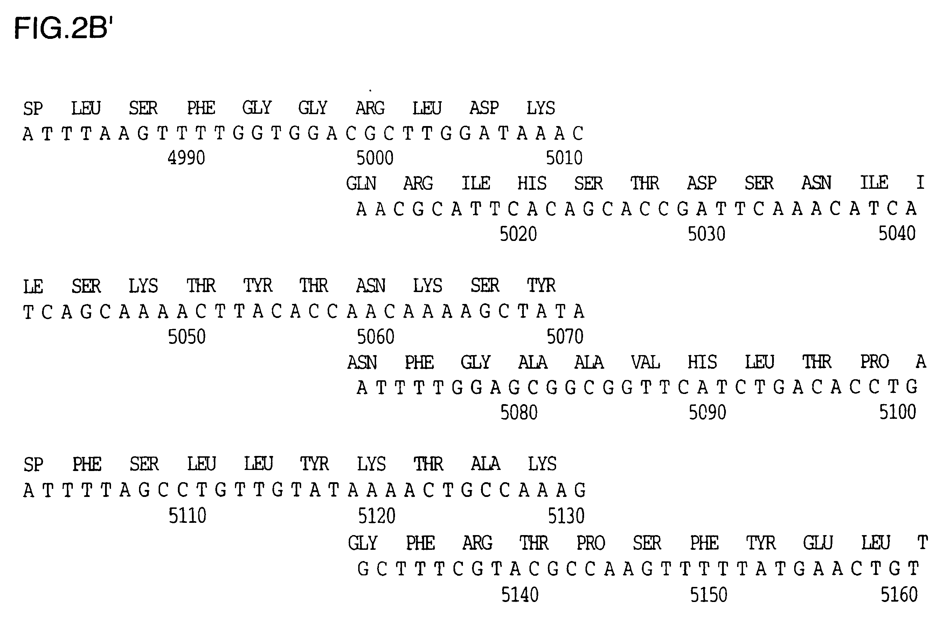

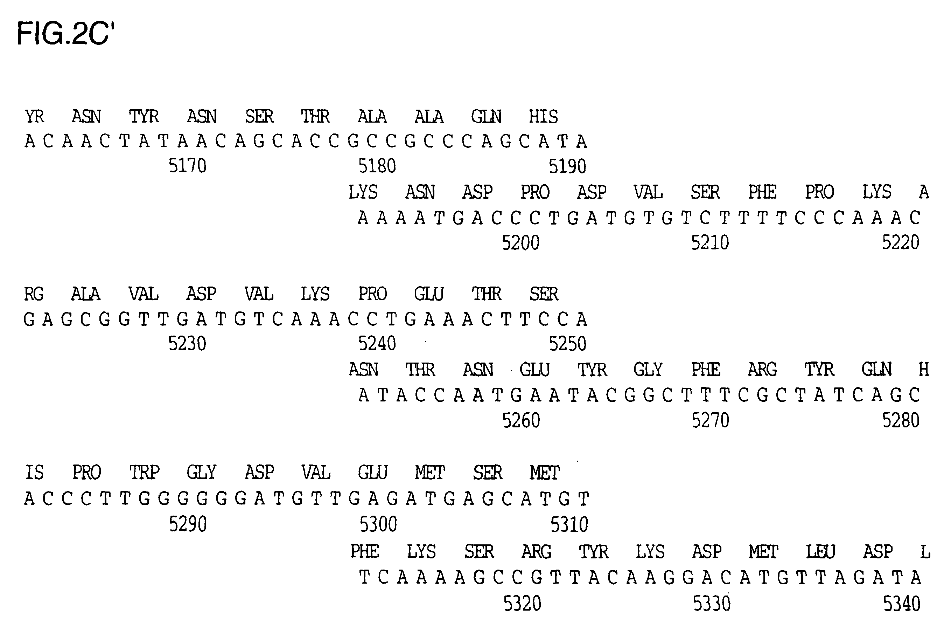

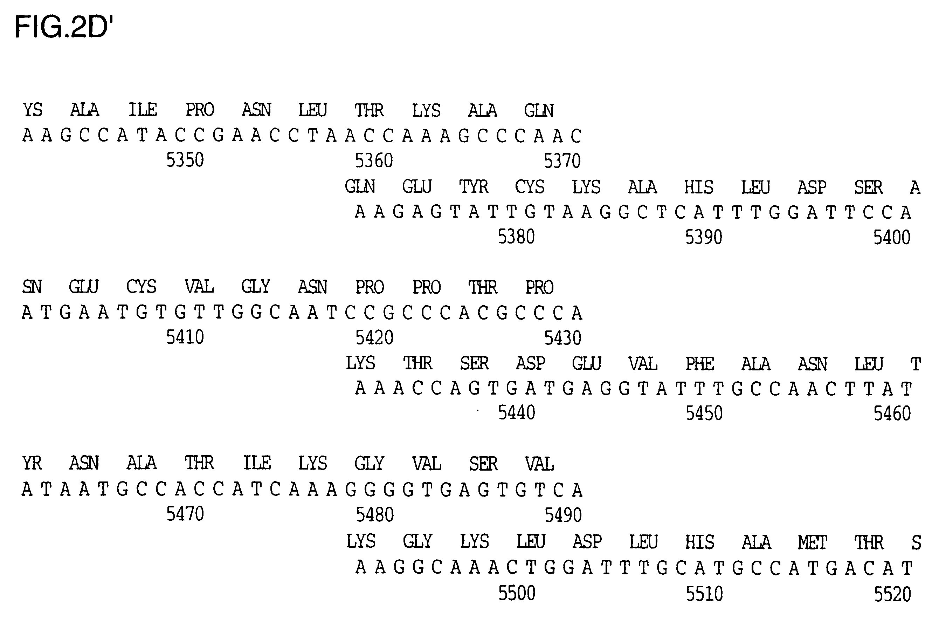

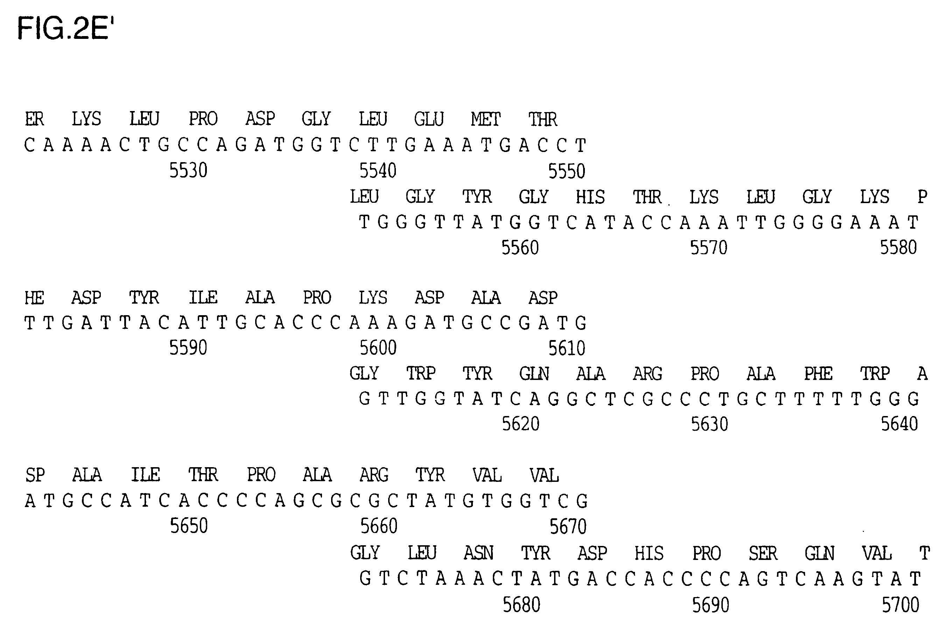

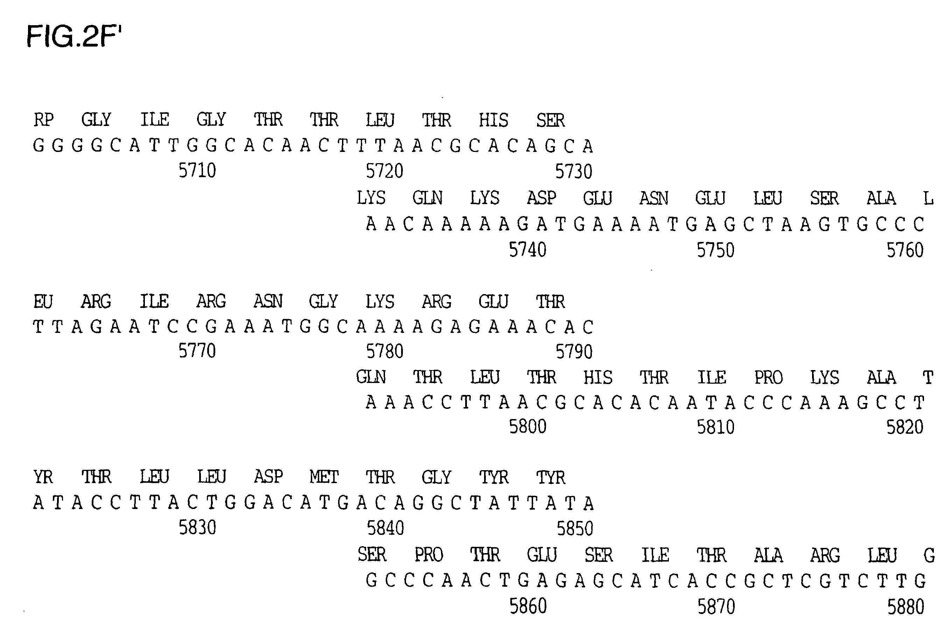

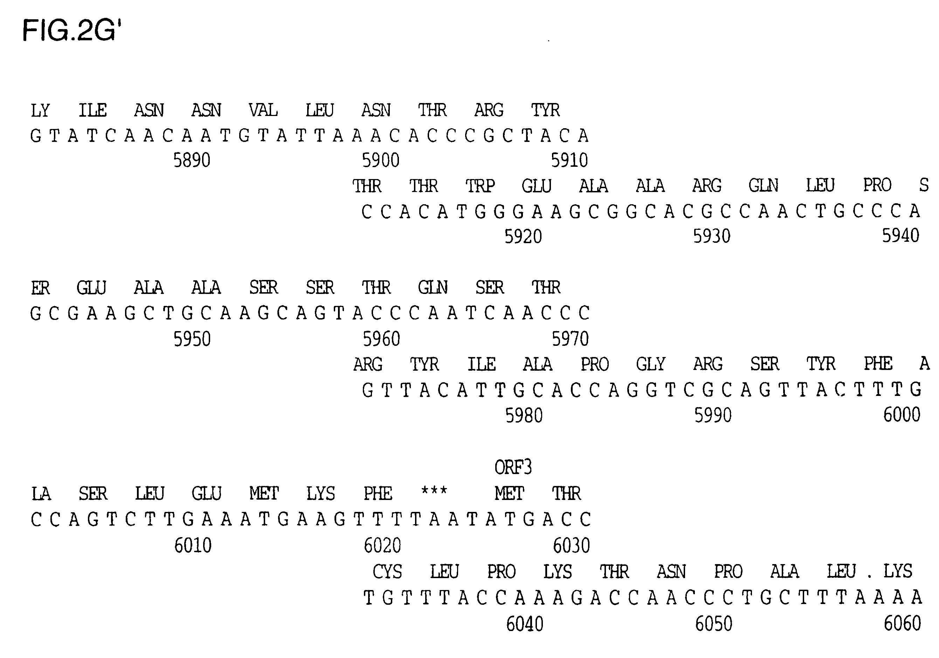

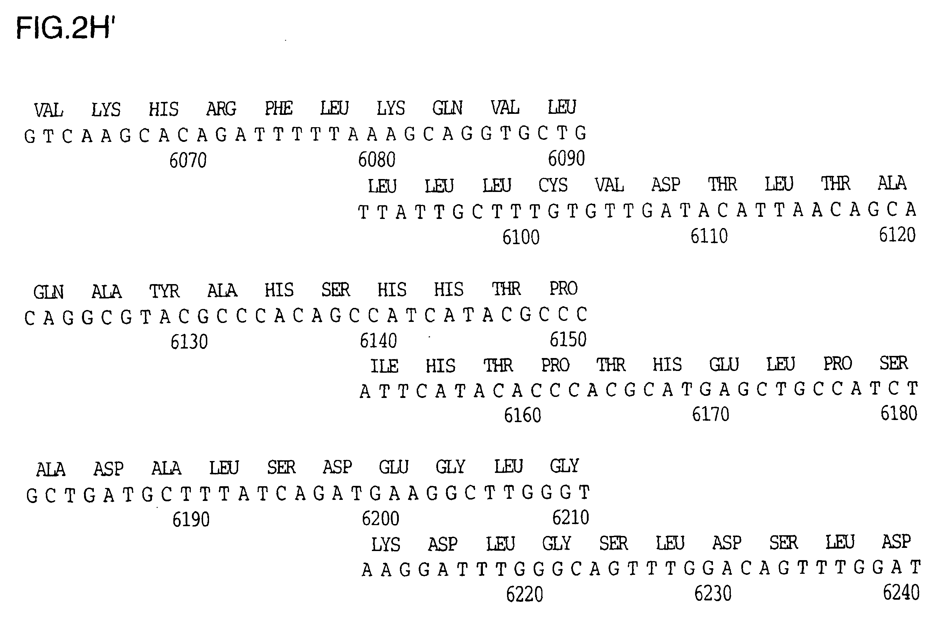

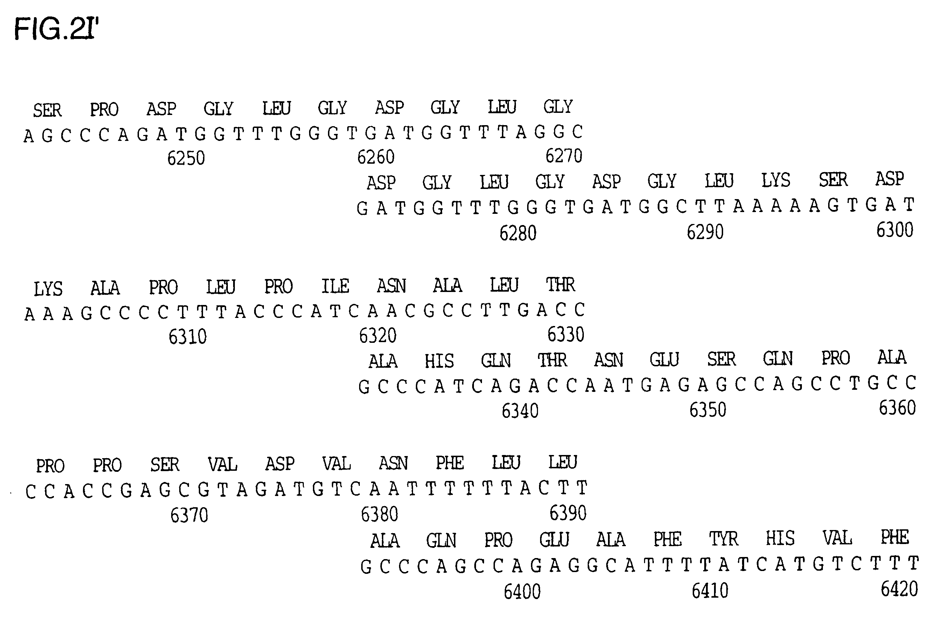

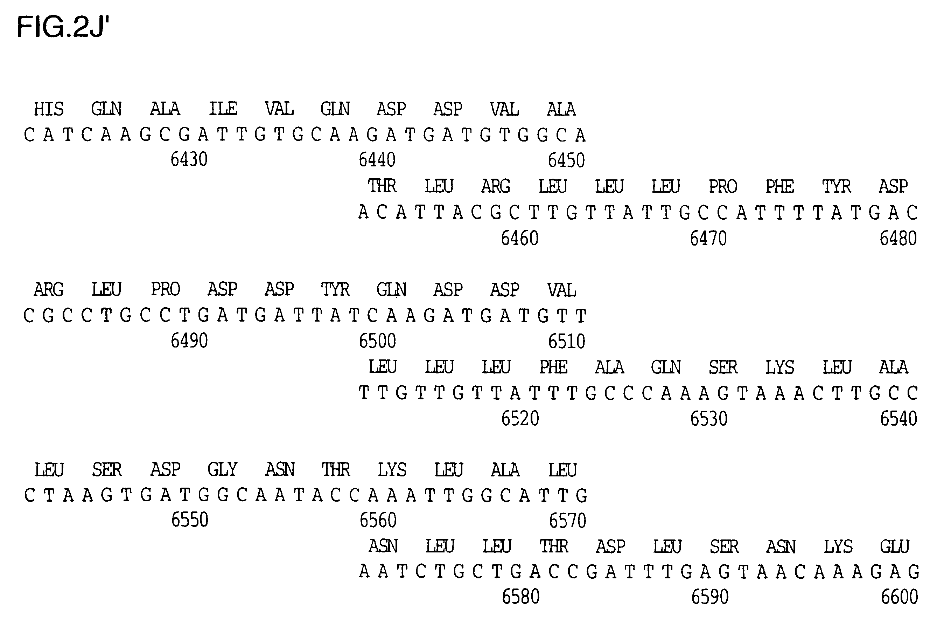

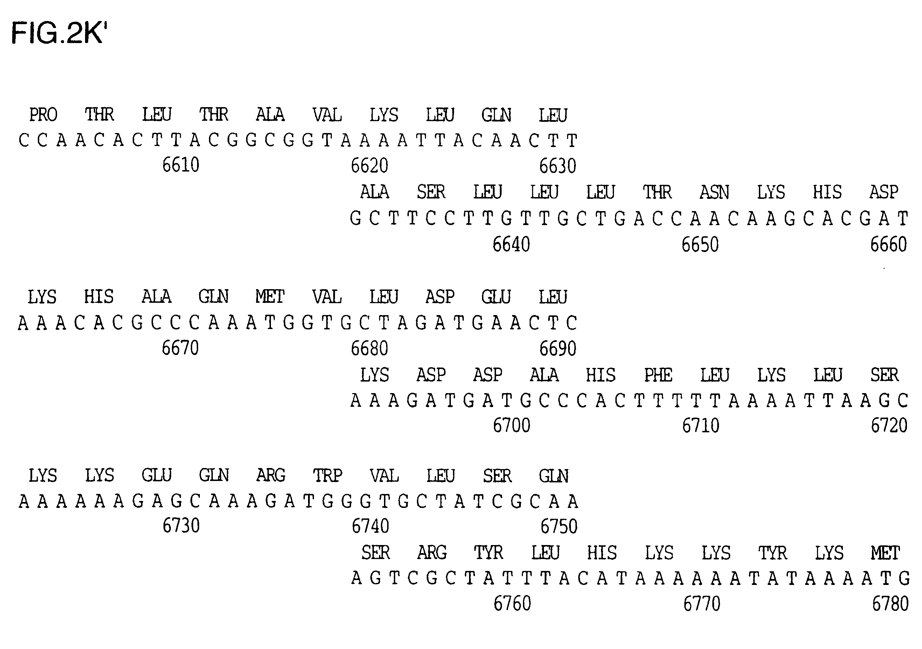

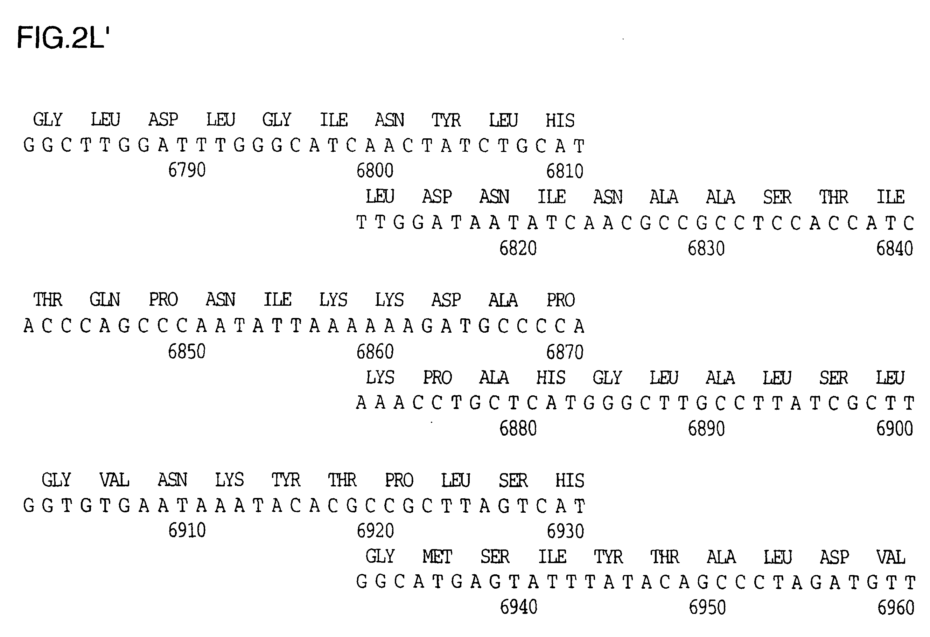

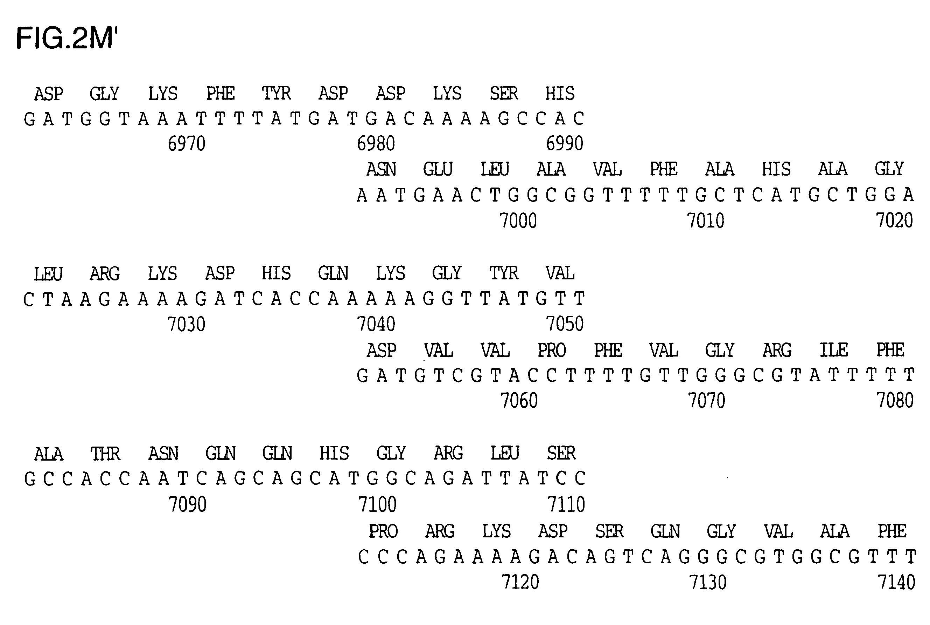

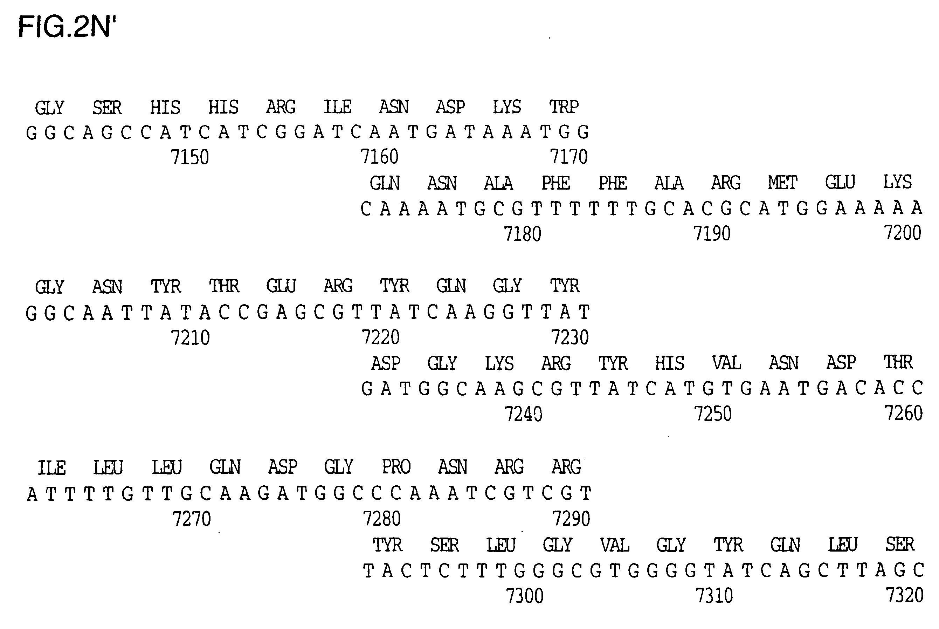

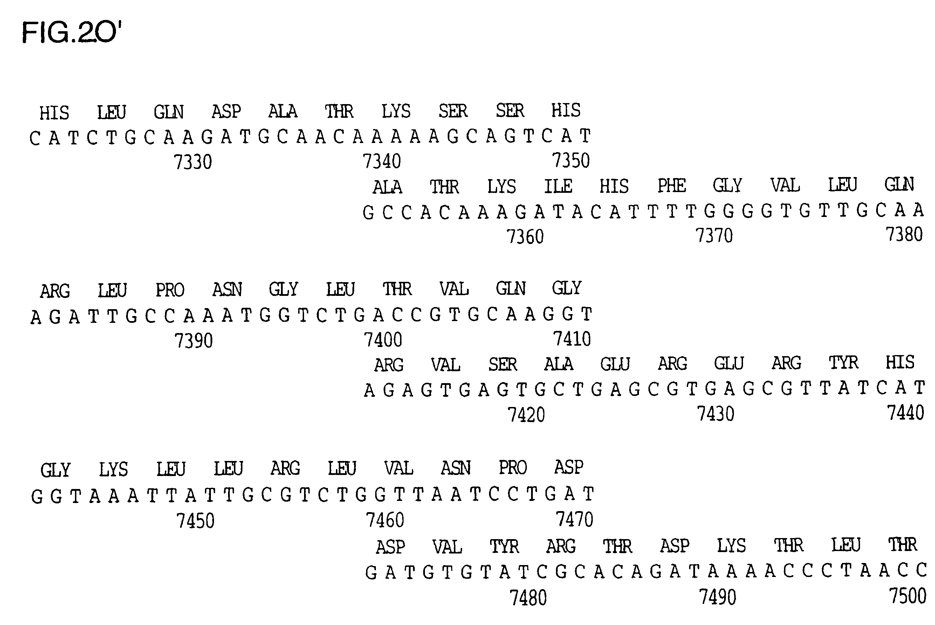

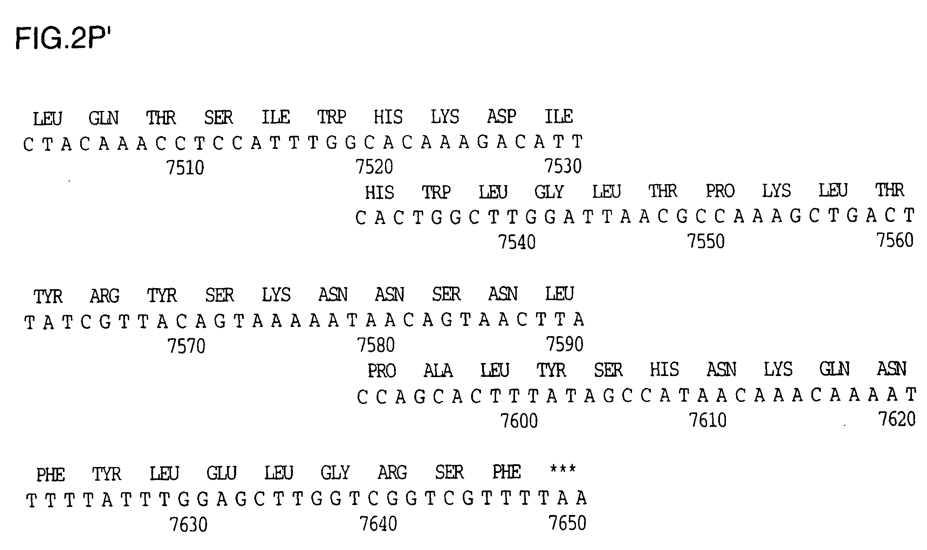

Figure 2 shows the nucleotide (SEQ ID No: 1, full sequence; SEQ ID No: 2, Lbp2 coding sequence; SEQ ID No: 3, Lbp1 coding sequence, first methionine; SEQ ID No: 4, Lbp1 coding sequence, second methionine; SEQ ID No: 5, ORF3 coding sequence) and deduced amino acid sequences (SEQ ID No: 11, Lbp2; SEQ ID No: 12, Lbp1, first methionine; SEQ ID No: 13, Lbp1, second methionine; SEQ ID No: 14, ORF3) of the putative *lfr* locus from *M. catarrhalis* 4223. There are three tandem genes in the putative *lfr* locus identified as *lbpB, lbpA* and *orf3.* Potential promoter elements found upstream of the *lbpB* and *lbpA* genes are indicated by underlining.

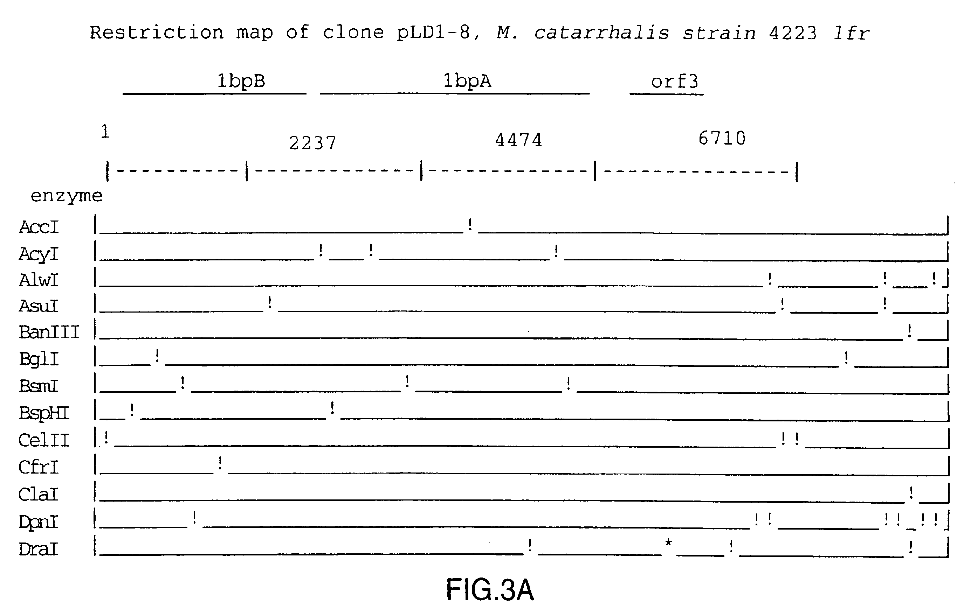

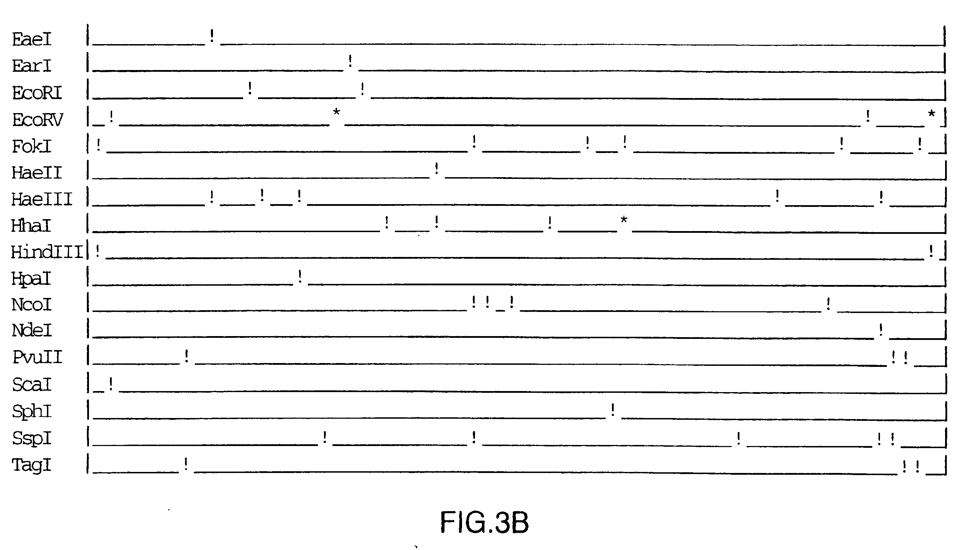

Figure 3 shows a restriction map of clone pLD1-8 containing the *lbpA, lbpB,* and *orf3* genes from *M. catarrhalis* isolate 4223.

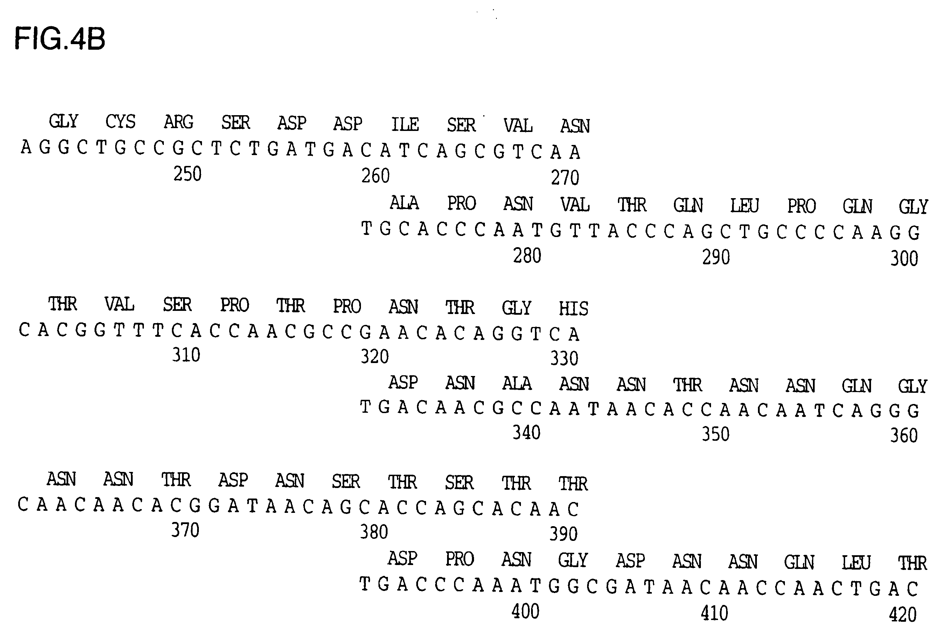

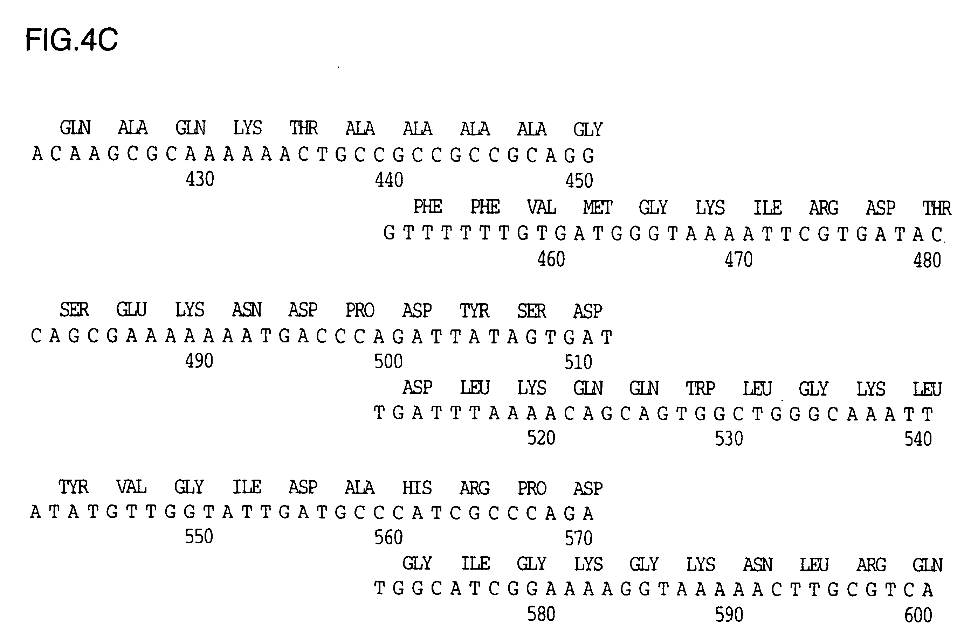

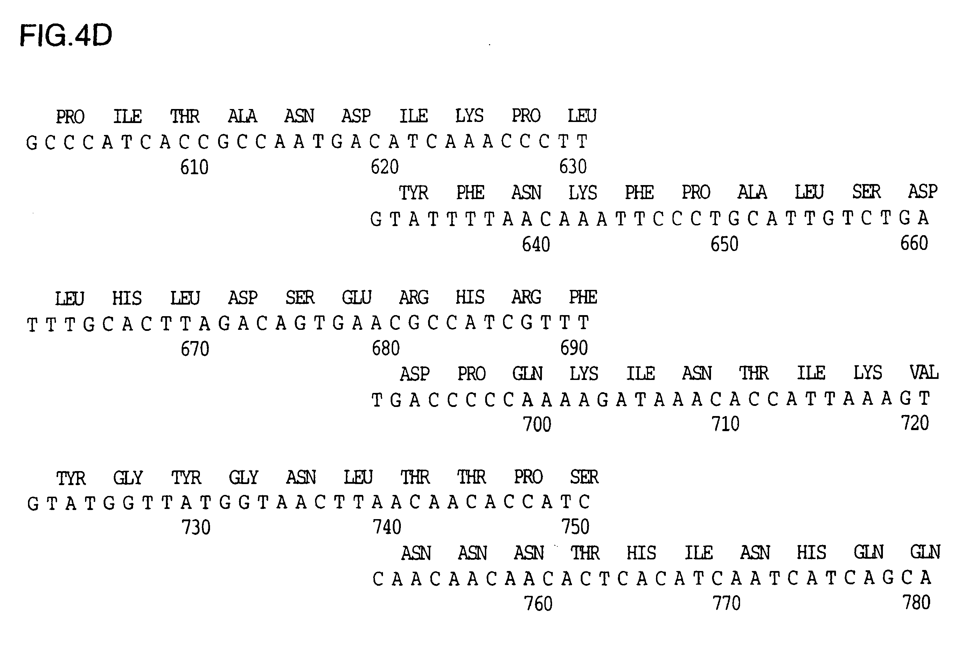

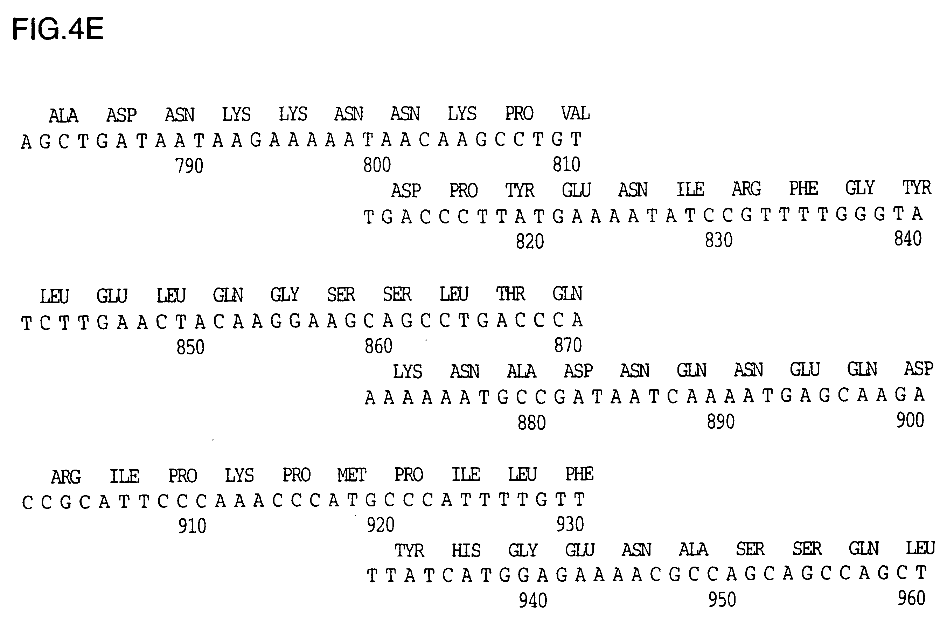

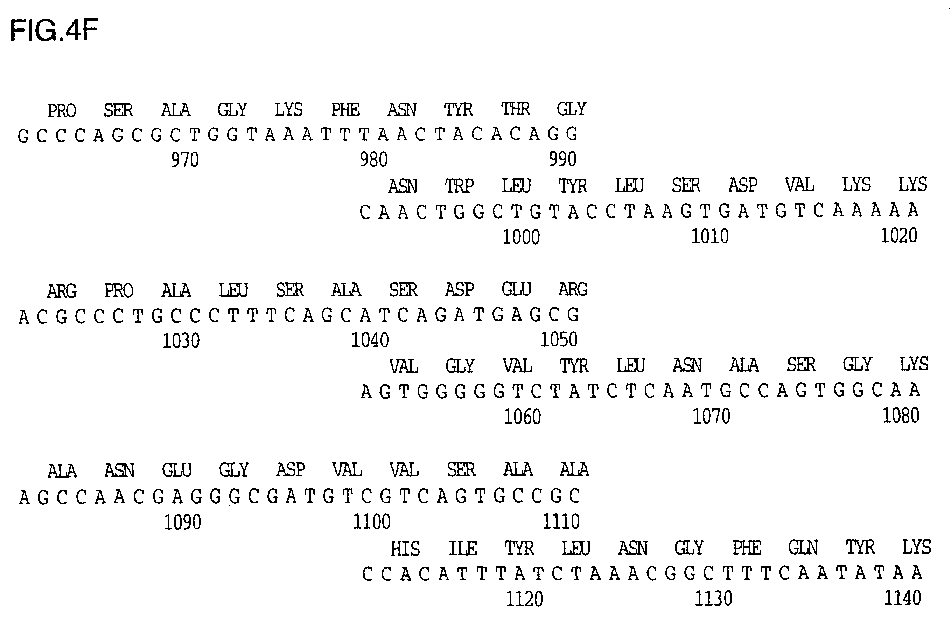

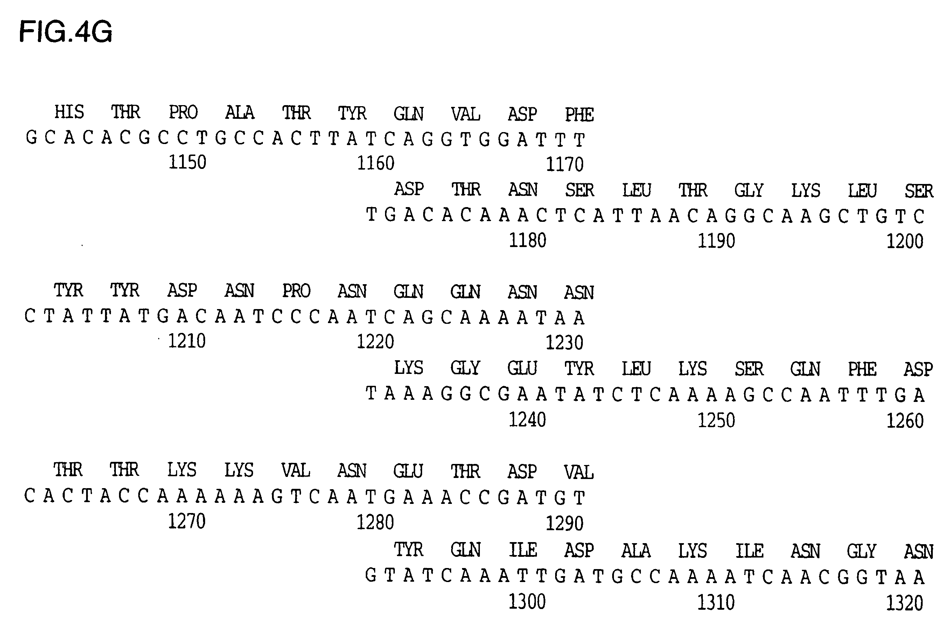

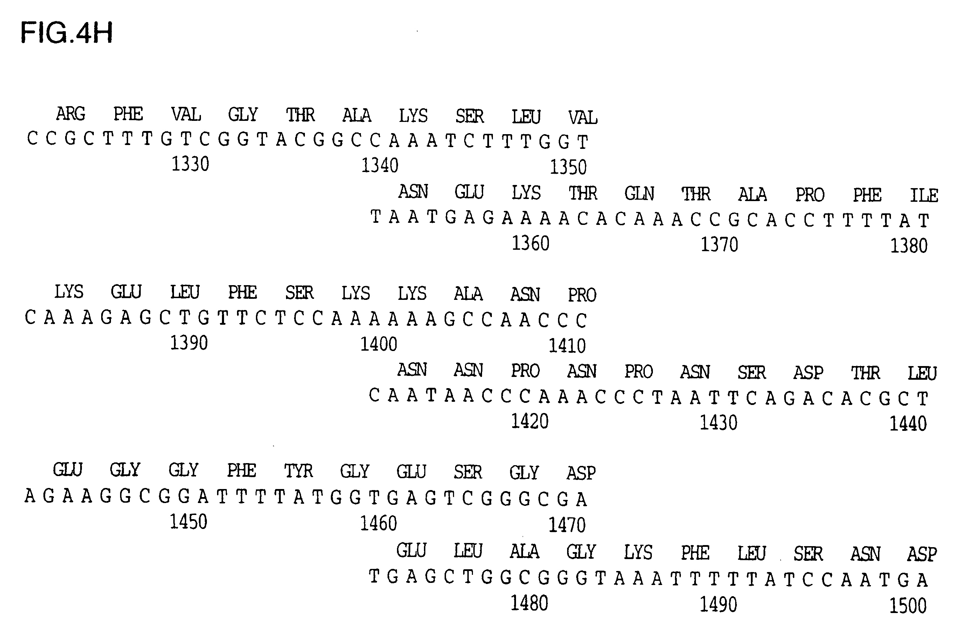

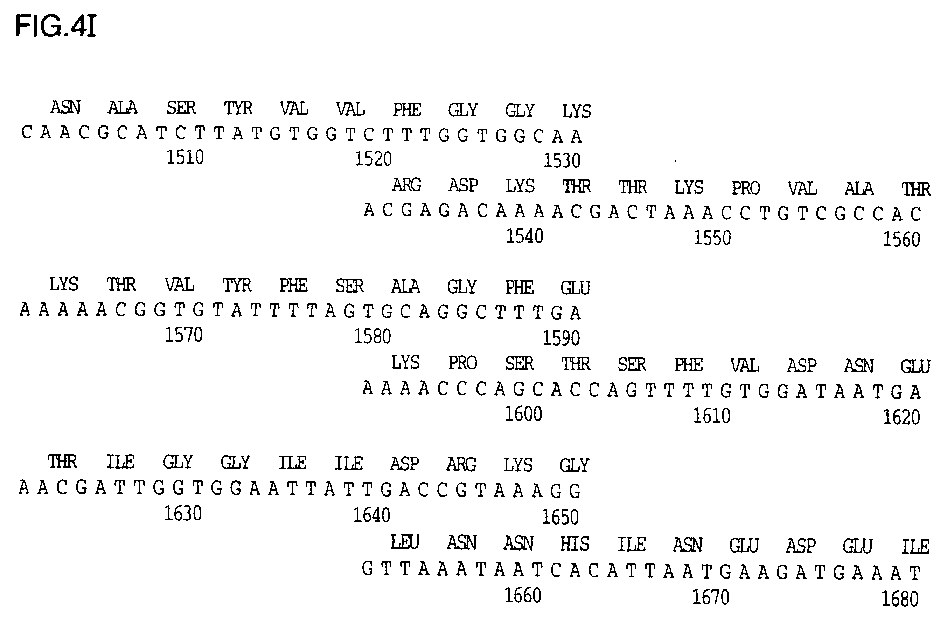

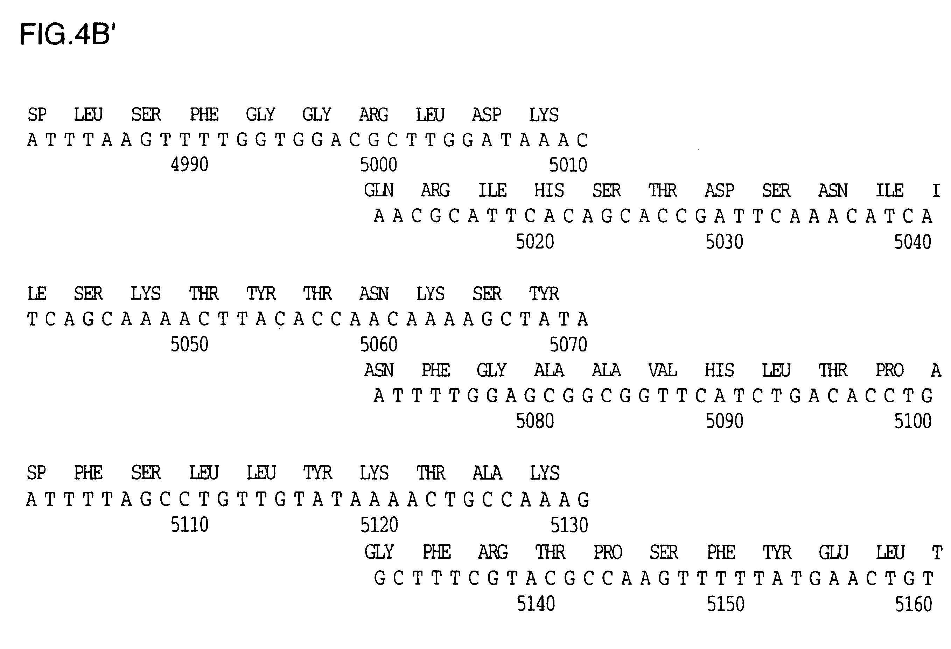

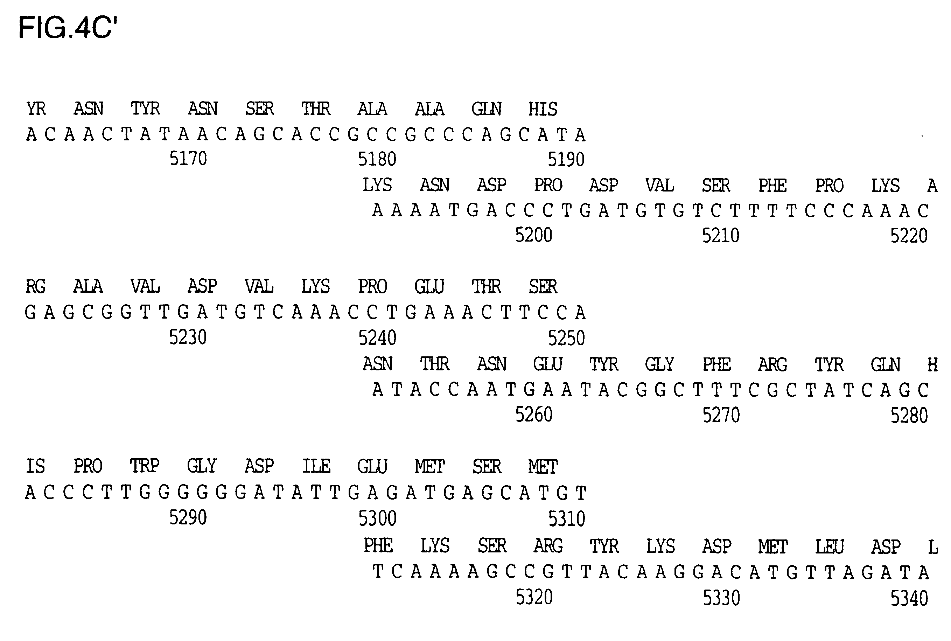

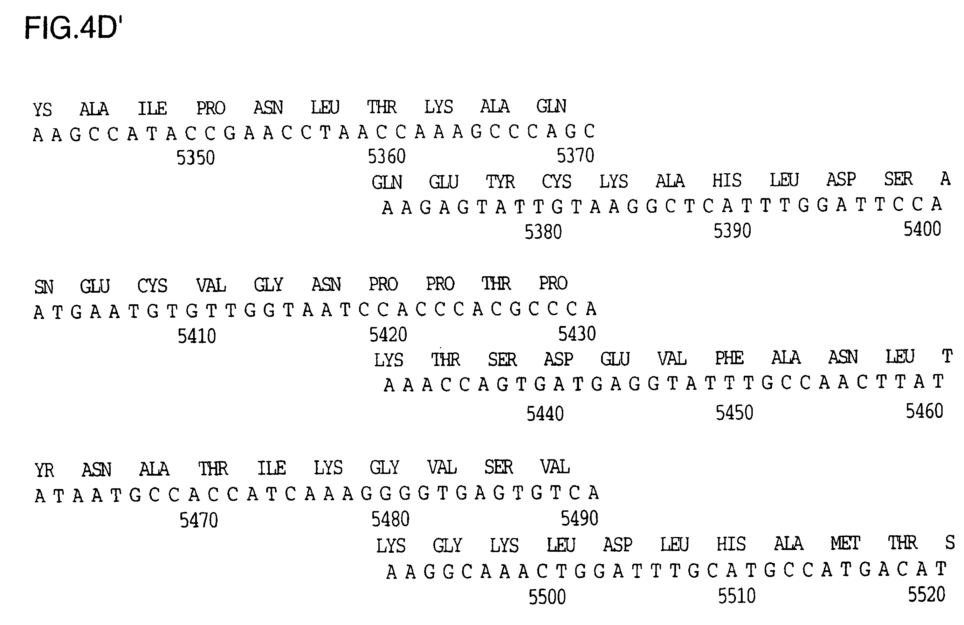

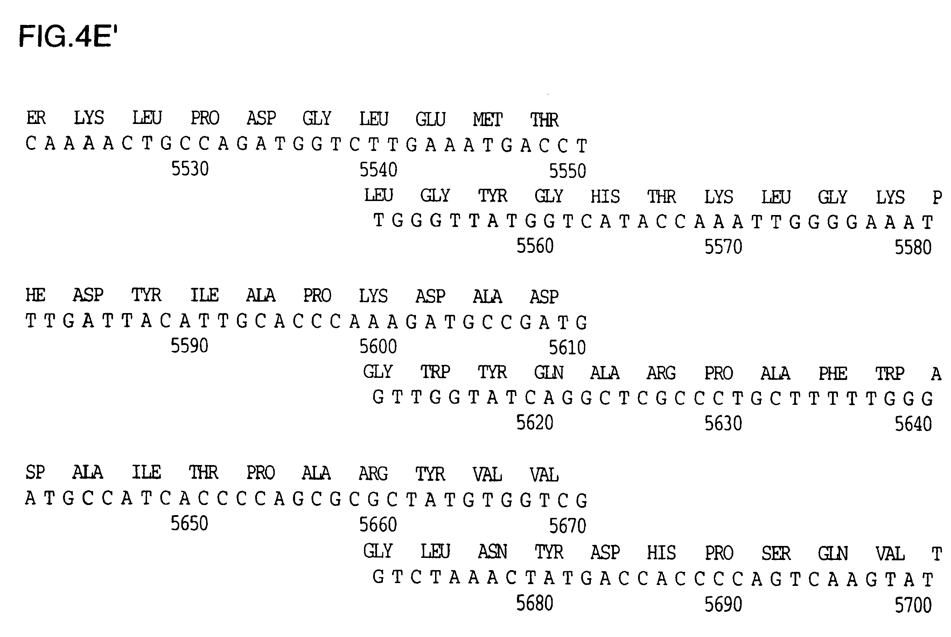

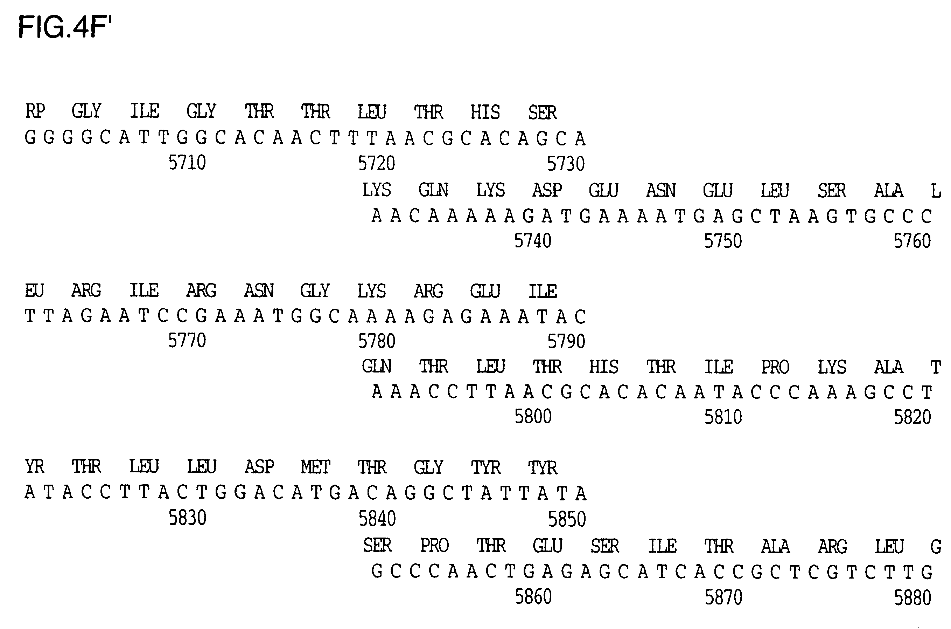

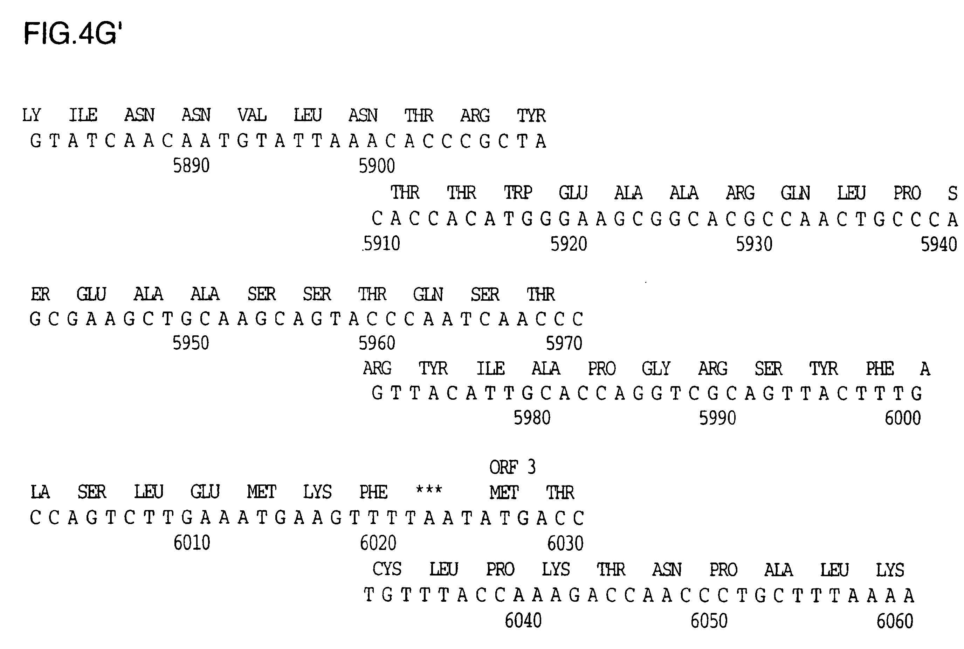

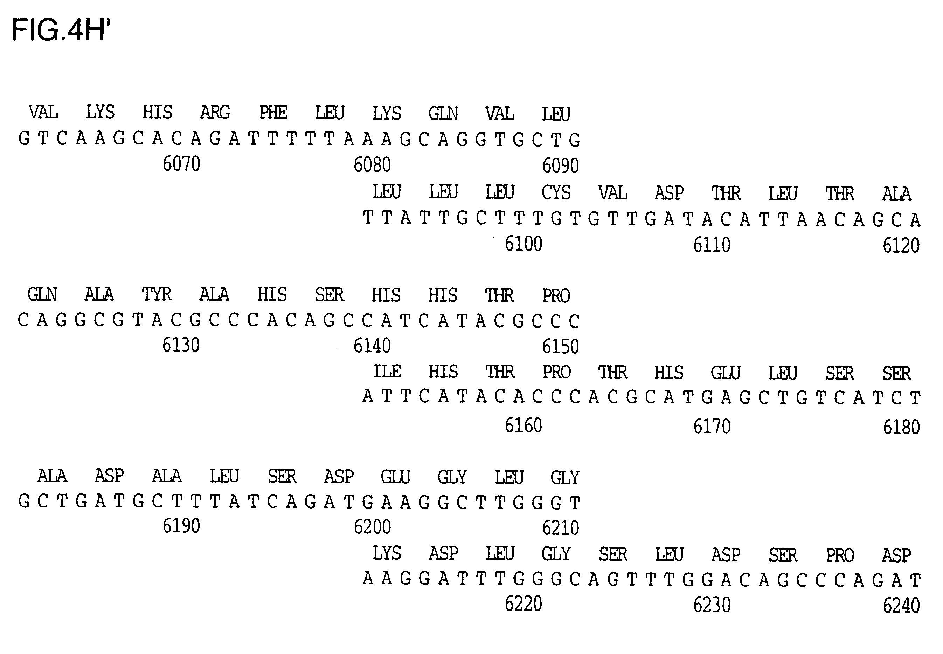

Figure 4 shows the nucleotide (SEQ ID No: 6, full sequence; SEQ ID No: 7, Lbp2 coding sequence; SEQ ID No: 8, Lbp1 coding sequence, first methionine; SEQ ID No: 9, Lbp1, second methionine; SEQ ID No: 10, ORF3 coding sequence) and deduced amino acid sequences (SEQ ID No: 15, Lbp2; SEQ ID No: 16, Lbp1, first methionine; SEQ ID No: 17, Lbp1, second methionine; SEQ ID No: 18, Lbp3) of the putative *lfr* locus from *M. catarrhalis* Q8. There are three tandem genes in the putative *lfr* locus identified as *lbpB, lbpA* and *orf3.* Potential promoter elements found upstream of the *lbpB* and *lbpA* genes are indicated by underlining.

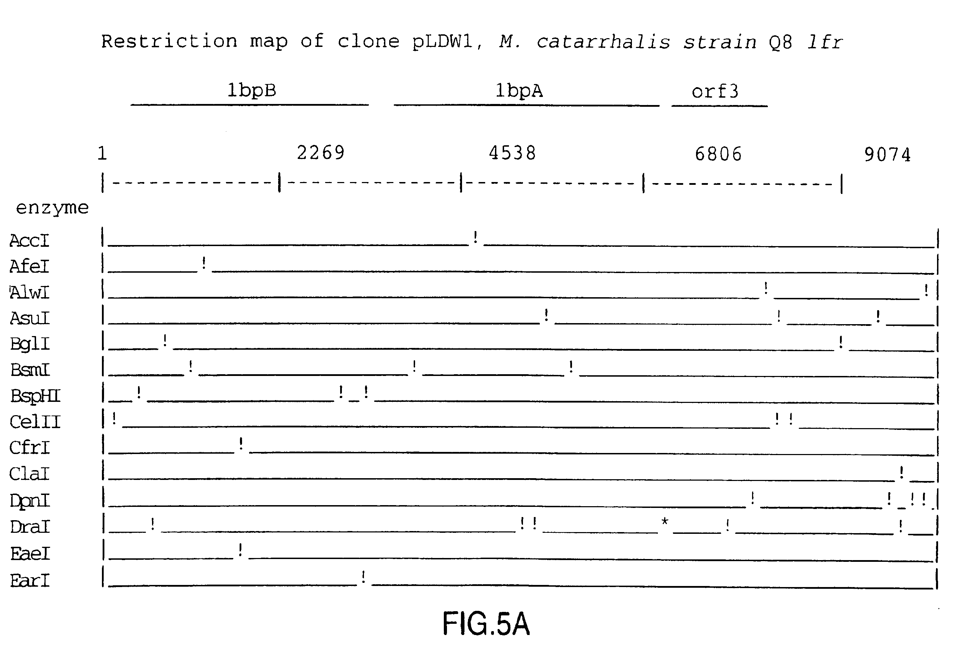

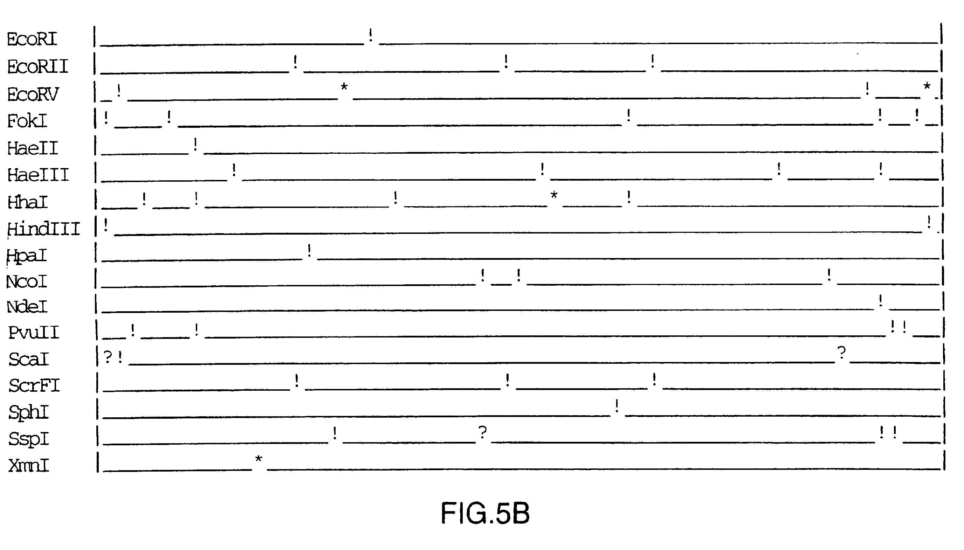

Figure 5 shows a restriction map of clone pLDW1 containing the *lbpA, lbpB* and *orf3* genes from *M. catarrhalis* isolate Q8.

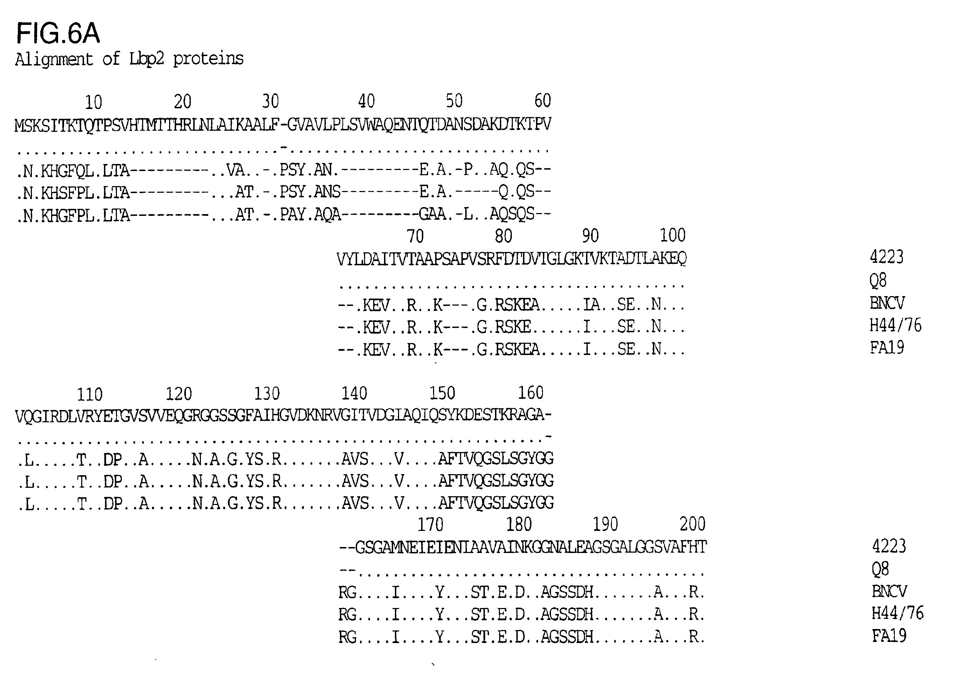

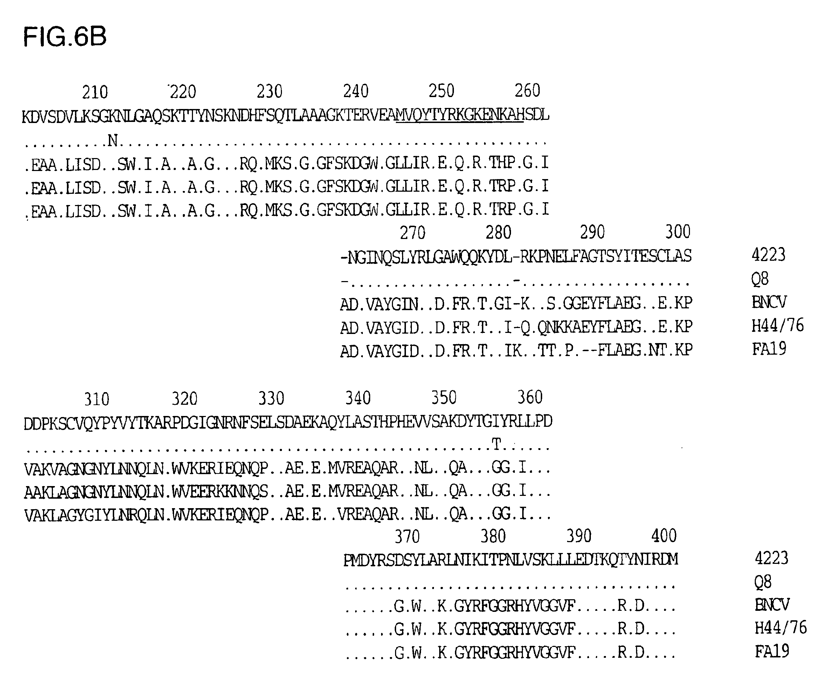

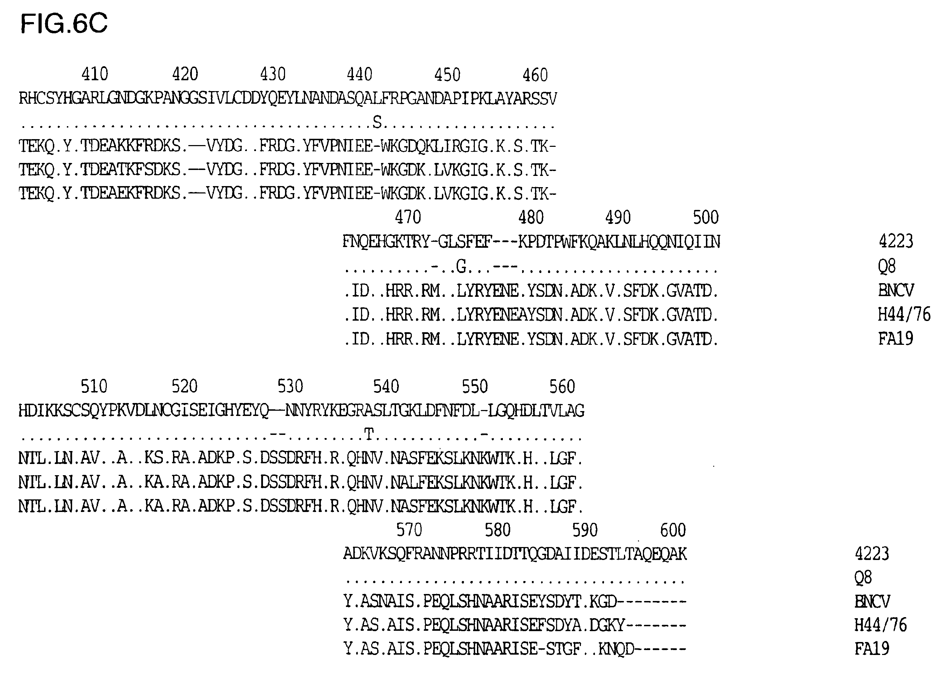

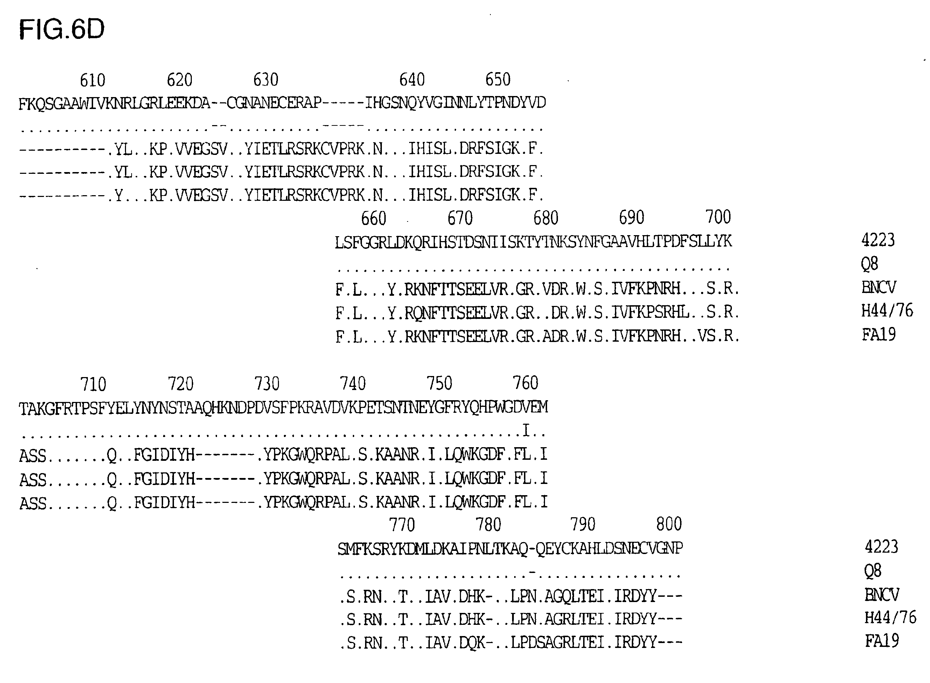

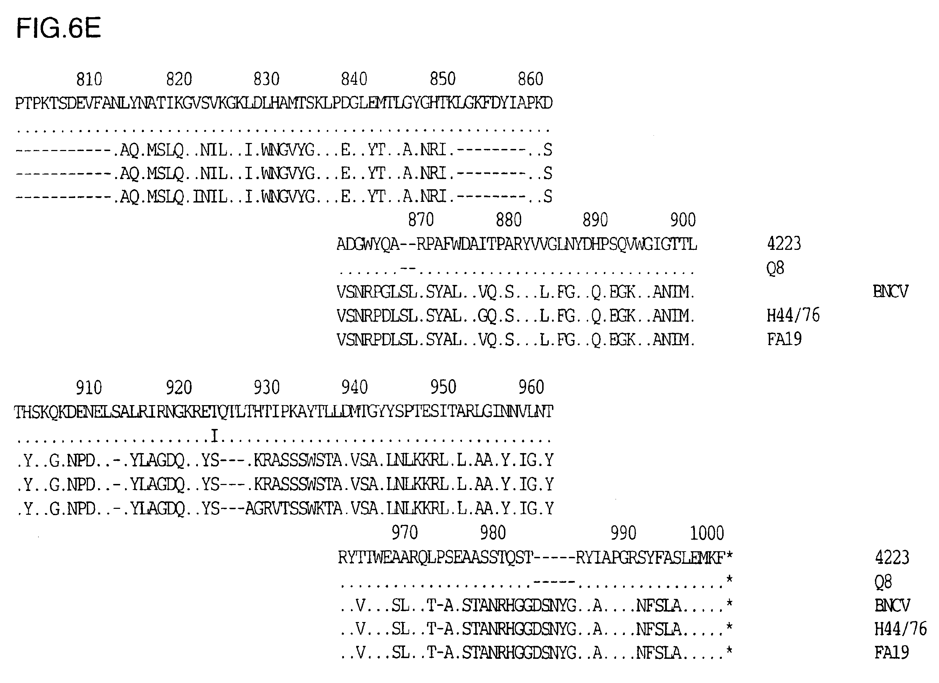

Figure 6 shows a comparison of the amino acid sequences of Lbp1 from *M. catarrhalis* strains 4223 (SEQ ID No: 12) and Q8 (SEQ ID No: 16), *N. meningitidis* strains BNCV (SEQ ID No: 23) and H44/76 (SEQ ID No: 75), and *N. gonorrhoeae* strain FA19 (SEQ ID No: 24). Also shown is the partial carboxy terminal sequence of Lbp2 from *N. meningitidis* strains BNCV (SEQ ID No: 76) and H44/76 (SEQ ID No: 77) and *N. gonorrhoeae* strain FA19 (SEQ ID No: 75). Dots indicate identical residues and dashes have been introduced to achieve maximum sequence alignment.

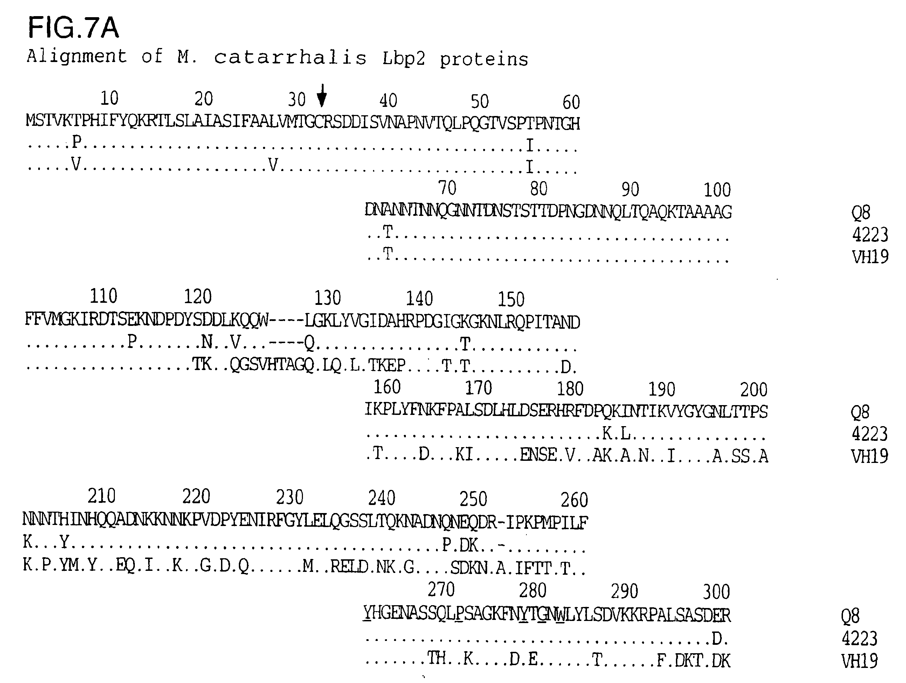

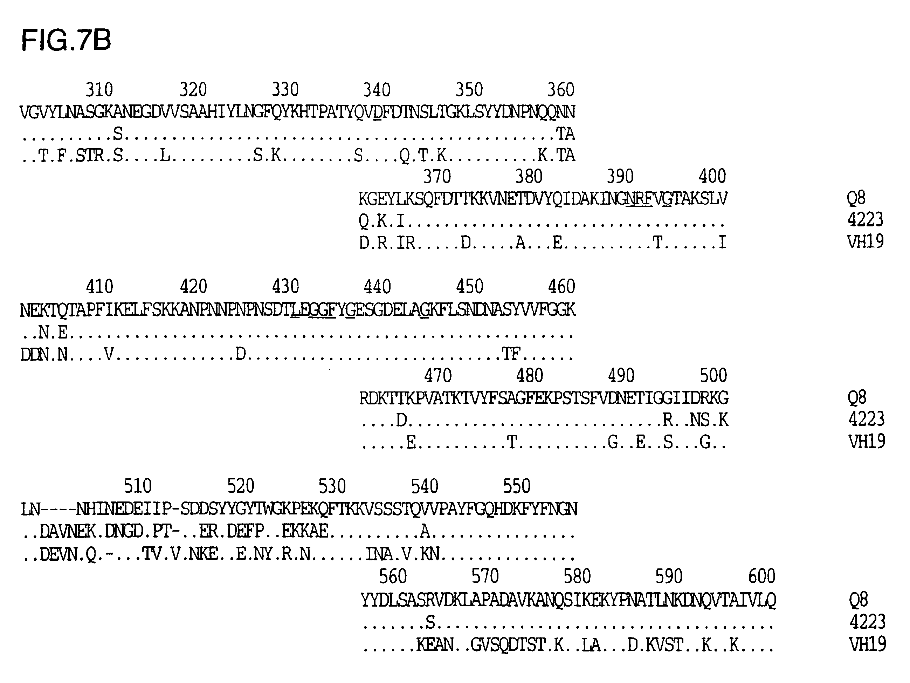

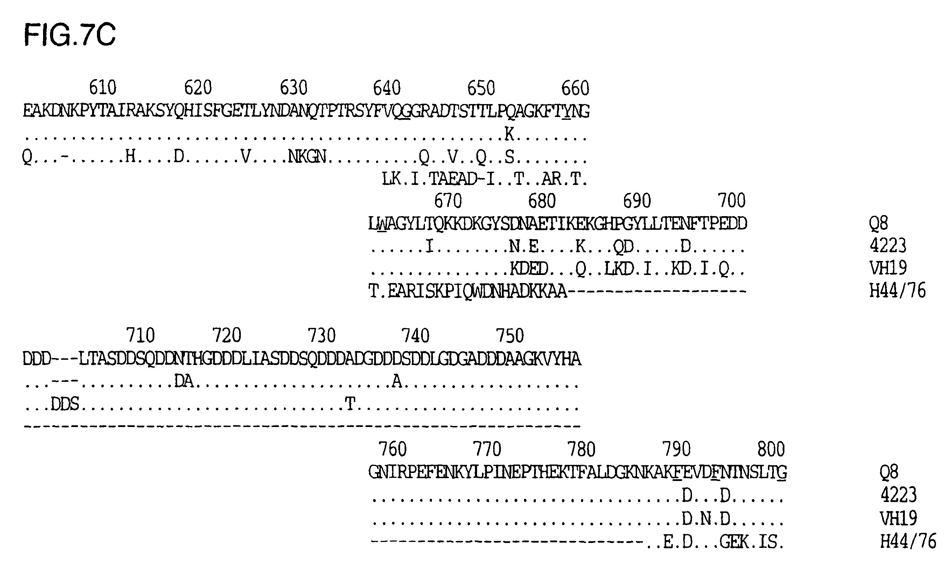

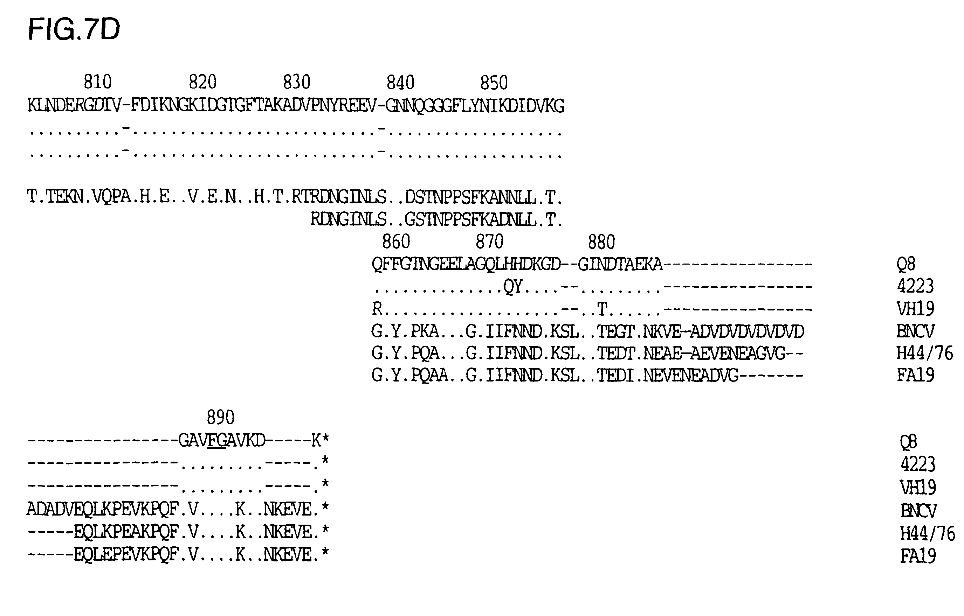

Figure 7 shows a comparison of the amino acid sequences of Lbp2 from *M. catarrhalis* strains 4223 (SEQ ID No: 11), Q8 (SEQ ID No: 15) and VH19 (SEQ ID No: 70). Dots indicate identical residues. The arrow indicates the lipidated cysteine of a potential mature Lbp2 lipoprotein. The residues conserved with Tbp2 proteins are underlined and the RGD sequence is italicized.

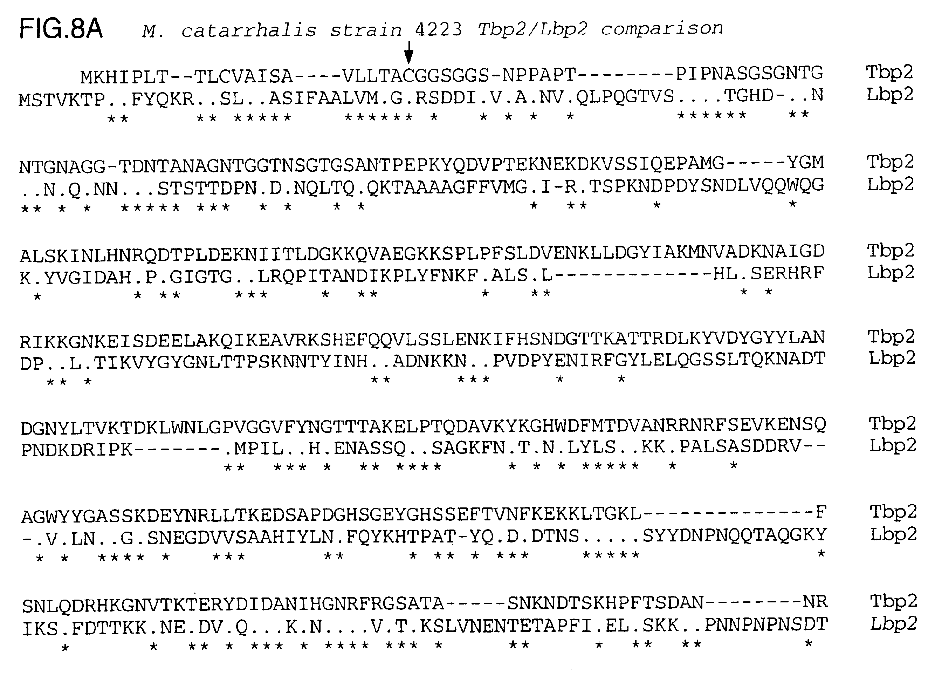

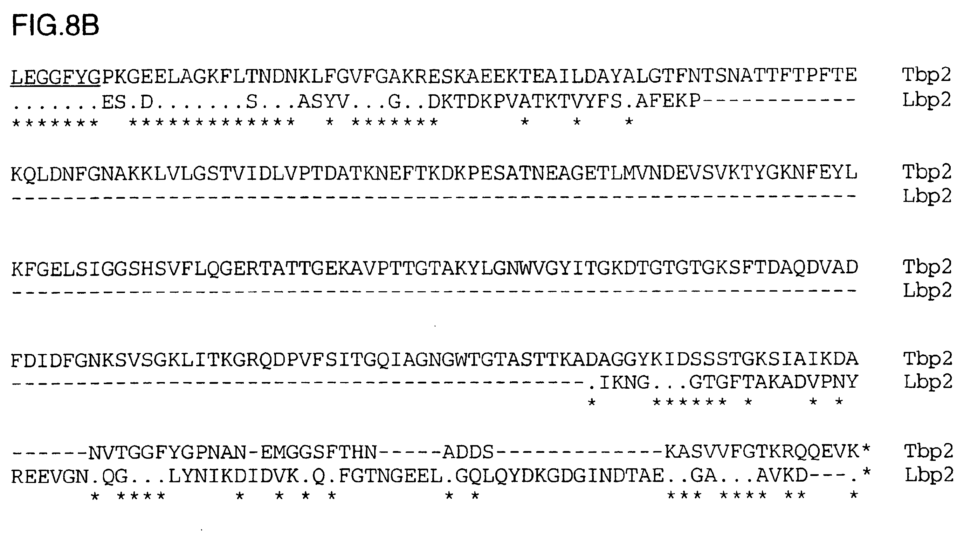

Figure 8 shows a comparison of the amino acid sequences of Tbp2 (USPA No: 08/613,009) (SEQ ID No: 25) and Lbp2 from *M. catarrhalis* strain 4223 (SEQ ID No: 11). Dots indicate identical residues and dashes have been inserted to achieve maximum sequence alignment. The asterisks indicate conserved residues and the putative site of lipidation for both proteins is indicated by the arrow.

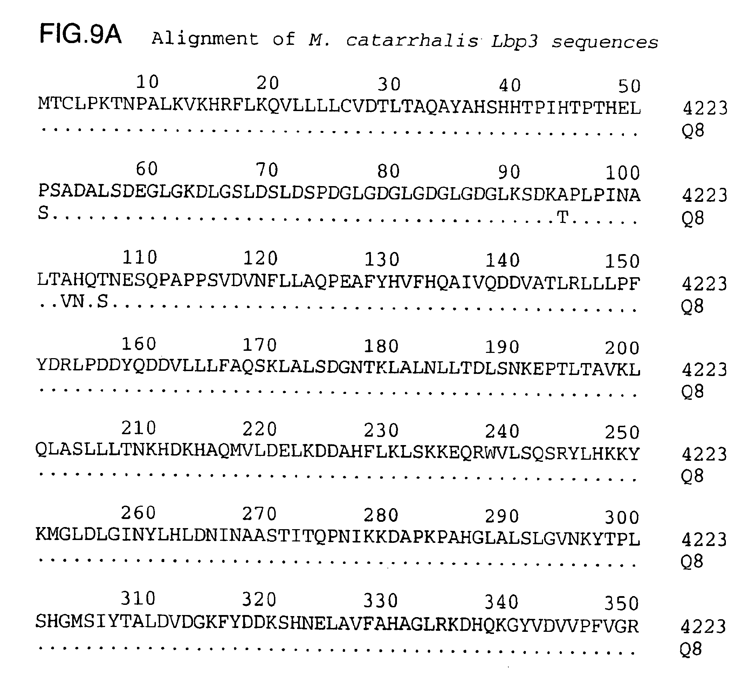

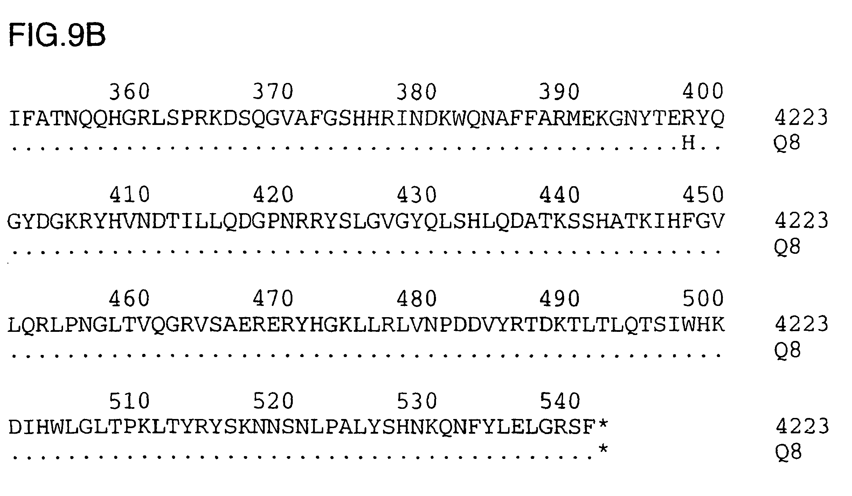

Figure 9 shows a comparison of the amino acid sequences of ORF3 from *M. catarrhalis* strains 4223 (SEQ ID No: 14) and Q8 (SEQ ID No: 18). Dots indicate identical residues and dashes have been introduced for maximum alignment.

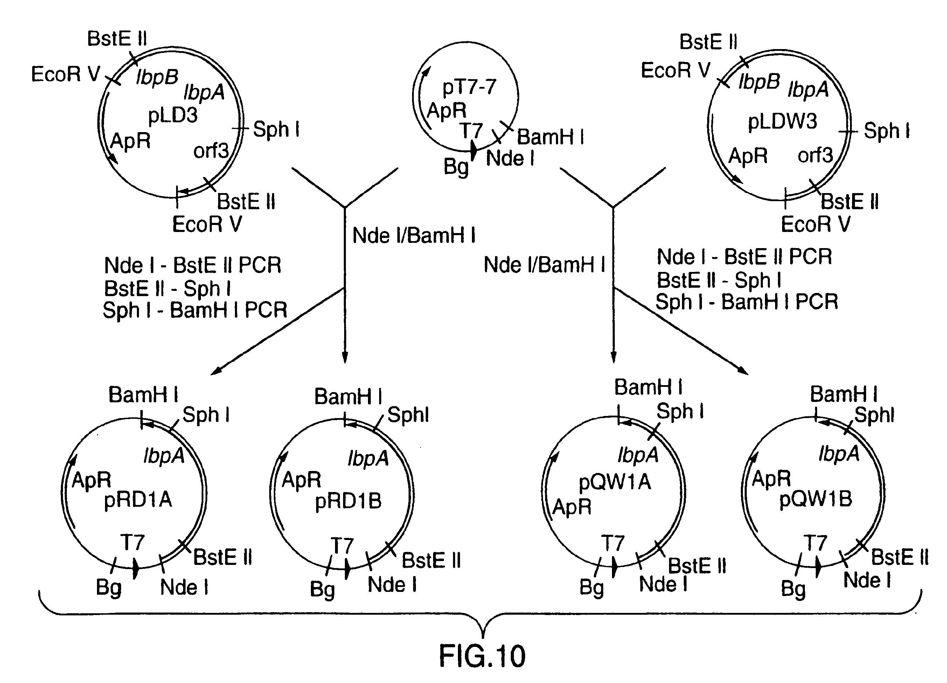

Figure 10 shows the construction of plasmids for expression of recombinant Lbp1 protein from *E. coli*. Plasmids pRD1A and pRD1B express 4223 Lbp1 from the first or second methionine residues, respectively. Plasmids pQW1A and pQW1B express Q8 Lbp1 from the first or second methionine residues, respectively.

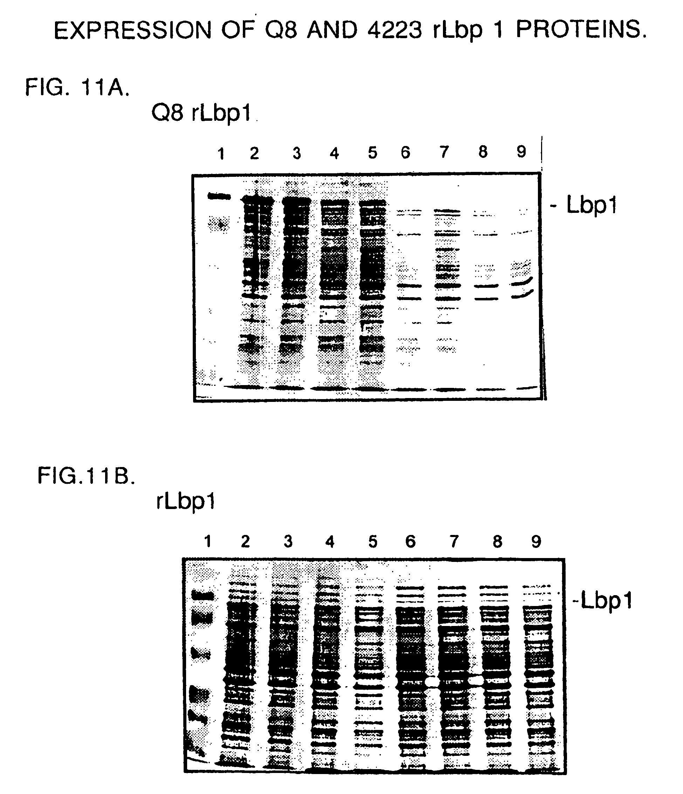

Figure 11, comprising panels A and B, shows the expression of recombinant Lbp1 (rLbp1) proteins from *E. coli.* Panel A shows the expression of the Q8 Lbp1 proteins and panel B shows the expression of the 4223 Lbp1 proteins. Lane 1, molecular weight marker. Lanes 2 and 3 demonstrate the induced expression of the longer Lbp1 starting from the first methionine residues and lanes 4 and 5 illustrate the expression of the shorter Lbp1 proteins starting from the second methionine residues. Lanes 6, 7, 8 and 9 are uninduced samples.

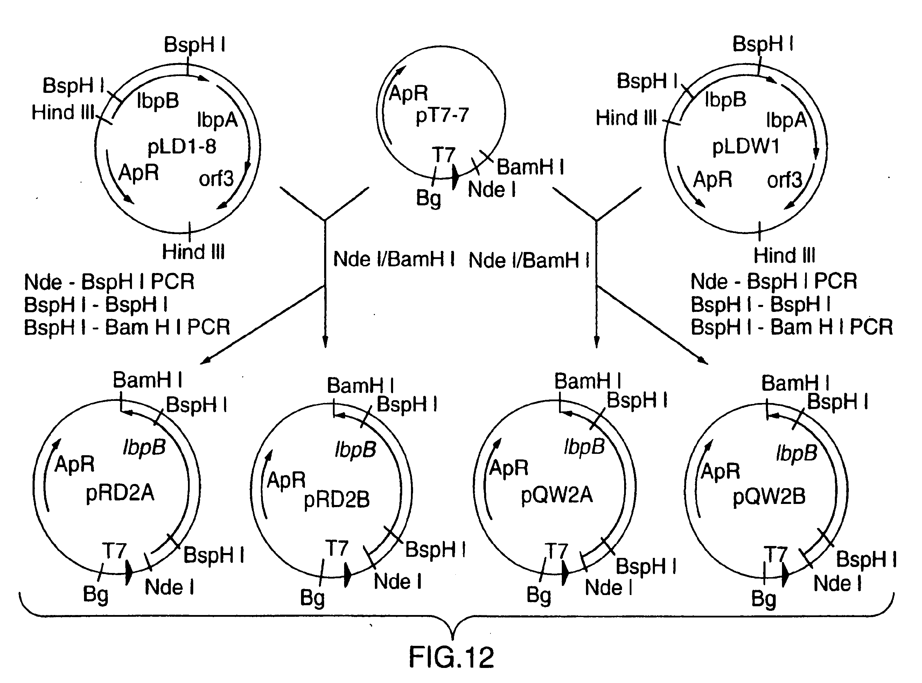

Figure 12 shows the construction of plasmids for expression of recombinant Lbp2 (rLbp2) protein from *E. coli*. Plasmids pRD2A and pRD2B express 4223 Lbp2 with or without the native leader sequence, respectively. Plasmids pQW2A and pQW2B express Q8 Lbp2 with or without the native leader sequence, respectively.

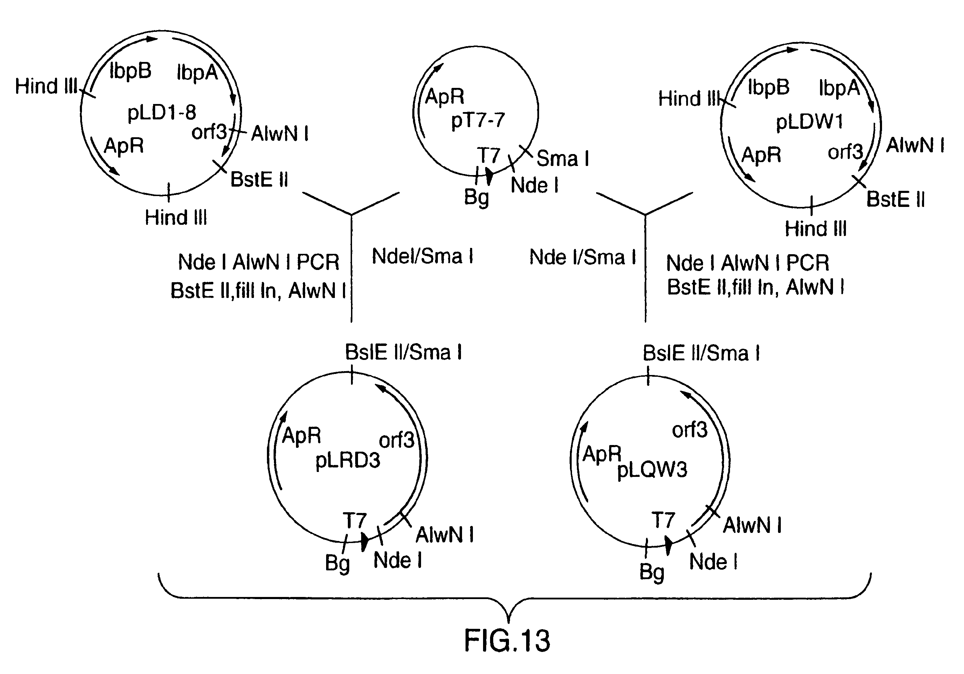

Figure 13 shows the construction of a plasmid for expression of recombinant ORF3 (rORF3) proteins from *E. coli.*

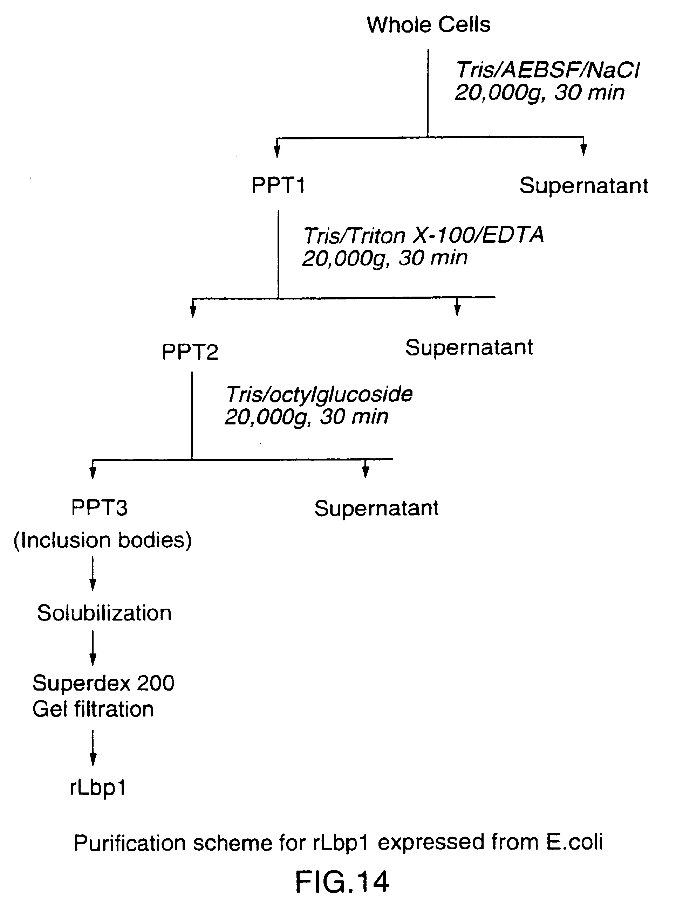

Figure 14 shows a purification scheme for rLbp1 expressed from *E. coli*.

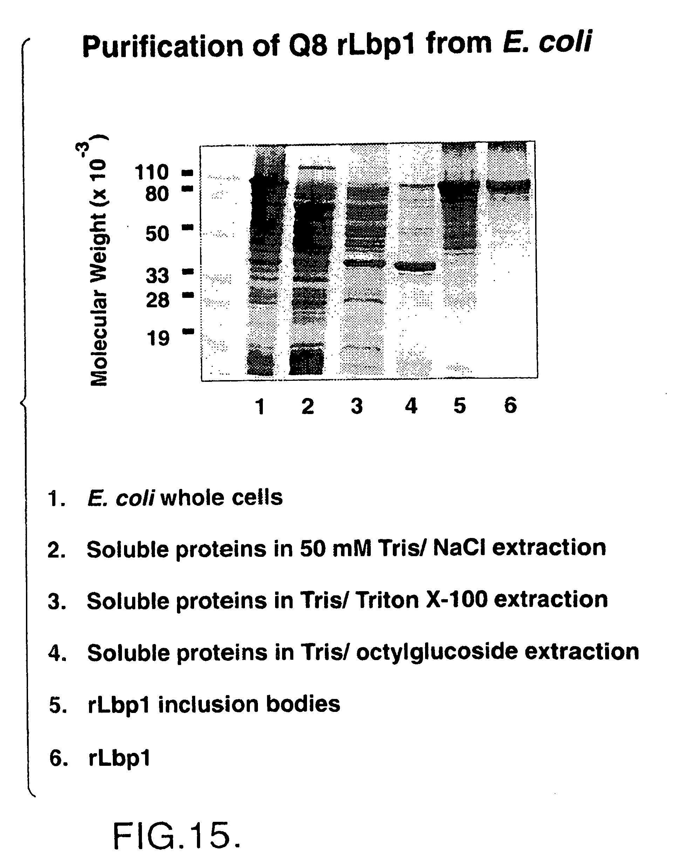

Figure 15 shows an SDS PAGE gel of the purification of Q8 Lbp1 from *E. coli*. Lane 1, BL21(DE3) lysate; lane 2, soluble proteins after 50 mM Tris/5 mM AEBSF/0.5 M NaCl, pH 8.0 extraction; lane 3, soluble proteins after 50 mM Tris/0.5% Triton X-100/10 mM EDTA, pH 8.0 extraction; lane 4, soluble proteins after 50 mM Tris-HC1/1% octylglucoside, pH 8.0 extraction; lane 5, solubilized inclusion bodies; lane 6, purified Lbp1.

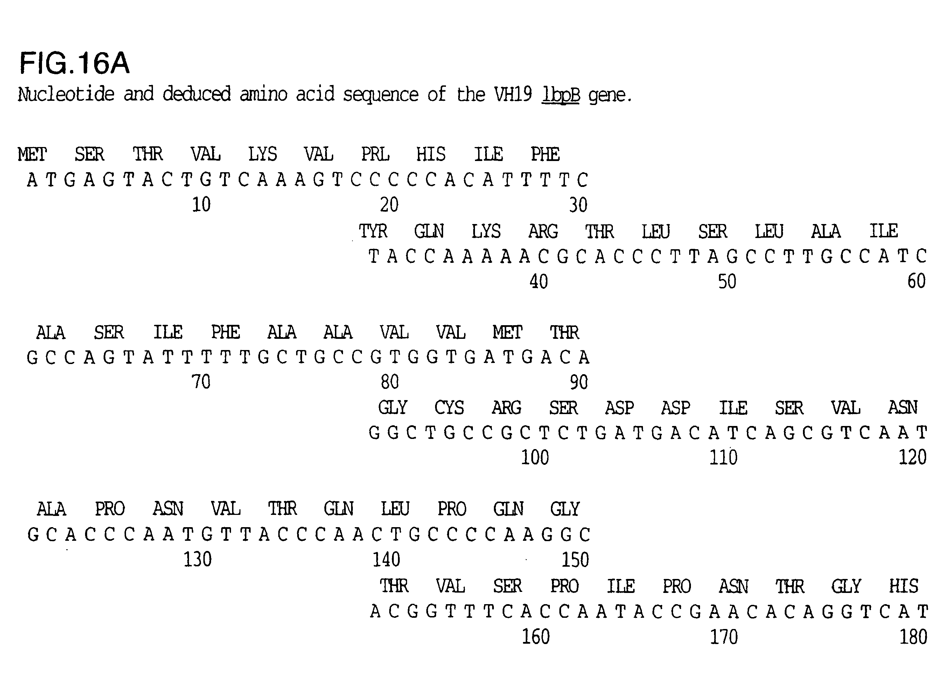

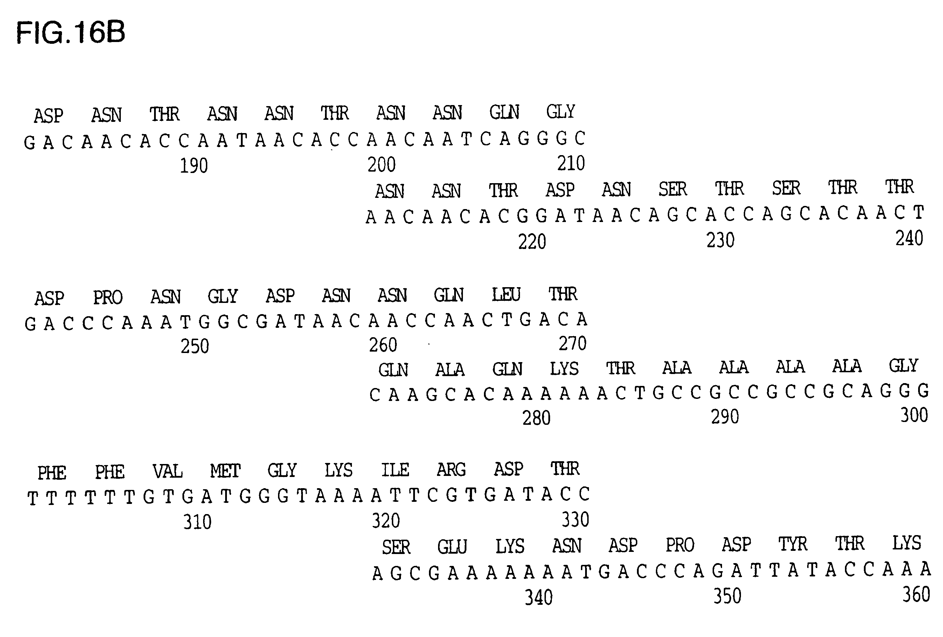

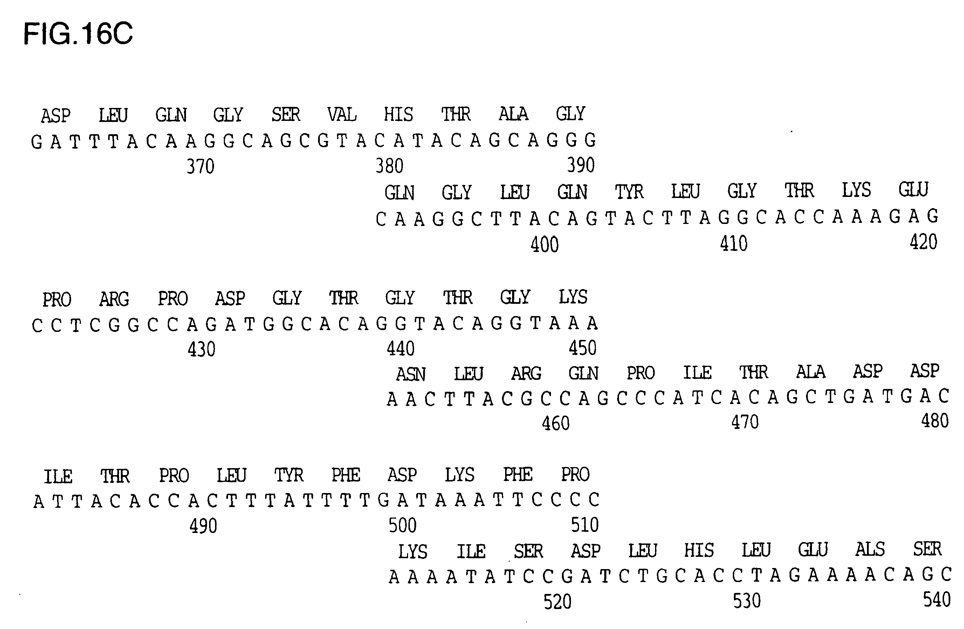

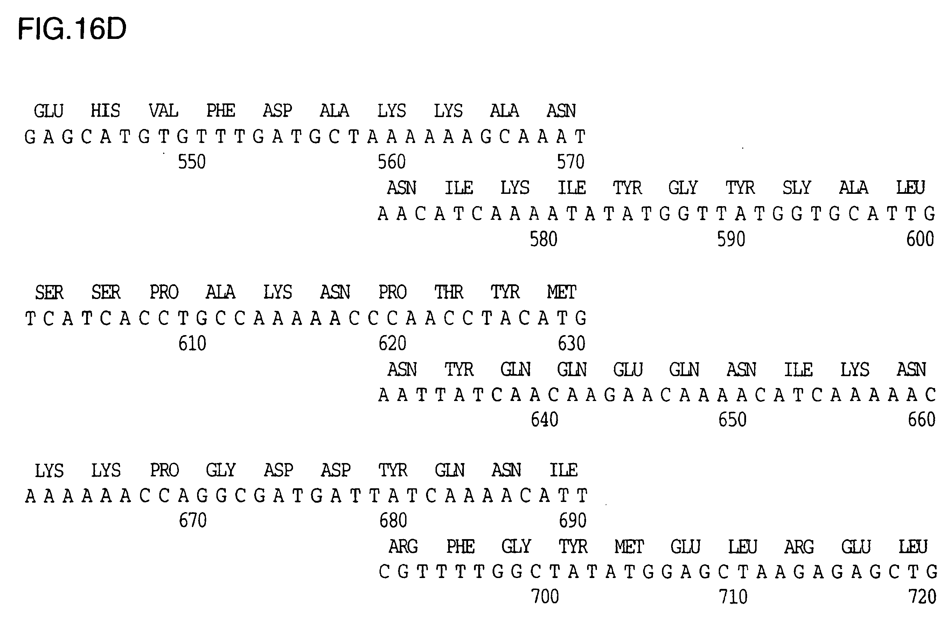

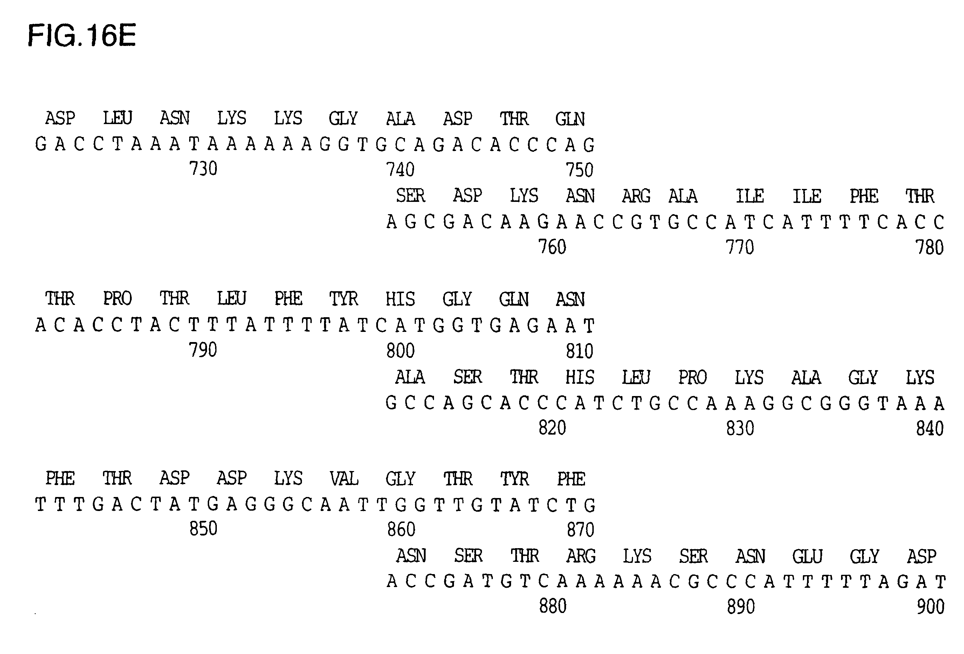

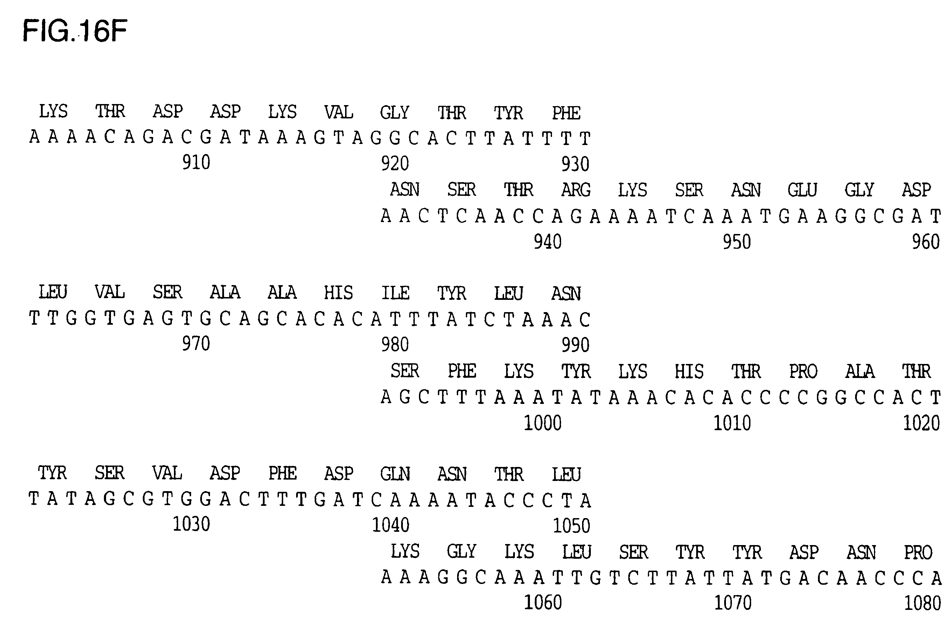

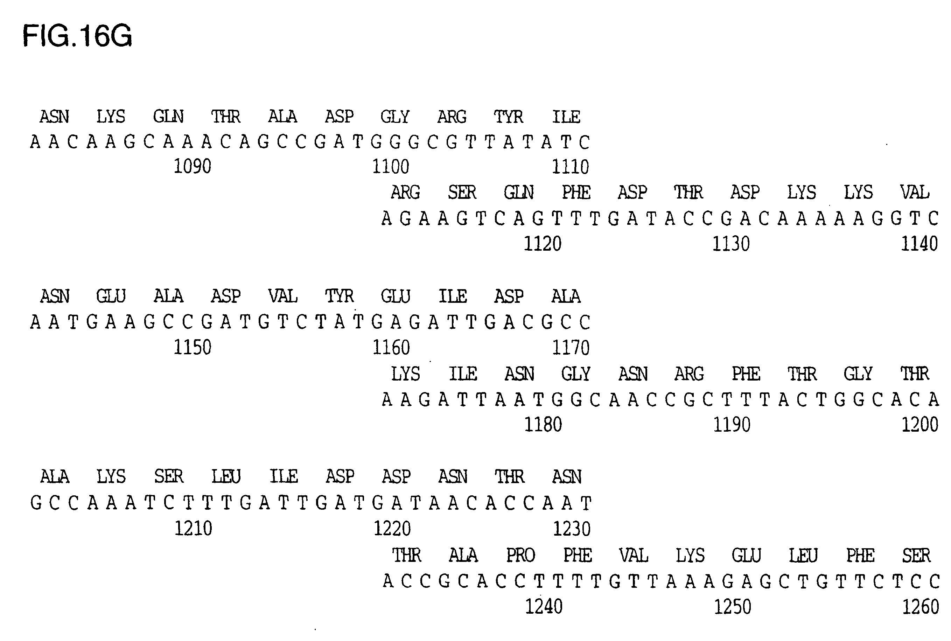

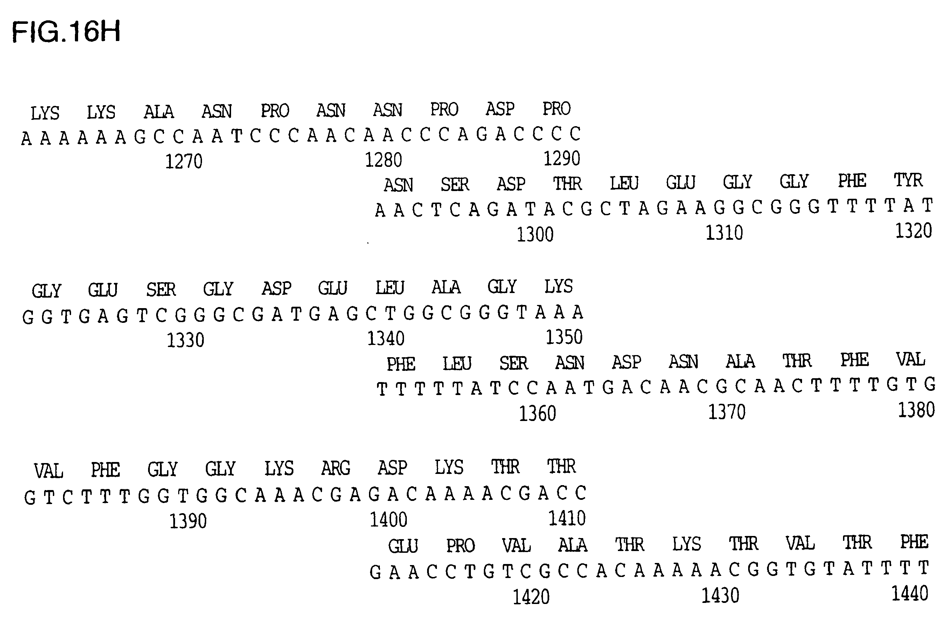

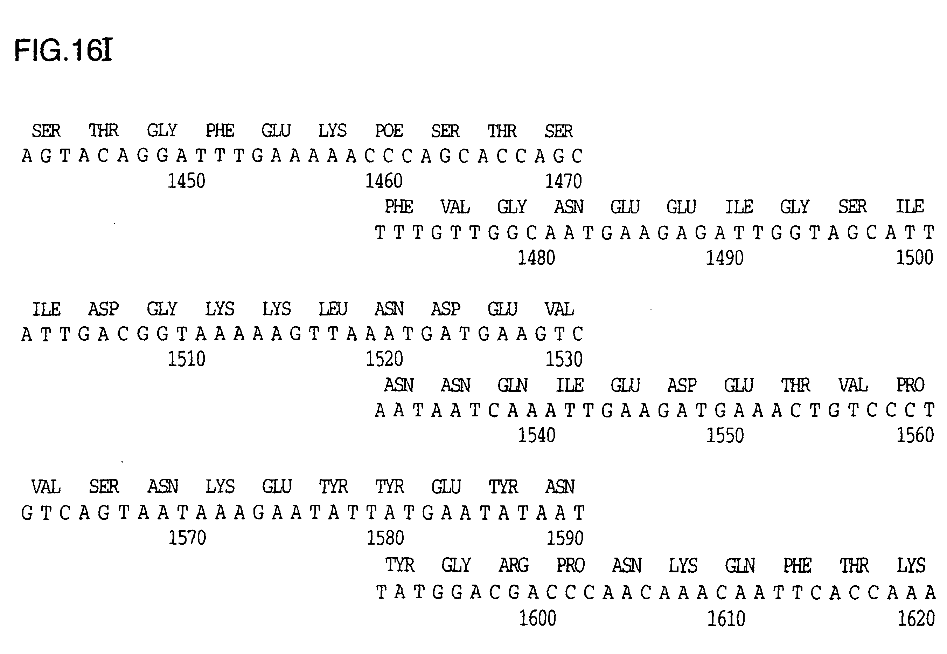

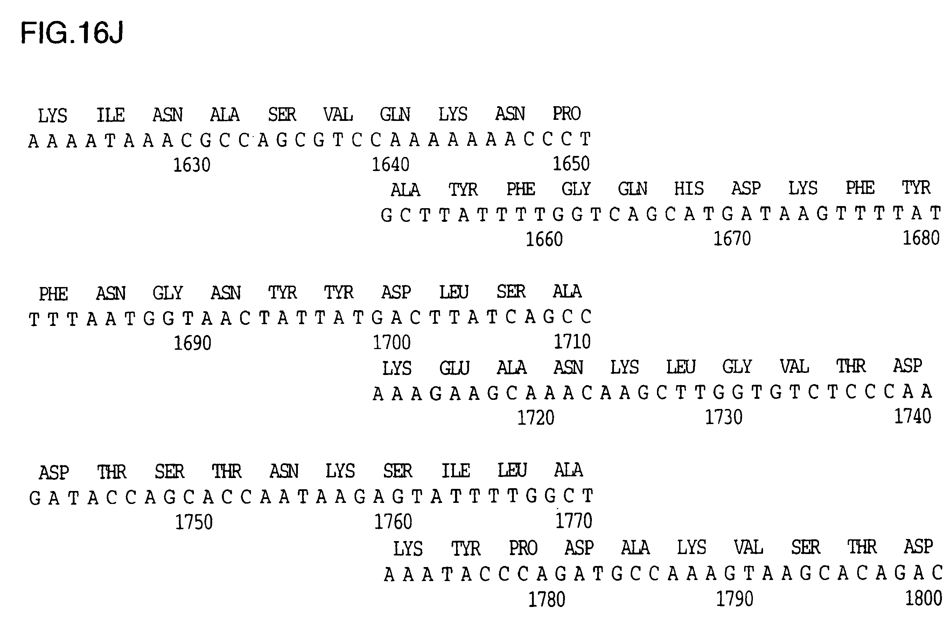

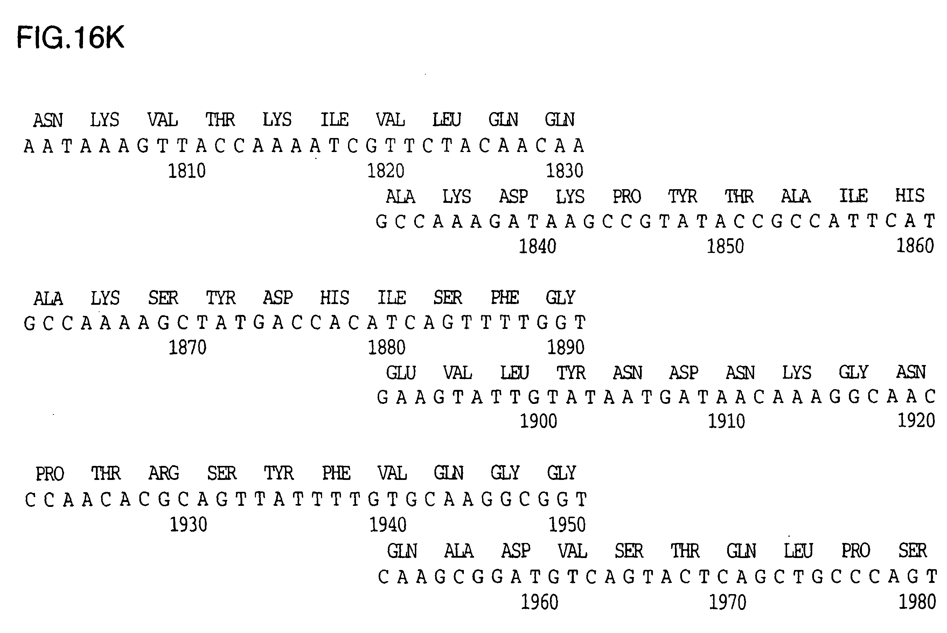

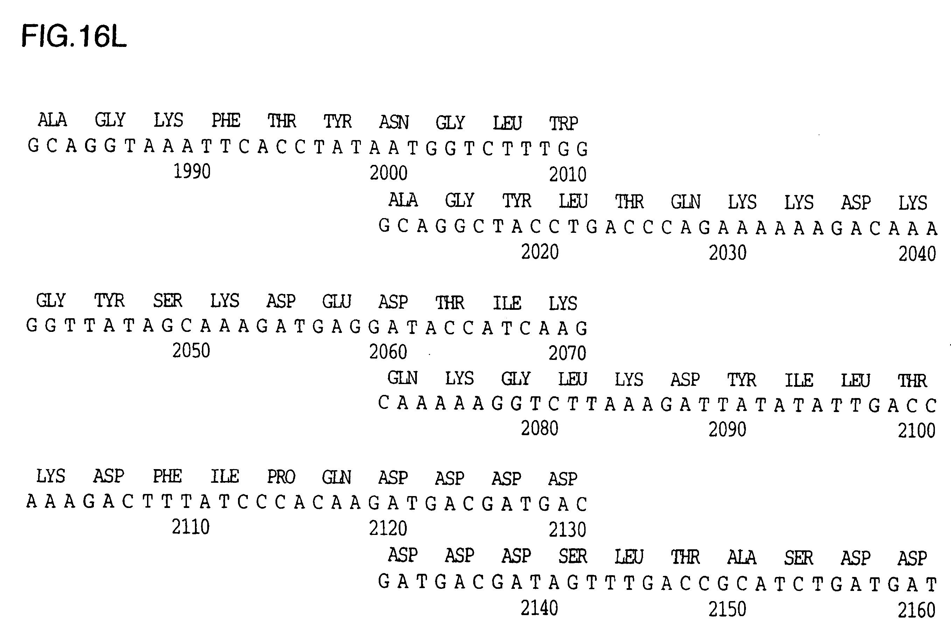

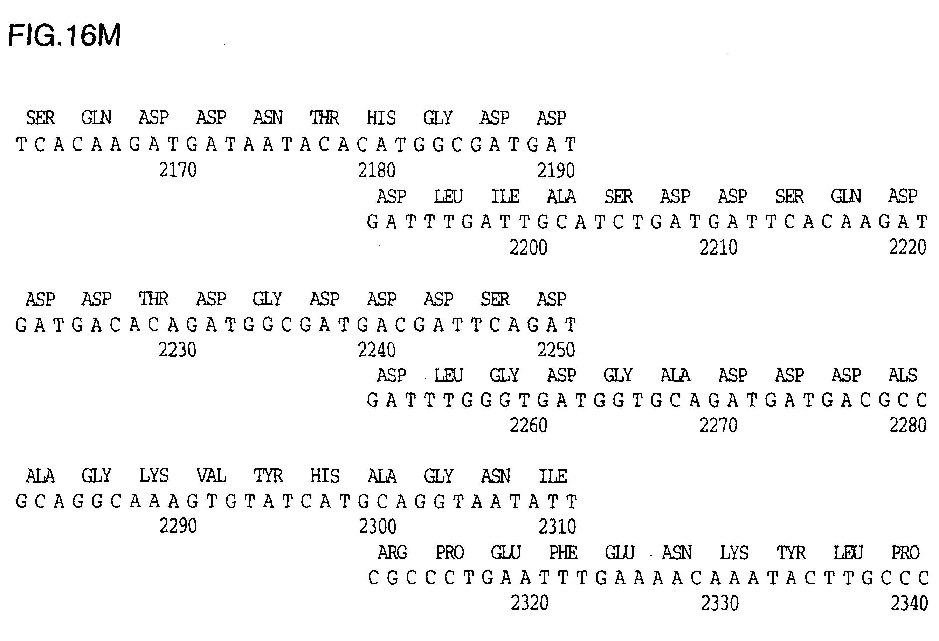

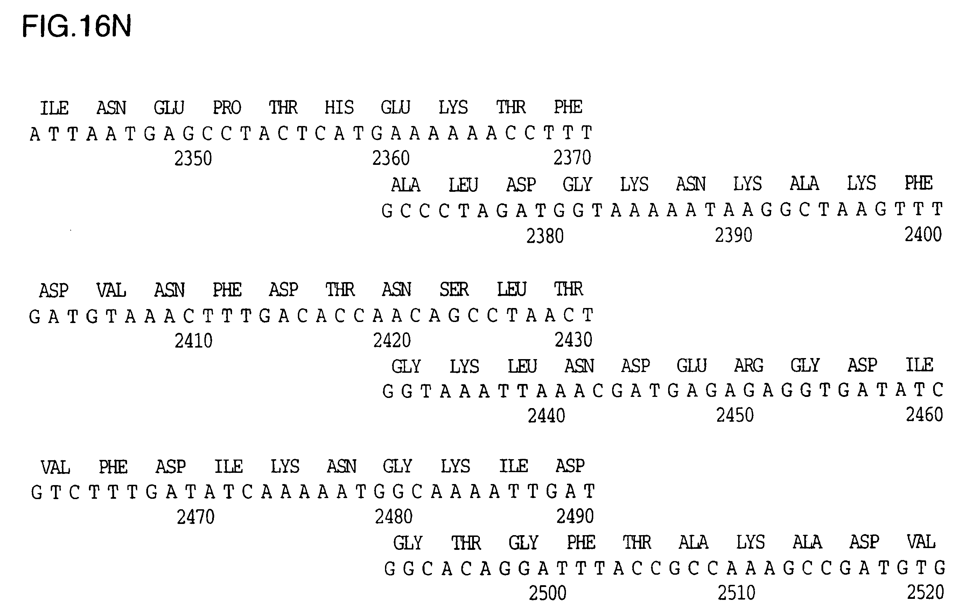

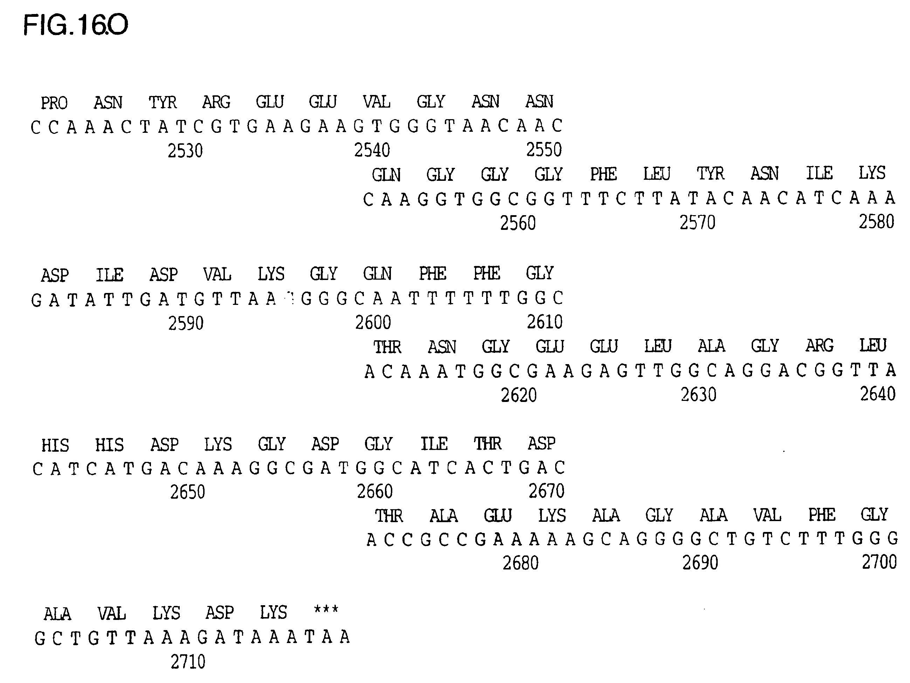

Figure 16 shows the nucleotide sequence (SEQ ID No: 69) of the *M. catarrhalis* strain VH19 *lbpB* gene and the deduced amino acid sequence (SEQ ID No: 70) of the corresponding Lbp2 protein.

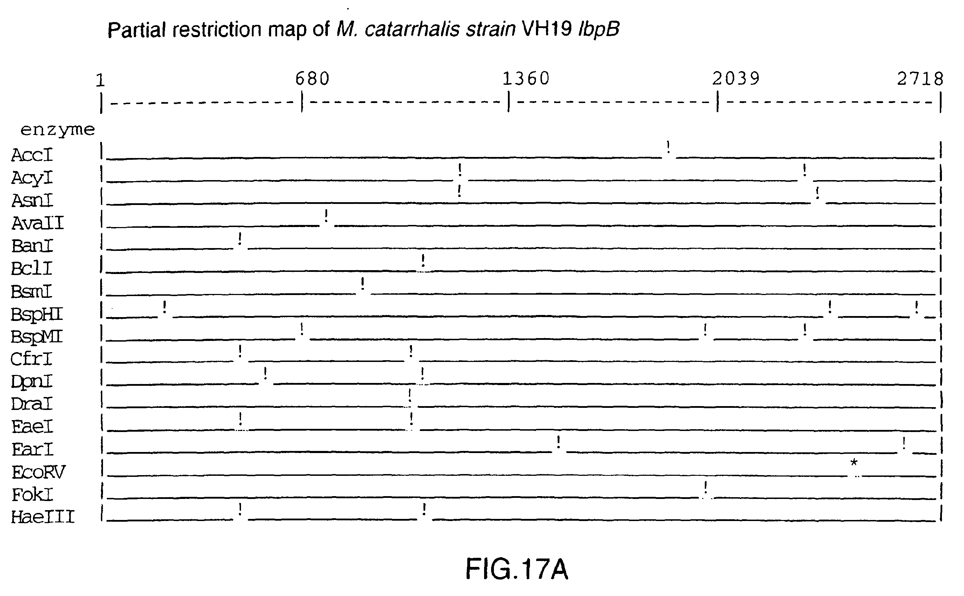

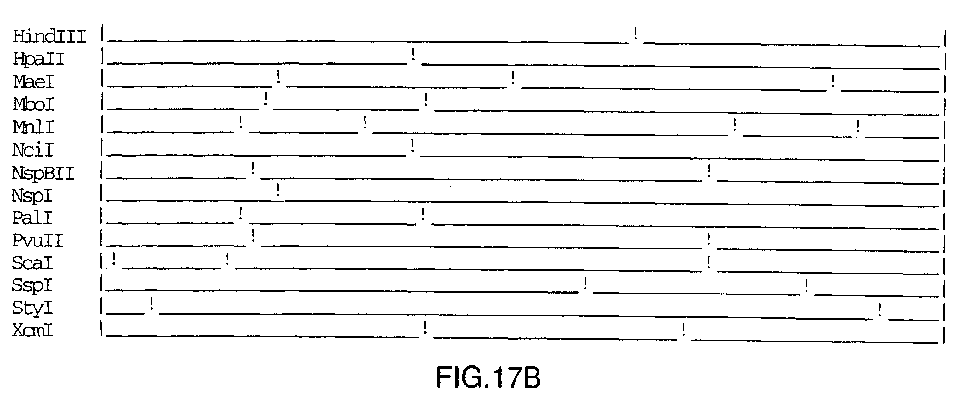

Figure 17 shows a partial restriction map of the *M. catarrhalis* strain VH19 *lbpB* gene.

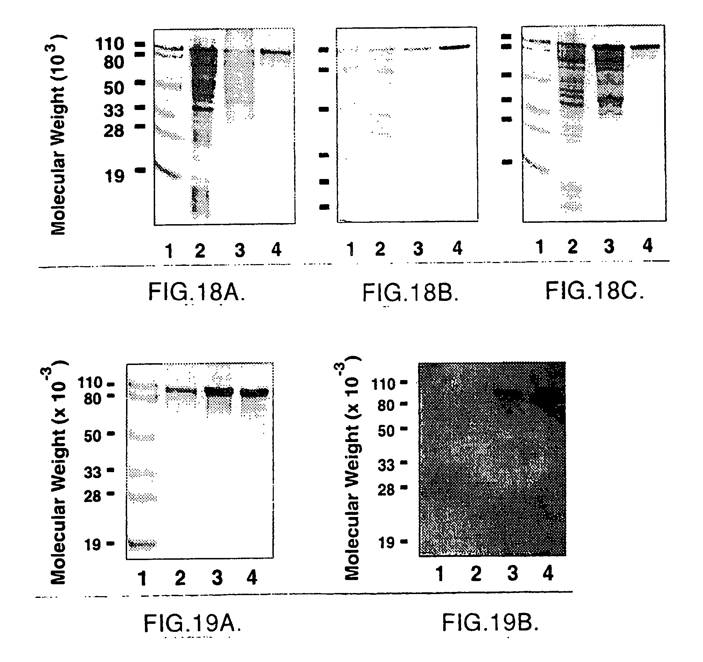

Figure 18, comprising panels A, B and C, shows SDS-PAGE gels of the purification of recombinant Lbp proteins. Panel A shows an SDS-PAGE gel of the purification of Q8 rLbp1. Panels B and C show the purification of Q8 rLbp2 and 4223 rLbp2, respectively. Lane 1, molecular weight markers; lane 2, whole cell lysates; lane 3, inclusion bodies; lane 4, purified protein.
Figure 19, comprising panels A and B, shows binding of recombinant Lbp proteins to lactoferrin. Panel A shows an SDS PAGE gel of purified recombinant proteins. Panel B shows the binding of recombinant proteins to human lactoferrin. Lane 1, molecular weight markers; lane 2, Q8 rLbp1; lane 3, Q8 rLbp2; lane 4, 4223 rLbp2.

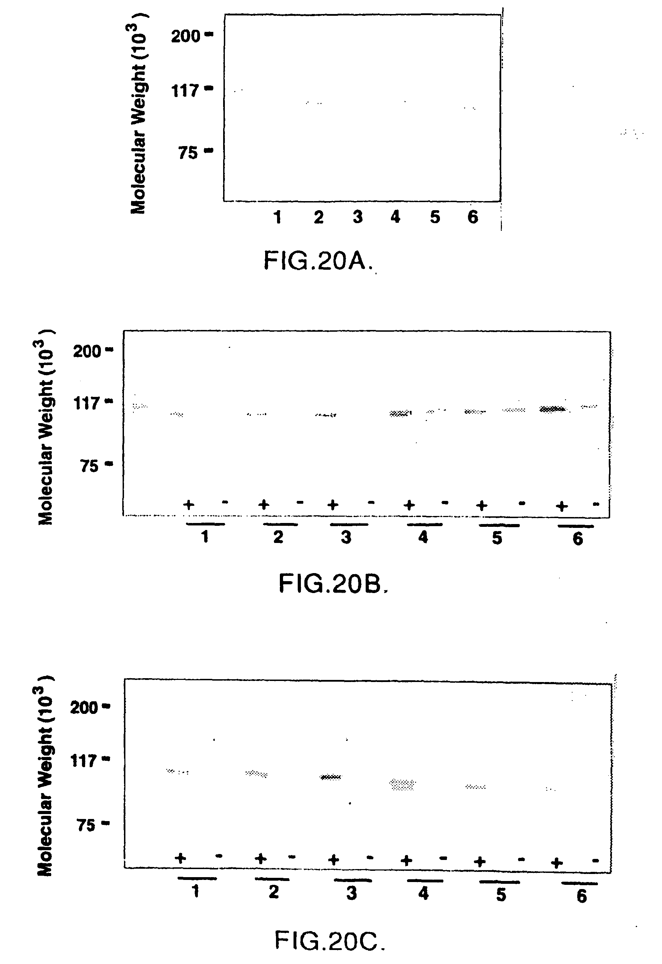

Figure 20, comprising panels A, B and C, shows an immunoblot of *M. catarrhalis* strains reacted with anti-rLbp1 and anti-rLbp2 antibodies. Panel A: whole cell lysates probed with anti-Q8 rLbp1 + anti-Q8 rLbp2 antisera. All cells were grown in the presence of EDDA. Panel B: whole cell lystaes probed with anti-Q8 rLbp1 antibody. Panel C: whole cell lysates probed with anti-Q8 rLbp2 antibody. Lane 1, strain Q8; lane 2, strain 4223; lane 3, strain VH19; lane 4, strain LES-1; lane 5, strain H-04; lane 6, strain 3. + indicates growth in the presence of EDDA and - indicates growth in the absence of EDDA.

### GENERAL DESCRIPTION OF THE INVENTION

*Moraxella catarrhalis* Q8 strain is conveniently used to provide the purified and isolated nucleic acid, which may be in the form of DNA molecules, comprising at least a portion of the nucleic acid coding for a lactoferrin receptor protein 2 (Lbp2) as typified by embodiments of the present invention. Moraxella strains are generally available from clinical sources and from bacterial culture collections, such as the American Type Culture Collection.

In this application, the terms "lactoferrin receptor" (LfR) and "lactoferrin binding proteins" (Lbp) are used to define a family of Lbp1, Lbp2 and/or ORF3 proteins which includes those having variations in their amino acid sequences including those naturally occurring in various strains of, for example, *Moraxella.*

Lactoferrin receptor proteins were purified from *M. catarrhalis* membrane preparations by affinity chromatography on biotinylated human lactoferrin. Cyanogen bromide fragments were generated and amino acid sequence analysis of a 13 kDa fragment provided an internal Lbp1 sequence of MVQYTYRKGKENKAH (SEQ ID No: 22) underlined in Figure 6. The C-terminus of *M. catarrhalis* Tbp1 (United States Patent Application No. 08/613,009), *N. meningitidis* Tbp1 (ref. 27) and *H. influenzae* Tbp1 (ref. 31) has a conserved LEMKF (SEQ ID No: 26) sequence. Oligonucleotide primers were generated based upon these two sequences and used to PCR amplify an approximately 2.2kb fragment of the *lbpA* gene from *M. catarrhalis* strains 4223, Q8 and VH19. Partial sequence analysis demonstrated that the amplified genes were *lbpA* and not *tbpA* (see Fig. 1). The 2.2 kb PCR fragments were used to screen genomic libraries.

Chromosomal DNA from 4223, Q8 and VH19 was partially digested with Sau3A I and 15 to 23 kb fragments were purified before cloning into BamH I arms of the lambda vector EMBL3. The libraries were screened with the PCR fragment and positive clones were subjected to three rounds of plaque purification. Phage clone 4223LfR.17 containing an approximately 16 kb insert from 4223 and phage clone Q8LfR.13 containing an approximately 16 kb insert from Q8 were selected for further analysis.

Restriction enzyme and Southern blot analyses revealed that an internal Hind III fragment of approximately 9 kb contained at least a portion of the *lbpA* gene for both phage clones. The approximately 9 kb Hind III fragments were subcloned into pUC or pBluescript-based plasmids and sequenced. In each case, they contained the complete *lbpA* gene as well as an upstream gene identified as *lbpB,* and a downstream gene designated as *orf3.* The *lbpB-lbpA* gene arrangement is the same as present for *Neisseria* strains, but there has been no identification of a third gene for these organisms.

The gene arrangement is different than that observed for the *M. catarrhalis tfr* operon which was *tbpA-orf-tbpB* (United States Patent Application No. 08/613,009). There are promoter elements found upstream of both the *lbpB* and *lbpA* genes from strains 4223 and Q8. The third ORF is located immediately downstream of *lbpA,* separated by a single nucleotide.

By analogy with the *N. meningitidis* and *N. gonorrhoeae* transferrin receptor operons (ref. 26, 27, 28), the lactoferrin receptor operon was presumed to consist of two genes encoding lactoferrin binding proteins 1 and 2 (Lbp1 and Lbp2) (ref. 29). However, we report here that, for *M. catarrhalis,* there also appears to be a third gene located immediately downstream of *lbpA* encoding a potential lactoferrin binding protein 3 (ORF3).

The *M. catarrhalis* 4223 and Q8 *lbpA* genes encode proteins of molecular mass about 110 kDa and that are highly conserved with only seven residues difference between them. The N-terminal sequence of the native Lbp protein is unknown and there are two possible ATG start codons at positions 1 or 16. The first of these is adjacent to consensus sequences for promoter elements and the second is followed by a putative signal sequence. The exact peptide sequence used to design the PCR amplification primers was not found. When compared with other known Lbp1 sequences from *N. meningitidis* (refs. 31, 24) or *N. gonorrhoeae* (ref. 25) there is about 32% sequence identity and about 50% sequence homology between the *M. catarrhalis* and the *Neisseria* proteins. There is some homology between the *M. catarrhalis* Lbp1 and Tbp1 proteins as shown in Figure 1, but it is very scattered.

The *M. catarrhalis* 4223, Q8 and VH19 *lbpB* genes encode 898, 894 and 906 amino acid proteins, respectively. The *M. catarrhalis* Lbp2 proteins from strains 4223 and Q8 are 92% identical and 95% homologous while that from VH19 is 77% identical and 84% similar to the 4223 and Q8 Lbp2 proteins (Figure 7). There is a consensus sequence for lipidation at the Cys³² residue, suggesting that Lbp2 is a lipoprotein like Tbp2. There is little homology between the *M. catarrhalis* Lbp2 and Tbp2 proteins (Fig. 8) with the exception of a previously identified peptide sequence (LEGGFY (SEQ ID No: 27)) that is also found in *N. meningitidis* and *H. influenzae* Tbp2 (ref. 30).

The sequence of the proposed *M. catarrhalis lfr-*related downstream *orf3* is conserved between strains 4223 and Q8. The encoded 4223 and Q8 ORF3 proteins when compared to the PIR and Swiss Prot protein databases were found to be previously unknown. The ORF3 protein may bind lactoferrin itself or may be an associated or regulatory protein for Lbp1 and/or Lbp2.

Expression vectors have been assembled from the *lbpA* and *lbpB* genes and recombinant Lbp1 and Lbp2 proteins isolated and purified, as described in detail in the Examples below.

Results shown in Table 1 below illustrate the ability of anti-Lbp1 guinea pig antiserum, produced by immunization with affinity purified Lbp1, to lyse *M. catarrhalis.* The results show that the antisera produced by immunization with Lbp1 protein isolated from *M. catarrhalis* isolate 4223 was bactericidal against a homologous non-clumping *M. catarrhalis* strain RH408 (a strain previously deposited in connection with United States Patent Application No. 08/328,589, assigned to the assignee hereof (WO 96/12733 published May 2, 1996)) derived from isolate 4223. In addition, antisera produced by immunization with Lbp1 protein isolated from *M. catarrhalis* 4223 were bactericidal against the heterologous non-clumping strain Q8. The results in Table 3 show that similarly-produced anti-Lbp2 guinea pig antiserum was bactericidal for the homologous strain and for three of five hetrologous strains. The ability of isolated and purified lactoferrin binding protein to generate bactericidal antibodies is *in vivo* evidence of utility of these proteins as vaccines to protect against disease caused by *Moraxella.* Provided within the scope of the present invention, is a vaccine against *Moraxella* comprising an immunogenically-effective amount of lactoferrin binding protein 2 of Moraxella catarrhalis Q8, or a nucleic acid molecule (DNA or RNA) encoding said lactoferrin binding protein 2, and a physiologically-acceptable carrier therefor. The lactoferrin binding protein 2 provided herein may also be used as a carrier protein for haptens, polysaccharide or peptides to make conjugate vaccines against antigenic determinants unrelated to lactoferrin binding proteins.

The lactoferrin binding protein as provided herein may be used as a carrier molecule to prepare chimeric molecules and conjugate vaccines (including glycoconjugates) against pathogenic bacteria, including encapsulated bacteria. Thus, for example, glycoconjugates of the present invention may be used to confer protection against disease and infection caused by any bacteria having polysaccharide antigens including lipooligosaccharides (LOS) and PRP. Such bacterial pathogens may include, for example, *Haemophilus influenzae, Streptococcus pneumoniae, Escherichia coli, Neisseria meningiti dis, Salmonella typhi, Streptococcus mutans, Cryptococcus neoformans, Klebsiella, Staphylotoccus aureus* and *Pseudomonas aeruginosa.* Particular antigens which can be conjugated to lactoferrin binding protein and methods to achieve such conjugations are described in U.S. Patent Application No. 08/433,522 filed November 23, 1993 (WO 94/12641), assigned to the assignee hereof.

The carrier function of lactoferrin binding protein 2 may be used, for example, to induce an immune response against abnormal polysaccharides of tumour cells, or to produce antitumour antibodies that can be conjugated to chemotherapeutic or bioactive agents.

The lactoferrin binding protein provided herein is useful as a diagnostic reagent, as an antigen or for the generation of anti-lactoferrin protein 2 binding antibodies, antigen for vaccination against disease caused by species of *Moraxella* and for detecting infection by *Moraxella* and other such bacteria.

The invention extends to lactoferrin binding protein 2 or nucleic acid molecules encoding the same from *Moraxella catarrhalis* Q8 for use as an active ingredient in a vaccine against disease caused by infection with *Moraxella.* The invention also extends to a pharmaceutical vaccinal composition containing lactoferrin binding protein 2 or nucleic acid molesules encoding the same from *Moraxella catarrhalis* Q8 and optionally, a pharmaceutically acceptable carrier and/or diluent.

Also within the scope of the present invention is the use of lactoferrin binding protein 2 of Moraxella catarrhalis Q8 or nucleic acid molesules encoding the same for the preparation of a pharmaceutical vaccinal composition for immunization against disease caused by infection with *Moraxella.*

It is clearly apparent to one skilled in the art, that the various embodiments of the present invention have many applications in the fields of vaccination, diagnosis, treatment of, for example, *Moraxella* infections and the generation of immunological and other diagnostic reagents. A further non-limiting discussion of such uses is further presented below.

### 1. Vaccine Preparation and Use

Immunogenic compositions, suitable to be used as vaccines, may be prepared from immunogenic lactoferrin receptor proteins encoded by the nucleic acid molecules as well as the nucleic acid molecules disclosed herein. The vaccine elicits an immune response which produces antibodies, including anti-lactoferrin receptor antibodies and antibodies that -are opsonizing or bactericidal. Should the vaccinated subject be challenged by *Moraxella,* the antibodies bind to the lactoferrin receptor and thereby prevent access of the bacteria to an iron source which is required for viability. Furthermore, opsonizing or bactericidal anti-lactoferrin receptor antibodies may also provide protection by alternative mechanisms.

Immunogenic compositions, including vaccines, may be prepared as injectables, as liquid solutions or emulsions. The lactoferrin receptor proteins, analogs and fragments thereof and encoding nucleic acid molecules as well as the nucleic acid molecules described herein may be mixed with pharmaceutically acceptable excipients which are compatible with the lactoferrin receptor proteins or nucleic acid molecules. Such excipients may include water, saline, dextrose, glycerol, ethanol, and combinations thereof. The immunogenic compositions and vaccines may further contain auxiliary substances, such as wetting or emulsifying agents, pH buffering agents, or adjuvants, to enhance the effectiveness of the vaccines. Immunogenic compositions and vaccines may be administered parenterally, by injection subcutaneously, intradermally or intramuscularly. Alternatively, the immunogenic compositions provided according to the present invention, may be formulated and delivered in a manner to evoke an immune response at mucosal surfaces. Thus, the immunogenic composition may be administered to mucosal surfaces by, for example, the nasal or oral (intragastric) routes. The immunogenic composition may be provided in combination with a targeting molecule for delivery to specific cells of the immune system or to mucosal surfaces. Some such targeting molecules include vitamin B12 and fragments of bacterial toxins, as described in WO 92/17167 (Biotech Australia Pty. Ltd.), and monoclonal antibodies, as described in U.S. Patent No. 5,194,254 (Barber et al). Alternatively, other modes of administration, including suppositories and oral formulations, may be desirable. For suppositories, binders and carriers may include, for example, polyalkalene glycols or triglycerides. Oral formulations may include normally employed incipients such as, for example, pharmaceutical grades of saccharine, cellulose and magnesium carbonate. These compositions may take the form of solutions, suspensions, tablets, pills, capsules, sustained release formulations or powders and contain about 1 to 95% of the lactoferrin receptor protein 2.

The vaccines are administered in a manner compatible with the dosage formulation, and in such amount as will be therapeutically effective, protective and immunogenic. The quantity to be administered depends on the subject to be treated, including, for example, the capacity of the individual's immune system to synthesize antibodies, and, if needed, to produce a cell-mediated immune response. Precise amounts of active ingredient required to be administered depend on the judgement of the practitioner. However, suitable dosage ranges are readily determinable by one skilled in the art and may be of the order of micrograms of the lactoferrin receptor protein 2, and/or nucleic acid molecules. Suitable regimes for initial administration and booster doses are also variable, but may include an initial administration followed by subsequent administrations. The dosage of the vaccine may also depend on the route of administration and will vary according to the size of the host.

The nucleic acid molecules encoding lactoferrin receptor 2 of *Moraxella* strain Q8 may be used directly for immunization by administration of the DNA directly, for example, by injection for genetic immunization or by constructing a live vector, such as *Salmonella,* BCG, adenovirus, poxvirus, vaccinia or poliovirus containing the nucleic acid molecules. A discussion of some live vectors that have been used to carry heterologous antigens to the immune system is contained in, for example, O'Hagan (ref. 18). Processes for the direct injection of DNA into test subjects for genetic immunization are described in, for example, Ulmer et al. (ref. 19).

Immunogenicity can be significantly improved if the antigens are co-administered with adjuvants, commonly used as an 0.05 to 1.0 percent solution in phosphate - buffered saline. Adjuvants enhance the immunogenicity of an antigen but are not necessarily immunogenic themselves. Adjuvants may act by retaining the antigen locally near the site of administration to produce a depot effect facilitating a slow, sustained release of antigen to cells of the immune system. Adjuvants can also attract cells of the immune system to an antigen depot and stimulate such cells to elicit immune responses.

Immunostimulatory agents or adjuvants have been used for many years to improve the host immune responses to, for example, vaccines. Intrinsic adjuvants, such as lipopolysaccharides, normally are the components of killed or attenuated bacteria used as vaccines. Extrinsic adjuvants are immunomodulators which are typically non-covalently linked to antigens and are formulated to enhance the host immune responses. Thus, adjuvants have been identified that enhance the immune response to antigens delivered parenterally. Some of these adjuvants are toxic, however, and can cause undesirable side-effects, making them unsuitable for use in humans and many animals. Indeed, only aluminum hydroxide and aluminum phosphate (collectively commonly referred to as alum) are routinely used as adjuvants in human and veterinary vaccines. The efficacy of alum in increasing antibody responses to diphtheria and tetanus toxoids is well established and an HBsAg vaccine has been adjuvanted with alum.

A wide range of extrinsic adjuvants can provoke potent immune responses to antigens. These include saponins complexed to membrane protein antigens (immune stimulating complexes), pluronic polymers with mineral oil, killed mycobacteria and mineral oil, Freund's complete adjuvant, bacterial products, such as muramyl dipeptide (MDP) and lipopolysaccharide (LPS), as well as lipid A, and liposomes.

To efficiently induce humoral immune responses (HIR) and cell-mediated immunity (CMI), immunogens are often emulsified in adjuvants. Many adjuvants are toxic, inducing granulomas, acute and chronic inflammations (Freund's complete adjuvant, FCA), cytolysis (saponins and pluronic polymers) and pyrogenicity, arthritis and anterior uveitis (LPS and MDP). Although FCA is an excellent adjuvant and widely used in research, it is not licensed for use in human or veterinary vaccines because of its toxicity.

Desirable characteristics of ideal adjuvants include:
(1) lack of toxicity;
(2) ability to stimulate a long-lasting immune response;
(3) simplicity of manufacture and stability in long-term storage;
(4) ability to elicit both CMI and HIR to antigens administered by various routes, if required;
(5) synergy with other adjuvants;
(6) capability of selectively interacting with populations of antigen presenting cells (APC);
(7) ability to specifically elicit appropriate T_{H}1 or T_{H}2 cell specific immune responses; and
(8) ability to selectively increase appropriate antibody isotype levels (for example, IgA) against antigens.

U.S. Patent No. 4,855,283 granted to Lockhoff et al on August 8, 1989, teaches glycolipid analogues including N-glycosylamides, N-glycosylureas and N-glycosylcarbamates, each of which is substituted in the sugar residue by an amino acid, as immuno-modulators or adjuvants. Thus, Lockhoff et al. 1991 (ref. 20) reported that N-glycolipid analogs displaying structural similarities to the naturally-occurring glycolipids, such as glycophospholipids and glycoglycerolipids, are capable of eliciting strong immune responses in both herpes simplex virus vaccine and pseudorabies virus vaccine. Some glycolipids have been synthesized from long chain-alkylamines and fatty acids that are linked directly with the sugars through the anomeric carbon atom, to mimic the functions of the naturally occurring lipid residues.

U.S. Patent No. 4,258,029 granted to Moloney, assigned to the assignee hereof, teaches that octadecyl tyrosine hydrochloride (OTH) functions as an adjuvant when complexed with tetanus toxoid and formalin inactivated type I, II and III poliomyelitis virus vaccine. Also, Nixon-George et al. 1990, (ref. 21) reported that octadecyl esters of aromatic amino acids complexed with a recombinant hepatitis B surface antigen, enhanced the host immune responses against hepatitis B virus.

### 2. Immunoassays

The lactoferrin receptor protein, of the present invention is useful as an immunogen, as an antigen in immunoassays including enzyme-linked immunosorbent assays (ELISA), RIAs and other non-enzyme linked antibody binding assays or procedures known in the art for the detection of anti-*Moraxella*, lactoferrin receptor protein 2 antibodies. In ELISA assays, the lactoferrin receptor protein 2, is immobilized onto a selected surface, for example, a surface capable of binding proteins or peptides such as the wells of a polystyrene microtiter plate. After washing to remove incompletely adsorbed lactoferrin receptor, a non-specific protein such as a solution of bovine serum albumin (BSA) or casein that is known to be antigenically neutral with regard to the test sample may be bound to the selected surface. This allows for blocking of nonspecific adsorption sites on the immobilizing surface and thus reduces the background caused by non-specific bindings of antisera onto the surface.

The immobilizing surface is then contacted with a sample, such as clinical or biological materials, to be tested in a manner conducive to immune complex (antigen/antibody) formation. This procedure may include diluting the sample with diluents, such as BSA, bovine gamma globulin (BGG) and/or phosphate buffered saline. (PBS)/Tween. The sample is then allowed to incubate for from about 2 to 4 hours, at temperatures such as of the order of about 25° to 37°C. Following incubation, the sample-contacted surface is washed to remove non-immunocomplexed material. The washing procedure may include washing with a solution such as PBS/Tween or a borate buffer.

Following formation of specific immunocomplexes between the test sample and the bound lactoferrin receptor protein, and subsequent washing, the occurrence, and even amount, of immunocomplex formation may be determined by subjecting the immunocomplex to a second antibody having specificity for the first antibody. If the test sample is of human origin, the second antibody is an antibody having specificity for human immunoglobulins and in general IgG. To provide detecting means, the second antibody may have an associated activity such as an enzymatic activity that will generate, for example, a color development upon incubating with an appropriate chromogenic substrate. Quantification may then achieved by measuring the degree of color generation using, for example, a spectrophotometer.

### 3. Use of Sequences as Hybridization Probes

The nucleotide sequences of the present invention, comprising the sequence of the lactoferrin receptor gene, now allow for the identification and cloning of the lactoferrin receptor genes from any species of *Moraxella.*

The nucleotide sequences comprising the sequence of the lactoferrin receptor genes of the present invention are useful for their ability to selectively form duplex molecules with complementary stretches of other *lfr* genes. Depending on the application, a variety of hybridization conditions may be employed to achieve varying degrees of selectivity of the probe toward the other *lfr* genes. For a high degree of selectivity, relatively stringent conditions are used to form the duplexes, such as low salt and/or high temperature conditions, such as provided by 0.02 M to 0.15 M NaCl at temperatures of between about 50°C to 70°C. For some applications, less stringent hybridization conditions are required such as 0.15 M to 0.9 M salt, at temperatures ranging from between about 20°C to 55°C. Hybridization conditions can also be rendered more stringent by the addition of increasing amounts of formamide, to destabilize the hybrid duplex. Thus, particular hybridization conditions can be readily manipulated, and will generally be a method of choice depending on the desired results. In general, convenient hybridization temperatures in the presence of 50% formamide are: 42°C for a probe which is 95 to 100% homologous to the target fragment, 37°C for 90 to 95% homology and 32°C for 85 to 90% homology.

Such hybridization conditions may be employed to determine DNA sequences which encode a functional lactoferrin receptor of *Moraxella* and which hybridize under stringent conditions to any one of the DNA sequences (a) or (b), described above.

In a clinical diagnostic embodiment, the nucleic acid sequences of the present invention may be used in combination with an appropriate means, such as a label, for determining hybridization. A wide variety of appropriate indicator means are known in the art, including radioactive, enzymatic or other ligands, such as avidin/biotin and digoxigenin-labelling, which are capable of providing a detectable signal. In some diagnostic embodiments, an enzyme tag such as urease, alkaline phosphatase or peroxidase, instead of a radioactive tag may be used. In the case of enzyme tags, colorimetric indicator substrates are known which can be employed to provide a means visible to the human eye or spectrophotometrically, to identify specific hybridization with samples containing *lfr* gene sequences.

The nucleic acid sequences of the present invention are useful as hybridization probes in solution hybridizations and in solid-phase procedures. In solid-phase procedures, the test DNA (or RNA) from samples, such as clinical samples, including exudates, body fluids (e. g., serum, amniotic fluid, middle ear effusion, sputum, bronchoalveolar lavage fluid) or even tissues, is adsorbed or otherwise affixed to a selected matrix or surface. The fixed, single-stranded nucleic acid is then subjected to specific hybridization with selected probes comprising the nucleic acid sequences of *lfr* genes or fragments thereof of the present invention under desired conditions. The selected conditions will depend on the particular circumstances based on the particular criteria required depending on, for example, the G+C contents, type of target nucleic acid, source of nucleic acid, size of hybridization probe etc. Following washing of the hybridization surface so as to remove non-specifically bound probe molecules, specific hybridization is detected, or even quantified, by means of the label. It is preferred to select nucleic acid sequence portions which are conserved among species of *Moraxella.* The selected probe may be at least 18bp and may be in the range of about 30 to 90 bp.

### 4. Expression of the Lactoferrin Receptor Protein 2 Gene

Plasmid vectors containing replicon and control sequences which are derived from species compatible with the host cell may be used for the expression of the lactoferrin receptor protein 2 gene in expression systems. The vector ordinarily carries a replication site, as well as marking sequences which are capable of providing phenotypic selection in transformed cells. For example, *E. coli* may be transformed using pBR322 which contains genes for ampicillin and tetracycline resistance and thus provides easy means for identifying transformed cells. The pBR322 plasmid, or other microbial plasmid or phage, must also contain, or be modified to contain, promoters which can be used by the host cell for expression of its own proteins.

In addition, phage vectors containing replicon and control sequences that are compatible with the host can be used as a transforming vector in connection with these hosts. For example, the phage in lambda GEM^{™}-11 may be utilized in making recombinant phage vectors which can be used to transform host cells, such as *E. coli* LE392.

Promoters commonly used in recombinant DNA construction include the β-lactamase (penicillinase) and lactose promoter systems and other microbial promoters, such as the T7 promoter system as described in U.S. Patent No. 4,952,496. Details concerning the nucleotide sequences of promoters are known, enabling a skilled worker to ligate them functionally with genes. The particular promoter used will generally be a matter of choice depending upon the desired results. Hosts that are appropriate for expression of the lactoferrin receptor gene of the invention may include *E. coli, Bacillus* species, *Haemophilus,* fungi, yeast, *Moraxella, Bordetella,* or the baculovirus expression system may be used.

In accordance with this invention, it is preferred to produce the lactoferrin receptor protein 2 by recombinant methods, particularly since the naturally occurring Lbp2 protein as purified from a culture of *Moraxella catarrhalis* Q8 may include trace amounts of toxic materials or other contaminants. This problem can be avoided by using recombinantly produced Lbp2 protein in heterologous systems which can be isolated from the host in a manner to minimize contaminants, including other proteins of the *Moraxella* strain, in the purified material. Particularly desirable hosts for expression in this regard include Gram positive bacteria which do not have LPS and are, therefore, endotoxin free. Such hosts include species of *Bacillus* and may be particularly useful for the production of non-pyrogenic lactoferrin receptor proteins, fragments or analogs thereof. Furthermore, recombinant methods of production permit the manufacture of Lbp2 separate from Lbp1 or orf3, which is distinct from the normal combined proteins present in *Moraxella.*

### Sequence Alignment and Analysis

Sequence alignments were performed using the ALIGN (Trademark) or GENALIGN (Trademark) computer programs (Inteligenetics Suite 5.4, Oxford Molecular). ALIGN® uses the Needleman-Wunsch algorithm (ref. 35) and its later modifications to locate regions of similarity between two sequences. Finding regions of maximum similarity between two sequences can be solved in a rigorous manner using the iterative matrix calculation of the Needleman and Wunsch 1997 algorithm. The analysis is restricted to regions with no internal deletions or insertions, joined by a minimum number of loop-outs or deletions. Sellers (ref. 36) developed a true metric measure of the "distance" between sequences and Waterman (ref. 37) extended this algorithm to include insertions and deletions of arbitrary length. Smith (ref. 38) improved the early algorithms to find the subsequences of maximum similarity. The algorithm has been used to analyze sequences as long as 5000 bases by dividing these sequences into segments of 200 to 400 bases, and then reassembling them into a final best match. This method of dividing the sequence and then reassembling it has proven quite robust. The algorithm permits the size of the segment to be specified which the program searches for similarities. The program then assembles the segments after checking overlaps of adjacent subsequences. The weighting of deletions and the relative size of overlaps may be controlled. The program displays the results to show the differences in closely related sequences.

GENALIGN® is a multiple alignment program. Up to 99 sequences using the Martinez/Regions (ref. 39) or Needleman-Wunsch (ref. 35) method may be analyzed for alignment. GENALIGN places the sequences in an order that puts the most closely aligned sequence pairs adjacent to each other. A consensus sequence is displayed under the multiple sequence alignments. The sequences used in developing the consensus sequence file for use in other programs. GENALIGN allows the parameters of the search to be changed so that alternate alignments of the sequences can be formed.

These programs are used employing their default settings. The default settings are as follows:

**FastDB**

| | |
|---|---|
| AMINO-Res-length = | 2 |
| DELetion-weight = | 5.00 |
| LEngth-factor = | 0 |
| Matching-weight = | 1.00 |
| NUCLEIC-Res-length = | 4 |
| SPread-factor = | 50 |

**Findseq**

| Search Parameters: | |
|---|---|
| Similarity matrix | Unitary |
| K-tuple | 4 |
| Mismatch penalty | 1 |
| Joining Penalty | 30 |
| Randomization group length | 0 |
| Cutoff score | 5 |

| Alignment Parameters: | |
|---|---|
| Window size | 32 |
| Gap penalty | 1.00 |
| Gap size penalty | 0.33 |

Such procedures may be used to determine DNA sequences which encode a functional lactoferrin receptor of *Moraxella* and which may have at least about 90% sequence identity with any one of the DNA sequences (a) or (b), described above.

### Biological Deposits

Certain vectors that contain at least a portion coding for a lactoferrin receptor protein from strains of *Moraxella catarrhalis* strain 4223 and Q8 and a strain of *M. catarrhalis* RH408 that are described and referred to herein have been deposited with the American Type Culture Collection (ATCC) located at 12301 Parklawn Drive, Rockville, Maryland 20852, USA, pursuant to the Budapest Treaty and prior to the filing of this application. Samples of the deposited vectors and bacterial strain will become available to the public and the restrictions imposed on access to the deposits will be removed upon grant of a patent based upon this United States patent application. In addition, the deposit will be replaced if viable samples cannot be dispensed by the Depository. The invention described and claimed herein is not to be limited in scope by the biological materials deposited, since the deposited embodiment is intended only as an illustration of the invention. Any equivalent or similar vectors or strains that encode similar or equivalent antigens as described in this application are within the scope of the invention.

### Deposit Summary

| **Deposit** | **ATCC Designation** | **Date deposited** |
|---|---|---|
| Plasmid pLD1-8 | 97, 997 | April 23, 1997 |
| Plasmid pLDW1 | 97, 998 | April 23, 1997 |
| Strain RH408 | 55, 637 | Dec. 9, 1994 |

### EXAMPLES

The above disclosure generally describes the present invention. A more complete understanding can be obtained by reference to the following specific Examples. These Examples are described solely for purposes of illustration and are not intended to limit the scope of the invention. Changes in form and substitution of equivalents are contemplated as circumstances may suggest or render expedient. Although specific terms have been employed herein, such terms are intended in a descriptive sense and not for purposes of limitations.

Methods of molecular genetics, protein biochemistry and immunology used but not explicitly described in this disclosure and these Examples are amply reported in the scientific literature and are well within the ability of those skilled in the art.

### Example 1

This Example illustrates the generation of oligonucleotide primers for PCR amplification of *M. catarrhalis lbpA.*

Native Lbp1 was purified by affinity chromatography using high stringency conditions as described in United States Patent Application No. 08/552,232, assigned to the assignee hereof and the disclosure of which is incorporated herein by reference, and in ref. 40.

The purified Lbp1 protein was digested overnight with cyanogen bromide, then fragments separated by SDS PAGE and submitted to sequence analysis on an AB1 model 477A. A 13 kDa protein fragment was found to have the N-terminal sequence MVQYTYRKGKENKAH (SEQ ID No: 22). A degenerate oligonucleotide primer (4393.RD) was prepared based upon this sequence: CAA TAT ACI CGT AAA GGT GAA AAT AAA GC (SEQ ID No: 29)
CAA TAT ACI CGT AAA GGC GAA AAC AAA GC (SEQ ID No: 30)
CAA TAT ACI CGT AAA GGT GAA AAC AAA GC (SEQ ID No: 31)
CAA TAT ACI CGT AAA GGC GAA AAT AAA GC (SEQ ID No: 32)
CAA TAT ACI CGC AAA GGC GAA AAC AAA GC (SEQ ID No: 33)
CAA TAT ACI CGC AAA GGC GAA AAT AAA GC (SEQ ID No: 34)
CAA TAT ACI CGC AAA GGT GAA AAT AAA GC (SEQ ID No: 35)
CAA TAT ACI CGC AAA GGT GAA AAC AAA GC (SEQ ID No: 36)
The Y⁶ and K¹⁰ residues were omitted from the sequence analysis report for the N-terminal sequence and the oligonucleotides used to PCR amplify the 2.2 kb fragment were incorrect, but nevertheless were successful.

There is a conserved C-terminal pentapeptide found in all known Lbp1 and Tbp1 protein sequences: LEMKF (SEQ ID No. 26). An oligonucleotide primer (4572.RD) was prepared based upon the complementary DNA sequence encoding this pentapeptide: 3' GAA CTT TAC TTC AAA ATT 5' 4572.RD (SEQ ID No: 38)

### Example 2

This Example illustrates the preparation of chromosomal DNA from *M. catarrhalis* strains 4223 and Q8.

*M. catarrhalis* isolate 4223 was inoculated into 100 ml of BHI broth, and incubated for 18 hr at 37°C with shaking. The cells were harvested by centrifugation at 10,000 x g for 20 min. The pellet was used for extraction of *M. catarrhalis* 4223 chromosomal DNA.

The cell pellet was resuspended in 20 ml of 10 MM Tris-HC1 (pH 7.5)-1.0 mM EDTA (TE). Pronase and SDS were added to final concentrations of 500 µg/ml and 1.0%, respectively, and the suspension was incubated at 37°C for 2 hr. After several sequential extractions with phenol, phenol:chloroform (1:1), and chloroform:isoamyl alcohol (24:1), the aqueous extract was dialysed, at 4°C, against 1.0 M NaCl for 4 hr, and against TE (pH 7.5) for a further 48 hr with three buffer changes. Two volumes of ethanol were added to the dialysate, and the DNA was spooled onto a glass rod. The DNA was allowed to air-dry, and was dissolved in 3.0 ml of water. Concentration was estimated, by UV spectrophotometry, to be about 290 µg/ml.

*M. catarrhalis* strain Q8 was grown in BHI broth. Cells were pelleted from 50 ml of culture by centrifugation at 5000 rpm for 20 minutes, at 4°C. The cell pellet was resuspended in 10 ml of TE (10 mM Tris-HCl, 1 mM EDTA, pH 7.5) and proteinase K and SDS were added to final concentrations of 500 µg/ml and 1%, respectively. The sample was incubated at 37°C for 4 hours until a clear lysate was obtained. The lysate was extracted twice with Tris-saturated phenol/chloroform (1:1), and twice with chloroform. The final aqueous phase was dialysed for 24 hours against 2 X 1000 ml of 1 M NaCl at 4°C, changing the buffer once, and for 24 hours against 2 x 1000 ml of TE at 4°, changing the buffer once. The final dialysate was precipitated with two volume of 100% ethanol. The DNA was spooled, dried and resuspended in 5 to 10 ml of TE buffer.

### Example 3

This Example illustrates the PCR amplification of a fragment of *M. catarrhalis lbpA* and the generation of probes for screening libraries.

PCR amplification was performed on chromosomal DNA isolated in Example 2 using primers 4393.RD and 4572.RD under the following cycling conditions: 25 cycles of 94°C for 1 min, 47°C for 30 sec and 72°C for 1 min. PCR4 is the amplification of the 4223 *lbpA* fragment and PCR5 is the amplification of the Q8 *lbpA* fragment. A specific band of about 2.2 kb was amplified and partial sequence analysis was performed to ensure that the gene product was related to *lbpA* and was not *tbpA*. The derived amino acid sequences are shown in Figure 1 and have been aligned with the complete 4223 Lbp1 sequence to show their placement and the 4223 Tbp1 sequence (USAN 08/613,009) to indicate their uniqueness.

The full-length 2.2 kb gene fragment was randomly labeled with ³²P and used to probe genomic libraries.

### Example 4

This Example illustrates the generation and screening of the EMBL 3 libraries.

Chromosomal DNA was prepared as described in Example 2. A series of Sau3AI restriction digests of chromosomal DNA, in final volumes of 10 µL each, were carried out in order to optimize the conditions necessary to generate maximal amounts of restriction fragments within a 15 to 23 kb size range. Using the optimized digestion conditions, a large-scale digestion was set up in a 100 µL volume, containing the following: 50 µL of chromosomal DNA (290 µg/ml), 33 µL water, 10 µL 10X Sau3A buffer (New England Biolabs), 1.0 µL BSA (10 mg/ml, New England Biolabs), and 6.3 µL Sau3A (0.04 U/µL). Following a 15 min. incubation at 37°C, the digestion was terminated by the addition of 10 µL of 100 mM Tris-HCl (pH 8.0)-10 mM EDTA-0.1% bromophenol blue-50% glycerol (loading buffer). Digested DNA was electrophoresed through a 0.5% agarose ge1 in 40 mM Tris acetate-2 mM Na₂EDTA.2H₂O (pH 8.5)(TAE buffer) at 50 V for 6 hr. The region containing restriction fragments within a 15 to 23 kb molecular size range was excised from the gel, and placed into dialysis tubing containing 3.0 ml of TAE buffer. DNA was electroeluted from the gel fragment by applying a field strength of 1.0 V/cm for 18 hr. Electroeluted DNA was extracted once each with phenol and phenol:chloroform (1:1), and precipitated with ethanol. The dried DNA was dissolved in 5.0 µL water.

Size-fractionated chromosomal DNA was ligated with *Bam*HI-digested EMBL3 arms (Promega), using T4 DNA ligase in a final volume of 9 µL. The entire ligation mixture was packaged into lambda phage using a commercial packaging kit (Amersham), following manufacturer's instructions.

The packaged DNA library was amplified on solid media. 0.1 ml aliquots of *Escherichia coli* strain NM539 in 10 mM MgSO₄ (OD₂₆₀ = 0.5) were incubated at 37°C for 15 min. with 15 to 25 µL of the packaged DNA library. Samples were mixed with 3 ml of 0.6% agarose containing 1.0% BBL trypticase peptone-0.5% NaCl (BBL top agarose), and mixtures were plated onto 1.5% agar plates containing 1.0% BBL trypticase peptone-0.5% NaCl, and incubated at 37°C for 18 hr. 3 ml quantities of 50 mM Tris-HCl (pH 7.5)-8 mM magnesium sulfate heptahydrate-100 mM NaCl-0.01% (w/v) gelatin (SM buffer) were added to each plate, and plates were left at 4°C for 7 hr. SM buffer containing phage was collected from the plates, pooled together, and stored in a screwcap tube at 4°C, with chloroform.

Ten µL aliquots of phage stock were combined each with 100 µL of *E. coli* strain LE392 in 10 mM MgSO4 (OD₂₆₀ = 0.5) (plating cells), and incubated at 37°C for 15 min. The samples were mixed with 3 m1 each of BBL top agarose, and the mixtures were poured onto 1.5% agarose plates containing 1% bacto tryptone-0.5% bacto yeast extract-0.05% NaCl (LB agarose; Difco) and supplemented with 200 µM EDDA. The plates were incubated at 37°C for 18 hr. Plaques were lifted onto nitrocellulose filters (Amersham Hybond-C Extra) which were hybridized with the 32P-labelled 2.2 kb PCR fragment. Several putative phage clones were obtained from each library and clones 4223LfR.17 and Q8LfR.13 were chosen for further analysis.

### Example 5

This Example illustrates the subcloning of the phage clones containing *M. catarrhalis lfr* genes.

Restriction enzyme analysis and Southern blotting using the screening probes, indicated that at least a portion of *lbpA* was localized to an about 9 kb Hind III fragment from each phage clone. The about 9 kb Hind III fragment from 4223LfR.17 was subcloned into pUC 18, generating clone pLD1-8. The about 9 kb Hind III fragment from Q8LfR.13 was subcloned into pBluescript, generating plasmid pLDW1. Internal about 5.5 kb EcoR V fragments were subcloned generating plasmids pLD3 and pLDW3 for the 4223 and Q8 genes, respectively.

### Example 6

This Example illustrates the sequence analysis of clones containing the *M. catarrhalis lfr* genes from strains 4223 and Q8.

Sequence analysis of the 5.5 kb EcoR V fragments from pLD3 and pLDW3, revealed that they each contained the 3'-end of *lbpB,* the complete *lbpA* gene, and a third complete gene designated *orf3.* The remainder of the *lbpB* genes was found on the about 9 kb Hind III fragments from pLDl-8 and pLDW1. Partial restriction enzyme analysis of the 4223 *lbpA, lbpB,* and *orf3* genes, based upon the nucleotide sequences is shown in Figure 3. Partial restriction enzyme analysis of the Q8 *lbpA, lbpB,* and *orf3* genes, based upon the nucleotide sequences is shown in Figure 5. The complete sequences of the *lbpB, lbpA,* and *orf3* genes comprising the putative *lfr* locus from *M. catarrhalis* 4223 and Q8 is shown in Figures 2 and 4, respectively. The intergenic distance between the *lbpB* and *lbpA* genes is 184 nucleotides, while a single nucleotide separates the *lbpA* and *orf3* genes. A putative promoter and ribosome binding site is indicated by underlining upstream of both *lbpB* and *lbpA.* A fourth potential gene was cloned on the approximately 9 kb Hind III fragments.

The N-terminal sequence of the native Lbp1 protein is unknown. Examination of the deduced amino acid sequence of the *lbpA* gene indicates that there are two possible ATG start codons at positions 1 and 16. The first position is downstream of strong promoter elements found in the *lbpB-lbpA* intergenic region and the second position is followed by a putative signal sequence. The *M. catarrhalis* 4223 and Q8 Lbp1 proteins (from the first ATG) have molecular mass values of about 110 kDa and are 99% identical. The deduced Lbp1 protein sequences from *M. catarrhalis* strains 4223 and Q8 are compared in Figure 6. They are also compared with the *iroA*/*lbpA* gene from *N. meningitidis* strain BNCV (ref. 24) and the *lbpA* gene from *N. gonorrhoeae* strain FA19 (ref. 25). The *M. catarrhalis* proteins are found to be about 32% identical and about 50% similar to the *Neisseria* proteins. As shown in Figure 1, there is very limited sequence homology between the *M. catarrhalis* Tbp1 and Lbp1 sequences.

The deduced Lbp2 protein sequences from *M. catarrhalis* strains 4223 and Q8 are compared in Figure 7. The 4223 and Q8 Lbp2 proteins both have molecular masses of about 99 kDa and are 92% identical and 95% similar to each other. A comparison to the *M. catarrhalis* Tbp2 proteins shows very little homology except the LEGGFY (SEQ ID No: 27) epitope previously identified in *H. influenzae* and *N. meningitidis* Tbp2 proteins (Fig. 8). A cysteine residue at position 32 is preceded by a consensus sequence for lipoproteins suggesting that Lbp2, like Tbp2, is a lipoprotein. An unusual feature of the Lbp2 proteins is the high combined aspartic acid and asparagine content which is nearly 20%. In addition, the 4223 Lbp2 amino acid composition from residues 698 to 751 is about 52% aspartic acid.

The 4223 and Q8 *lfr orf3* genes would encode proteins of molecular mass about 60 kDa, respectively. A notable feature of the ORF3 protein is a potential signal sequence, a terminal phenylalanine which is often associated with membrane anchored proteins, an internal repeat sequence of DGLG (SEQ ID No: 39), and a high leucine content of 15%. The deduced Lbp3 protein sequences are compared in Figure 9. These proteins are 98% identical and 99% similar.

### Example 7

This Example illustrates the construction of vectors to express *M. catarrhalis* Lbp1 from the first methionine in *E. coli.*

There are two possible start codons at the beginning of the *lbpA* gene and hence two expression, constructs were made. The construction scheme for 4223 or Q8 *lbpA* expressed from the first methionine is shown in Figure 10. An approximately 200 bp fragment of the 5'-end of *lbpA* from the ATG to a BstE II site was PCR amplified using primers 5405.RD and 5407.RD. An Nde I site was engineered at the 5'-end to facilitate cloning into the pT7-7 vector.
NdeI M S K S I T (SEQ ID No: 40)
5' GGAATTCCAT ATG TCA AAA TCT ATC ACA AA 3' 5405.RD (SEQ ID No: 41)
BstE II L D A I T V T A A (SEQ ID No: 42)
5' T TTA GAT GCC ATC ACG GTA ACC GCC GCC CC 3' (SEQ ID No: 43)

In order to subclone the *lbpA* gene into pT7-7, a approximately 515 bp fragment of the 3'-end of the gene from an Sph I site to the stop codon was PCR amplified using primers 5281.RD and 5282.RD and a BamH1 site was engineered at 3'-end. 5' GGC AAA CTG GAT TTG CAT GCC ATG ACA TCA 3' 5281. RD (SEQ ID No: 46)
S L E M K F * (SEQ ID No: 47)
5' AGT CTT GAA ATG AAG TTT TAA 3' (SEQ ID No: 48)

For the Q8 subclone, plasmid pLDW3, prepared as described in Example 5, was digested with BstE II and Sph I generating a 2.3kb fragment of *lbpA* which was ligated with the Nde I-BstE II and SphI-BamH I PCR fragments and cloned into pT7-7 digested with NdeI and BamH I. The resulting plasmid pQW1A thus contains the full-length Q8 *lbpA* gene from the first methionine, under the control of the T7 promoter. DNA from pQW1A was purified and transformed by electroporation into electrocompetent BL21(DE3) cells to generate strain QW1A which was grown and induced using IPTG. Expressed proteins were resolved by SDS-PAGE and the induced Lbp1 protein was visualized by Coomassie blue staining (Fig. 11).

For the 4223 subclone, plasmid pLD3, prepared as described in Example 5 was digested with BstEII and SphI, generating a 2.3 kb fragment of *lbpA,* which was ligated with the Nde I-BstE II and SphI-BamH I PCR fragments and cloned into pT7-7 digested with NdeI and BamH I. The resulting plasmid pRD1A thus contains the full-length 4223 *lbpA* gene from the first possible methionine under the control of the T7 promoter. DNA from pRD1A was purified and transformed by electroporation into electrocompetent BL21(DE3) cells to generate strain RD1A which was grown and induced using IPTG. Expressed proteins were resolved by SDS-PAGE and the induced Lbp1 protein was visualized by Coomassie blue staining (Fig. 11).

The Q8 Lbp1 protein was expressed at very high levels but the 4223 Lbp1 protein was expressed at substantially lower levels.

### Example 8

This Example illustrates the extraction and purification of rLbp1 from *E. coli.* The procedure is illustrated generally in Figure 14.

*E. coli* cells from a 500 ml culture, prepared as described in Example 7, were resuspended in 40 ml of 50 mM Tris-HCl, pH 8.0 containing 5 mM AEBSF (protease inhibitor) and 0.1 M NaCl, and disrupted by sonication (3 x 10 min, 70% duty circle). The extract was centrifuged at 20,000 x g for 30 min and the resultant supernatant, which contained greater than 95% of the soluble proteins from *E. coli,* was discarded. The remaining pellet (Figure 14, PPT1) was further extracted in 40 ml of 50 mM Tris, pH 8.0 containing 0.5% Triton X-100 and 10 mM EDTA. The mixture was stirred at 4°C for at least 1 hour and then centrifuged at 20,000 x g for 30 min and the supernatant containing residual soluble proteins and the majority of the membrane proteins was discarded. The resultant pellet (Figure 14, PPT2) was further extracted in 40 ml of 50 mM Tris, pH 8.0 containing 1% octylglucoside. The mixture was stirred at 4°C for at least 1 hour and then centrifuged at 20,000 x g for 30 min. The supernatant containing residual contaminating proteins was discarded. The resultant pellet (Figure 14, PPT3) obtained after the above extractions contained the Lbp1 protein as inclusion bodies.

The rLbp1 protein was solubilized from the inclusion bodies in 50 mM Tris, pH 8.0, containing 6 M guanidine and 5 mM DTT. After centrifugation, the resultant supernatant was further purified on a Superdex 200 gel filtration column equilibrated in 50 mM Tris-HCl, pH 8.0, containing 2 M guanidine and 5 mM DTT. The fractions were analysed by SDS-PAGE and those containing purified rLbp1 were pooled. Triton X-100 was added to the pooled rLbp1 fraction to a final concentration of 0.1%. The fraction was dialysed overnight at 4°C against PBS, and then centrifuged at 20,000 x g for 30 min. The purified rLbp1 was stored at -20°C. Samples from the purification were analyzed by SDS-PAGE (Fig. 15).

### Example 9

This Example illustrates the construction of vectors to express *M. catarrhalis* Lbp1 from the second methionine in *E. coli.*

The construction scheme for 4223 or Q8 *lbpA* expressed from the second methionine is shown in Figure 10. An approximately 200 bp fragment of the 5'-end of *lbpA* from the ATG to a BstE II site was PCR amplified using primers 5406.RD and 5407.RD. An Nde I site was engineered at the 5'-end to facilitate cloning into the pT7-7 vector. 5' GGAATTCCAT ATG ACC ACG CAC CGC TTA AA 3' 5406.RD (SEQ ID No: 51) 5' T TTA GAT GCC ATC ACG GTA ACC GCC GCC CC 3'
3' A AAT CTA CGG TAG TGC CAT TGG CGG CGG GG 5' 5407.RD

The 3'-end of the *lbpA* gene was PCR amplified from the SphI restriction site to the stop codon using primers 5281.RD and 5282.RD as described in Example 8. The 2.3 kb BstE II-Sph I fragments described in Example 8 were ligated to the Nde I-BstE II and Sph I-BamH I PCR fragments and cloned into pT7-7 that had been digested with NdeI and BamH I. Plasmid pQW1B thus contains a full-length Q8 *lbpA* gene from the second methionine and plasmid pRD1B contains a full-length 4223 *lbpA* gene from the second methionine under the direction of the T7 promoter. DNA was purified and transformed by electroporation into electrocompetent BL21(DE3) cells to generate recombinant strains which were grown and induced using IPTG. Expressed proteins were resolved by SDS-PAGE and the induced Lbp1 proteins were visible by Coomassie blue staining (Fig. 11).

As seen for the longer protein in Example 8, the shorter Lbp1 from Q8 was expressed to much higher levels than the corresponding 4223 protein.

### Example 10

This Example illustrates the construction of vectors to express *M. catarrhalis* Lbp2 with a leader sequence from *E. coli.*

The construction scheme is illustrated in Figure 12. There are two BspH I sites within the *lbpB* genes of strains 4223 and Q8. The 5'-end of the *lbpB* gene was PCR amplified from the ATG start codon through the first BspH I site generating an approximately 201 bp fragment. An NdeI site was engineered at the ATG to facilitate cloning into the pT7-7 expression vector. The oligonucleotides used for amplification are illustrated below:
NdeI M S T V K T P H (SEQ ID No; 52)
5' GGAATTCCAT ATG AGT ACT GTC AAA ACC CCC CAC A 3' 5533.RD (SEQ ID No: 53) 5' A ATA CCG AAC ACA GGT CAT GAC AAC ACC AAT 3' (SEQ ID No: 55)
T TAT GGC TTG TGT CCA GTA CTG TTG TGG TTA 5' 5534.RD (SEQ ID No: 56)

The 3'-end of the *lbpB* gene was PCR amplified from the second BspH I site to the TAA stop codon generating a 381 bp fragment. A BamH I site was introduced after the stop codon for cloning purposes. The oligonucleotides used for amplification are illustrated below:
N E P T H E K T F A (SEQ ID No: 57)
5' AAT GAG CCT ACT CAT GAA AAA ACC TTT GCC 3' S535.RD (SEQ ID No: 58)
G A V F G A V K D K * (SEQ ID No: 59)
5' GG GCT GTC TTT GGG GCT GTT AAA GAT AAA TAA 3' (SEQ ID No: 60)

Plasmids pLD1-8 or pLDW1, prepared as described in Example 4, were digested with BspH I to release a 2.1 kb internal fragment of the *lbpB* gene which was ligated with the 5'- and 3'-PCR fragments and cloned into pT7-7 that had been digested with NdeI and BamH I. The resulting plasmids, pLD2A and pLDW2A, contain the full-length 4223 and Q8 *lbpB* genes under the control of the T7 promoter, respectively.

### Example 11

This Example illustrates the construction of vectors to express the mature *M. catarrhalis* Lbp2 proteins from *E. coli*.

The construction scheme is illustrated in Figure 12. The putative mature Lbp2 lipoproteins start at the Cys³² residue. A scheme similar to that described in Example 10 can be used to generate expression clones. To amplify the 5'-end of the *lbpB* gene, a sense PCR primer is designed that includes an NdeI site for subsequent cloning and an ATG start codon for initiation of translation followed immediately by the Cys³² residue. The antisense primer is the same as that described in Example 9 (5534.RD) and includes the BspH I cloning site. The amplified fragment is ~112 bp long. The oligonucleotides are illustrated below:
NdeI M C R S D D I S V N (SEQ ID No: 62)
5' GGAATTCCAT ATG TGC CGC TCT GAT GAC ATC AGC GTC AAT 3' RD (SEQ ID No: 63) 5' A ATA CCG AAC ACA GGT CAT GAC AAC ACC AAT 3' (SEQ ID No: 55)
3' T TAT GGC TTG TGT CCA GTA CTG TTG TGG TTA 5' 5534.RD (SEQ ID No: 56)

The BspH I-BamH I 3'-end of the *lbpB* gene is PCR amplified as in Example 9 and the plasmid expressing mature Lbp2 is constructed by ligating the 5'- and 3'-PCR fragments with the 2.1 kb BspH I fragment and vector pT7-7 digested with NdeI and BamH I. The resulting plasmids, pLD2B and pLDW2B, contain the *lbpB* gene encoding the mature Lbp2 proteins from strains 4223 and Q8 under the direction of the T7 promoter, respectively.

### Example 12

This Example illustrates the construction of a vector to express the *M. catarrhalis lfr* Lbp3 from *E. coli.*

The construction scheme is illustrated in Figure 13. Oligonucleotides were used to generate the 5'-end of the *orf3* gene from the ATG start codon to an AlwN I site. An NdeI site was engineered at the 5'-end for subsequent cloning into pT7-7. The oligonucleotides are shown below: TTT TTA AAG CAG GTG 3' 5532.RD (SEQ ID No: 65)
AAA AAT TTC GTC 5' 5457.RD (SEQ ID No: 66)

The pLD1-8 or pLDW1 plasmid, prepared as described in Example 5, was digested with BstE II generating a 4.6 kb fragment which was filled in with Klenow polymerase before being digested with AlwNI. The resultant 1.8 kb fragment was ligated with the annealed NdeI-AlwN I oligonucleotides and cloned into pT7-7 that had been digested with NdeI and SmaI. The resulting plasmids, pLRD3 and pLQW3, contain the full-length *orf3* genes from strains 4223 and Q8 under the direction of the T7 promoter, respectively.

### Example 13

This Example describes the cloning and sequencing of the *lbpB* gene from *M. catarrhalis* strain VH19.

Chromosomal DNA was prepared from *M. catarrhalis* strain VH19, as described previously in Example 2. Oligonucleotide primers were designed based upon the flanking sequence of the 4223 *lbpB* gene. The sense primer was 5' AAGCTTAGCATGATGGCATCGGCT 3' (SEQ ID No: 67) and the antisense primer was 5' TTAGCCCAAGGCAAATCTGGTGCA 3' (SEQ ID No: 68). PCR was performed in buffer containing 10mM Tris-HCl (pH 8.3), 50 mM potassium chloride and 1.5 mM magnesium chloride. Each 100 µl reaction mixture contained 1 µg chromosomal DNA, 0.1 µg each primer, 2.5 units amplitaq DNA polymerase (Perkin Elmer Cetus, Foster City, California) and 10 mM of each dNTP (Perkin Elmer Cetus). The cycling conditions were 24 cycles of 94°C for 1 min, 47°C for 30 sec and 72°C for 1 min. Specific 2.9 kb fragments were amplified from two independent reactions and subcloned into pCR II (Invitrogen, Carlsbad, California), generating plasmids pVH19pcr1 and pVH19pcr2 for sequence analysis. A third PCR amplification was performed without subcloning the resultant DNA. Plasmid DNA from pVH19pcr1 and pVH19pcr2 was prepared from 50 ml overnight cultures using the Qiagen Plasmid Midi kit (Qiagen Inc, Chatsworth, California). PCR amplified DNA was purified for direct sequencing using a Qiagen PCR purification kit. DNA samples were sequenced on an ABI model 373A DNA sequencer using dye terminator chemistry. Oligonucleotide primers 17 to 25 bases in length were used to sequence both strands of the DNA.

The nucleotide sequence (SEQ ID No: 69) of the VH19 *lbpB* gene and the deduced amino acid sequence of the corresponding Lbp2 protein (SEQ ID No: 70) are shown in Figure 16. The encoded VH19 Lbp2 protein is 906 amino acids and is 77% identical and 84% similar to the 4223 and Q8 Lbp2 proteins. There is a putative lipoprotein signal sequence which is very similar to the 4223 and Q8 signal sequences. The high Asp and Asn content found in the 4223 and Q8 Lbp2 proteins is also present in the VH19 LbpB protein, as is the RGD sequence. A partial restriction map of the VH19 *lbpB* gene is shown in Figure 17.

An alignment of the Lbp2 proteins from *M. catarrhalis* strains 4223, Q8 and VH19 is shown in Figure 7. The *M. catarrhalis* Lbp2 proteins are also compared with partial Lbp2 sequences from *N. meningitis* strains BNCV (ref. 31) and H44/76 (ref. 24) and *N. gonorrhoeae* strain FA19 (ref. 25). There are small scattered regions of sequence homology to the known bacterial Tbp2 proteins (ref. 32). Residues that are conserved among the Tbp2 proteins and the *M. catarrhalis* Lbp2 proteins are underlined in Figure 7 and include the LEGGFYG (SEQ ID No: 75) motif.

### Example 14

This Example describes the construction of vectors for expression of the *M. catarrhalis* Lbp2 protein.

By analogy with Tbp2 proteins, Lbp2 was assumed to be a lipoprotein and constructs were designed for expression of Lbp2 with or without a lipopeptide signal sequence. There is a unique Bgl I site in *lbpb.* To express the full-length Lbp2 protein with leader sequence (construct A), an approximately 429 bp 5'-fragment from the Met¹ start codon to the Bgl I site was PCR amplified and to express the mature protein (construct B), an approximately 329 bp 5'-fragment from the putative Cys³² start to the Bgl I site was PCR amplified. The following sense primers were used:
Nde I M S T V K T P H (SEQ ID No: 52)
5' GGAATTCCAT ATG AGT ACT GTC AAA ACC CCC CAC A 3' (SEQ ID No: 53)
for construct A or
Nde I M C R S D D I S V N (SEQ ID No: 62)
5' GGAATTCCAT ATG TGC CGC TCT GAT GAC ATC AGC GTC AAT 3' (SEQ ID No: 63)
for construct B and the anti-sense primer was:
G K N L R G P I (SEQ ID No: 72)
5' GGT AAA AAC TTG CGT CAG CCC ATC 3' (SEQ ID No: 73) The Q8 lfr-containing plasmid, pLDW1 (Example 5), was digested with Bgl I and EcoR I to release a 2.3 kb *lbpB* fragment which was ligated with the Nde I - Bgl I PCR' fragment and cloned into pT7-7 that had been digested with Nde I and EcoR I. The resulting plasmids, pQW2A and pQW2B, thus contain the Q8 *lbpB* gene encoding the full-length or mature Lbp2 proteins under the direction of the T7 promoter. The plasmids expressing the 4223 full-length or mature Lbp2 proteins were constructed in a similar manner and designated pRD2A and pRD2B. There was no measurable expression of rLbp2 from constructs containing the signal sequence, however the mature rLbp2 proteins were expressed at 5 to 10% of total proteins as inclusion bodies and were purified by the same process as that described for rLbp1 in Example 8. Samples from the purification were analyzed by SDS-PAGE (Figure 18).

### Example 15

This Example describes the functional characterization of the recombinant lactoferrin binding proteins.

Human lactoferrin (Sigma) was conjugated to horseradish peroxidase using an EZ-Link maleimide activated horseradish peroxidase (HRP) kit (Pierce, Rockford, Illinois) according to the manufacturer's instructions. The lactoferrin binding activity of rLbp1 or rLbp2 was assessed by modifying the procedure described for transferrin binding proteins (ref. 17). Briefly, purified rLbp1 or rLbp2 was subjected to discontinuous electrophoresis through a 12.5% SDS PAGE gel. The proteins were electrophoretically transferred to a polyvinylidene difluoride (PVDF) membrane (Millipore, Bedford, Massachusetts) and incubated with horseradish peroxidase-conjugated human lactoferrin (1:20 dilution) at 4°C overnight. LumiGLO substrate (Kirkegaard and Perry Laboratories, Inc., Gaithersburg, Maryland) was used for chemiluminescent detection of HRP activity according to the manufacturer's instructions. The Q8 rLbp1 protein did not bind human lactoferrin under these conditions, but the 4223 rLbp2 and Q8 rLbp2 proteins did (Fig. 19).

### Example 16

This Example describes the immunization of animals and immunoassays.

Groups of two guinea pigs (Hartley outbred, Charles River, Quebec) were immunized intramuscularly (i.m.) with 5 µg doses of purified rLbp1 or rLbp2 protein emulsified in CFA or IFA. Anti-Lbp antibody titers in guinea pig immune sera were determined by antigen-specific ELISA. Microtiter wells (Nunc-MAXISORB, Nunc, Denmark) were coated with 50 µl of protein (0.5 µg ml ¹). The reactive titer of an antiserum was defined as the reciprocal of the highest dilution consistently showing a two-fold increase in absorbance at 450 nm over that obtained with the pre-immune serum samples. The recombinant proteins elicited high titre antibodies as shown in Tables 1 and 2.

### Example 17

This Example describes the antigenic conservation of Lbp1 and Lbp2 in *M. catarrhalis* strains.

To demonstrate the iron-dependent expression of the *lbpA* and *lbpB* genes, representative *M. catarrhalis* strains were grown in BHI ± 25 mM EDDA. Whole cell lysates were separated by SDS PAGE and electrophoretically transferred to nitrocellulose membrane. Guinea pig anti-Q8 rLbp1, anti-Q8 rLbp2 and anti-4223 rLbp2 antisera were used as first antibodies and horseradish peroxidase-conjugated protein G (ZYMED) was used as secondary antibody. To assess antigenic conservation, approximately 90 *M. catarrhalis* strains, obtained from North America or Finland were grown in BHI + 25 mM EDDA, and immunoblots were probed with guinea pig anti-4223 rLbp2 antibody, as above. All strains showed a protein band reactive with anti-rLbp2 antibody. There was very little size heterogeneity for the Lbp2 proteins from the 90 *M. catarrhalis* strains, ranging from approximately 100 kDa to 105 kDa. Representative immunoblots are illustrated in Fig. 19.

### Example 18

This Example describes the assay used to determine the bactericidal antibody activity of anti-Lbp antibodies.

The assay was performed as described by ref. 33. Briefly, the *M. catarrhalis* strains were grown to an OD₅₇₈ of 0.5 in BHI medium containing 25 mM EDDA. The bacteria were diluted so that the pre-bleed control plates contained 100 to 300 cfu. Guinea pig anti-rLbp1 or anti-rLbp2 antisera and pre-bleed controls, were heated to 56°C for 30 min to inactivate endogenous complement and were diluted 1:64 with veronal buffer containing 0.1% BSA (VBS). Guinea pig complement (Biowhittaker, Walkersville, Maryland) was diluted 1:10 in VBS. Twenty-five µl each of diluted antiserum, bacteria and complement were added to duplicate wells of a 96 well microtiter plate (Nunc). The plates were incubated at 37°C for 60 min, gently shaking at 70 rpm on a rotary platform. Fifty µl of each reaction mixture were plated onto Mueller Hinton agar plates (Becton-Dickinson, Cockeysville, Maryland) which were incubated at 37°C for 24 h, then room temperature for 24 h, before the bacteria were counted. Antisera were determined to be bactericidal if ≥ 50% of bacteria were killed compared with negative controls.

Six strains of different geographical and anatomical origins were tested. The data in Table 3 illustrates that anti-4223 rLbp2 antibody was bactericidal for the homologous strain and three of five heterologous strains.

### SUMMARY OF THE DISCLOSURE

In summary of this disclosure, the present invention provides purified and isolated DNA molecules containing a lactoferrin receptor gene encoding Lbp2 of *Moraxella catarrhalis* Q8, the sequences of this lactoferrin receptor gene, and the derived amino acid sequences thereof. The genes and DNA sequences are useful for diagnosis, immunization, and the generation of diagnostic and immunological reagents. Immunogenic compositions, including vaccines, based upon expressed recombinant Lbp1 and/or Lbp2 and/or ORF3, portions thereof, or analogs thereof, can be prepared for prevention of diseases caused by *Moraxella.*

**Table 1**

| **Bactericidal antibody titres for anti-native Lbp1** | | | | |
|---|---|---|---|---|
| | **Bactericidal titre - RH408** | | **Bactericidal titre - Q8** | |
| **Antibody** | **Pre-immune** | **Immune** | **Pre-immune** | **Immune** |
| Anti-4223 Lbp1 | <8 | 114-330 | <8 | 128-512 |

| | | | | |
|---|---|---|---|---|
| Bactericidal titres are expressed as the reciprocal dilution of antiserum capable of killing 50% of *M. catarrhalis* cells | | | | |

**Table 2 ELISA titers for guinea pig anti-Lbp antibodies raised against recombinant lactoferrin binding proteins**

| Coated antigen | Anti-Q8 rLbp1 | Anti-Q8 rLbp2 | Anti-4223 rLbp2 |
|---|---|---|---|
| Q8 rLbp1 | 3,200 | - | - |
| | 25,600 | | |
| Q8 rLbp2 | - | 1,638,400 | 409,600 |
| | | 1,638,400 | 409,600 |
| 4223 rLbp2 | - | 409,600 | 819,200 |
| | | 409,600 | 819,200 |

**Table 3. Bactericidal antibody activity of guinea pig anti-rLbp2 antibodies**

| Strain | locale¹ | source² | Lbp2 size | Bactericidal antibody activity³ | |
|---|---|---|---|---|---|
| | | | | Anti-4223 rLbp2 | Anti-Q8 rLbp2 |
| 4223 | New York | MEF | 105 kDa | ++ | - |
| Q8 | Quebec | sputum | 105 kDa | ± | - |
| VH19 | Texas | MEF | 105 kDa | + | NT⁴ |
| LES-1 | Finland | MEF | 102 kDa | - | NT |
| H-04 | Nova Scotia | MEF | 100 kDa | + | NT |
| 3 | New York | sputum | 100 kDa | ++ | NT |

| | | | | | |
|---|---|---|---|---|---|
| ¹ geographic locale where strain was isolated ² anatomical source of clinical isolate. MEF is middle ear fluid from otitis media patients ³ killing by antiserum diluted 1:64, compared to negative controls: - indicates 0-25% killing; ± indicates 26-49% killing; + indicates 50-75% killing; ++ indicates 76-100% killing. ⁴ NT = not tested | | | | | |

### REFERENCES

1. Brorson, J-E., A. Axelsson, and S.E. Holm. 1976. Studies on Branhamella catarrhalis (Neisseria catarrhalis) with special reference to maxillary sinusitis. Scan. J. Infect. Dis. 8:151-155.
2. Catlin, B.W., 1990. Branhamella catarrhalis: an organism gaining respect as a pathogen. Clin. Microbiol. Rev. 3: 293-320.
3. Hager, H., A. Verghese, S. Alvarez, and S.L. Berk. 1987. Branhamella catarrhalis respiratory infections. Rev. Infect. Dis. 9:1140-1149.
4. McLeod, D.T., F. Ahmad, M.J. Croughan, and M.A. Calder. 1986. Bronchopulmonary infection due to M. catarrhalis. Clinical features and therapeutic response. Drugs 31(Suppl.3):109-112.
5. Nicotra, B., M. Rivera, J.I. Luman, and R.J. Wallace. 1986. Branhamella catarrhalis as a lower respiratory tract pathogen in patients with chronic lung disease. Arch.Intern.Med. 146:890-893.
6. Ninane, G., J. Joly, and M. Kraytman. 1978. Bronchopulmonary infection due to Branhamella catarrhalis 11 cases assessed by transtracheal puncture. Br.Med.Jr. 1:276-278.
7. Srinivasan, G., M.J. Raff, W.C. Templeton, S.J. Givens, R.C. Graves, and J.C. Mel. 1981. Branhamella catarrhalis pneumonia. Report of two cases and review of the literature. Am. Rev. Respir. Dis. 123:553-555.
8. West, M., S.L. Berk, and J.K. Smith. 1982. Branhamella catarrhalis pneumonia., South. Med. J. 75:1021-1023.
9. Christensen, J.J., and B. Bruun. 1985. Bacteremia caused by a beta-lactamase producing strain of Branhamella catarrhalis. Acta. Pathol. Microbiol. Immunol. Scand. Sect. B 93:273-275.
10. Craig, D.B., and P.A. Wehrle. 1983. Branhamella catarrhalis septic arthritis. J. Rheumatol. 10:985-986.
11. Guthrie, R., K. Bakenhaster, R. Nelson, and R. Woskobnick. 1988. Branhamella catarrhalis sepsis: a case report and review of the literature. J. Infect. Dis. 158:907-908.
12. Hiroshi, S., E.J. Anaissie, N. Khardori, and G.P. Bodey. 1988. Branhamella catarrhalis septicemia in patients with leukemia. Cancer 61:2315-2317.
13. O'Neill, J.H., and P.W. Mathieson. 1987. Meningitis due to Branhamella catarrhalis. Aust. N.Z. J. Med. 17:241-242.
14. Murphy, T.F. 1989. The surface of Branhamella catarrhalis: a systematic approach to the surface antigens of an emerging pathogen. Pediatr. Infect. Dis. J. 8:S75-S77.
15. Van Hare, G.F., P.A. Shurin, C.D. Marchant, N.A. Cartelli, C.E.Johnson, D. Fulton, S. Carlin, and C.H. Kim. Acute otitis media caused by Branhamella catarrhalis: biology and therapy. Rev. Infect. Dis. 9:16-27.
16. Jorgensen, J.H., Doern, G.V., Maher, L.A., Howell, A.W., and Redding, J.S., 1990 Antimicrobial resistance among respiratory isolates of Haemophilus influenza, Moraxella catarrhalis, and Streptococcus pneumoniae in the United States. Antibicrob. Agents Chemother. 34: 2075-2080.
17. Schryvers, A.B. and Lee, B.C. (1988) Comparative analysis of the transferrin and lactoferrin binding proteins in the family Neisseriaceae. Can. J. Microbiol. 35, 409-415.
18. O'Hagan, DT. 1992. Oral deleivery of vaccines. Formulation and clinical pharmaco kinetic considerations. Clin. Pharmacokinet 22(t): 1-10.
19. Ulmer et al. 1993. Curr. Opinion Invest. Drugs 2:983-989.
20. Lockhoff, O., 1991. Glycolipids as immunomodulators: Synthesis and properties.
21. Nixon-George A., et al., 1990. The adjuvant effect of stearyl tyrosine on a recombinant subunit hepatitis B surface antigen. J Immunol 144 (12): 4798-4802.
22. Wallace, R.J. et al., 1990. Antibiotic susceptibilites and drug resistance in Moraxella (Branhaemella) catarrhalis. Am. J. Med. 88(5A): 465-505.
23. Nissinen A, et al., 1995. Development of beta-lactamase-mediated resistance to penicillin in middle-ear isolates of Moraxella catarrhalis in Finnish children, 1978-1993. Clin Infect Dis 21 (5): 1193-1196.
24. Pettersson, A., et al., 1994. Identification of iroa Gene Product of Neisseria meningitides as a Lactoferrin Receptor. J. Bacteriol. 176(6): 1764-1766.
25. Biswas GD, Sparring PF. 1995. Characterization of lbpa, the structural gene for a lactoferrin receptor in Neisseria gonorrhoeae. Infect Inimun 63 (8): 2958-2967.
26. Legrain M, et al. 1993. Cloning and characterization of Neisseria meningitides genes encoding the transferrin-binding proteins Tbpl and Tbp2. Gene 130 (1): 73-80.
27. Cornelissen CN, Biswas GD, Sparling PF. 1993. Expression of gonococcal transferrin-binding protein 1 causes Escherichia coli to bind human transferrin. J Bacteriol 175 (8): 2448-2450.
28. Anderson JE, Sparling PF, Cornelissen CN. 1994. Gonococcal transferrin-binding protein 2 facilitates but is not essential for transferrin utilization. J Bacteriol 176 (11): 31623170.
29. Ogunnariwo JA, Schryvers AB. 1996. Rapid identification and cloning of bacterial transferrin and lactoferrin receptor protein genes. J Bacteriol 178 (24): 7326-7328.
30. Loosmore SM, et al. 1996. Cloning and expression of the Haemophilus influenzae transferrin receptor genes. Mol Microbiol 19 (3): 575-586.
31. Pettersson, A. et al. 1993. Molecular Characterization of the 98-Kilodalton Iron-Regulated Outer membrane Protein of Neisseria meningitides. Infect. Immun. 61 (ti): 47244733.
32. Ogunnariwo, J.A., Woo, T.K.W., Lo, R.Y.C., Gonzalez, G.C., and Schryvers, A.B. (1997) Characterization of the Pasteurella haemolytica transferrin receptor genes and the recombinant receptor proteins. Microbial Pathog 23:273-284.
33. Yang, Y.P., Myers, L.E., McGuinness, U., Chong, P., Kwok, Y., Klein, M.H., and Harkness, R.E. (1997) The outer membrane protein, CD, extracted from Moraxella (Branhamella) catarrhalis is a potential vaccine antigen that induces bactericidal antibodies. FEMS Immun Med Microbiol 17:187-199.
34. Pettersson, A., Klarenbeek, V., van Deurzen, J., Poolman, J.T., and Tommassen, J. (1994a) Molecular characterization of the structural gene for the lactoferrin receptor of the meningococcal strain H44/76. Microb Pathog 17:395-408.
35. Needleman, S.B., and Wunsch, C.D. 1970, J. Mol Biol. 48:443-453.
36. Sellers, P.H. 1974 On the theory and computation of evolutionary distances. J. Appl. Math(Siam) 26:787-793.
37. Waterman, M.S., Smith, T.F., and Beyer, W.A. 1976. Advan. Math. 20:367-387.
38. Smith, T.F., and Waterman, M.S. 1981 Identification of common molecular subsequences. J. Mol. Biol. 147:195-197.
39. Sobel, E. and Martinez, H.M. 1985 A Multiple Sequence Alignment Program. Nucleic Acid Res. 14:363-374.
40. Bonnat, R.A., Yu, R.H. and Schryvers, A.B. 1995, Biochemical Analysis of Lactoferrin Receptors in the Neisseriaceae: Identification of a Second Lactoferrin Receptor Protein. Microb. Pathog. 19:285-297.

## Claims

1. A purified and isolated nucleic acid molecule encoding a lactoferrin receptor protein 2 (Lbp2) of *Moraxella catarrhalis* Q8 and having:
(a) a DNA sequence encoding the amino acid sequence:
(b) a DNA sequence complementary to the DNA sequence as defined in (a).

2. A nucleic acid molecule as claimed in Claim 1, having the DNA sequence: a DNA sequence complementary thereto.

3. An expression vector for transformation of a host and comprising a nucleic acid molecule as claimed in any preceding claim.

4. A host cell transformed in culture with an expression vector as claimed in Claim 3.

5. A method of preparing a substantially pure recombinant lactoferrin receptor protein 2 (Lbp2) of *Moraxella catarrhalis* Q8 as defined in Claim 1 which method comprises:
culturing a host cell transformed with an expression vector as claimed in Claim 3 under conditions permitting expression of the receptor protein as inclusion bodies,
purifying the inclusion bodies free from cellular material and soluble proteins,
solubilizing lactoferrin receptor protein free from the purified inclusion bodies, and
purifying the lactoferrin receptor protein free from other solubilized materials.

6. A method as claimed in Claim 5 wherein said lactoferrin receptor protein is at least 70% pure.

7. A method as claimed in Claim 6 wherein said lactoferrin receptor protein is at least 90% pure.

8. A recombinant lactoferrin receptor protein as defined In Claim 1 and producible by a transformed host as claimed in claim 4 wherein the receptor protein is devoid of other proteins of the *Moraxella* strain.

9. An immunogenic composition comprising:-
(a) at least one active component selected from: a nucleic acid molecule as claimed in any one of Claims 1 to 2, or a lactoferrin receptor protein as claimed in Claim 8;
and
(b) a pharmaceutically-acceptable carrier.

10. A method of determining the presence, in a sample, of nucleic acid encoding a lactoferrin receptor protein 2 of *Moraxella* as defined in Claim 1, the method comprising:
(a) contacting the sample with the nucleic acid as claimed in Claims 1 to 2 to produce duplexes comprising the nucleic acid molecule and any nucleic acid molecule encoding the lactoferrin receptor protein present in the sample and specifically hybridizable therewith; and
(b) determining production of any said duplexes.

11. A diagnostic kit for determining the presence, in a sample, of nucleic acid encoding a lactoferrin receptor protein 2 of *Moraxella* as defined in Claim 1 said kit comprising:-
(a) a nucleic acid molecule as claimed in any one of Claims 1 to 2;
(b) means for contacting the nucleic acid molecule with the sample to produce duplexes comprising the nucleic acid molecule and any said nucleic acid present in the sample and hybridizable with the nucleic acid molecule; and
(c) means for determining production of any said duplexes.

12. A nucleic add molecule as claimed in any one of Claims 1 to 2 for use as a pharmaceutical.

13. A recombinant lactoferrin receptor protein as claimed in Claim 8 for use as a pharmaceutical.

## Patentansprüche

1. Ein gereinigtes und isoliertes Nukleinsäuremolekül, welches ein Lactoferrinrezeptorprotein 2 (Lbp2) von *Moraxella catarrhalis* Q8 codiert und welches aufweist:
(a) eine DNA-Sequenz, welche die Aminosäuresequenz: codiert, oder
(b) eine DNA-Sequenz, welche zu der DNA-Sequenz, wie sie in (a) definiert ist, komplementär ist.

2. Ein Nukleinsäuremolekül wie in Anspruch 1 beansprucht, welches die DNA-Sequenz: oder
eine DNA-Sequenz, die dazu komplementär ist, aufweist.

3. Ein Expressionsvektor zur Transformation eines Wirtes und umfassend ein Nukleinsäuremolekül wie in irgendeinem vorhergehenden Anspruch beansprucht.

4. Eine Wirtszelle, transformiert in Kultur mit einem Expressionsvektor wie in Anspruch 3 beansprucht.

5. Ein Verfahren zur Herstellung eines im Wesentlichen reinen rekombinanten Lactoferrinrezeptorproteins 2 (Lbp2) von *Moraxella catarrhalis* Q8 wie in Anspruch 1 definiert, wobei das Verfahren umfasst:
das Kultivieren einer Wirtszelle, welche mit einem Expressionsvektor wie in Anspruch 3 definiert transformiert ist, unter Bedingungen, welche die Expression des Rezeptorproteins als Einschlusskörper erlauben,
das Reinigen der Einschlusskörper, so dass diese frei von zellulärem Material und löslichen Proteinen sind,
das Solubilisieren des Lactoferrinrezeptorproteins, so dass dieses frei von den gereinigten Einschlusskörpern ist, und
das Reinigen des Lactoferrinrezeptorproteins, so dass dieses frei von anderen solubilisierten Materialien ist.

6. Ein Verfahren wie in Anspruch 5 beansprucht, wobei das Lactoferrinrezeptorprotein zu wenigstens 70% rein ist.

7. Ein Verfahren wie in Anspruch 6 beansprucht, wobei das Lactoferrinrezeptorprotein zu wenigstens 90% rein ist.

8. Ein rekombinantes Lactoferrinrezeptorprotein wie in Anspruch 1 definiert und erzeugbar durch einen transformierten Wirt wie in Anspruch 4 definiert, wobei das Rezeptorprotein frei von anderen Proteinen des Moraxella-Stamms ist.

9. Eine immunogene Zusammensetzung, umfassend:
(a) wenigstens eine aktive Komponente, die ausgewählt ist aus: einem Nukleinsäuremolekül wie in irgendeinem der Ansprüche 1 bis 2 beansprucht oder einem Lactoferrinrezeptorprotein wie in Anspruch 8 beansprucht; und
(b) einen pharmazeutisch verträglichen Träger.

10. Ein Verfahren zur Bestimmung des Vorliegens in einer Probe von Nukleinsäure, welche ein Lactoferrinrezeptorprotein 2 von *Moraxella* wie in Anspruch 1 definiert codiert, wobei das Verfahren umfasst:
(a) das Inkontaktbringen der Probe mit der Nukleinsäure wie in den Ansprüchen 1 bis 2 beansprucht, um Doppelstränge zu erzeugen, welche das Nukleinsäuremolekül und irgendein Nukleinsäuremolekül, welches das Lactoferrinrezeptorprotein codiert, das in der Probe vorliegt und spezifisch damit hybridisierbar ist, umfassen; und
(b) das Bestimmen der Erzeugung irgendwelcher dieser Doppelstränge.

11. Ein diagnostischer Kit zur Bestimmung des Vorliegens in einer Probe von Nukleinsäure, welche ein Lactoferrinrezeptorprotein 2 von *Moraxella* wie in Anspruch 1 definiert codiert, wobei der Kit umfasst:
(a) ein Nukleinsäuremolekül wie in irgendeinem der Ansprüche 1 bis 2 beansprucht;
(b) Mittel zum Inkontaktbringen des Nukleinsäuremoleküls mit der Probe, um Doppelstränge zu erzeugen, welche das Nukleinsäuremolekül und irgendeine der Nukleinsäuren, die in der Probe vorliegen und mit dem Nukleinsäuremolekül hybridisierbar sind, umfassen; und
(c) Mittel zur Bestimmung der Erzeugung irgendwelcher dieser Doppelstränge.

12. Ein Nukleinsäuremolekül wie in irgendeinem der Ansprüche 1 bis 2 beansprucht zur Verwendung als ein Pharmazeutikum.

13. Ein rekombinantes Lactoferrinrezeptorprotein wie in Anspruch 8 beansprucht zur Verwendung als ein Pharmazeutikum.

## Revendications

1. Molécule d'acide nucléique purifiée et isolée codant pour une protéine de récepteur de lactoferrine 2 (Lbp2) de *Moraxella catarrhalis* Q8 et ayant
(a) une séquence d'ADN codant pour la séquence d'aminoacides:
(b) une séquence d'ADN complémentaire à la séquence d'ADN définie dans (a).

2. Molécule d'acide nucléique selon la revendication 1, ayant la séquence d'ADN: une séquence d'ADN qui lui est complémentaire.

3. Vecteur d'expression pour la transformation d'un hôte et comprenant une molécule d'ADN selon l'une quelconque des revendications précédentes.

4. Cellule hôte transformée en culture avec un vecteur d'expression selon la revendication 3.

5. Procédé de préparation d'une protéine de récepteur de lactoferrine 2 (Lbp2) essentiellement pure de *Moraxella catarrhalis* Q8 telle que définie dans la revendication 1, ce procédé comprenant:
la culture d'une cellule hôte transformée avec un vecteur d'expression selon la revendication 3 dans des conditions permettant l'expression de la protéine de récepteur sous forme de corps d'inclusion;
la purification des corps d'inclusion pour les débarrasser du matériel cellulaire et des protéines solubles,
la solubilisation de la protéine de récepteur de lactoferrine pour la débarrasser des corps d'inclusion purifiés, et
la purification de la protéine de récepteur de lactoferrine pour la débarrasser des autres matières solubilisées.

6. Procédé selon la revendication 5, dans lequel ladite protéine de récepteur de lactoferrine a une pureté d'au moins 70 %.

7. Procédé selon la revendication 6, dans lequel ladite protéine de récepteur de lactoferrine a une pureté d'au moins 90 %.

8. Protéine de récepteur de lactoferrine recombinée telel que définie dans la revendication 1 et pouvant être produite par un hôte transformé selon la revendication 4, la protéine de récepteur étant dépourvue d'autres protéines de la souche de *Moraxella.*

9. Composition immunogène comprenant:
(a) au moins un constituant actif choisi parmi: une molécule d'acide nucléique selon l'une quelconque des revendications 1 à 2, ou une protéine de récepteur de lactoferrine selon la revendication 8; et
(b) un support pharmaceutiquement acceptable.

10. Procédé de détermination de la présence, dans un échantillon, d'un acide nucléique codant pour une protéine de récepteur de lactoferrine 2 de *Moraxella* telle que définie dans la revendication 1, le procédé comprenant:
(a) la mise en contact de l'échantillon avec l'acide nucléique selon les revendications 1 à 2 pour produire des duplex comprenant la molécule d'acide nucléique et toute molécule d'acide nucléique codant pour la protéine de récepteur de lactoferrine présente dans l'échantillon et pouvant s'hybrider de façon spécifique avec elle; et
(b) la détermination de la production desdits duplex.

11. Trousse de diagnostic pour la détermination de la présence, dans un échantillon, d'acide nucléique codant pour une protéine de récepteur de lactoferrine 2 de *Moraxella* telle que définie dans la revendication 1, ladite trousse comprenant:
(a) une molécule d'acide nucléique selon l'une quelconque des revendications 1 à 2;
(b) des moyens de mise en contact de la molécule d'acide nucléique avec l'échantillon pour produire des duplex comprenant la molécule d'acide nucléique et toute molécule d'acide nucléique codant pour la protéine de récepteur de lactoferrine présente dans l'échantillon et pouvant s'hybrider de façon spécifique avec elle; et
(c) des moyens de détermination de la production desdits duplex.

12. Molécule d'acide nucléique selon l'une quelconque des revendications 1 à 2, à utiliser comme produit pharmaceutique.

13. Protéine de récepteur de lactoferrine recombinée selon la revendication 8, à utiliser comme produit pharmaceutique.
